# EUROPEAN PATENT SPECIFICATION

(11) **EP 3 717 637 B1**
(45) Date of publication and mention of the grant of the patent: **22.04.2026**
(21) Application number: 18811749.3
(22) Date of filing: 21.11.2018
(51) Int. Cl.: C12N 7/02, C12N 7/04, C12N 15/70

(54) **PHAGE AND TRANSDUCTION PARTICLES**
PHAGEN- UND TRANSDUKTIONSPARTIKEL
PHAGE ET PARTICULES DE TRANSDUCTION

(30) Priority: 29.11.2017 GB 201719896; 17.05.2018 GB 201808063; 21.05.2018 US 201815985658
(43) Date of publication of application: 07.10.2020
(73) Proprietor: SNIPR Biome ApS., 2100 Copenhagen (DK)
(72) Inventor: HAABER, Jakob Krause, 2100 Copenhagen (DK); SEMSEY, Szabolcs, 2100 Copenhagen (DK)
(74) Representative: CMS Cameron McKenna Nabarro Olswang LLP
(86) International application number: PCT/EP2018/082053
(87) International publication number: WO 2019/105821

(56) References cited:
- WO-A1-2017/009399
- WO-A2-2005/046579
- ROBERT J CITORIK ET AL: "Sequence-specific antimicrobials using efficiently delivered RNA-guided nucleases", NATURE BIOTECHNOLOGY, vol. 32, no. 11, 21 September 2014 (2014-09-21), pages 1141 - 1145, XP055545744, ISSN: 1087-0156, DOI: 10.1038/nbt.3011
- RUSSELL J. KROM ET AL: "Engineered Phagemids for Nonlytic, Targeted Antibacterial Therapies", NANO LETTERS, vol. 15, no. 7, 8 July 2015 (2015-07-08), US, pages 4808 - 4813, XP055387647, ISSN: 1530-6984, DOI: 10.1021/acs.nanolett.5b01943
- L. CHASTEEN ET AL: "Eliminating helper phage from phage display", NUCLEIC ACIDS RESEARCH, vol. 34, no. 21, 6 November 2006 (2006-11-06), pages e145 - e145, XP055545748, ISSN: 0305-1048, DOI: 10.1093/nar/gkl772
- DAVID BIKARD ET AL: "Exploiting CRISPR-Cas nucleases to produce sequence-specific antimicrobials", NATURE BIOTECHNOLOGY, vol. 32, no. 11, 5 October 2014 (2014-10-05), pages 1146 - 1150, XP055545750, ISSN: 1087-0156, DOI: 10.1038/nbt.3043
- DIANA P. PIRES ET AL: "SUMMARY", MICROBIOLOGY AND MOLECULAR BIOLOGY REVIEWS, vol. 80, no. 3, 1 June 2016 (2016-06-01), US, pages 523 - 543, XP055545757, ISSN: 1092-2172, DOI: 10.1128/MMBR.00069-15

## Description

### TECHNICAL FIELD

The disclosure relates to the production of phage using DNAs (eg, plasmids and helper phage, or plasmids with chromosomally integrated helper phage genes), as well as the phage, helper phage and kits, and the invention relates to compositions and methods of use involving these. The particles are particularly useful for delivering toxic payloads into target bacteria for antibacterial action. Embodiments enable production of highly pure compositions of such particles for medical or environmental use and for containment of the particles, which may be useful for containing antibacterial action, controlling dosing or reducing the risk of acquisition of undesirable foreign genes.

### BACKGROUND

The use of helper phage to package phagemid DNA into phage virus particles is known. An example is the M13KO7 helper phage, a derivative of M13, used in *E coli* host cells. Other examples are R408 and CM13

R. J. Citorik et al "Sequence-specific antimicrobials using efficiently delivered RNA-guided nuclease" Nature Biotechnology, Vol. 31, No. 11, 21 September 2014 discusses the use of CRISPR-Cas technology to create antimicrobials whose spectrum of activity is chosen by design.

WO2017/009399 relates to the improvement of endonuclease-based antimicrobials by blocking DNA repair of double-strand break(s) (DSB(s)) in prokaryotic cells, using CRISPR-Cas technology.

### SUMMARY OF THE INVENTION

The invention is as defined in the claims, including the following statements:
An antibacterial composition comprising a population of first phages, wherein the first phages comprise a first DNA and require helper phages for replication of first phage particles, wherein the first DNA comprises a phage packaging signal for producing the first phage particles, wherein the first phages are capable of infecting target bacteria, and each first phage comprises antibacterial means for killing target bacteria,
wherein each first phage is a non self-replicative transduction particle,
wherein the phage DNA is comprised by a medium or high copy number plasmid,
and wherein
   (a) the antibacterial means encodes a Cas nuclease, or
   (b) wherein each first phage particle comprises a nucleotide sequence of interest (NSI) that is capable of expressing a protein or RNA in the target bacteria, wherein the presence in the target bacteria of the NSI-encoded protein or RNA mediates target cell killing, or downregulation of target cell growth or propagation,
wherein the NSI encodes a component of a CRISPR/Cas system that is toxic to the target bacteria, and wherein the component comprises
   (i) a DNA sequence encoding a guide RNA (eg, a single guide RNA) or comprising a CRISPR array for producing guide RNA, wherein the guide RNA is capable of targeting the genome of the target bacteria; and/or
   (ii) a DNA sequence encoding one or more components of Cascade.

A method of producing a phage composition, the method comprising expressing the phage proteins in a host bacterial cell comprising first and second DNAs, wherein
(i) the DNAs together comprise all phage structural protein genes required to produce a packaged phage particle comprising a copy of the first DNA;
(ii) the first DNA comprises none or at least one, but not all, of the genes; and wherein the one or more second DNAs comprise the remainder of the genes;
(iii)the first DNA comprises a phage packaging signal for producing the packaged phage particle; and
(iv) the second DNA is devoid of a nucleotide sequence required for packaging the second DNA into phage particles;

wherein packaged first phage particles are produced that comprise the first DNA, wherein the first phage require the second DNA for replication thereof to produce further first phage; and optionally separating an amount of first phage from cellular material wherein an amount of purified phage is obtained;
wherein each first phage comprises antibacterial means for killing target bacteria,
wherein the phage DNA is comprised by a medium or high copy number plasmid,
and wherein
   (a) the antibacterial means encodes a Cas nuclease, or
   (b) wherein each first phage particle comprises a nucleotide sequence of interest (NSI) that is capable of expressing a protein or RNA in the target bacteria, wherein the presence in the target bacteria of the NSI-encoded protein or RNA mediates target cell killing, or downregulation of target cell growth or propagation,
wherein the NSI encodes a component of a CRISPR/Cas system that is toxic to the target bacteria, and wherein the component comprises
   (i) a DNA sequence encoding a guide RNA (eg, a single guide RNA) or comprising a CRISPR array for producing guide RNA, wherein the guide RNA is capable of targeting the genome of the target bacteria; and/or
   (ii) a DNA sequence encoding one or more components of Cascade.

Any part of the description not within the scope of the claims and these statements does not form a part of the invention but serves in the understanding of the claimed invention.

The reference to methods of treatment by therapy or surgery or *in vivo* diagnosis methods in the examples of this description are to be interpreted as references to compounds, pharmaceutical compositions and medicaments of the present invention for use in those methods.
There is provided:
A non-self replicative transduction particle comprising a vector described herein.
"Transduction particles" may be phage or smaller than phage and are particles that are capable of transducing nucleic acid encoding the antibiotic or component thereof into target bacterial cells.

A composition comprising a plurality of transduction particles, wherein each particle comprises a vector described herein, wherein the transduction particles are capable of transferring the nucleic acid encoding the agent or component, or copies thereof into target bacterial cells, wherein
(i) target cells are killed by the antibacterial agent;
(ii) growth or proliferation of target cells is reduced; or
(iii) target cells are sensitised to an antibiotic, whereby the antibiotic is toxic to the cells.

A composition comprising a plurality of non-self replicative transduction particles, wherein each particle comprises a plasmid described herein, wherein the transduction particles are capable of transferring the nucleic acid encoding the agent or component, or copies thereof into target bacterial cells, wherein the agent is a CRISPR/Cas system and the component comprises a nucleic acid encoding a crRNA or a guide RNA that is operable with a Cas in a target bacterial cell to guide the Cas to a target nucleic acid sequence of the cell to modify the sequence, whereby
(i) target cells are killed by the antibacterial agent;
(ii) growth or proliferation of target cells is reduced; or
(iii) target cells are sensitised to an antibiotic, whereby the antibiotic is toxic to the cells.

A bacterial host cell comprising a first phage and a vector or particledescribed herein, wherein the agent is toxic to cells of the same species as the host cell, and wherein the host cell has been engineered so that the agent is not toxic to the host cell.

A bacterial host cell comprising a first phage, wherein the cell is comprised by a kit, the kit further comprising a composition described herein, wherein the agent is toxic to cells of the same species as the host cell, and wherein the host cell has been engineered so that the agent is not toxic to the host cell.

A bacterial host cell comprising a first phage and a vector or particle as described herein, wherein the agent is not toxic to the host cell, but the agent is toxic to second cells of a species or strain that is different from the species or strain of the host cell, whereby the second cell is exposed to the antibacterial agent.

A bacterial host cell comprising a vector or particle as described herein and nucleic acid under the control of one or more inducible promoters, wherein the nucleic acid encodes all structural proteins necessary to produce a transduction particle that packages a copy of the plasmid, wherein the agent is not toxic to the host cell, but the agent is toxic to second cells of a species or strain that is different from the species or strain of the host cell, whereby the second cell is exposed to the antibacterial agent.

A plasmid comprising
(a) A nucleotide sequence encoding an antibacterial agent or component thereof for expression in target bacterial cells;
(b) A constitutive promoter for controlling the expression of the agent or component;
(c) An optional *terS* nucleotide sequence;
(d) An origin of replication *(ori);* and
(e) A phage packaging sequence (optionally *pac, cos* or a homologue thereof); and
   the plasmid being devoid of
(f) All nucleotide sequences encoding phage structural proteins necessary for the production of a transduction particle (optionally a phage), or the plasmid being devoid of at least one of such sequences; and
(g) Optionally *terL.*

A bacterial host cell comprising the genome of a helper phage that is incapable of self-replication, optionally wherein the genome is present as a prophage, and a plasmid as described herein, wherein the helper phage is operable to package copies of the plasmid in transduction particles, wherein the particles are capable of infecting bacterial target cells to which the antibacterial agent is toxic.

A method of making a plurality of transduction particles, the method comprising culturing a plurality of host cells described herein, optionally inducing a lytic cycle of the helper phage, and incubating the cells under conditions wherein transducing particles comprising packaged copies of the plasmid are created, and optionally separating the particles from the cells to obtain a plurality of transduction particles.

A plurality of transduction particles obtainable by the method described herein for use in medicine, eg, for use in treating or preventing an infection of a human or animal subject by target bacterial cells, wherein transducing particles are administered to the subject for infecting target cells and killing the cells using the antibacterial agent.

A method of making a plurality of transduction particles, the method comprising
(a) Producing host cells whose genomes comprise nucleic acid encoding structural proteins necessary to produce transduction particles that can package first DNA, wherein the genomes are devoid of a phage packaging signal, wherein the expression of the proteins is under the control of inducible promoter(s);
(b) Producing first DNA encoding an antibacterial agent or a component thereof, wherein the DNA comprises a phage packaging signal;
(c) Introducing the DNA into the host cells;
(d) Inducing production of the structural proteins in host cells, whereby transduction particles are produced that package the DNA;
(e) Optionally isolating a plurality of the transduction particles; and
(f) Optionally formulating the particles into a pharmaceutical composition for administration to a human or animal for medical use.

A plurality of transduction particles obtainable by the method.

### BRIEF DESCRIPTION OF THE DRAWINGS

Figure 1: The genetic map of P2 genome with non-essential genes boxed;
Figure 2: Schematic of SaPIbov1;
Figure 3: (A) Maps of P2 and P4 - genomic architecture and regulatory network of phage P2 and satellite phage P4; the grey boxed region in P2 was deleted and replaced with a a kanamycin marker sequence, and the box shown in dashed lines shows the region of P4 including the *cos* site that was included in the CGV; (B) p94 genomic map;
Figure 4: Schematic view of the SA100 production srain - the defective helper phage will only turn on phage production following induction: since the phage packaging DNA sequence has been removed from the helper and placed on the CGV^{™}, only CGV^{™} molecules will be packaged into the synthetic phage particles;

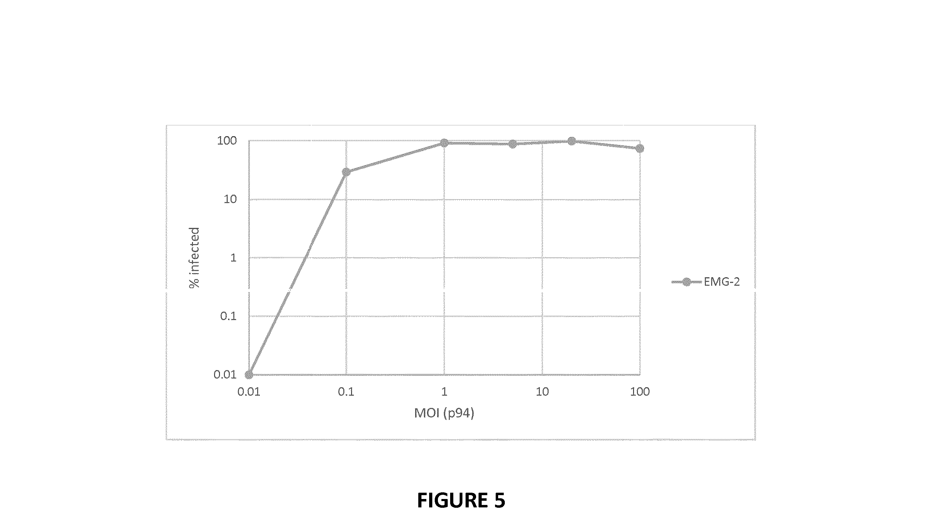

Figure 5: Efficiency of infection - percent infected EMG-2 cells with increasing ratio (MOI) of SA100 particles to EMG-2;
Figure 6: CGV^{™} delivery by SA100 - total CFU and CGV^{™}-containing CFU after infection of MG1655_pks cells (A) with SA100 containing p114 or p94 or X1-blue_pks cells (B) containing p114;
Figure 7: Killing of target cells by SA100-delivered CGV^{™} - killing efficiency of the two target strains by SA100 delivered CGVs^{™} (2nd, 4th and 6th bars) using wild-type and optimised copy number CGV^{™} compared to non-infected controls;
Figure 8: Plotted is the abundance as expressed in colony forming units (CFU) per 5 milliliter faecal sample (n=15). Two groups, CRISPR induced and non-induced are shown. No difference was observed between the groups when the CFU was deteremined on agar plates containing streptomycin; and
Figure 9: Plotted is the abundance as expressed in colony forming units (CFU) per 5 milliliter faecal sample (n=15). Two groups, CRISPR induced and non-induced are shown. The statistical difference is calculated using the Student t-test, showing a statistically significant increase in abundance after 48 hours in the CRISPR non-induced group (p-value=0.011) on agar plates supplemented with spectinomycin and streptomycin. This shows that the CGV (containing a spectonomycin marker) can be delivered by non-self-replicative particles SA100.

### DETAILED DESCRIPTION

The disclosure relates to the production of phage using DNAs (eg, plasmids with helper phage), as well as the phage and helper phage, and the invention relates to the compositions and methods involving these. The invention finds utility, for example, for containing phage in environments *ex vivo* and *in vivo,* reducing the risk of acquisition of antibiotic resistance or other genes by phage, as well as controlling dosing of phage in an environment. The contamination of useful phage populations by helper phage may in examples also be restricted or eliminated, thereby controlling phage propagation and enhancing the proportion of desired phage in phage compositions, such as medicaments, herbicides and other agents where phage may usefully be used. Thus, there is provided:

A kit comprising
a) A first DNA; and
b) One or more second DNAs;
Wherein
(i) the DNAs together comprise all phage structural protein genes required to produce a packaged phage particle comprising a copy of the first DNA;
(ii) the first DNA comprises none or at least one, but not all, of the genes; and wherein the one or more second DNAs comprise the remainder of the genes;
(iii) the first DNA comprises a phage packaging signal for producing the packaged phage particle; and
(iv) the second DNA is devoid of a nucleotide sequence required for packaging the second DNA into phage particles;
wherein the DNAs are operable when co-existing in a host bacterium for producing packaged phage that comprise the first DNA, wherein the phage require the second DNA for replicaton thereof to produce further phage particles.

For example the second DNA is devoid of a packaging signal for packaging second DNA. Additionally or alternatively, the second DNA is devoid of a nucleotide sequence required for replication of helper phage. Optionally, the nucleotide sequence enodes a sigma factor or comprises a sigma factor recognition site, a DNA polymerisation recognition site, or a promoter of a gene required for helper phage DNA replication when the second DNA is comprised by a helper prophage.

The second DNA may be comprised by an M13 or M13-based helper phage. M13 encodes the following proteins required for phage packaging:-
a. pIII: host recognition
b. pV: coat protein
c. pVII, pVIII, pIX: membrane proteins
d. pI, pIV, pXI: Channel for translocating the phage to the extracellular space.

Optionally, the second DNA is devoid of one or more of the genes coding for these proteins, eg, is devoid of a gene endoding pIII, a gene encoding pV, a gene endoding pVII, a gene endoding pVIII, a gene endoding pIX, a gene endoding pI, a gene endoding pIV and/or a gene endoding XI.

The phage particle of (i) may be capable of infecting a target bacterium, the phage comprising a nucleotide sequence of interest (NSI) that is capable of expressing a protein or RNA in the target bacterium, or wherein the NSI comprises a regulatory element that is operable in the target bacterium. Optionally, the NSI is capable of recombination with the target cell chromosome or an episome comprised by the target cell to modify the chromosome or episome. Optionally, this is carried out in a method wherein the chromosome or episome is cut (eg, at a predetermined site using a guided nuclease, such as a Cas, TALEN, zinc finger or meganuclease; or a restriction endonuclease) and simultaneously or sequentially the cell is infected by a phage particle that comprises the first DNA, wherein the DNA is introduced into the cell and the NSI or a sequence thereof is introduced into the chromosome or episome at or adjacent the cut site. Optionally, the first DNA comprises one or more components of a CRISPR/Cas system operable to perform the cutting (eg, comprising at least a nucleotide sequence encoding a guide RNA or crRNA for targeting the site to be cut) and further comprising the NSI.

The presence in the target bacterium of the NSI or its encoded protein or RNA may mediate target cell killing, or downregulation of growth or propagation of target cells, or mediate switching off of expression of one or more RNA or proteins encoded by the target cell genome, or downregulation thereof.

The presence in the target bacterium of the NSI or its encoded protein or RNA may mediate upregulation of growth or propagation of the target cell, or mediate switching on of expression of one or more RNA or proteins encoded by the target cell genome, or upregulation thereof.

The NSI may encode a component of a CRISPR/Cas system that is toxic to the target bacterium.

The DNA may be a first DNA as defined in any preceding paragraph.

The first DNA may be comprised by a vector (eg, a plasmid or shuttle vector).

The second DNA may be comprised by a vector (eg, a plasmid or shuttle vector), helper phage (eg, a helper phagemid) or is integrated in the genome of a host bacterial cell.

There is provided a bacterial cell comprising the first and second DNAs. Optionally, the cell is devoid of a functional CRISPR/Cas system before transfer therein of a first DNA, eg, a first DNA comprising a component of a CRISPR/Cas system that is toxic to the target bacterium. There is provided an antibacterial composition comprising a plurality of cells, wherein each cell is optionally according to this paragraph, for administration to a human or animal subject for medical use.

A method of producing phage is provided, the method comprising expressing in a host bacterial cell the phage protein genes, wherein packaged phage are produced that comprise the first DNA, wherein the phage require the second DNA for replicaton thereof to produce further phage particles. Optionally, the method comprises isolating the phage particles.

A composition comprising a population of phage particles obtainable by the method is provided for administration to a human or animal subject for use in treating an infection of target bacterial cells, wherein the phage are capable of infecting and killing the target cells.

A method of treating an environment *ex vivo,* the method comprising exposing the environment to a population of phage particles obtainable by the method is provided, wherein the environment comprises target bacteria and the phage infect and kill the target bacteria. In an example thje subject is further administered an agent simultaneously or sequentially with the phage administration. In an example, the agent is a herbicide, pesticide, insecticide, plant fertilizer or cleaning agent.

Optionally, the method is for containing the treatment in the environment.

Optionally, the method is for controlling the dosing of the phage treatment in the environment.

Optionally, the method is for reducing the risk of acquisition of foreign gene sequence(s) by the phage in the environment.

Described herein is a method of treating an infection of target bacteria in a human or animal subject is provided, the method comprising exposing the bacteria to a population of phage particles obtainable by the production method, wherein the phage infect and kill the target bacteria.

Optionally, the method described herein is for treating is for containing the treatment in the subject.

Optionally, the method described herein is for treating is for containing the treatment in the environment in which the subject exists.

Optionally, the method described herein is for treating is for controlling the dosing of the phage treatment in the subject.

Optionally, the method described herein is for treating is for reducing the risk of acquisition of foreign gene sequence(s) by the phage in the subject.

Optionally, the method described herein is for treating or for reducing the risk of acquisition of foreign gene sequence(s) by the phage in the environment in which the subject exists.

Optionally, target bacteria herein are comprised by a microbiome of the subject, eg, a gut microbiome. Altertnatively, the microbiome is a skin, scalp, hair, eye, ear, oral, throat, lung, blood, rectal, anal, vaginal, scrotal, penile, nasal or tongue microbiome.

The subject may be further administered a medicament simultaneously or sequentially with the phage administration. In an example, the medicament is an antibiotic, antibody, immune checkpoint inhibitor (eg, an anti-PD-1, anti-PD-L1 or anti-CTLA4 antibody), adoptive cell therapy (eg, CAR-T therapy) or a vaccine.

**In** an example, helper phage may be employed for packaging the phage nucleic acid of interest. Thus, there is provided the following:-

**In a** first aspect, there is provided a population of helper phage, wherein the helper phage are capable of packaging first phage nucleic acid to produce first phage particles, wherein the first phage are different from the helper phage and the helper phage are incapable themselves of producing helper phage particles.

**In** a second aspect, there is provided composition comprising a population of first phage, wherein the first phage require helper phage according to Aspect 1 for replication of first phage particles; and optionally wherein less than 20, 15, 10, 5, 4, 3, 2, 1, 0.5, 0.4, 0.2 or 0.1% of total phage particles comprised by the composition are particles of such helper phage.

**In** an example, the composition comprises helper phage and less than 1% of total phage particles comprised by the composition are particles of such helper phage. In an example the composition comprises helper phage and less than 0.5% of total phage particles comprised by the composition are particles of such helper phage. In an example the composition comprises helper phage and less than 0.1% of total phage particles comprised by the composition are particles of such helper phage. In an example the composition comprises helper phage and less than 0.01% of total phage particles comprised by the composition are particles of such helper phage. In an example the composition comprises helper phage and less than 0.001% of total phage particles comprised by the composition are particles of such helper phage. In an example the composition comprises helper phage and less than 0.0001% of total phage particles comprised by the composition are particles of such helper phage. In an example the composition comprises helper phage and less than 0.00001% of total phage particles comprised by the composition are particles of such helper phage. In an example the composition comprises helper phage and less than 0.000001% of total phage particles comprised by the composition are particles of such helper phage. In an example the composition comprises helper phage and less than 0.0000001% of total phage particles comprised by the composition are particles of such helper phage. In an example the composition comprises helper phage and less than 0.00000001% of total phage particles comprised by the composition are particles of such helper phage.

**In** an example, the population comprises at least 10³,10⁴, 10⁵ or 10⁶ phage particles, as indicated a transduction assay, for example. In an example, the population comprises at least 10³ phage particles and eg, no more than 10¹⁴ particles. In an example, the population comprises at least 10⁴ phage particles and eg, no more than 10¹⁴ particles. In an example, the population comprises at least 10⁵ phage particles and eg, no more than 10¹⁴ particles. In an example, the population comprises at least 10⁶ phage particles and eg, no more than 10¹⁴ particles. To have a measure of the first phage concentration, for example, one can perform a standard transduction assay when the first phage genome contains an antibiotic marker. Thus, in this case the first phage are capable of infecting target bacteria and in a sample of 1ml the population comprises at least 10³,10⁴, 10⁵ or 10⁶ transducing particles, which can be determined by infecting susceptible bacteria at a multiplicity of infection <0.1 and determining the number of infected cells by plating on a selective agar plate corresponding to the antibiotic marker in vitro at 20 to 37 degrees centigrade, eg, at 20 or 37 degrees centrigrade.

Optionally at least 99.9, 99.8, 99.7, 99.6, 99.5, 99.4, 99.3, 99.2, 99.1, 90, 85, 80, 70, 60, 50 or 40% of total phage particles comprised by the composition are particles of first phage.

In an example, the first phage genome comprises an f1 origin of replication.

In an example, the helper phage are E coli phage. In an example, the first phage are *E coli, C dificile, Streptococcus, Klebsiella, Pseudomonas, Acitenobacter, Enterobacteracea, Firmicutes* or *Bacteroidetes* phage. In an example, the helper phage are engineered M13 phage.

In an example, the first phage genome comprises a phagemid, wherein the phagemid comprises a packaging signal for packaging first phage particles in the presence of the helper phage.

The first phage particles may contain a nucleotide sequence of interest (NSI), eg, as defined herein, such as a NSI that encodes a component of a CRISPR/Cas system operable in target bacteria that can be infected by the first phage particles. Once inside the target bacteria, the first phage DNA is incapable of being packaged to form first phage particles in the absence of the helper phage. This usefully contains the activity of the first phage genome and its encoded products (protieins and/or nucleic acid), as well as limits or controls dosing of the NSI and its encoded products in an environment comprising the target bacteria that have been exposed to the first phage. This is useful, for example to control the medical treatment of an environment comprised by a human or animal subject, plant or other environment (eg, soil or a foodstiff or food ingredient).

In an example the helper phage or composition as described in the first or second aspect, wherein the genome of each first phage is devoid of genes encoding first phage structural proteins.

Optionally the composition comprises helper phage DNA, and further optionally the DNA comprises helper DNA fragments.

**In** an example, the helper phage are in the form of prophage.

Thus, the prophage is integrated in the chromosome of a host cell.

Examples of phage structural proteins are phage coat proteins, collar proteins and phage tail fibre proteins.

In an example, the composition as described in the second aspect comprises no helper phage DNA comprising a sequence of 20 contiguous nucleotides or more (eg, from 20 to 25, 30, 35, 40, 50 or 100 nucleotides), eg, no helper phage DNA.

This can be determined, for example, using DNA probes (designed on the basis of the known heper phge genome sequence) with PCR, as is conventional. In an example, the composition may comprise residual helper prophage DNA, but essentially otherwise is devoid of helper DNA.

In an example, the composition as described in the second aspect, excluding where the helper phage are in the form of prophage, wherein the helper phage are capable of infecting host bacteria and the composition does not comprise host bacteria.

In an example, the composition as described in the second aspect is a lysate of host bacterial cells, wherein the lysate comprises helper prophage DNA, eg, such DNA comprises 20 contiguous nucleotides or more (eg, from 20 to 25, 30, 35, 40, 50 or 100 nucleotides) of helper phage DNA.

Optionally, the composition is a lysate of host bacterial cells, wherein the lysate has been processed (eg, filtered) to remove all or some helper phage DNA; or the composition is a lysate of host bacterial cells that is devoid of cellular material.

In an example, the composition as described in the second aspect does not comprise helper phage particles.

In an example, in the composition as described in the second aspect, at least 95% (eg, 100%) of phage particles comprised by the composition are first phage particles.

In another example, the composition comprises second phage particles, wherein the second phage are different from the first phage and are not helper phage.

**In** an example, in the composition as described in the second aspect, the population comprises at least 103, 104, 105 or 106 phage particles, as indicated in a transduction assay.

**In** an example, the first phage of the helper phage or composition as descibred in the first or second aspect are capable of replicating in host bacteria in the presence of the helper phage (eg, helper prophage), and wherein the first phage comprise antibacterial means for killing target bacteria of a first strain or species, wherein the target bacteria are of a different strain or species and the antibacterial means is not operable to kill the target bacteria.

**In** an example, the composition as described in the second aspect, comprises a population of phage, the population comprising
a) A first sub-population of first phage that require a helper phage for packaging the first phage;
b) A second sub-population of phage comprising the helper phage, wherein the helper phage are as recited in any preceding Aspect.

**In** an example, the helper phage, or the helper phage in composition, as described in the first or second aspect are phagemids.

**In a** third aspect, there is provided a composition comprising
a) A population of helper phage as described in the first and second aspects above; and
b) A population of nucleic acid vectors comprising vector DNA that comprises a first phage packaging signal;
c) wherein the helper phage are capable of packaging the vector DNA to produce first phage.

**In** an example, in the composition as described in the third aspect, the vectors are phage.

**In** an example, in the composition as described in the third aspect, the vectors are plasmids or phagemids.

Optionally, the vectors are shuttle vectors (eg, pUC vectors) that can be replicated in first bacteria, wherein the vectors can further be replicated and packaged into first phage in second bacteria (host bacteria) in the presence of the helper phage, wherein the first bacteria are of a strain or species that is different to the strain or species of the host bacteria.

Optionally, the first phage are capable of infecting third bacteria of a strain or species that is different to the second (and optionally also the first) bacteria.

**In** an example, the first phage of the composition as described in the third aspect are capable of replicating in host bacteria in the presence of the helper phage (eg, helper prophage), wherein the first phage comprise antibacterial means for killing target bacteria of a first strain or species, wherein the host bacteria are of a different strain or species and the antibacterial means is not operable to kill the host bacteria.

**In** an example, in the helper phage or composition as described in the first, second or third aspect, the genome is devoid of a packaging signal (eg, SEQ ID NO: 2 below), and wherein the helper phage are incapable of self-replication.

Optionally, the signal is a pac or cos sequence.

**In** an example, in the helper phage or composition as described in the first, second or third aspect, the genome is devoid of a nucleotide sequence required for production of helper phage particles.

Optionally, the nucleotide sequence enodes a sigma factor (eg, sigma-70) or comprises a sigma factor recognition site, a DNA polymerisation recognition site, or a promoter of a gene required for helper phage DNA replication.

**In** an example, in the helper phage or composition as described in the first, second or third aspect, the helper phage genome is capable of replication in a host cell.

Thus, the genome is capable of nucleic acid replication but not packaging of helper phage.

**In** an example, in the helper phage or composition as described in the first, second or third aspect, the helper phage are temperate phage or the helper phage are lytic phage.

**In** an example, in the helper phage or composition as described in the first, second or third aspect, the first phage are capable of infecting target bacteria, the first phage comprising a nucleotide sequence of interest (NSI) that is capable of expressing a protein or RNA (eg, gRNA or crRNA) in target bacteria, or wherein the NSI comprises a regulatory element that is operable in target bacteria.

Optionally, the presence in target bacteria of the NSI or its encoded protein or RNA mediates target cell killing, or downregulation of growth or propagation of target cells, or mediates switching off of expression of one or more RNA or proteins encoded by the target cell genomes, or downregulation thereof, or the presence in target bacteria of the NSI or its encoded protein or RNA mediates upregulation of growth or propagation of target cells, or mediates switching on of expression of one or more RNA or proteins encoded by the target cell genomes, or upregulation thereof.

**In** an example, in the antibacterial composition as described in the second or third aspect, the first phage are capable of infecting target bacteria and each first phage comprises engineered antibacterial means for killing target bacteria.

By use of the term "engineered" it will be readily apparent to the skilled addressee that the relevant means has been introduced and is not naturally-occurring in the phage. For example, the means is recombinant, artificial or synthetic.

Optionally, the antibacterial means comprises one or more components of a CRISPR/Cas system.

Optionally, the component(s) comprise (i) a DNA sequence encoding a guide RNA (eg, a single guide RNA) or comprising a CRISPR array for producing guide RNA, wherein the guide RNA is capable of targeting the genome of target bacteria; (ii) a Cas nuclease-encoding DNA sequence; and/or (iii) a DNA sequence encoding one or more components of Cascade.

In an example, a Cas herein is a Cas9. In an example, a Cas herein is a Cas3. The Cas may be identical to a Cas encoded by the target bacteria.

Optionally, the antibacterial means comprises a nucleic acid encoding a guided nuclease, such as a Cas nuclease, TALEN, zinc finger nuclease or meganuclease.

In an example, in the helper phage or composition as described in the first, second or third aspect, the helper phage is for use in medicine practised on a human or animal subject, or the composition is a pharmaceutical composition for use in medicine practised on a human or animal subject.

In an example, the animal is a livestock or companion pet animal (eg, a cow, pig, goat, sheep, horse, dog, cat or rabbit). In an example, the animal is an insect (an insect at any stage of its lifecycle, eg, egg, larva or pupa). In an example, the animal is a protozoan. In an example, the animal is a cephalopod.

In an example, the composition as described in the second or third aspect is a herbicide, pesticide, food or beverage processing agent, food or beverage additive, petrochemical or fuel processing agent, water purifying agent, cosmetic additive, detergent additive or environmental (eg, soil) additive or cleaning agent.

In an example, the helper phage or composition as described in the first, second or third aspectis for use in a contained method of treating a disease or condition of a human or animal subject, wherein the disease or condition is mediated by the target bacteria and the target bacteria are comprised by the subject, the method comprising administering the composition to the subject, whereby the target bacteria are exposed to the antibacterial means and killed and propagation of the first phage is contained.

The inability of the first phage to self-replicate and to require helper phage or second DNA to do this usefully provides containment in the location (eg, gut) of action of the composition and/or in the environment of the subject, eg, when exposed to secretions such as urine and faeces of the subject that otherwise may contain replicated first phage. Inability of the helper phage or second DNA to self-package limits availability of factors required by the first phage to form packaged particles, hence providing containment by limiting first phage propagation. This may be useful, for example, to contain an antibacterial acitivity provided by the first phage, such as a CRISPR/Cas killing principle.

In a fourth aspect, there is provided a bacterial cell or a plurality of bacterial cells comprising the helper phage or composition as described in the first, second or third aspect, wherein the first phage are capable of replication in the presence of the helper phage in the cell.

The cell may, for example, act as a carrier for the genome of the first phage, wherein the first phage DNA is capable of horizontal transfer from the carrier to the target bacteria once the carrier bacteria have been administered to an environment to be treated, eg, a soil or a human gut or other environment described herein. In an example, the environment is comprised by a human or animal subject and the carrier are commensal or probiotic in the subject. For example the carrier bacteria are Lactobacillus (eg, L reuteri or L lactis), E coli or Streptococcus (eg, S thermophilus) bacteria. The horizontal transfer can be transfer of a plasmid (such as a conjugative plasmid) to the target bacteria or first phage infection of the target bacteria, wherein the first phage have been prior packaged in the carrier. The use of a carrier is useful too for oral administration or other routes where the carrier can provide protection for the phage, helper or composition from the acid stomach or other harsh environments in the subject. Furthermore, the carrier can be formulated into a beverage, for example, a probiotic drink, eg, an adapted Yakult (trademark), Actimel (trademark), Kevita (trademark), Activia (trademark), Jarrow (trademark) or similar drink for human consumption.

In an example, the cell(s) as described in the fourth aspectare for administration to a human or animal subject for medical use, comprising killing target bacteria using first phage, wherein the target bacteria mediate as disease or condition in the subject.

In an example, when the subject is a human, the subject is not an embryo.

Optionally, the cell(s) comprises helper phage and is symbiotic or probiotic in the subject.

**In a** fifth aspect, there is provided a method of killing target bacteria in an environment, optionally wherein the method is not practised on a human or animal body, wherein the method comprises exposing the environment to the cell(s) as described in the fourth aspect, or a composition obtained or obtainable by the method as described in the twelfth aspect, wherein the environment is or has been exposed to first phage or said vectors to produce first phage in the presence of the helper phage, wherein the first phage are capable of replication in the environment and kill target bacteria.

**In** an example, in the cell(s) as described in the fourth aspect or in the method as described in the fifth aspect, the cell is an E coli, Lactobacillus (eg, L lactis or retueri) or Streptococcus (eg, thermophilus) cell.

**In** an example, in the cell(s) or method as described in the fourth or fifth aspect, the subject is administered or has been administered a cell comprising first phage.

**In** an example, the composition as described in the second or third aspect is in combination with a target bacterial cell wherein the first phage are capable of infecting the target bacterial cell.

**In** a sixth aspect, there is provided the use of the helper phage, composition or cell(s) as described in the first, second, third or fourth aspect, or a composition obtained or obtainable by the method as described in the twelfth aspect, in the manufacture of an antibacterial agent that kills target bacteria, for containment of the antibacterial in an environment, eg, containment ex vivo; or containment in a human or animal subject comprising the environment.

**In** a seventh aspect, there is provided the use of the helper phage, composition or cell(s) as described in the first, second, third or fourth aspect, or a composition obtained or obtainable by the method as described in the twelfth aspect, in the manufacture of an antibacterial agent that kills the target bacteria, for reducing the risk of acquisition by the first phage of foreign genes.

For example, this is useful for reducing the risk of antibiotic resistance genes by the phage, such as when the phage are in the presence of other phage or plasmids in the environment.

**In** an eighth aspect, there is provided the use of the helper phage, composition or cell(s) as described in the first, second, third or fourth aspect, or a composition obtained or obtainable by the method as described in the twelfth aspect, in the manufacture of an antibacterial agent that kills the target bacteria, for reducing the risk of acquisition by the first phage of one or more antibiotic resistance genes.

**In** a ninth aspect, there is provided a method of reducing the risk of acquisition by first phage of foreign genes, the method comprising
a) Providing the composition as described in the second or third aspect, or a composition obtained or obtainable by the method as described in the twelfth aspect; and
b) Exposing target bacteria to the composition, wherein the first phage infect the target bacteria;
c) wherein the helper phage are incapable of self-replication and propagation of first phage is thereby limited, wherein propagation of first phage is prevented or reduced, thereby reducing the risk of acquisition of first phage of foreign genes (eg, antibiotic resistance genes).

**In** a tenth aspect, there is provided a method of containing an antibacterial activity in an environment (e.g., ex vivo), the method comprising
a) Providing an antibacterial composition as described in the second or third aspect, or a composition obtained or obtainable by the method as described in the twelfth aspect; and
b) Exposing target bacteria in the environment to the composition, wherein the bacteria are exposed to the first phage and antibacterial means and are killed;
c) wherein the helper phage are incapable of self-replication and propagation of first phage is thereby limited, wherein propagation of first phage is prevented or reduced, thereby containing the antibacterial activity.

**In** an eleventh aspect, there is provided a method of controlling the dosing of first phage in an environment (e.g., ex vivo), the method comprising
a) Providing an antibacterial composition as described in the second or third aspect, or a composition obtained or obtainable by the method as described in the twelfth aspect; and
b) Exposing target bacteria in the environment to the composition, wherein the bacteria are infected by first phage;
c) wherein the helper phage are incapable of self-replication and propagation of first phage is thereby limited, wherein propagation of first phage is prevented or reduced, thereby controlling dosing of first phage in the environment.

**In** an example, in the method as described in the fifth, tenth or eleventh aspect, or in the use as described in the sixth aspect, the environment is a human or animal microbiome, e.g., a gut microbiome, or the environment is a microbiome of soil; a plant, part of a part (e.g., a leaf, fruit, vegetable or flower) or plant product (e.g., pulp); water; a waterway; a fluid; a foodstuff or ingredient thereof; a beverage or ingredient thereof; a medical device; a cosmetic; a detergent; blood; a bodily fluid; a medical apparatus; an industrial apparatus; an oil rig; a petrochemical processing, storage or transport apparatus; a vehicle or a container, or the environment is an ex vivo bodily fluid (e.g., urine, blood, blood product, sweat, tears, sputum or spit), bodily solid (e.g., faeces) or tissue of a human or animal subject that has been administered the composition, or the environment is an in vivo bodily fluid (e.g., urine, blood, blood product, sweat, tears, sputum or spit), bodily solid (e.g., faeces) or tissue of a human or animal subject that has been administered the composition.

**In** a twelfth aspect, there is provided a method of producing first phage, wherein the first phage require helper phage to replicate, the method comprising
a) Providing DNA comprising a packaging signal;
b) Introducing the DNA into a host bacterial cell;
c) Wherein the host bacterial cell comprises helper phage or wherein helper phage are introduced into the bacterial cell simultaneously or sequentially with step (b);
d) Wherein the helper phage are according to any preceding Aspect;
e) Causing or allowing the helper phage to produce phage coat proteins, wherein the packaging signal is recognised in the host cell, whereby first phage are produced using the proteins, the first phage packaging the DNA;
f) Wherein helper phage particle production in the host cell is inhibited or reduced, thereby limiting the availability of helper phage particles;
g) Optionally lysing the host cell and obtaining the first phage;
h) Thereby producing a composition comprising first phage which require the helper phage for replication, wherein further production of first phage particles is prevented or reduced by the limitation of helper phage availability in the composition.

The DNA may be comprised by a phagemid or cloning vector (eg, a shuttle vector, eg, a pUC vector).

There may be a modest amount of helper phage DNA replication to enable first phage protein production efficiently, or should replication of helper phage DNA may be eliminated totally eliminated.

**In** an example, in the method as described in the twelfth aspect, in (c) the helper phage are prophage integrated in the bacterial cell chromosome.

Optionally, (e) comprises inducing replication of helper phage DNA and/or expression of the proteins, eg, using UV, mitomycin.

**In** an example, in the method as described in the twelfth aspect, (g) comprises further separating the first phage from cellular material or helper phage DNA.

In an example, in the method as described in the twelfth aspect, the composition comprises a population of first phage particles, wherein the composition does not comprise helper phage DNA and/or particles.

In an example, in the method as described in the twelfth aspect, the DNA of (a) comprises engineered antibacterial means for killing target bacteria.

Optionally, the antibacterial means comprises one or more components of a CRISPR/Cas system.

Optionally, the component(s) comprise (i) a DNA sequence encoding a guide RNA (eg, a single guide RNA) or comprising a CRISPR array for producing guide RNA, wherein the guide RNA is capable of targeting the genome of target bacteria; (ii) a Cas (eg, Cas9, Cas3, Cpf1, CasX or CasY) nuclease-encoding DNA sequence; and/or (iii) a DNA sequence encoding one or more components of Cascade (eg, CasA).

Optionally, the antibacterial means comprises a nucleic acid encoding a guided nuclease, such as a Cas nuclease, TALEN, zinc finger nuclease or meganuclease.

In an example, the helper phage, composition or cell(s) as described in the first, second, third or fourth aspect, or a composition obtained or obtainable by the method as described in the twelfth aspect, is for antibacterial treatment of target bacteria in a human or animal subject whereby the antibacterial treatment is contained in the subject, or is for antibacterial treatment of target bacteria in a gut of a human or animal subject whereby the antibacterial activity in one or more bodily excretions of the subject is reduced.

This is useful as a safety measure to reduce or eliminate first phage activity outside the subject.

Optionally, the antibacterial activity in one or more bodily excretions of the subject is eliminated.

In an example, the helper phage, composition or cell(s) as described in the first, second, third or fourth aspect, or a composition obtained or obtainable by the method as described in the twelfth aspect, is for controlling the dosing of antibacterial treatment of target bacteria in a human or animal subject, eg, in the gut of the subject.

Usefully, propagation of the first phage is restricted or eliminated, so dosing in the subject can be controlled, or even pre-determined within a narrow expected range. This is useful, for example, for medicaments comprising the first phage or composition, and may be aid approval of such medicines before FDA and simiar authorities.

Alternatively, the dosing is dosing of an environment, such as soil etc disclosed herein, wherein limitation of the first phage or composition activity is also desirable to limit spread of activities in natural and other terrains.

In an example, the helper phage, composition or cell(s) as described in the first, second, third or fourth aspect, or a composition obtained or obtainable by the method as described in the twelfth aspectis for fixing the dosing of antibacterial treatment of target bacteria in a human or animal subject, eg, in the gut of the subject.

In a thirteenth aspect, there is provided a phage production system, for producing phagecomprising a nucleotide sequence of interest (NSI-phage), the system comprising components (i) to (iii):-
a) A first DNA;
b) A second DNA; and
c) a NSI-phage production factor (NPF) or an expressible nucleotide sequence that encodes a NPF;
   Wherein
d) The first DNA encodes a helper phage;
e) The second DNA comprises the nucleotide sequence of interest (NSI);
f) When the system is comprised by a bacterial host cell, helper phage proteins are expressed from the first DNA to form phage that package the second DNA in the presence of the NPF, thereby producing NSI-phage; and
g) The system is devoid of a helper phage production factor (HPF) that is required for forming helper phage particles that package the first DNA, or is devoid of an expressible nucleotide sequence that encodes a functional HPF; or the system comprises a nucleotide sequence that comprises or encodes a functional HPF, the system further comprising means for targeted inactivation in the host cell of the HPF sequence to eliminate or minimise production of helper phage comprising the first DNA;
Whereby the system is capable of producing a product comprising a population of NSI-phage, wherein each NSI-phage requires a said helper phage for propagation, optionally wherein the NSI-phage in the product are not mixed with helper phage or less than 20% of total phage comprised by the product are said helper phage.

A relatively low level of helper phage particle production may be achieved if there is a residual capability of helper phage to replicate to produce particles, such as for example in the case that a helper phage packaging signal or other HPF nucleotide sequence in the helper phage genome is mutated (eg, by deletion, substitution or addition of nucleotides therein) to knock down the ability to form phage particles. Preferably, there is no production of helper phage particles, such as by deleting all or part of the sequence from the helper phage genome or inactivating the sequence.

In a fourteenth aspect, there is provided a method of producing first phage, wherein the first phage require helper phage to replicate, the method comprising
a) Providing in host cells the system as described in the thirteenth aspect;
b) Causing or allowing the helper phage proteins to be produced, whereby the second DNA is packaged to produce first phage; and
c) Optionally lysing the host cells and obtaining a composition comprising first phage.

In an example, in the method as described in the fourteenth aspect, step (c) comprises separating the first phage from cellular material.

In an example, in the method as described in the fourteenth aspect, the composition comprises a population of first phage, wherein less than 20, 10, 5, 4, 3, 2, 1, 0.5 or 0.1% of total phage comprised by the composition are helper phage.

In an example, in the method as described in the fourteenth aspect, the second DNA comprises engineered antibacterial means for killing target bacteria.

Optionally, the antibacterial means comprises one or more components of a CRISPR/Cas system.

Optionally, the component(s) comprise (i) a DNA sequence encoding a guide RNA (eg, a single guide RNA) or comprising a CRISPR array for producing guide RNA, wherein the guide RNA is capable of targeting the genome of target bacteria; (ii) a Cas nuclease-encoding DNA sequence; and/or (iii) a DNA sequence encoding one or more components of Cascade.

Optionally, the antibacterial means comprises a nucleic acid encoding a guided nuclease, such as a Cas nuclease, TALEN, zinc finger nuclease or meganuclease

In an example, in the system or method as described in the thirteenth or fourteenth aspect, the first phage are capable of infecting target bacteria, the NSI being capable of expressing a protein or RNA in target bacteria, or wherein the NSI comprises a regulatory element that is operable in target bacteria.

Optionally, the presence in target bacteria of the NSI or its encoded protein or RNA mediates target cell killing, or downregulation of growth or propagation of target cells, or mediates switching off of expression of one or more RNA or proteins encoded by the target cell genomes, or downregulation thereof, or the presence in target bacteria of the NSI or its encoded protein or RNA mediates upregulation of growth or propagation of target cells, or mediates switching on of expression of one or more RNA or proteins encoded by the target cell genomes, or upregulation thereof.

In an example, in the system or method as described in the thirteenth or fourteenth aspect, each of the NPF and HPF is a packaging signal, eg, SEQ ID NO: 2 or a sequence that is at least 70, 80, 90, 95, 96, 97, 98 or 99% identical thereto, or is a homologue from a different species.

Optionally, each signal is a pac or cos sequence, or is a homologue.

In an example, in the system or method as described in the thirteenth or fourteenth aspect, the HPF is a nucleotide sequence required for replication of helper phage.

In an example, in the system or method as described in the thirteenth or fourteenth aspect, the HPF enodes a sigma factor (eg, sigma-70) or comprises a sigma factor recognition site, a DNA polymerisation recognition site, or a promoter of a gene required for helper phage DNA replication, a helper phage integrase, a helper phage excissionase or a helper phage origin of replication.

In a fifthteenth aspect, there is provided a composition comprising a population of first phage obtainable by the method as described in the fourteenth aspect, wherein the genome of each first phage is devoid of genes encoding phage proteins.

Optionally, the first phage comprise antibacterial means as described herein.

Optionally, the composition comprises DNA identical to the first DNA or fragments thereof.

Optionally, the DNA of the composition is identical to the first DNA and is devoid of a helper phage packaging signal.

In an example, the composition as described in the fifthteenth aspectis for antibacterial treatment of target bacteria in a human or animal subject whereby the antibacterial treatment is contained in the subject, or is for controlling the dosing of antibacterial treatment of target bacteria in a human or animal subject, eg, in the gut of the subject, or is for fixing the dosing of antibacterial treatment of target bacteria in a human or animal subject, eg, in the gut of the subject, or is for antibacterial treatment of target bacteria in a gut of a human or animal subject whereby the antibacterial activity in one or more bodily excretions of the subject is reduced.

Optionally, the antibacterial activity in one or more bodily excretions of the subject is eliminated.

In a sixthteenth aspect, there is provided an isolated DNA comprising all structural protein genes of a helper phage genome that are required for producing phage particles, wherein the DNA is devoid of a helper phage production factor (HPF) that is required for producing packaged helper phage, optionally wherein the DNA comprises one or more promoters for expression of the genes when the DNA is integrated in the genone of a host bacterial cell.

In an example, the DNA as described in the sixthteenth aspect is devoid of any phage packaging signals.

In an example, in the DNA as described in the sixthteenth aspect, the HPF is a sigma factor-encoding nucleotide sequence or comprises a sigma factor recognition site, a DNA polymerisation recognition site, a promoter of a gene required for helper phage DNA replication, a helper phage integrase-encoding nucleotide sequence, a helper phage excissionase-encoding nucleotide sequence or a helper phage origin of replication.

In an example, the DNA as described in the sixthteenth aspect comprises a nucleotide sequence encoding a CRISPR/Cas system repressor.

In an example, the DNA as described in the sixthteenth aspect is integrated in the chromosome of a host bacterial cell, wherein the genes are expressible in the host cell.

Optionally, the cell is devoid of an active CRISPR/Cas system.

In an example, the DNA as described in the sixthteenth aspect is in combination with a second DNA, wherein the second DNA comprises the HPF, and/or wherein the second DNA comprises a phage packaging signal and optionally the first DNA is devoid of a phage packaging signal.

Optionally, the second DNA is comprised by a phagemid or a plasmid (eg, a shuttle vector).

In an example, the kit, DNA(s), first phage, helper phage or composition is comprised by a medical container, eg, a syringe, vial, IV bag, inhaler, eye dropper or nebulizer. In an example, the kit, DNA(s), first phage, helper phage or composition is comprised by a sterile container. In an example, the kit, DNA(s), first phage, helper phage or composition is comprised by a medically-compatible container. In an example, the kit, DNA(s), first first phage, helper phage or composition is comprised by a fermentation vessel, eg, a metal, glass or plastic vessel.

In an example, the kit, DNA(s), first phage, helper phage or composition is comprised by a medicament, e,g in combination with instructions or a packaging label with directions to administer the medicament by oral, IV, subcutaneous, intranasal, intraocular, vaginal, topical, rectal or inhaled administration to a human or animal subject. In an example, the kit, DNA(s), first phage, helper phage or composition is comprised by an oral medicament formulation. In an example, the kit, DNA(s), first phage, helper phage or composition is comprised by an intranasal or ocular medicament formulation. In an example, the kit, DNA(s), first phage, helper phage or composition is comprised by a personal hygiene composition (eg, shampoo, soap or deodorant) or cosmetic formulation. In an example, the kit, DNA(s), first phage, helper phage or composition is comprised by a detergent formulation. In an example, the kit, DNA(s), first phage, helper phage or composition is comprised by a cleaning formulation, eg, for cleaning a medical or industrial device or apparatatus. In an example, the kit, DNA(s), first phage, helper phage or composition is comprised by foodstuff, foodstuff ingredient or foodstuff processing agent. In an example, the kit, DNA(s), first phage, helper phage or composition is comprised by beverage, beverage ingredient or beverage processing agent. In an example, the kit, DNA(s), first phage, helper phage or composition is comprised by a medical bandage, fabric, plaster or swab. In an example, the kit, DNA(s), first phage, helper phage or composition is comprised by a herbicide or pesticide. In an example, the kit, DNA(s), first phage, helper phage or composition is comprised by an insecticide.

**In** an example, the first phage is a is a *Corticoviridae, Cystoviridae, Inoviridae, Leviviridae, Microviridae, Myoviridae, Podoviridae, Siphoviridae,* or *Tectiviridae* virus. In an example, the helper phage is a is a *Corticoviridae, Cystoviridae, Inoviridae, Leviviridae, Microviridae, Myoviridae, Podoviridae, Siphoviridae,* or *Tectiviridae* virus. In an example, the helper phage is a filamentous M13, a *Noviridae,* a tailed phage (eg, a *Myoviridae. Siphoviridae* or *Podoviridae*), or a non-tailed phage (eg, a Tectiviridae).

In an example, both the first and helper phage are *Corticoviridae.* In an example, both the first and helper phage are *Cystoviridae.* In an example, both the first and helper phage are *Inoviridae.* In an example, both the first and helper phage are *Leviviridae.* In an example, both the first and helper phage are *Microviridae.* In an example, both the first and helper phage are *Podoviridae.* In an example, both the first and helper phage are *Siphoviridae.* In an example, both the first and helper phage are *Tectiviridae.*

**In** an example, the CRISPR/Cas component(s) are component(s) of a Type I CRISPR/Cas system. In an example, the CRISPR/Cas component(s) are component(s) of a Type II CRISPR/Cas system. In an example, the CRISPR/Cas component(s) are component(s) of a Type III CRISPR/Cas system. In an example, the CRISPR/Cas component(s) are component(s) of a Type IV CRISPR/Cas system. In an example, the CRISPR/Cas component(s) are component(s) of a Type V CRISPR/Cas system. In an example, the CRISPR/Cas component(s) comprise a Cas9-encoding nucleotide sequence (eg, *Spyogenes* Cas9, *S aureus* Cas9 or *S thermophilus* Cas9). In an example, the CRISPR/Cas component(s) comprise a Cas3-encoding nucleotide sequence (eg, *E coli* Cas3, C *difficile* Cas3 or Salmonella Cas3). In an example, the CRISPR/Cas component(s) comprise a Cpf-encoding nucleotide sequence. In an example, the CRISPR/Cas component(s) comprise a CasX-encoding nucleotide sequence. In an example, the CRISPR/Cas component(s) comprise a CasY-encoding nucleotide sequence.

**In** an example, the first DNA, first phage or vector encode a CRISPR/Cas component or protein of interest from a nucleotide sequence comprising a promoter that is operable in the target bacteria.

**In** an example, the host bacteria and/or target bacteria are *E coli.* In an example, the host bacteria and/or target bacteria are *C dificile* (eg, the vector is a shuttle vector operable in *E coli* and the host bacteria are *C dificile*). In an example, the host bacteria and/or target bacteria are *Streptococcus,* such as *S thermophilus* (eg, the vector is a shuttle vector operable in *E coli* and the host bacteria are *Streptococcus*). In an example, the host bacteria and/or target bacteria are *Pseudomonas,* such as *P aeruginosa* (eg, the vector is a shuttle vector operable in *E coli* and the host bacteria are P *aeruginosa).* In an example, the host bacteria and/or target bacteria are *Klebsiella* (eg, the vector is a shuttle vector operable in *E coli* and the host bacteria are *Klebsiella).* In an example, the host bacteria and/or target bacteria are *Salmonella, eg, S typhimurium* (eg, the vector is a shuttle vector operable in *E coli* and the host bacteria are *Salmonella).*

Optionally, host and/or target bacteria is a gram negative bacterium (eg, a spirilla or vibrio). Optionally, host and/or target bacteria is a gram positive bacterium. Optionally, host and/or target bacteria is a mycoplasma, chlamydiae, spirochete or mycobacterium. Optionally, host and/or target bacteria is *a Streptococcus (eg, pyogenes* or *thermophilus).* Optionally, host and/or target bacteria is *a Staphylococcus (eg, aureus,* eg, MRSA). Optionally, host and/or target bacteria is an *E. coli (eg,* O157: H7) host, eg, wherein the Cas is encoded by the vecor or an endogenous host Cas nuclease activity is de-repressed. Optionally, host and/or target bacteria is a *Pseudomonas* (eg, aeruginosa). Optionally, host and/or target bacteria is a *Vibro (eg, cholerae* (eg, 0139) or *vulnificus).* Optionally, host and/or target bacteria is a *Neisseria (eg, gonnorrhoeae* or *meningitidis).* Optionally, host and/or target bacteria is a *Bordetella (eg, pertussis).* Optionally, host and/or target bacteria is a *Haemophilus (eg, influenzae).* Optionally, host and/or target bacteria is a *Shigella (eg, dysenteriae).* Optionally, host and/or target bacteria is *a Brucella (eg, abortus).* Optionally, host and/or target bacteria is a *Francisella* host. Optionally, host and/or target bacteria is *a Xanthomonas* host. Optionally, host and/or target bacteria is a *Agrobacterium* host. Optionally, host and/or target bacteria is a *Erwinia* host. Optionally, host and/or target bacteria is *a Legionella* (eg, *pneumophila).* Optionally, host and/or target bacteria is a *Listeria (eg, monocytogenes).* Optionally, host and/or target bacteria is a *Campylobacter (eg, jejuni).* Optionally, host and/or target bacteria is a *Yersinia (eg, pestis).* Optionally, host and/or target bacteria is a *Borelia (eg, burgdorferi).* Optionally, host and/or target bacteria is *a Helicobacter (eg, pylori).* Optionally, host and/or target bacteria is a *Clostridium (eg, dificile* or *botulinum).* Optionally, host and/or target bacteria is a *Erlichia (eg, chaffeensis*). Optionally, host and/or target bacteria is a *Salmonella (eg, typhi* or *enterica,* eg, serotype typhimurium, eg, DT 104). Optionally, host and/or target bacteria is a *Chlamydia (eg, pneumoniae).* Optionally, host and/or target bacteria is a *Parachlamydia* host. Optionally, host and/or target bacteria is a *Corynebacterium (eg, amycolatum*). Optionally, host and/or target bacteria is a *Klebsiella (eg, pneumoniae).* Optionally, host and/or target bacteria is an *Enterococcus* (eg, *faecalis* or *faecim,* eg, linezolid-resistant). Optionally, host and/or target bacteria is an *Acinetobacter (eg, baumannii, eg,* multiple drug resistant).
Further examples of target cells and targeting of antibiotic resistance in such cells are as follows:-
In an example, the target bacteria are Staphylococcus *aureus* cells, eg, resistant to an antibiotic selected from methicillin, vancomycin, linezolid, daptomycin, quinupristin, dalfopristin and teicoplanin.

In an example, the target bacteria are *Pseudomonas aeuroginosa* cells, eg, resistant to an antibiotic selected from cephalosporins (eg, ceftazidime), carbapenems (eg, imipenem or meropenem), fluoroquinolones, aminoglycosides (eg, gentamicin or tobramycin) and colistin.

In an example, the target bacteria are *Klebsiella* (eg, pneumoniae) cells, eg, resistant to carbapenem.

In an example, the target bacteria are *Streptoccocus* (eg, thermophilus, pneumoniae or pyogenes) cells, eg, resistant to an antibiotic selected from erythromycin, clindamycin, beta-lactam, macrolide, amoxicillin, azithromycin and penicillin.

In an example, the target bacteria are *Salmonella* (eg, serotype Typhi) cells, eg, resistant to an antibiotic selected from ceftriaxone, azithromycin and ciprofloxacin.

In an example, the target bacteria are *Shigella* cells, eg, resistant to an antibiotic selected from ciprofloxacin and azithromycin.

In an example, the target bacteria are mycobacterium tuberculosis cells, eg, resistant to an antibiotic selected from Resistance to isoniazid (INH), rifampicin (RMP), fluoroquinolone, amikacin, kanamycin and capreomycin and azithromycin.

In an example, the target bacteria are *Enterococcus* cells, eg, resistant to vancomycin.

In an example, the target bacteria are *Enterobacteriaceae* cells, eg, resistant to an antibiotic selected from a cephalosporin and carbapenem.

In an example, the target bacteria are E. *coli* cells, eg, resistant to an antibiotic selected from trimethoprim, itrofurantoin, cefalexin and amoxicillin.

In an example, the target bacteria are *Clostridium* (eg, dificile) cells, eg, resistant to an antibiotic selected from fluoroquinolone antibiotic and carbapenem.

In an example, the target bacteria are *Neisseria gonnorrhoea* cells, eg, resistant to an antibiotic selected from cefixime (eg, an oral cephalosporin), ceftriaxone (an injectable cephalosporin), azithromycin and tetracycline.

In an example, the target bacteria are *Acinetoebacter baumannii* cells, eg, resistant to an antibiotic selected from beta-lactam, meropenem and a carbapenem.

In an example, the target bacteria are *Campylobacter* cells, eg, resistant to an antibiotic selected from ciprofloxacin and azithromycin.

In an example, the target cell(s) produce Beta (β)-lactamase.

In an example, the target cell(s) are bacterial cells that are resistant to an antibiotic recited in any one of paragraphs [00182] to [00195].

### Mobile Genetic Elements, Genomic Islands, Pathogenicity Islands etc.

Genetic variation of bacteria and archaea can be achieved through mutations, rearrangements and horizontal gene transfers and recombinations. Increasing genome sequence data have demonstrated that, besides the core genes encoding house-keeping functions such as essential metabolic activities, information processing, and bacterial structural and regulatory components, a vast number of accessory genes encoding antimicrobial resistance, toxins, and enzymes that contribute to adaptation and survival under certain environmental conditions are acquired by horizontal gene transfer of mobile genetic elements (MGEs). Mobile genetic elements are a heterogeneous group of molecules that include plasmids, bacteriophages, genomic islands, chromosomal cassettes, pathogenicity islands, and integrative and conjugative elements. Genomic islands are relatively large segments of DNA ranging from 10 to 200 kb often integrated into tRNA gene clusters flanked by 16-20 bp direct repeats. They are recognized as discrete DNA segments acquired by horizontal gene transfer since they can differ from the rest of the chromosome in terms of GC content (%G+C) and codon usage.

Pathogenicity islands (PTIs) are a subset of horizontally transferred genetic elements known as genomic islands. There exists a particular family of highly mobile PTIs in *Staphylococcus aureus* that are induced to excise and replicate by certain resident prophages. These PTIs are packaged into small headed phage-like particles and are transferred at frequencies commensurate with the plaque-forming titer of the phage. This process is referred to as the SaPI excision replication-packaging (ERP) cycle, and the high-frequency SaPI transfer is referred to as SaPI-specific transfer (SPST) to distinguish it from classical generalized transduction (CGT). The SaPIs have a highly conserved genetic organization that parallels that of bacteriophages and clearly distinguishes them from all other horizontally acquired genomic islands. The SaPI1-encoded and SaPIbov2-encoded integrases are used for both excision and integration of the corresponding elements, and it is assumed that the same is true for the other SaPIs. Phage 80α can induce several different SaPIs, including SaPI1, SaPI2, and SaPIbov1, whereas φ11 can induce SaPIbov1 but neither of the other two SaPIs.

Reference is made to "Staphylococcal pathogenicity island DNA packaging system involving cos-site packaging and phage-encoded HNH endonucleases", Quiles-Puchalt et al, PNAS April 22, 2014. 111 (16) 6016-6021. Staphylococcal pathogenicity islands (SaPIs) are highly mobile and carry and disseminate superantigen and other virulence genes. It was reported that SaPIs hijack the packaging machinery of the phages they victimise, using two unrelated and complementary mechanisms. Phage packaging starts with the recognition in the phage DNA of a specific sequence, termed *"pac"* or *"cos"* depending on the phage type. The SaPI strategies involve carriage of the helper phage *pac-* or cos-like sequences in the SaPI genome, which ensures SaPI packaging in full-sized phage particles, depending on the helper phage machinery. These strategies interfere with phage reproduction, which ultimately is a critical advantage for the bacterial population by reducing the number of phage particles.

Staphylococcal pathogenicity islands (SaPIs) are the prototypical members of a widespread family of chromosomally located mobile genetic elements that contribute substantially to intra- and interspecies gene transfer, host adaptation, and virulence. The key feature of their mobility is the induction of SaPI excision and replication by certain helper phages and their efficient encapsidation into phage-like infectious particles. Most SaPIs use the headful packaging mechanism and encode small terminase subunit (TerS) homologs that recognize the SaPI-specific *pac* site and determine SaPI packaging specificity. Several of the known SaPIs do not encode a recognizable TerS homolog but are nevertheless packaged efficiently by helper phages and transferred at high frequencies. Quiles-Puchalt *et al* report that one of the non-terS-coding SaPIs, SaPIbov5, and found that it uses two different, undescribed packaging strategies. SaPIbov5 is packaged in full-sized phage-like particles either by typical pac-type helper phages, or by cos-type phages-i.e., it has both *pac* and cossites and uses the two different phage-coded TerSs. This is an example of SaPI packaging by a *cos* phage, and in this, it resembles the P4 plasmid of *Escherichia coli. Cos*-site packaging in *Staphylococcus aureus* is additionally unique in that it requires the HNH nuclease, carried only by *cos* phages, in addition to the large terminase subunit, for cos-site cleavage and melting.

Characterization of several of the phage-inducible SaPIs and their helper phages has established that the *pac* (or headful) mechanism is used for encapsidation. In keeping with this concept, some SaPIs encode a homolog of TerS, which complexes with the phage-coded large terminase subunit TerL to enable packaging of the SaPI DNA in infectious particles composed of phage proteins. These also contain a morphogenesis *(cpm)* module that causes the formation of small capsids commensurate with the small SaPI genomes. Among the SaPI sequences first characterized, there were several that did not include either a TerS homolog or a *cpm* homolog, and the same is true of several subsequently identified SaPIs from bovine sources and for many phage-inducible chromosomal islands from other species. It was assumed, for these several islands, either that they were defective derivatives of elements that originally possessed these genes, or that *terS* and *cpm* genes were present but not recognized by homology.

Quiles-Puchalt *et al* observed that an important feature of ϕSLT/SaPIbov5 packaging is the requirement for an HNH nuclease, which is encoded next to the ϕSLT terminase module. Proteins carrying HNH domains are widespread in nature, being present in organisms of all kingdoms. The HNH motif is a degenerate small nucleic acid-binding and cleavage module of about 30-40 aa residues and is bound by a single divalent metal ion. The HNH motif has been found in a variety of enzymes playing important roles in many different cellular processes, including bacterial killing; DNA repair, replication, and recombination; and processes related to RNA. HNH endonucleases are present in a number of cos-site bacteriophages of Gram-positive and -negative bacteria, always adjacent to the genes encoding the terminases and other morphogenetic proteins. Quiles-Puchalt *et al* have demonstrated that the HNH nucleases encoded by ϕ12 and the closely related ϕSLT have nonspecific nuclease activity and are required for the packaging of these phages and of SaPIbov5. Quiles-Puchalt *et al* have shown that HNH and TerL are jointly required for cos-site cleavage. Quiles-Puchalt *et al* have also observed that only *cos* phages of Gram-negative as well as of Gram-positive bacteria encode HNH nucleases, consistent with a special requirement for cos-site cleavage as opposed to *pac-site* cleavage, which generates flush-ended products. The demonstration that HNH nuclease activity is required for some but not other *cos* phages suggests that there is a difference between the TerL proteins of the two types of phages-one able to cut both strands and the other needing a second protein to enable the generation of a double-stranded cut.

The phage and compositions described herein are optionally for an industrial or domestic use, or is used in a method for such use. For example, it is for or used in agriculture, oil or petroleum industry, food or drink industry, clothing industry, packaging industry, electronics industry, computer industry, environmental industry, chemical industry, aeorspace industry, automotive industry, biotechnology industry, medical industry, healthcare industry, dentistry industry, energy industry, consumer products industry, pharmaceutical industry, mining industry, cleaning industry, forestry industry, fishing industry, leisure industry, recycling industry, cosmetics industry, plastics industry, pulp or paper industry, textile industry, clothing industry, leather or suede or animal hide industry, tobacco industry or steel industry.

The phage and compositions described herein are optionally for use in an industry or the environment is an industrial environment, wherein the industry is an industry of a field selected from the group consisting of the medical and healthcare; pharmaceutical; human food; animal food; plant fertilizers; beverage; dairy; meat processing; agriculture; livestock farming; poultry farming; fish and shellfish farming; veterinary; oil; gas; petrochemical; water treatment; sewage treatment; packaging; electronics and computer; personal healthcare and toiletries; cosmetics; dental; non-medical dental; ophthalmic; non-medical ophthalmic; mineral mining and processing; metals mining and processing; quarrying; aviation; automotive; rail; shipping; space; environmental; soil treatment; pulp and paper; clothing manufacture; dyes; printing; adhesives; air treatment; solvents; biodefence; vitamin supplements; cold storage; fibre retting and production; biotechnology; chemical; industrial cleaning products; domestic cleaning products; soaps and detergents; consumer products; forestry; fishing; leisure; recycling; plastics; hide, leather and suede; waste management; funeral and undertaking; fuel; building; energy; steel; and tobacco industry fields.

In an example, the first DNA, first phage or vector comprises a CRISPR array that targets target bacteria, wherein the array comprises one, or two or more spacers (eg, 2, 3, 4, 5, 6, 7, 8, 9, 10, 20, 30, 40, 50 or more spacers) for targeting the genome of target bacteria.

In an example, the target bacteria are comprised by an environment as follows. In an example, the environment is a microbiome of a human, eg, the oral cavity microbiome or gut microbiome or the bloodstream. In an example, the environment is not an environment in or on a human. In an example, the environment is not an environment in or on a non-human animal. In an embodiment, the environment is an air environment. In an embodiment, the environment is an agricultural environment. In an embodiment, the environment is an oil or petroleum recovery environment, eg, an oil or petroleum field or well. In an example, the environment is an environment in or on a foodstuff or beverage for human or non-human animal consumption.

In an example, the environment is a human or animal microbiome (eg, gut, vaginal, scalp, armpit, skin or oral cavity microbiome). In an example, the target bacteria are comprised by a human or animal microbiome (eg, gut, vaginal, scalp, armpit, skin or oral cavity microbiome).

In an example, the DNAs, phage or compositions described herein are administered intranasally, topically or orally to a human or non-human animal, or is for such administration. The skilled person aiming to treat a microbiome of the human or animal will be able to determine the best route of administration, depending upon the microbiome of interest. For example, when the microbiome is a gut microbiome, administration can be intranasally or orally. When the microbiome is a scalp or armpit microbiome, administration can be topically. When the microbiome is in the mouth or throat, the administration can be orally.

In any use or method herein, in an embodiment the first phage are contacted with the target bacteria at a multiplicity of infection (MOI) of at least 0.5, 1, 2, 3, 4, 5, 6, 7, 8, 9, 10, 15, 20, 25, 30, 35, 40, 45, 50, 55, 60, 65, 70, 75, 80, 85, 90, 95, 100, 110, 120, 130, 140, 150, 160, 170, 180, 190, 200, 300, 400, 500, 600 or 700. For example, the MOI is from 20 to 200, from 20 to 100, fro 50 to 200, from 50 to 100, from 75 to 150, 100 or about 100, or 200 or about 200. In an example, this may be determined by obtaining a sample of the microbiome containing the target bacteria (eg, a sample of a waterway or gut microbiome of a subject) and determining the number of CFU/ml or mg in the sample and using this to titrate the phage dose at the desired MOI to be exposed to the microbiome or administered to the environment or subject to be treated.

In an example, the environment is harboured by a beverage or water (eg, a waterway or drinking water for human consumption) or soil. The water is optionally in a heating, cooling or industrial system, or in a drinking water storage container.

In an example, the host and/or target bacteraia are *Firmicutes* selected from *Anaerotruncus, Acetanaerobacterium, Acetitomaculum, Acetivibrio, Anaerococcus, Anaerofilum, Anaerosinus, Anaerostipes, Anaerovorax, Butyrivibrio, Clostridium, Capracoccus, Dehalobacter, Dialister, Dorea, Enterococcus, Ethanoligenens, Faecalibacterium, Fusobacterium, Gracilibacter, Guggenheimella, Hespellia, Lachnobacterium, Lachnospira, Lactobacillus, Leuconostoc, Megamonas, Moryella, Mitsuokella, Oribacterium, Oxobacter, Papillibacter, Proprionispira,Pseudobutyrivibrio, Pseudoramibacter, Roseburia, Ruminococcus, Sarcina, Seinonella, Shuttleworthia, Sporobacter, Sporobacterium, Streptococcus, Subdoligranulum, Syntrophococcus, Thermobacillus, Turibacter* and *Weisella.*

In an example, the kit, DNA(s), first phage, helper phage, composition, use or method is for reducing pathogenic infections or for re-balancing gut or oral microbiota eg, for treating or preventing obesity or disease in a human or animal. For example, the first phage, helper phage, composition, use or method is for knocking-down *Clostridium dificile* or *E coli* bacteria in a gut microbiota of a human or animal.

In an example, the packaging signal, NPF and/or HPF consists or comprises SEQ ID NO: 2 or a structural or functional homologue thereof.

In an example, the packaging signal, NPF and/or HPF consists or comprises SEQ ID NO: 2 or a nucleotide sequence that is at least 70, 75, 80, 85, 90, 91, 92, 93, 94, 95, 96, 97, 98 or 99% identical thereto.

In an example, the disease or condition is a cancer, inflammatory or autoimmune disease or condition, eg, obesity, diabetes IBD, a GI tract condition or an oral cavity condition.

Optionally, the environment is comprised by, or the target bacteria are comprised by, a gut microbiota, skin microbiota, oral cavity microbiota, throat microbiota, hair microbiota, armpit microbiota, vaginal microbiota, rectal microbiota, anal microbiota, ocular microbiota, nasal microbiota, tongue microbiota, lung microbiota, liver microbiota, kidney microbiota, genital microbiota, penile microbiota, scrotal microbiota, mammary gland microbiota, ear microbiota, urethra microbiota, labial microbiota, organ microbiota or dental microbiota. Optionally, the environment is comprised by, or the target bacteria are comprised by, a plant (eg, a tobacco, crop plant, fruit plant, vegetable plant or tobacco, eg on the surface of a plant or contained in a plant) or by an environment (eg, soil or water or a waterway or acqueous liquid).

Optionally, the disease or condition of a human or animal subject is selected from

| | |
|---|---|
| (a) | A neurodegenerative disease or condition; |
| (b) | A brain disease or condition; |
| (c) | A CNS disease or condition; |
| (d) | Memory loss or impairment; |
| (e) | A heart or cardiovascular disease or condition, eg, heart attack, stroke or atrial fibrillation; |
| (f) | A liver disease or condition; |
| (g) | A kidney disease or condition, eg, chronic kidney disease (CKD); |
| (h) | A pancreas disease or condition; |
| (i) | A lung disease or condition, eg, cystic fibrosis or COPD; |
| (j) | A gastrointestinal disease or condition; |
| (k) | A throat or oral cavity disease or condition; |
| (l) | An ocular disease or condition; |
| (m) | A genital disease or condition, eg, a vaginal, labial, penile or scrotal disease or condition; |
| (n) | A sexually-transmissible disease or condition, eg, gonorrhea, HIV infection, syphilis or Chlamydia infection; |
| (o) | An ear disease or condition; |
| (p) | A skin disease or condition; |
| (q) | A heart disease or condition; |
| (r) | A nasal disease or condition |
| (s) | A haematological disease or condition, eg, anaemia, eg, anaemia of chronic disease or cancer; |
| (t) | A viral infection; |
| (u) | A pathogenic bacterial infection; |
| (v) | A cancer; |
| (w) | An autoimmune disease or condition, eg, SLE; |
| (x) | An inflammatory disease or condition, eg, rheumatoid arthritis, psoriasis, eczema, asthma, ulcerative colitis, colitis, Crohn's disease or IBD; |
| (y) | Autism; |
| (z) | ADHD; |
| (aa) | Bipolar disorder; |
| (bb) | ALS [Amyotrophic Lateral Sclerosis]; |
| (cc) | Osteoarthritis; |
| (dd) | A congenital or development defect or condition; |
| (ee) | Miscarriage; |
| (ff) | A blood clotting condition; |
| (gg) | Bronchitis; |
| (hh) | Dry or wet AMD; |
| (ii) | Neovascularisation (eg, of a tumour or in the eye); |
| (jj) | Common cold; |
| (kk) | Epilepsy; |
| (ll) | Fibrosis, eg, liver or lung fibrosis; |
| (mm) | A fungal disease or condition, eg, thrush; |
| (nn) | A metabolic disease or condition, eg, obesity, anorexia, diabetes, Type I or Type II diabetes. |
| (oo) | Ulcer(s), eg, gastric ulceration or skin ulceration; |
| (pp) | Dry skin; |
| (qq) | Sjogren's syndrome; |
| (rr) | Cytokine storm; |
| (ss) | Deafness, hearing loss or impairment; |
| (tt) | Slow or fast metabolism (ie, slower or faster than average for the weight, sex and age of the subject); |
| (uu) | Conception disorder, eg, infertility or low fertility; |
| (vv) | Jaundice; |
| (ww) | Skin rash; |
| (xx) | Kawasaki Disease; |
| (yy) | Lyme Disease; |
| (zz) | An allergy, eg, a nut, grass, pollen, dust mite, cat or dog fur or dander allergy; |
| (aaa) | Malaria, typhoid fever, tuberculosis or cholera; |
| (bbb) | Depression; |
| (ccc) | Mental retardation; |
| (ddd) | Microcephaly; |
| (eee) | Malnutrition; |
| (fff) | Conjunctivitis; |
| (ggg) | Pneumonia; |
| (hhh) | Pulmonary embolism; |
| (iii) | Pulmonary hypertension; |
| (jjj) | A bone disorder; |
| (kkk) | Sepsis or septic shock; |
| (lll) | Sinusitus; |
| (mmm) | Stress (eg, occupational stress); |
| (nnn) | Thalassaemia, anaemia, von Willebrand Disease, or haemophilia; |
| (ooo) | Shingles or cold sore; |
| (ppp) | Menstruation; |
| (qqq) | Low sperm count. |

### NEURODEGENERATIVE OR CNS DISEASES OR CONDITIONS FOR TREATMENT OR PREVENTION

In an example, the neurodegenerative or CNS disease or condition is selected from the group consisting of Alzheimer disease , geriopsychosis, Down syndrome, Parkinson's disease, Creutzfeldt-jakob disease, diabetic neuropathy, Parkinson syndrome, Huntington's disease, Machado-Joseph disease, amyotrophic lateral sclerosis, diabetic neuropathy, and Creutzfeldt Creutzfeldt- Jakob disease. For example, the disease is Alzheimer disease. For example, the disease is Parkinson syndrome.

In an example, wherein the method disclosure herein is practised on a human or animal subject for treating a CNS or neurodegenerative disease or condition, the method causes downregulation of Treg cells in the subject, thereby promoting entry of systemic monocyte-derived macrophages and/or Treg cells across the choroid plexus into the brain of the subject, whereby the disease or condition (eg, Alzheimer's disease) is treated, prevented or progression thereof is reduced. In an embodiment the method causes an increase of IFN-gamma in the CNS system (eg, in the brain and/or CSF) of the subject. In an example, the method restores nerve fibre and//or reduces the progression of nerve fibre damage. In an example, the method restores nerve myelin and//or reduces the progression of nerve myelin damage. In an example, the method disclosed herein treats or prevents a disease or condition disclosed in WO2015136541 and/or the method can be used with any method disclosed in WO2015136541 (eg, for providing disclosure of such methods, diseases, conditions and potential therapeutic agents that can be administered to the subject for effecting treatement and/or prevention of CNS and neurodegenerative diseases and conditions, eg, agents such as immune checkpoint inhibitors, eg, anti-PD-1, anti-PD-L1, anti-TIM3 or other antibodies disclosed therein).

### CANCERS FOR TREATMENT OR PREVENTION BY THE METHOD

Cancers that may be treated include tumours that are not vascularized, or not substantially vascularized, as well as vascularized tumours. The cancers may comprise non-solid tumours (such as haematological tumours, for example, leukaemias and lymphomas) or may comprise solid tumours. Types of cancers to be treated as disclosed herein include, but are not limited to, carcinoma, blastoma, and sarcoma, and certain leukaemia or lymphoid malignancies, benign and malignant tumours, and malignancies e.g., sarcomas, carcinomas, and melanomas. Adult tumours/cancers and paediatric tumours/cancers are also included.

Haematologic cancers are cancers of the blood or bone marrow. Examples of haematological (or haematogenous) cancers include leukaemias, including acute leukaemias (such as acute lymphocytic leukaemia, acute myelocytic leukaemia, acute myelogenous leukaemia and myeloblasts, promyeiocytic, myelomonocytic, monocytic and erythroleukaemia), chronic leukaemias (such as chronic myelocytic (granulocytic) leukaemia, chronic myelogenous leukaemia, and chronic lymphocytic leukaemia), polycythemia vera, lymphoma, Hodgkin's disease, non-Hodgkin's lymphoma (indolent and high grade forms), multiple myeloma, Waldenstrom's macroglobulinemia, heavy chain disease, myeiodysplastic syndrome, hairy cell leukaemia and myelodysplasia.

Solid tumours are abnormal masses of tissue that usually do not contain cysts or liquid areas. Solid tumours can be benign or malignant. Different types of solid tumours are named for the type of cells that form them (such as sarcomas, carcinomas, and lymphomas). Examples of solid tumours, such as sarcomas and carcinomas, include fibrosarcoma, myxosarcoma, liposarcoma, chondrosarcoma, osteosarcoma, and other sarcomas, synovioma, mesothelioma, Ewing's tumour, leiomyosarcoma, rhabdomyosarcoma, colon carcinoma, lymphoid malignancy, pancreatic cancer, breast cancer, lung cancers, ovarian cancer, prostate cancer, hepatocellular carcinoma, squamous eel! carcinoma, basal cell carcinoma, adenocarcinoma, sweat gland carcinoma, medullary thyroid carcinoma, papillary thyroid carcinoma, pheochromocytomas sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, Wilms' tumour, cervical cancer, testicular tumour, seminoma, bladder carcinoma, melanoma, and CNS tumours (such as a glioma (such as brainstem glioma and mixed gliomas), glioblastoma (also known as glioblastoma multiforme) astrocytoma, CNS lymphoma, germinoma, medu!loblastoma, Schwannoma craniopharyogioma, ependymoma, pineaioma, hemangioblastoma, acoustic neuroma, oligodendroglioma, menangioma, neuroblastoma, retinoblastoma and brain metastases).

### AUTOIMMUNE DISEASES FOR TREATMENT OR PREVENTION BY THE METHOD

1. Acute Disseminated Encephalomyelitis (ADEM)
2. Acute necrotizing hemorrhagic leukoencephalitis
3. Addison's disease
4. Agammaglobulinemia
5. Alopecia areata
6. Amyloidosis
7. Ankylosing spondylitis
8. Anti-GBM/Anti-TBM nephritis
9. Antiphospholipid syndrome (APS)
10. Autoimmune angioedema
11. Autoimmune aplastic anemia
12. Autoimmune dysautonomia
13. Autoimmune hepatitis
14. Autoimmune hyperlipidemia
15. Autoimmune immunodeficiency
16. Autoimmune inner ear disease (AIED)
17. Autoimmune myocarditis
18. Autoimmune oophoritis
19. Autoimmune pancreatitis
20. Autoimmune retinopathy
21. Autoimmune thrombocytopenic purpura (ATP)
22. Autoimmune thyroid disease
23. Autoimmune urticaria
24. Axonal & neuronal neuropathies
25. Balo disease
26. Behcet's disease
27. Bullous pemphigoid
28. Cardiomyopathy
29. Castleman disease
30. Celiac disease
31. Chagas disease
32. Chronic fatigue syndrome
33. Chronic inflammatory demyelinating polyneuropathy (CIDP)
34. Chronic recurrent multifocal ostomyelitis (CRMO)
35. Churg-Strauss syndrome
36. Cicatricial pemphigoid/benign mucosal pemphigoid
37. Crohn's disease
38. Cogans syndrome
39. Cold agglutinin disease
40. Congenital heart block
41. Coxsackie myocarditis
42. CREST disease
43. Essential mixed cryoglobulinemia
44. Demyelinating neuropathies
45. Dermatitis herpetiformis
46. Dermatomyositis
47. Devic's disease (neuromyelitis optica)
48. Discoid lupus
49. Dressler's syndrome
50. Endometriosis
51. Eosinophilic esophagitis
52. Eosinophilic fasciitis
53. Erythema nodosum
54. Experimental allergic encephalomyelitis
55. Evans syndrome
56. Fibromyalgia
57. Fibrosing alveolitis
58. Giant cell arteritis (temporal arteritis)
59. Giant cell myocarditis
60. Glomerulonephritis
61. Goodpasture's syndrome
62. Granulomatosis with Polyangiitis (GPA) (formerly called Wegener's Granulomatosis)
63. Graves' disease
64. Guillain-Barre syndrome
65. Hashimoto's encephalitis
66. Hashimoto's thyroiditis
67. Hemolytic anemia
68. Henoch-Schonlein purpura
69. Herpes gestationis
70. Hypogammaglobulinemia
71. Idiopathic thrombocytopenic purpura (ITP)
72. IgA nephropathy
73. IgG4-related sclerosing disease
74. Immunoregulatory lipoproteins
75. Inclusion body myositis
76. Interstitial cystitis
77. Juvenile arthritis
78. Juvenile diabetes (Type 1 diabetes)
79. Juvenile myositis
80. Kawasaki syndrome
81. Lambert-Eaton syndrome
82. Leukocytoclastic vasculitis
83. Lichen planus
84. Lichen sclerosus
85. Ligneous conjunctivitis
86. Linear IgA disease (LAD)
87. Lupus (SLE)
88. Lyme disease, chronic
89. Meniere's disease
90. Microscopic polyangiitis
91. Mixed connective tissue disease (MCTD)
92. Mooren's ulcer
93. Mucha-Habermann disease
94. Multiple sclerosis
95. Myasthenia gravis
96. Myositis
97. Narcolepsy
98. Neuromyelitis optica (Devic's)
99. Neutropenia
100. Ocular cicatricial pemphigoid
101. Optic neuritis
102. Palindromic rheumatism
103. PANDAS (Pediatric Autoimmune Neuropsychiatric Disorders Associated with Streptococcus)
104. Paraneoplastic cerebellar degeneration
105. Paroxysmal nocturnal hemoglobinuria (PNH)
106. Parry Romberg syndrome
107. Parsonnage-Turner syndrome
108. Pars planitis (peripheral uveitis)
109. Pemphigus
110. Peripheral neuropathy
111. Perivenous encephalomyelitis
112. Pernicious anemia
113. POEMS syndrome
114. Polyarteritis nodosa
115. Type I, II, & III autoimmune polyglandular syndromes
116. Polymyalgia rheumatica
117. Polymyositis
118. Postmyocardial infarction syndrome
119. Postpericardiotomy syndrome
120. Progesterone dermatitis
121. Primary biliary cirrhosis
122. Primary sclerosing cholangitis
123. Psoriasis
124. Psoriatic arthritis
125. Idiopathic pulmonary fibrosis
126. Pyodenna gangrenosum
127. Pure red cell aplasia
128. Raynauds phenomenon
129. Reactive Arthritis
130. Reflex sympathetic dystrophy
131. Reiter's syndrome
132. Relapsing polychondritis
133. Restless legs syndrome
134. Retroperitoneal fibrosis
135. Rheumatic fever
136. Rheumatoid arthritis
137. Sarcoidosis
138. Schmidt syndrome
139. Scleritis
140. Scleroderma
141. Sjogren's syndrome
142. Sperm & testicular autoimmunity
143. Stiff person syndrome
144. Subacute bacterial endocarditis (SBE)
145. Susac's syndrome
146. Sympathetic ophthalmia
147. Takayasu's arteritis
148. Temporal arteritis/Giant cell arteritis
149. Thrombocytopenic purpura (TTP)
150. Tolosa-Hunt syndrome
151. Transverse myelitis
152. Type 1 diabetes
153. Ulcerative colitis
154. Undifferentiated connective tissue disease (UCTD)
155. Uveitis
156. Vasculitis
157. Vesiculobullous dermatosis
158. Vitiligo
159. Wegener's granulomatosis (now termed Granulomatosis with Polyangiitis (GPA).

### INFLAMMATORY DISEASES FOR TREATMENT OR PREVENTION BY THE METHOD

1. Alzheimer
2. ankylosing spondylitis
3. arthritis (osteoarthritis, rheumatoid arthritis (RA), psoriatic arthritis)
4. asthma
5. atherosclerosis
6. Crohn's disease
7. colitis
8. dermatitis
9. diverticulitis
10. fibromyalgia
11. hepatitis
12. irritable bowel syndrome (IBS)
13. systemic lupus erythematous (SLE)
14. nephritis
15. Parkinson's disease
16. ulcerative colitis.
There is also provided the following (which may optionally be combined with any of the disclosure above):-
**In** a first aspect, there is provided an antibacterial composition comprising a population of first phage, wherein the first phage require helper phage for replication of first phage particles, wherein the helper phage are capable of packaging first phage nucleic acid to produce first phage particles, wherein the first phage are different from the helper phage and the helper phage are incapable themselves of producing helper phage particles, wherein the first phage are capable of infecting target bacteria and each first phage comprises antibacterial means for killing bacteria.

**In** an example, in the composition as described in the first aspect, at least 95% of phage particles comprised by the composition are first phage particles.

Optionally, the composition comprises helper phage. Optionally, the composition comprises no more than 1 helper phage particle per 1 x 10⁶ or more of phage particles. Optionally, the composition comprises no more than 1 helper phage particle per 1 x 10⁸ or more of phage particles. Optionally, the composition comprises no more than 1 helper phage particle per 1 x 10⁹ or more of phage particles. Optionally, the composition comprises no more than 1 helper phage particle per 1 x 10¹⁰ or more of phage particles.

**In** an example, in the composition as described in the first aspect, each first phage comprises one or more components of a CRISPR/Cas system, wherein the component(s) comprise a DNA sequence encoding a guide RNA (optionally a single guide RNA) or comprising a CRISPR array for producing guide RNA, wherein the guide RNA is capable of targeting the genome of target bacteria.

**In** an example, in the composition as described in the first aspect, each first phage genome is devoid of genes encoding phage proteins.

**In** a second aspect, there is provided a kit comprising
a) A first DNA; and
b) One or more second DNAs;
Wherein
(i) the DNAs together comprise all phage structural protein genes required to produce a packaged phage particle comprising a copy of the first DNA;
(ii) the first DNA comprises none or at least one, but not all, of the genes; and wherein the one or more second DNAs comprise the remainder of the genes;
(iii)the first DNA comprises a phage packaging signal for producing the packaged phage particle; and
(iv) the second DNA is devoid of a nucleotide sequence required for packaging the second DNA into phage particles;
(v) wherein the DNAs are operable when co-existing in a host bacterium for producing packaged phage (first phage) that comprise the first DNA, wherein the first phage require the second DNA for replication thereof to produce further first phage particles.

**In** an example, in the kit as described in the second aspect, the phage particle of (i) is capable of infecting a target bacterium, the phage comprising a nucleotide sequence of interest (NSI) that is capable of expressing a protein or RNA in the target bacterium, wherein the presence in the target bacterium of the NSI-encoded protein or RNA mediates target cell killing, or downregulation of target cell growth or propagation.

Optionally, the phage particle of (i) is capable of infecting a target bacterium, the phage comprising a nucleotide sequence of interest (NSI) that is capable of expressing a protein or RNA in the target bacterium, or wherein the NSI comprises a regulatory element that is operable in the target bacterium.

Optionally, the presence in the target bacterium of the NSI or its encoded protein or RNA mediates target cell killing, or downregulation of target cell growth or propagation, or mediates switching off of expression of one or more RNA or proteins encoded by the target cell genome, or downregulation thereof, or the presence in the target bacterium of the NSI or its encoded protein or RNA mediates upregulation of growth or propagation of the target cell, or mediates switching on of expression of one or more RNA or proteins encoded by the target cell genome, or upregulation thereofy.

Optionally, the NSI encodes a component of a CRISPR/Cas system that is toxic to the target bacterium, or the NSI comprises engineered antibacterial means for killing target bacteria.

In an example, in the kit as described in the second aspect, the packaged phage particle genome is devoid of genes encoding phage proteins.

In an example, in the kit as described in the second aspect, the first DNA comprises none of the structural protein genes.

In an example, in the kit as described in the second aspect, the second DNA is devoid of a phage packaging signal.

In an example, in the kit as described in the second aspect, each signal is a pac or cos sequence, or is a homologue thereof.

In an example, in the kit as described in the second aspect, each signal comprises SEQ ID NO: 2 or a sequence that is at least 70, 80, 90, 95, 96, 97, 98 or 99% identical thereto, or is a homologue from a different species.

In a third aspect, there is provided an isolated DNA, wherein the DNA is a first DNA as described in the second aspect.

In an example, in the kit or DNA as described in the second or third aspect, the first DNA is comprised by a vector (optionally, a plasmid, phagemid or shuttle vector).

In an example, in the kit or DNA as described in the second or third aspect, the second DNA is comprised by a vector (optionally a plasmid, phagemid or shuttle vector), helper phage (optionally a helper phagemid) or is integrated in the genome of a host bacterial cell.

In a fourth aspect, there is provided a host bacterial cell comprising the first and second DNAs as described in the second aspect; or comprising the DNA described in the third aspect.

In a fifth aspect, there is provided a method of producing a phage composition, the method comprising expressing in a cell of the fourth aspect the phage proteins, wherein packaged first phage particles are produced that comprise the first DNA, wherein the first phage require the second DNA for replication thereof to produce further first phage; and optionally separating an amount of first phage from cellular material wherein an amount of purified phage is obtained.

In an example, the purified phage are mixed with a pharmaceutically-acceptable excipient, carrier or diluent (eg, an aqueous liquid or water) to produce a pharmaceutical composition.

In an example any composition or kit described herein is in combination with a label or instructions for use to treat and/or prevent a disease or condition in a human; optionally wherein the label or instructions comprise a marketing authorisation number (eg, an FDA or EMA authorisation number); optionally wherein the kit comprises an injection pen or IV container that comprises the first DNA or first phage.

In an example, the method of the fifth aspect, comprises isolating the first phage particles.

In a sixth aspect, there is provided a composition (eg, antibacterial composition, eg, for medcial use) comprising a population of first phage particles obtainable by the method of the fifth aspect.

In an example, the first phage particles are obtained by the method.

In an example, any composition described herein comprises at least 1 x 10³ first phage per ml or mg, such as when the composition is comprised by a fluid (eg, a liquid) or solid. In an example, any composition of the invention comprises at least 1 x 10⁴ first phage per ml or mg. In an example, any composition of the invention comprises at least 1 x 10⁵ first phage per ml or mg. In an example, any composition of the invention comprises at least 1 x 10⁶ first phage per ml or mg. In an example, any composition of the invention comprises at least 1 x 10⁷ first phage per ml or mg. In an example, any composition of the invention comprises at least 1 x 10⁸ first phage per ml or mg. In an example, any composition of the invention comprises at least 1 x 10⁹ first phage per ml or mg. In an example, any composition of the invention comprises at least 1 x 10¹⁰ first phage per ml or mg. In an example, any composition of the invention comprises at least 1 x 10¹¹ first phage per ml or mg. In an example, any composition of the invention comprises at least 1 x 10¹² first phage per ml or mg. In an example, any composition of the invention comprises at least 1 x 10¹³ first phage per ml or mg. In an example, any composition of the invention comprises at least 1 x 10¹⁴ first phage per ml or mg.

In an example, any composition of the invention comprises up to 1 x 10¹⁴ first phage per ml or mg, such as when the composition is comprised by a fluid (eg, a liquid) or solid. In an example, any composition of the invention comprises up to 1 x 10¹³ first phage per ml or mg. In an example, any composition of the invention comprises up to 1 x 10¹² first phage per ml or mg. In an example, any composition of the invention comprises up to 1 x 10¹¹ first phage per ml or mg. In an example, any composition of the invention comprises up to 1 x 10¹⁰ first phage per ml or mg. In an example, any composition of the invention comprises up to 1 x 10⁹ first phage per ml or mg.

In an example, any composition of the invention comprises at least 1 x 10³ to 1 x 10¹⁰, 1 x 10¹¹, 1 x 10¹², 1 x 10¹³ or 1 x 10¹⁴ first phage per ml or mg, such as when the composition is comprised by a fluid (eg, a liquid) or solid. In an example, any composition of the invention comprises at least 1 x 10⁴ to 1 x 10¹⁰, 1 x 10¹¹, 1 x 10¹², 1 x 10¹³ or 1 x 10¹⁴ first phage per ml or mg. In an example, any composition of the invention comprises at least 1 x 10⁵ to 1 x 10¹⁰, 1 x 10¹¹, 1 x 10¹², 1 x 10¹³ or 1 x 10¹⁴ first phage per ml or mg. In an example, any composition of the invention comprises at least 1 x 10⁶ to 1 x 10¹⁰, 1 x 10¹¹, 1 x 10¹², 1 x 10¹³ or 1 x 10¹⁴ first phage per ml or mg. In an example, any composition of the invention comprises at least 1 x 10⁷ to 1 x 10¹⁰, 1 x 10¹¹, 1 x 10¹², 1 x 10¹³ or 1 x 10¹⁴ first phage per ml or mg. In an example, any composition of the invention comprises at least 1 x 10⁸ to 1 x 10¹⁰, 1 x 10¹¹, 1 x 10¹², 1 x 10¹³ or 1 x 10¹⁴ first phage per ml or mg. In an example, any composition of the invention comprises at least 1 x 10⁹ to 1 x 10¹⁰, 1 x 10¹¹, 1 x 10¹², 1 x 10¹³ or 1 x 10¹⁴ first phage per ml or mg. In an example, any composition of the invention comprises at least 1 x 10¹⁰ to 1 x 10¹⁰, 1 x 10¹¹, 1 x 10¹², 1 x 10¹³ or 1 x 10¹⁴ first phage per ml or mg. In an example, any composition of the invention comprises at least 1 x 10¹¹ to 1 x 10¹⁰, 1 x 10¹¹, 1 x 10¹², 1 x 10¹³ or 1 x 10¹⁴ first phage per ml or mg. In an example, any composition of the invention comprises at least 1 x 10¹² to 1 x 10¹⁰, 1 x 10¹¹, 1 x 10¹², 1 x 10¹³ or 1 x 10¹⁴ first phage per ml or mg. In an example, any composition of the invention comprises at least 1 x 10¹³ to 1 x 10¹⁰, 1 x 10¹¹, 1 x 10¹², 1 x 10¹³ or 1 x 10¹⁴ first phage per ml or mg. In an example, any composition of the invention comprises at least 1 x 10¹⁴ to 1 x 10¹⁰, 1 x 10¹¹, 1 x 10¹², 1 x 10¹³ or 1 x 10¹⁴ first phage per ml or mg.

In an example, the composition comprises one or more doses of the first phage for administration to a subject for medical use, eg, to treat or prevent a disease or condition in the subject. In an example, the composition comprises a single dose. In an example, the composition comprises (or comprises at least) 2, 3, 4, 5, 6, 7, 8, 9, 10, 11, 12, 13, 14, 15, 16, 17, 18, 19, 20, 21, 22, 23, 24, 25, 26, 27, 28, 29 or 30 doses. In an example, each dose is (or is at least) a 0.5, 1, 2, 3, 4, 5, 10, 20, 25, 30, 40, 50, 75, 100, 125, 200 or 250mg or ml dose comprising said phage (ie, the dose is said amount and comprises phage and an excipient, diluent or carrier for example).

In an example, the composition comprises one or more doses of the first phage for administration to a subject for non-medical use, eg, for agricultural use. In an example, the composition comprises a single dose. In an example, the composition comprises (or comprises at least) 2, 3, 4, 5, 6, 7, 8, 9, 10, 11, 12, 13, 14, 15, 16, 17, 18, 19, 20, 21, 22, 23, 24, 25, 26, 27, 28, 29 or 30 doses. In an example, each dose is (or is at least) a 0.5, 1, 2, 3, 4, 5, 10, 20, 25, 30, 40, 50, 75, 100, 125, 200, 250, 500, 750, 1000, 2000, 3000, 4000, 5000, 10000, 50000, 100000 mg or ml dose comprising said phage (ie, the dose is said amount and comprises phage and an excipient, diluent or carrier for example). The dose may be dissolved or diluted in a solvent (eg, an aqueous solvent or water) before use for contacting with target bacteria. In an example 1 imperial gallon comprises one dose of the first phage, eg, for agricultural use, such as crop spraying, or for animal or livestock use, such as use as a beverage.

In an example, in the method of the fifth aspect or in the composition of the sixth aspect, the second DNA is comprised by helper phage DNA and less than 5% of total phage particles comprised by the composition are helper phage particles.

In an example, in the method or composition as described in the fifth or sixth aspect, the second DNA is comprised by helper phage DNA and the composition comprises no more than 1 helper phage particle per 1 x 10⁶ or more of phage particles.

This has been demonstrated in Example 3 and shown to be efficacious for target bacteria killing in Example 4. In an embodiment, the composition comprises no more than 1 helper phage particle per 1 x 10⁷ or more of phage particles. In an embodiment, the composition comprises no more than 1 helper phage particle per 1 x 10⁸ or more of phage particles. In an embodiment, the composition comprises no more than 1 helper phage particle per 1 x 10⁹ or more of phage particles. In an embodiment, the composition comprises no more than 1 helper phage particle per 1 x 10¹⁰ or more of phage particles.

In an embodiment, the composition comprises no more than 1 helper phage particle per 1 x 10⁶ of phage particles. In an embodiment, the composition comprises no more than 1 helper phage particle per 1 x 10⁷ of phage particles. In an embodiment, the composition comprises no more than 1 helper phage particle per 1 x 10⁸ of phage particles. In an embodiment, the composition comprises no more than 1 helper phage particle per 1 x 10⁹ of phage particles.

In an embodiment, the composition comprises no more than 1 helper phage particle per 1 x 10⁶ to 1 x 10⁹ of phage particles. In an embodiment, the composition comprises no more than 1 helper phage particle per 1 x 10⁶ to 1 x 10⁸ of phage particles. In an embodiment, the composition comprises no more than 1 helper phage particle per 1 x 10⁶ to 1 x 10⁷ of phage particles. In an embodiment, the composition comprises no more than 1 helper phage particle per 1 x 10⁷ to 1 x 10⁹ of phage particles. In an embodiment, the composition comprises no more than 1 helper phage particle per 1 x 10⁷ to 1 x 10⁸ of phage particles.

In an example, the proportion of helper phage is determined as plaque forming units (PFU), eg, PFU/ml of the phage composition comprising said number of phage particles. In example, the proportion of non-helper phage (ie, first phage) is determined as number of transduced units of target bacteria (TFU), eg, TFU/ml of the phage composition. PFU is determined on lawns of a susceptible indicator bacterium while TFU is determined in a transduction assay in which a culture of susceptible indicator bacteria is infected with the phage composition ensuring surplus of indicator cells (i.e. at a low multiplicity of infection (MOI < 0.01) and number of transduced cells are determined by plating on selective plates.

Thus, the composition may comprise one or more helper phage particles. The level of helper particles is, however, extremely low. This is beneficial as the composition is relatively pure (and useful, for example, therefore as a medicament). It is also useful as the chances of the first phage being replicated is extremely low, providing the advantages of dosing control of phage, containment of phage (eg, in a human or animal body or an environment), and the lack of phage replication reduces the chances of acquiring undesirable genes (eg, antibiotic resistance genes) by the phage.

In an example, in the composition as described in the first aspect, in the method as described in the fifth aspect, or in the composition as described in the sixth aspect, each first phage particle comprises a nucleotide sequence of interest (NSI) that is capable of expressing a protein or RNA in a target bacterium, wherein the presence in the target bacterium of the NSI-encoded protein or RNA mediates target cell killing, or downregulation of target cell growth or propagation.

Optionally, the NSI encodes a component of a CRISPR/Cas system that is toxic to target cells.

Optionally, the component comprises (i) a DNA sequence encoding a guide RNA (eg, a single guide RNA) or comprising a CRISPR array for producing guide RNA, wherein the guide RNA is capable of targeting the genome of target bacteria; (ii) a Cas nuclease-encoding DNA sequence; and/or (iii) a DNA sequence encoding one or more components of Cascade.

Optionally, the NSI encodes a Cas nuclease and a guide RNA of the system (or wherein the NSI encodes a Cas nuclease and a CRISPR array for producing guide RNA), wherein the guide RNA is capable of targeting the genome of target bacteria, wherein the guide RNA is capable of guiding the Cas in target cells to mediate target cell killing, or downregulation of target cell growth or propagation.

Optionally, the NSI encodes a Cas9, and a tracrRNA and a CRISPR array for producing guide RNA.

In an example, in the method or composition as described in the first, fifth or sixth aspect, the first phage particles comprise no phage structural protein genes.

In an example, in the kit, DNA, method or composition as described in any previous aspect, the first DNA or first phage DNA is comprised by a high copy number plasmid.

Optionally, the first DNA or first phage DNA is comprised by a medium copy number plasmid.

The meaning of low, medium and high copy number ori and plasmids is known to the skilled addressee and these are terms of art. As is known by the skilled person, copy number denotes the average number of plasmid copies per cell. For example, a low copy number plasmid is a plasmid that exists in from 1 to 10 copies per bacterial cell in which the plasmid is harboured; a medium copy number plasmid exists in from 11 to 50 (eg, 11 to 40 or 20 to 30 or 40) copies per cell; and a high copy number is >50 (eg, up to 100, 200, 250, 300, 400, 500, 600 or 700) copies per cell. In an example, the plasmid or vector comprising first DNA is a medium copy number plasmid or vector. In an example, the plasmid or vector comprising first DNA is a high copy number plasmid or vector. An example of common ori and plasmids is shown in Table 7.

Optionally, the NSI comprises engineered antibacterial means for killing target bacteria.

Optionally, the antibacterial means comprises a nucleic acid encoding a guided nuclease, such as a Cas nuclease, TALEN, zinc finger nuclease or meganuclease

In an example, the composition as described in the first or sixth aspect is for administration to a human or animal subject for medical use.

**In** an example, the composition as described in the first or sixth aspect is for administration to a human or animal subject for treating an infection of target bacterial cells, wherein the first phage are capable of infecting and killing the target cells, optionally wherein the infection is a gut microbiome infection.

Optionally, the target cells are E coli cells the first DNA is comprised by a high copy number plasmid.

**In** an example, the gut microbiome is an upper GI tract microbiome. In an example the target cells are comprised by the upper GI tract of the subject. In an example, the first phage are delivered to the upper GI tract of the subject.

**In** an example, the gut microbiome is a stomach or small intestine microbiome. In an example the target cells are comprised by the stomach or small intestine of the subject. In an example, the first phage are delivered to the stomach or small intestine of the subject.

Optionally, the composition comprising engineered antibacterial means for killing target bacteria is for use in a contained method of treating a disease or condition of a human or animal subject, wherein the disease or condition is mediated by target bacteria and the target bacteria are comprised by the subject (optionally comprised by a gut microbiome), the method comprising administering the composition to the subject, whereby the target bacteria are exposed to the antibacterial means and killed and propagation of the first phage is contained.

**In** a seventh aspect, there is provided a method of treating an environment *ex vivo,* the method comprising exposing the environment to a composition comprising a population of first phage particles, wherein the composition is obtainable by the method as described in the fifth or sixth aspect, or the composition is as described in the first or sixth aspect, wherein the environment comprises target bacteria and the first phage infect and kill the target bacteria.

Optionally, the method is for containing the treatment in the environment.

**In** an example, the composition as described in the first or sixth aspect is for controlling the dosing of the first phage treatment in the subject; or the method as described in the seventh aspect is for controlling the dosing of the first phage treatment in the environment.

In an example, the composition as described in the first or sixth aspect is for reducing the risk of acquisition of foreign gene sequence(s) by the first phage in the subject; or the method as described in the seventh apsect for reducing the risk of acquisition of foreign gene sequence(s) by the first phage in the environment.

### EXAMPLES

### Example 1: Efficient phage CRISPR delivery vehicle production

### Background

We designed a strategy for efficient production of phage particles comprising components of a CRISPR/Cas system for killing target *E coli* Nissle strain bacteria. So our phage composition will consist of a lysate primarily containing CRISPR/Cas system components packaged in phage particles which will be devoid of phage protein-encoding sequences and which will have no or a very low proportion of helper phage. Also the strategy will work alternatively in less well characterised phage/bacterial strain combinations. Worked exemplification is provided in Examples 3 and 4.

### Outline of strategy for CRISPR/Cas component packaging in hitherto unknown phages

(a) Identify a high or medium copy number cloning/lshuttle vector (capable of cloning and propagation in a first *E coli* strain (cloning production strain) and then transfer to a second bacterial host strain of interest (target host strain)), the vector containing an *E coli* ori for replication in the *E coli* cloning production strain;
(b) Isolate temperate phage against the target host (second) bacterium;
(c) To produce the cloning production strain, identify or engineer a phage production strain of the host target bacteria (or other bacteria) that has an inactive CRISPR/Cas system (eg, a repressed Cas3 or other nuclease) and/or lacks the target protospacer found in the second strain, and which can be infected and lysogenized with the temperate phage; or repress or inactivate the system in the production strain;
(d) In that production strain make a lysogen using the temperate phage (helper phage) and test that it can be induced;
(e) Identify the packaging sequence (*pac* or *cos*) using PhageTerm (https://www.ncbi.nlm.nih.gov/pmc/articles/PMC5557969/) on whole genome sequenced phage;
(f) Delete the *pac*/*cos* packaging signal sequence in the helper phage in the production strain bacteria;
(g) Incorporate the packaging signal in the shuttle vector along with a CRISPR-array or single gRNA-encoding sequence (and optionally other components of the CRISPR/Cas system, such as a Cas9-encoding nucleotide sequence and optionally tracrRNA-encoding sequence, or Cas3 and/or Cascade-encoding sequence);
(h) Transform the vector into production host strain;
(i) Induce (eg, UV or mitomycin C induce) and harvest phage comprising the CRISPR/Cas component(s). Alternatively, use a system with inducible RecA in *trans* to simulate SOS (needs to be activated RecA).

### Example of the above specifically for E coli Nissle using phage P2:

Nissle is useful due to its GRAS (Generally Regarded as Safe) status and P2 has a relatively broad host range (most *E coli, Shigella, Klebsiella, Salmonella* in addtition to DNA delivery into *e.g. Pseudomonas;* Kahn et al 1991, "Bacteriophage P2 and P4", Methods in Enzymology, vol 204, pp264-280).

We will use pUC19 or other high or medium copy number cloning vector. Temperate phage P2 can lysogenize Nissle. Most *E coli* K strains have an inactive CRISPR/Cas system and can be infected by P2 and thus all regular cloning hosts can be used (here exemplified by *E coli* TOP10).

P2 is introduced into TOP10 to produce a lysogen. P2 cannot be induced with mitomycin C or UV but we will use the epsilon anti-repressor from the parasite phage P4 that derepresses P2 and makes it go into lytic phase. We will express this gene from an inducible promoter in the production host strain.

The 325 bp packaging signal sequence of SEQ ID NO: 2 will be used.

The packaging sequence will be deleted in the P2 prophage of the lysogenic production TOP10 strain.

A pUC19 shuttle vector encoding a guide RNA that targets the genome of the target Nissle strain (or alternatively comprising a CRISPR array for producing such a guide RNA) will be constructed and the packaging signal SEQ ID NO: 2 will be added. If the target Nissle harbours its own endogenous CRISPR/Cas system, we will use an activation strategy to activate the endogenous Cas3 by including Cas activating genes in the vector. If not, we will include an exogenous Cas3-encoding nucleotide sequence (and optionally one or more nucleotide sequences encoding one or more required Cascade components) in the vector for expression in the target Nissle. We will transform the vector into the TOP10 production strain, induce the P4 anti-repressor and harvest phage comprising the CRISPR/Cas component(s).

Since the induced (helper) phage DNA does not contain a packaging signal we will be able to isolate particles with only the vector DNA packaged. Thus, we will obtain a composition comprising such phage which can be used to infect target Nissle *E coli* bacteria and introduce the CRISPR/Cas component(s) therein for killing the target bacteria.

### Example 2 (Reference): MGEs, Genomics Islands etc

Overview of possible different MGE packaging strategies follow.

Applicable to different types of phages:
- Identify packaging signal and structural genes in the helper phage
- Delete packaging signal in helper phage and place on plasmid comprising MGE
- Place both helper and plasmid in production strain
- Induce structural gene transcription of helper to get production of helper-phage-packaged MGEs

For using parasitic mobile elements (P4 phage or SaPI etc) activation of helper phage structural genes is done by induction of a helper phage activator obtained from the parasitic element Delta in P4 or one, more or al of ptiA/B/M in SaPI.

If one wants smaller size particles one can choose to package in a parasite-size capsid (typically 10-20 kb) by including in the MGE or vector P4 Sid and psu or cpmA/B from a SaPI.

One can use defective helper phages where at least the packaging signal has been removed and structural genes are either on a plasmid or integrated as a cryptic prophage in the production host. If for some reason one cannot use this approach and need to use functional helper phages, one will include in the MGE or vector the genes on the parasite that hijack the phage packaging machinery to preferentially package parasite DNA (in our case CGV^{™}) over phage DNA.

**List of the minimal genes one could include on a plasmid vector from P4.**
P4 sequence: see https://www.ncbi.nlm.nih.gov/nuccore/x51522
Cos packaging site: SEQ ID NO: 3
The homologous sequence between P2 and P4; this may be used as an alternative packaging signal in the MGE or vector: SQ ID NO: 4
For small capsid size (packages 11.4kb instead of 33.5 kb) Sid and/or Psu can be included in the MGE or vector:-
   Sid: SEQ ID NO: 5
   Psu: SEQ ID NO: 6

To activate helper phage P2, Delta from P4 can be included in a host cell genome (provided separately in a host cell, not on the MGE or vector to be packaged)
Delta: SEQ ID NO: 7

**Minimum genes to include in the host chromosome/episome from P2.**
P2 sequence (acc.number: NC_001895)

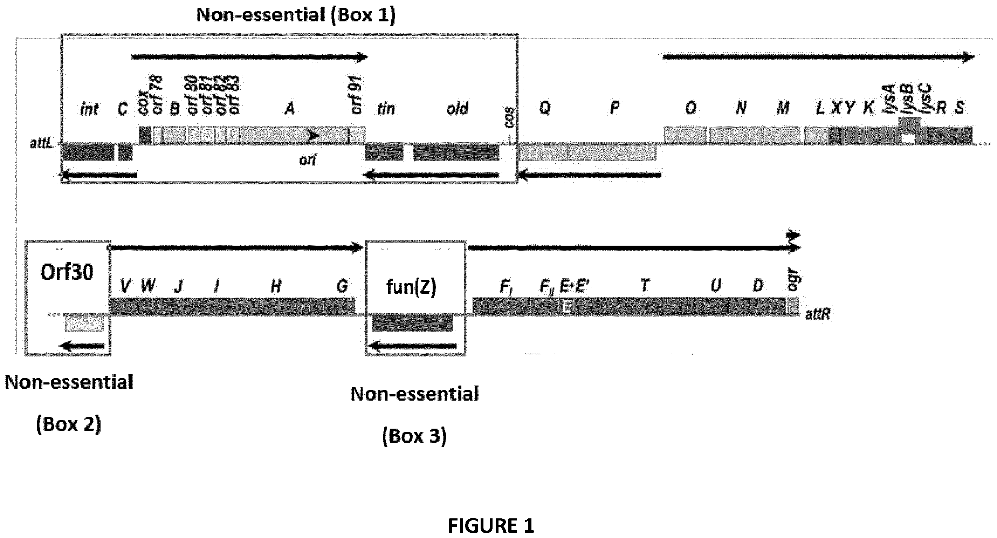

Figure 1 shows the genetic map of P2 genome with non-essential genes boxed - one, more or all of these can be excluded (but *Cos* is always deleted). *Cos* is deleted and preferably the whole region from int through *Cos.* This region may, for example, be swapped with a resistance marker while the *orf30* and *fun(Z)* genes are left intact or may be deleted.

Thus in an example, *int* through to *Cos* (ie, including *int* and *Cos)* are omitted from the helper phage DNA or second DNA or a homologous region is omitted when different phage are used as the basis for the helper phage DNA or second DNA.
**The sequences of various stretches of the P2 genome are as follows:-**
"Q" through "S": SEQ ID NO: 8
"V" through "G": SEQ ID NO: 9
"FI" through "ogr": SEQ ID NO: 10

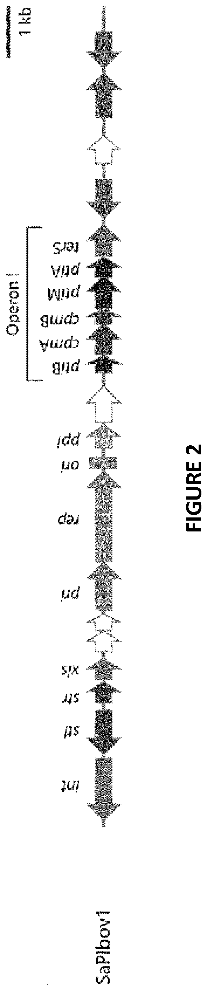

**Minimal genes to include from a SaPI on a vector or MGE.**
Several different SaPI systems exist. Figure 2 shows one of the well characterized SaPIs (SaPIbov1), which exploits phages phi11 or phi80alpha as helper phage. SaPIbov1 sequence (acc.number: AF217235.1)

### Packaging signal

If one uses a defective helper phage with deleted packaging signal one can use that signal from the helper phage and include it in the DNA to be packaged. An example from S. aureus phi11 (acc. number: AF424781) is SEQ ID NO: 11.

For small capsid size (packages 15.8 kb instead of 43.6 kb), one can include *cpmA* and/or *cpmB* in the MGE or vector (SEQ ID Nos: 12 and 13).

To activate helper phage phi11 one can include one, more or all of *ptiA, B* and *M* (provided separately in the production host cell and not on the MGE or vector to be packaged) (SEQ ID Nos: 14-16).

**Minimum genes to include in the host chromosome/episome from phi11.**
Phi11 sequence (acc.number: AF424781)
gene #29 (terS) through gene #53 (lysin): SEQ ID NO: 17

**A list of phage that work with SaPIs**
Different SaPIs are linked to different helper phages (see Table 2 below)

One can mutate the helper phage to only contain structural genes to direct the phage to package in smaller capsids. If only looking at the genes responsible for small capsid packaging *(cpmA* and *cpmB)* these are highly conserved among staphylococci indicating that they will function to redirect packaging in a variety of phages broader than the list below (Table 2).

### Example 3 (Reference): Design, construction and in vitro efficacy of a synthetic transduction particle delivery platform

### Executive summary

SA 100 is a synthetic DNA delivery vehicle comprising non-self-replicative transduction particles comprising phage coat proteins packaging DNA plasmids that comprise nucleic acid sequences endoding CRISPR/Cas components, wherein the particles are capable of infecting target *E coli* host cells to introduce the DNAs therein. Introduced DNAs are then able to be used in the host cells to produce the components. We call such DNAs CRISPR-Guided Vectors (CGVs^{™}). As shown in this example, we made such particles using a defective helper phage. Upon production of the synthetic particles we obtain a pure synthetic lysate devoid of any phage nucleic acid encoding phage proteins. We show that this lysate is capable of very efficient delivery of a CGV^{™} to *E. coli in vitro.* The particles are non-self-replicative and require the helper phage for replication; thus usefully the lysate is pure and removes the possibility of replication of the particles containing the CGV^{™}. This is useful to contain the action of the antibacterial, to allow for more controlled (eg, predetermined) dosing for administration to a human or animal subject or an environement for example, and the elimination of replication in the subject or environment reduces the chances of phage acquiring undesirable genes or traits from surrounding phage or bacteria (eg, undesirable antibiotic resistance genes) - and even then the limitation of particle replication described herein affords a way of reducing or eliminating spread of such genes or traits. This may be of interest to authorities such as the US FDA or USDA who consider such aspects when approving medicines or antibacterials for use in the environment.

### Study objectives

Objective 1: Design and engineer the fully synthetic phage-based CGV delivery platform SA100.

Objective 2: Use SA100 to demonstrate CGV delivery to *E.coli.*

### Materials and methods

### Bacterial strains and growth conditions

We made a bacterial production strain for producing the synthetic SAE100 transduction partiles. *E coli* strain C-2323 harboring phage P2 was used as starting material for engineering our SA100/CGV production strain (gift from Gail E. Christie at the Virginia Commonwealth University). Other strains used for testing SA100 and infectivity were EMG-2, C-1a and C-1792, (purchased from the Coli Genetic Stock Center http://cgsc2.biology.yale.edu/).

All bacterial cultures were grown in LB medium supplemented with 2mM MgCl₂. When appropriate, cultures were supplemented with 50µg/mL kanamycin or 50µg/mL spectinomycin for selection of defective prophage and CGV, respectively. In SA100 infection studies, the medium was supplemented with 2.5 mM CaCl: to aid adsorption of the SA100 particles to bacterial cells. For induction of the CRISPR/Cas system, cells were exposed to 0.5mM IPTG and 2mM theophylline.

### Construction of defective helper prophage

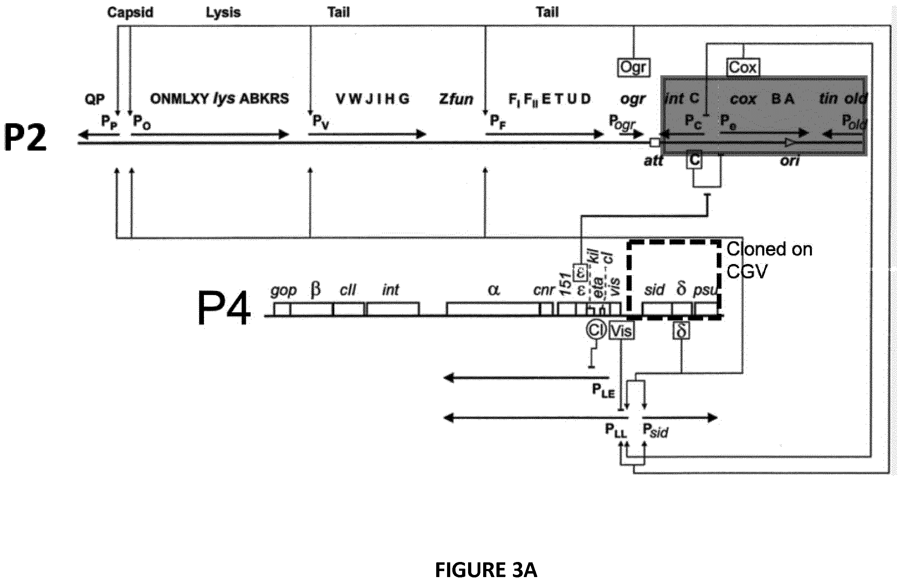

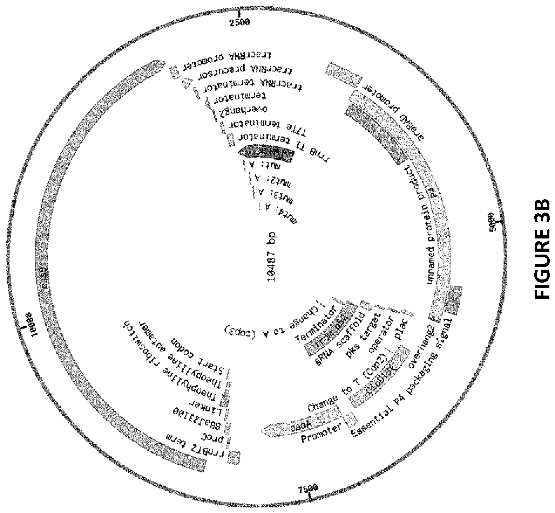

The pTK-red recombineering plasmid (p72) was transformed in strain C-2323.
A KamR kanamycin marker gene was amplified using oligos with 50 bp homology to the regions of the P2 prophage immediately up- and downstream of the approximately 10 kbp region of P2 that we wanted to delete (see Figure 3). Following successful replacement of the desired region with the KanR marker gene, the thermosensitive pTK-red recombineering plasmid was cured.

### Engineering CGV (p94) for packaging in the SA100 particles

CGV p77 was constructed using plasmid p53 as a template. Plasmid p53 is a plasmid encoding components of a CRISPR/Cas system, the plasmid comprising nucleic acid sequences endoding a tracrRNA and the Cas9 protein from *Streptococcus pyogenes* (SpCas). Nucleic acid sequence for produing crRNAs was obtained, expression driven by a plac promoter. The two fragments were assembled using Gibson assembly (NEB E5510S) and transformed into *E. coli* competent cells. Assembled p77 plasmid was verified by full sequencing.

p77 harboring the inducible CRISPR/Cas system was used as the backbone for insertion of a fragment comprising an arabinose inducible promoter transcribing the region *sid* through *cos* from the satellite phage P4 (Figure 3).

Both fragments were PCR amplified and cloned by restriction enzyme cloning. The resulting SA100 packable CGV (p94) was sequence verified.

### Production of SA100 packaged CGVs

CGV p94 was transformed into the previously constructed strain harboring the defective prophage thereby generating production strain #189.

The production strain was grown exponentially for approximately 10 generations in the presence of kanamycin and spectinomycin, ensuring balanced growth of the population. At optical density (OD₆₀₀) of 1 the culture was induced with 1% arabinose and incubation was continued until optical density measurements were low and stable. After spinning away cell debris the lysate was filtered (0.45 µm) and stored at +4C until further use.

### Transduction protocol to determine SA100 titers.

Determination of titers was done by mixing the SA100 lysate with a suitable bacterial strain ensuring at least 100-fold surplus of bacterial cells in presence of 2.5 mM CaCl₂. Following 30 min incubation, the bacteria were diluted and plated on LB containing spectinomycin for enumeration of bacteria that had received the p94 CGV by transduction. To verify purity of the SA100 lysate, undiluted lysate was spotted on a lawn of bacteria (C-1a) known to support P2 proliferation using standard soft-agar overlay. Absence of plaque formation was used to verify purity.

### DNA delivery assay

A transduction protocol was used in which the SA100 lysate was diluted before infecting the bacterial culture to obtain multiplicities of infection (MOI) ranging from 0.01 to 100 effectively spanning a 4 log-ratio of SA100 to bacterial cells.

Following 30 min infection in the presence of 2.5 mM CaCl₂ the bacteria were enumerated on LB plates with and without spectinomycin selection for delivery of the p94 CGV. The obtained numbers were used to calculate the percentage of the population infected at a given MOI.

### Results

### Design of the SA100 vehicle

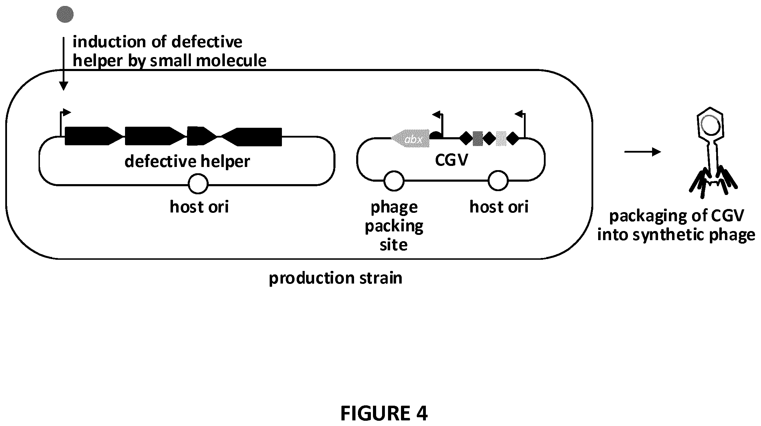

The overall design of the SA100 *E. coli* production strain harboring CGV and defective prophage is outlined schematically in Figure 4. We used the native *E. coli* phage P2 integrated as a prophage on the chromosome of the production strain as our starting material. In the prophage, we deleted genes that are essential for phage to initiate and carry out its own proliferation thus generating a defective helper phage.

We then placed expression of the structural genes needed to produce SA100 transduction particles under the control of an inducible promoter allowing us to turn on production of particles. Finally, we removed the packaging sequence normally used by the phage to package its DNA into the phage particles from the phage DNA and placed it on our CGV (see *cos* sites shown in Figure 3). Upon induction of the phage structural genes we got packaging of CGV DNA into the particles that were subsequently released by lysis of the production cells.

### Engineering defective helper phage

E. *coli* strain C-2323 harboring phage P2 on the chromosome was used as starting material for engineering the production strain with the defective helper phage. A region on the P2 phage comprising integrase, promoters for initiation of phage proliferation, origin of replication, DNA replication genes and the *cos* site (DNA sequence recognised when packaging DNA into phage particles) (see boxed P2 DNA shown in Figure 3), was replaced by a kanamycin marker using recombineering. The site-specific integration of the marker and deletion of approximately 10 kbp of phage DNA was verified by sequencing.

### Engineering CGV to enable SA100 packaging

We used a single vector (p77) with the CRIPSR/Cas system expressed from inducible promoters for engineering our SA100 packable CGV.

To enable activation of the defective P2 helper phage and CGV packaging into synthetic transduction particles, we cloned the genetic region comprising *sid-delta-psu-cos (cos* packaging site) from the satellite phage P4 into the p77 plasmid. The cloned P4 region (SEQ ID NO: 18), which is known to be able to activate the P2 phage was already cloned in another vector p93 where it was expressed from an arabinose inducible promoter. The resulting CGV (p94), containing all elements necessary for P2 activation and SA100 packaging, was verified by complete sequencing. P4 genomic architecture and the region cloned in p94 is shown in the lower box in Figure 3A and the p94 genomic map is shown in Figure 3B.

### Production of SA100 packaged CGVs

The p94 CGV was electroporated into the strain harboring the defective prophage resulting in production strain #189.

SA100 packaged CGVs were produced as described above. Titers of the SA100 packaged CGV was approximately 10¹⁰ TFU/mL determined by transduction of SA100 packaged p94 into 3 strains of *E. coli.* As expected, no contamination of the native P2 phage could be observed (Table 3 below). Accordingly, we can assume a native phage contamination of less than 1 in 1e9 per synthetic particle.

### CGV delivery to E. coli using SA100

To determine the number of phages required for 100% infection of an E. *coli* population, we performed an infection experiment varying the ratio of SA100 particles to bacterial cells, the so-called multiplicity-of-infection (MOI) from 0.01 to 100.

We observed that a MOI of 1 meaning one SA100 particle per *E. coli* cell, was sufficient for 100% infection of the bacterial population (Figure 4).

### Discussion and conclusions

SA100, a fully synthetic phage-based CGV delivery vehicle was designed and constructed. Upon expression in a production strain also harboring a compatible CGV, a high titer lysate of SA100 packaged CGVs devoid of native phage contamination was produced. Finally, the obtained SA100 packaged CGVs were efficiently delivered into E. *coli.*

### Example 4: CGV in vitro killing of E. coli following delivery by the SA100 synthetic transduction particle delivery platform

### Executive summary

We used the SA100 synthetic DNA delivery vehicle to deliver optimised CRISPR guided vectors (CGV^{™}) to two *E. coli* target strains. Upon induction of the system we could demonstrate up to 4 logs killing of the target population.

### Introduction

We have previously engineered the phage-based synthetic DNA delivery vehicle SA100 (Example 3) for delivery of CGVs to *E. coli* target cells. Here, we optimised a CGV for delivery by the SA100 vehicle and subsequently tested the efficacy in killing E. *coli* target cells.

### Study objectives

Objective 1: Optimize CGV for delivery by SA100.
Objective 2: kill *E.coli* by SA100 delivered CGVs

### Materials and methods

### Bacterial strains and culture conditions

All bacterial cultures were grown in LB medium supplemented with 2mM MgCl₂. When appropriate, cultures were supplemented with 50µg/mL kanamycin or 50µg/mL spectinomycin for selection of defective prophage and CGV, respectively. In SA100 infection studies, the medium was supplemented with 2.5 mM CaCl₂ to aid adsorption of the SA100 particles to bacterial cells. For induction of the CRISPR/Cas system, cells were exposed to 0.5mM IPTG and 2mM theophylline. Target strains MG1655_pks and XL1-blue_pks were constructed by recombineering of a 20-nucleotide sequence complementary to the guide RNA spacer sequence in our CGVs into the lacZ gene of MG1655 and XL1-blue thereby generating target strains MG1655_pks and XL1-blue_pks, respectively.

### Production of SA100 packaged CGVs

CGVs (p94 and p114) were separately transformed into a respective strain harboring the defective prophage thereby generating production strain #189 and #226, respectively.

The production strains were grown exponentially for approximately 10 generations in the presence of kanamycin and spectinomycin ensuring balanced growth of the population. At optical density (OD₆₀₀) of 1 cultures were concentrated x10 and induced with 1% arabinose and incubation was continued until optical density measurements were low and stable. After spinning away cell debris the lysate was filtered (0.45 µm), further concentrated approximately x4 using Amicon Ultra-15 centrifugal filters and stored at +4C until further use.

### CGV delivery and killing

Exponentially growing cultures of the *E. coli* target strains were infected with SA100 packaged CGVs p94 or p114 at a MOI of 20 in the presence of 2.5mM CaCl₂.

Following 30 min incubation at 37 degreees centigrade, the bacterial cultures were serially diluted and plated on LB plates containing IPTG and theophylline (for inducing the expression of CRISPR/Cas components) to investigate CGV mediated killing or on LB+spectinomycin to investigate CGV delivery to the target populations.

### Results

### Optimising CGV DNA by increasing copy number

CGV p94 was previously shown to be efficiently delivered to *E. coli* (Example 3). This CGV contains the CloDF 13 origin of replication, in which a single SNP will increase copy number from 7 to 80 according to Stuitje et al. 1981, "Identification of mutations affecting replication control of plasmid Clo DF13", Nature, vol 290, pp264-267. By incorporating the SNP into an oligo used for PCR amplification of the whole CGV, we created p114 containing the mutated CloDF13 ori (SEQ ID NO: 23). Sequence verification was carried out.

### CGV delivery by SA100

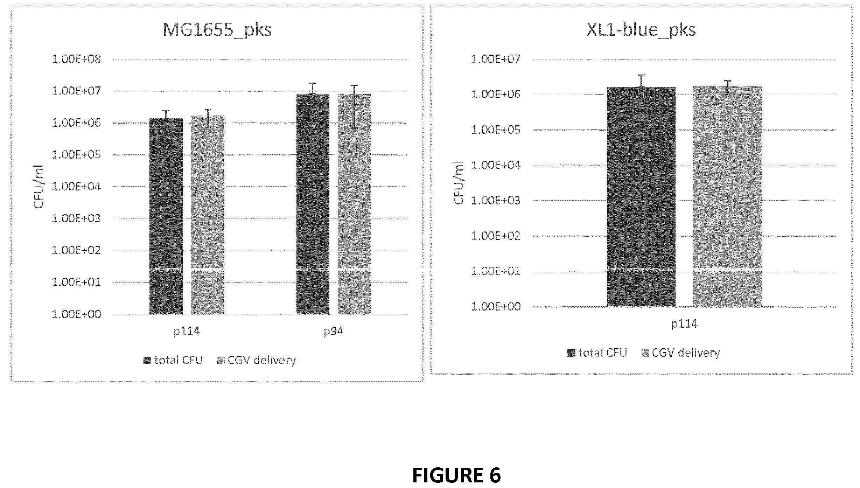

We previously showed efficient CGV delivery using p94 packaged in SA100 (Example 3). Here, we compared delivery of CGVs p94 and p114 to MG1655_pks (Fig 6A and additionally p114 delivery to XL1-blue_pks (Fig 6B. Using a multiplicity of infection (MOI) of 20 we demonstrate 100% delivery to the target populations.

### Killing efficacy of SA100 delivered CGV

Using the same experimental setup as above (target strains, SA 100 packaged CGVs and MOI of 20) we investigated the ability of the delivered CGVs to kill the target cell upon induction of the expression of the CRISPR/Cas system.

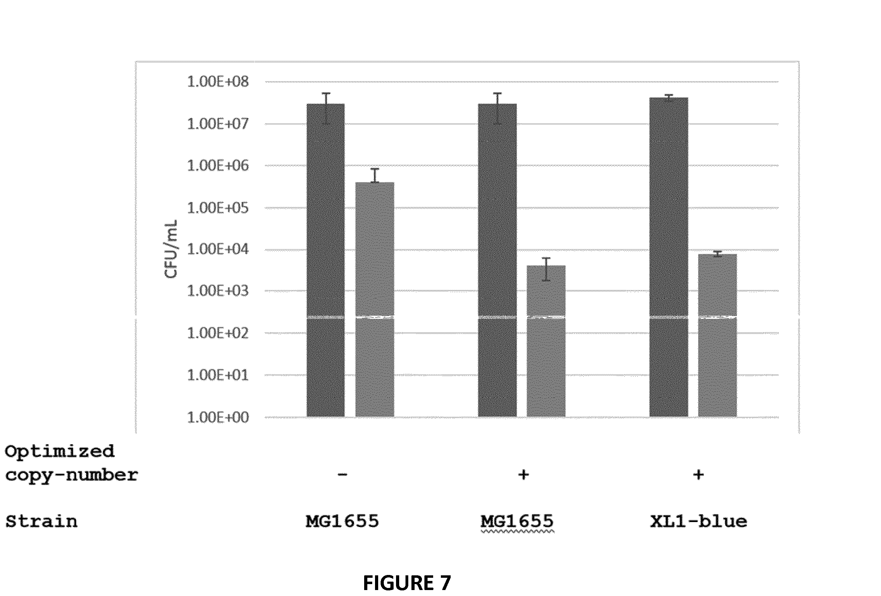

The p94 CGV targeting the pks DNA sequence in MG1655_pks was able to reduce the target strain approximately 2 logs while p 114 targeting the same sequence reduced the target population approximately 4 logs (Fig 7). The same magnitude of killing was observed when p114 was targeting pks in the XL1-blue_pks target population (Fig 7).

### Discussion and conclusions

Following SA100 delivery of CGVs to target cells we were able to increase killing of the target population from approximately 2 logs using p94 to 4 logs using p114 probably due to a high copy number CGV.

### Example 5: In vivo antibacterial delivery to gut microbiome using non-self-replicative particles & target cell killing in microbiome setting

### Executive summary

*E*. *coli* AMG1655-pks was used to colonize the murine gut. We show statistically significant delivery of CGV^{™} pSNP114 into MG1655-PKS by SA100 delivery vehicle (synthetic non-self-replicative phage particles, see Examples 3 and 4). We observered a reduction in survival upon activation of the CGV^{™} targeting *E. coli* MG1655-pks.

### Introduction

We have previously shown that *E*. *coli* ATCC43888 colonizes the a murine gut of NMRI mice model (data not shown) and that activating of a CRISPR array in *E*. *coli* ATCC43888 in the mouse gut reduces survival, where guide RNA produced using the array target the *E coli* genome for Cas cutting (data not shown). In this Example we set out to assess the survival of a CRISPR/Cas-based antibacterial (a CGV^{™}) delivered by a Synthetic Nanoboiotic^{™}, namely SA100, into MG1655-pks.

### Study objective:

Establish survival reduction of *E. coli* MG1655-pks in the murine gut microbiome by delivery of CGV^{™} pSNP114 with Synthetic Nanoboiotic^{™} SA100.

### Materials and methods

*E coli* was *E coli* MG1655 with pks fragment inserted, with a strep resistance marker, phylo group A.

Cells were grown overnight in LB media supplemented with 50 µg/mL spectinomycin. Next day the OD was measured and the cultures were diluted down to 10⁸ CFU/mL. Vehicle, induced and uninduced groups are shown in Table 4.
- 15 female NMRI mice, 26-30 gram (Taconic)
- Inducer solution containing theophylline and L-arabinose

### Laboratory animal facilities and housing of mice

The temperature was 21°C +/- 2 °C and could be regulated by heating and cooling, and light/dark period was in 12-hours intervals of 6 a.m.- 6 p.m./6 p.m - 6 a.m. Mice had free access to food and to domestic quality drinking water until 3 days prior to the study. Mice were housed 6-7 mice /cage in the standard facility with bedding from Aspen Wood from Tapvei and paper strands from Sizzle-nest as nesting material until 1 day prior to the study and there after moved into the GMO facility 1 mouse per cage. The From day 0 the wooden bedding was changed to white paper bedding to facilitate collection of faeces from the cages

### Preparation of streptomycin drinking water

6 liters of 5 g/L of streptomycin was prepared by dissolving 6.94 g streptomycin sulphate / L of sterile water. Mice were given streptomycin water starting day -3 and throughout the study.

### Inoculation of mice

Inocula was formulated prior to inoculation as ready to use suspensions. Mice were inoculated with 0.25 ml by oral gavage at 8 am day 0. A titre of approximately 10¹¹ TFU/ml was used.

### Treatment of mice

Mice were dosed with inducers and 10¹¹ TFU/mL SA100 by oral gavage at 7 am and 1 pm day 1 and again at 7 am day 2. Inducers were mixed immediately prior to each treatment time point prior to the treatment mice were gavaged with antacid.

### Collection of faecal samples from cages

Immediately prior to inoculation 0.5 ml faecal samples were collected from cages into three 15 ml Nunc tubes. Day 1 and 2 after inoculation, 0.5 ml faecal samples were collected prior to treatment at 7 am and mice were moved to a new cage. Day 2 after inoculation faecal samples were also collected 4 hours after treatment

### CFU determination

0.5 ml faecal matter was dissolved in 5 ml sterile saline by vortexing multiple times during 1-4 hours. Colony counts were determined by 10 fold serially dilution of the faecal samples in sterile 0.9% NaCl and 20 µL spots were applied to agar plates. The lower detection limit of faecal samples was 50 CFU/ml. All agar plates were incubated 18-22 hrs at 35°C in ambient air.

Delivery of the CGV was enumerated by plating on LB + streptomycin + spectinomycin plates plates. Abudance of the target strain was enumerated by plating on LB with streptomycin only.

### Clinical monitoring of mice

The body weight of the mice were monitored throughout the study and the mice were scored 0 - 6 based on their behavior and clinical signs (Table 5). Mice were euthanized if reaching score 3 or 20% weight loss.

### Results

### Microbiome analysis of mice prior to inoculation

No bacteria was detected in 2 out of 3 of the pooled samples collected prior to inoculation. One of the pooled samples had 6.54 log10 CFU/ml of small white colonies. MALDI analysis indicated *Paenibacillus odorifer* with a score value of 1.71 and as second most likely *Staphylococcus xylosus* with a score value of 1.43 both with a low rank quality. Also in the 2 other pooled samples 3-4 log10 CFU/ml of small white colonies appeared after 48 hours of incubation.

At 24 h after inoculation the CFU levels in the faecal samples ranged from 6.1 - 8.6 log10 CFU/sample as determined on the LB+ strep agar plates. Similar levels were observed day 2 after inoculation. No significant differences were observed with in each of the 2 inoculation groups.

On the LB+ strep + spec agar plates, no colonies were observed day 1 in in any of the groups. Day 2 CFU counts ranging from 1 to 5 log10 CFU/sample was observed in the inducer treated groups but not in the vehicle treated groups.

### Layout of animal study

Twenty four mice were used in each murine intervention study.

### Clinical mice score

Mice did not show any clinical signs of infection or discomfort during the entire study period.

### Induction killing of MG1655-pks

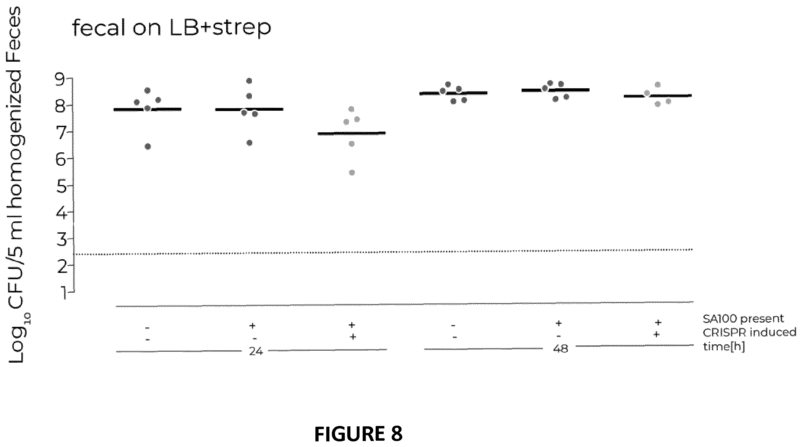

Faecal matter extracts were plated out on on plates containing streptomycin to determine the CFU of the *E. coli* MG1655-PKS strain (Fig 8). Streptomycin CFU counts show no difference between the various treatment groups.

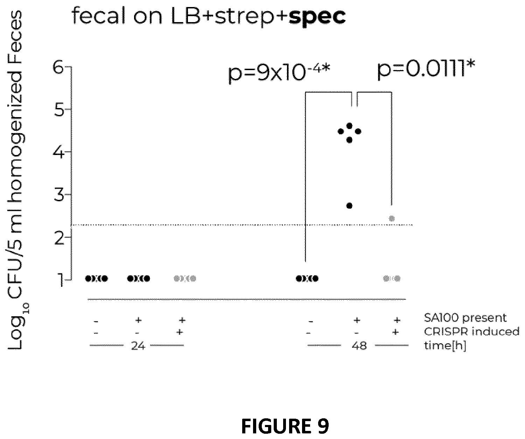

When plating the faecal samples on spectomycin and streptomycin to determine delivery of CGV pSNP114 (because this CGV carries a spectomycin resistance marker) we observe a significant increase in CFU, and thus CGV delivery, after 48 hours (Fig 9). When the CRISPR system was induced we observed a significant decrease in the mean CFU counts.

### Discussion and conclusions

We successfully demonstrated guided nuclease antibacterial (CGV^{™}) delivery using Synthetic Nanobiotic^{™} platform, SA100, into *E. coli* MG1655-pks in the murine gut model. Thus, delivery of an antibacterial using non-self-replicative particles to a microbiome *in vivo* was established. A statistically significant reduction in CFU counts was observed using the activated CRISPR system on the CGV^{™}.

**Table 1: Example Bacteria**

| Optionally, the host cells are selected from this Table and/or the target cells are selected from this Table (eg, wherein the host and target cells are of a different species; or of the same species but are a different strain or the host cells are engineered but the target cells are wild-type or *vice versa).* For example the host cells are *E coli* cells and the target cells are *C dificile, E coli, Akkermansia, Enterobacteriacea, Ruminococcus, Faecalibacterium, Firmicutes, Bacteroidetes, Salmonella, Klebsiella, Pseudomonas, Acintenobacter* or *Streptococcus* cells. | | | | |
|---|---|---|---|---|
| **Abiotrophia** | **Acidocella** | **Actinomyces** | **Alkalilimnicola** | **Aquaspirillum** |
| *Abiotrophia defectiva* | *Acidocella aminolytica* | *Actinomyces bovis* | *Alkalilimnicola ehrlichii* | *Aquaspirillum polymorphum* |
| | *Acidocella facilis* | *Actinomyces denticolens* | | *Aquaspirillum putridiconchylium* |
| **Acaricomes** | | *Actinomyces europaeus* | **Alkaliphilus** | |
| *Acaricomes phytoseiuli* | **Acidomonas** | *Actinomyces georgiae* | *Alkaliphilus oremlandii* | *Aquaspirillum serpens* |
| **Acetitomaculum** | *Acidomonas methanolica* | *Actinomyces gerencseriae* | *Alkaliphilus transvaalensis* | |
| | | *Actinomyces hordeovulneris* | | **Aquimarina** |
| *Acetitomaculum ruminis* | **Acidothermus** | | **Allochromatium** | *Aquimarina latercula* |
| | *Acidothermus cellulolyticus* | | *Allochromatium vinosum* | |
| **Acetivibrio** | | *Actinomyces howellii* | | **Arcanobacterium** |
| *Acetivibrio cellulolyticus* | **Acidovorax** | *Actinomyces hyovaginalis* | **Alloiococcus** | *Arcanobacterium haemolyticum* |
| *Acetivibrio ethanolgignens* | *Acidovorax anthurii* | *Actinomyces israelii* | *Alloiococcus otitis* | |
| *Acetivibrio multivorans* | *Acidovorax caeni* | *Actinomyces johnsonii* | | *Arcanobacterium pyogenes* |
| | *Acidovorax cattleyae* | *Actinomyces meyeri* | **Allokutzneria** | |
| **Acetoanaerobium** | *Acidovorax citrulli* | *Actinomyces naeslundii* | *Allokutzneria albata* | **Archangium** |
| *Acetoanaerobium noterae* | *Acidovorax defluvii* | *Actinomyces neuii* | | *Archangium gephyra* |
| | *Acidovorax delafieldii* | *Actinomyces odontolyticus* | | |
| | *Acidovorax facilis* | *Actinomyces oris* | | |
| **Acetobacter** | *Acidovorax konjaci* | *Actinomyces radingae* | **Altererythrobacter** | **Arcobacter** |
| *Acetobacter aceti* | *Acidovorax temperans* | *Actinomyces slackii* | *Altererythrobacter ishigakiensis* | *Arcobacter butzleri* |
| *Acetobacter cerevisiae* | *Acidovorax valerianellae* | *Actinomyces turicensis* | | *Arcobacter cryaerophilus* |
| *Acetobacter cibinongensis* | | *Actinomyces viscosus* | | *Arcobacter halophilus* |
| *Acetobacter estunensis* | **Acinetobacter** | | **Altermonas** | *Arcobacter nitrofigilis* |
| *Acetobacter fabarum* | *Acinetobacter baumannii* | **Actinoplanes** | *Altermonas haloplanktis* | *Arcobacter skirrowii* |
| *Acetobacter ghanensis* | *Acinetobacter baylyi* | *Actinoplanes auranticolor* | *Altermonas macleodii* | |
| *Acetobacter indonesiensis* | *Acinetobacter bouvetii* | *Actinoplanes brasiliensis* | **Alysiella** | **Arhodomonas** |
| *Acetobacter lovaniensis* | *Acinetobacter calcoaceticus* | *Actinoplanes consettensis* | *Alysiella crassa* | *Arhodomonas aquaeolei* |
| *Acetobacter malorum* | *Acinetobacter gerneri* | *Actinoplanes deccanensis* | | **Arsenophonus** |
| *Acetobacter nitrogenifigens* | *Acinetobacter haemolyticus* | *Actinoplanes derwentensis* | *Alysiella filiformis* | *Arsenophonus nasoniae* |
| *Acetobacter oeni* | *Acinetobacter johnsonii* | *Actinoplanes digitatis* | **Aminobacter** | |
| *Acetobacter orientalis* | *Acinetobacter junii* | *Actinoplanes durhamensis* | *Aminobacter aganoensis* | |
| *Acetobacter orleanensis* | *Acinetobacter lwoffi* | *Actinoplanes ferrugineus* | *Aminobacter aminovorans* | |
| *Acetobacter pasteurianus* | *Acinetobacter parvus* | *Actinoplanes globisporus* | *Aminobacter niigataensis* | **Arthrobacter** |
| *Acetobacter pornorurn* | *Acinetobacter radioresistens* | *Actinoplanes humidus* | | *Arthrobacter agilis* |
| *Acetobacter senegalensis* | *Acinetobacter schindleri* | *Actinoplanes italicus* | **Aminobacterium** | *Arthrobacter albus* |
| *Acetobacter xylinus* | *Acinetobacter soli* | *Actinoplanes liguriensis* | *Aminobacterium mobile* | *Arthrobacter aurescens* |
| | *Acinetobacter tandoii* | *Actinoplanes lobatus* | | *Arthrobacter chlorophenolicus* |
| **Acetobacterium** | *Acinetobacter tjernbergiae* | *Actinoplanes missouriensis* | **Aminomonas** | |
| *Acetobacterium bakii* | | | *Aminomonas paucivorans* | |
| *Acetobacterium carbinolicum* | *Acinetobacter towneri* | *Actinoplanes palleronii* | | *Arthrobacter citreus* |
| *Acetobacterium dehalogenans* | *Acinetobacter ursingii* | *Actinoplanes philippinensis* | **Ammoniphilus** | *Arthrobacter crystallopoietes* |
| *Acetobacterium fimetarium* | *Acinetobacter venetianus* | *Actinoplanes rectilineatus* | *Ammoniphilus oxalaticus* | *Arthrobacter cumminsii* |
| *Acetobacterium malicum* | | *Actinoplanes regularis* | Ammoniphilus oxalivorans | *Arthrobacter globiformis* |
| *Acetobacterium paludosum* | **Acrocarpospora** | *Actinoplanes teichomyceticus* | | *Arthrobacter histidinolovorans* |
| *Acetobacterium tundrae* | *Acrocarpospora corrugata* | | **Amphibacillus** | |
| *Acetobacterium wieringae* | *Acrocarpospora macrocephala* | *Actinoplanes utahensis* | *Amphibacillus xylanus* | *Arthrobacter ilicis* |
| *Acetobacterium woodii* | | | **Amphritea** | *Arthrobacter luteus* |
| | *Acrocarpospora pleiomorpha* | **Actinopolyspora** | *Amphritea balenae* | *Arthrobacter methylotrophus* |
| **Acetofilamentum** | | *Actinopolyspora halophila* | *Amphritea japonica* | *Arthrobacter mysorens* |
| *Acetofilamentum rigidum* | | *Actinopolyspora mortivallis* | | *Arthrobacter nicotianae* |
| | **Actibacter** | | ***Amycolatopsis*** | *Arthrobacter nicotinovorans* |
| **Acetohalobium** | *Actibacter sediminis* | | *Amycolatopsis alba* | *Arthrobacter oxydans* |
| *Acetohalobium arabaticum* | | **Actinosynnema** | | |
| | **Actinoalloteichus** | *Actinosynnema mirum* | *Amycolatopsis albidoflavus* | *Arthrobacter pascens* |
| **Acetomicrobium** | *Actinoalloteichus cyanogriseus* | | *Amycolatopsis azurea* | *Arthrobacter phenanthrenivorans* |
| *Acetomicrobium faecale* | | **Actinotalea** | *Amycolatopsis coloradensis* | |
| *Acetomicrobium flavidum* | *Actinoalloteichus hymeniacidonis* | *Actinotalea fermentans* | *Amycolatopsis lurida* | *Arthrobacter polychromogenes* |
| | | | *Amycolatopsis mediterranei* | |
| **Acetonema** | *Actinoalloteichus spitiensis* | **Aerococcus** | *Amycolatopsis rifamycinica* | *Atrhrobacter protophormiae* |
| *Acetonema longum* | | *Aerococcus sanguinicola* | *Amycolatopsis rubida* | *Arthrobacter psychrolactophilus* |
| | | *Aerococcus urinae* | | |
| | | *Aerococcus urinaeequi* | *Amycolatopsis sulphurea* | *Arthrobacter ramosus* |
| **Acetothermus** | **Actinobaccillus** | *Aerococcus urinaehominis* | *Amycolatopsis tolypomycina* | *Arthrobacter sulfonivorans* |
| *Acetothermus paucivorans* | *Actinobacillus capsulatus* | *Aerococcus viridans* | | *Arthrobacter sulfureus* |
| | *Actinobacillus delphinicola* | | **Anabaena** | *Arthrobacter uratoxydans* |
| **Acholeplasma** | *Actinobacillus hominis* | **Aeromicrobium** | *Anabaena cylindrica* | *Arthrobacter ureafaciens* |
| *Acholeplasma axanthum* | *Actinobacillus indolicus* | *Aeromicrobium erythreum* | *Anabaena flos-aquae* | *Arthrobacter viscosus* |
| *Acholeplasma brassicae* | *Actinobacillus lignieresii* | | *Anabaena variabilis* | *Arthrobacter woluwensis* |
| *Acholeplasma cavigenitalium* | *Actinobacillus minor* | **Aeromonas** | | |
| *Acholeplasma equifetale* | *Actinobacillus muris* | *Aeromonas allosaccharophila* | **Anaeroarcus** | **Asaia** |
| *Acholeplasma granularum* | *Actinobacillus pleuropneumoniae* | | *Anaeroarcus burkinensis* | *Asaia bogorensis* |
| *Acholeplasma hippikon* | | *Aeromonas bestiarum* | | |
| *Acholeplasma laidlawii* | *Actinobacillus porcinus* | *Aeromonas caviae* | **Anaerobaculum** | **Asanoa** |
| *Acholeplasma modicum* | *Actinobacillus rossii* | *Aeromonas encheleia* | *Anaerobaculum mobile* | *Asanoa ferruginea* |
| *Acholeplasma morum* | *Actinobacillus scotiae* | *Aeromonas enteropelogenes* | **Anaerobiospirillum** | **Asticcacaulis** |
| *Acholeplasma multilocale* | *Actinobacillus seminis* | | *Anaerobiospirillum succiniciproducens* | *Asticcacaulis biprosthecium* |
| *Acholeplasma oculi* | *Actinobacillus succinogenes* | *Aeromonas eucrenophila* | | *Asticcacaulis excentricus* |
| *Acholeplasma palmae* | *Actinobaccillus suis* | *Aeromonas ichthiosmia* | *Anaerobiospirillum thomasii* | |
| *Acholeplasma parvum* | *Actinobacillus ureae* | *Aeromonas jandaei* | | **Atopobacter** |
| *Acholeplasma pleciae* | | *Aeromonas media* | **Anaerococcus** | *Atopobacter phocae* |
| *Acholeplasma vituli* | **Actinobaculum** | *Aeromonas popoffii* | *Anaerococcus hydrogenalis* | |
| | *Actinobaculum massiliense* | *Aeromonas sobria* | *Anaerococcus lactolyticus* | |
| | *Actinobaculum schaalii* | *Aeromonas veronii* | | |
| | *Actinobaculum suis* | | *Anaerococcus prevotii* | |
| **Achromobacter** | *Actinomyces urinale* | **Agrobacterium** | *Anaerococcus tetradius* | **Atopobium** |
| *Achromobacter denitrificans* | | *Agrobacterium gelatinovorum* | *Anaerococcus vaginalis* | *Atopobium fossor* |
| *Achromobacter insolitus* | **Actinocatenispora** | | | *Atopobium minutum* |
| *Achromobacter piechaudii* | *Actinocatenispora rupis* | | **Anaerofustis** | *Atopobium parvulum* |
| *Achromobacter ruhlandii* | *Actinocatenispora thailandica* | **Agrococcus** | *Anaerofustis stercorihominis* | *Atopobium rimae* |
| *Achromobacter spanius* | | *Agrococcus citreus* | | *Atopobium vaginae* |
| | *Actinocatenispora sera* | *Agrococcus jenensis* | **Anaeromusa** | |
| **Acidaminobacter** | | | *Anaeromusa acidaminophila* | **Aureobacterium** |
| *Acidaminobacter hydrogenoformans* | **Actinocorallia** | **Agromonas** | | *Aureobacterium barkeri* |
| | *Actinocorallia aurantiaca* | *Agromonas oligotrophica* | **Anaeromyxobacter** | |
| | *Actinocorallia aurea* | | *Anaeromyxobacter dehalogenans* | **Aurobacterium** |
| **Acidaminococcus** | *Actinocorallia cavernae* | **Agromyces** | | *Aurobacterium liquefaciens* |
| *Acidaminococcus fermentans* | *Actinocorallia glomerata* | Agromyces *fucosus* | | **Avibacterium** |
| *Acidaminococcus intestini* | *Actinocorallia herbida* | Agromyces *hippuratus* | **Anaerorhabdus** | *Avibacterium avium* |
| | | *Agromyces luteolus* | *Anaerorhabdus furcosa* | |
| | *Actinocorallia libanotica* | | | |
| **Acidicaldus** | | *Agromyces mediolanus* | | *Avibacterium gallinarum* |
| *Acidicaldus organivorans* | *Actinocorallia longicatena* | *Agromyces ramosus* | **Anaerosinus** | *Avibacterium paragallinarum* |
| **Acidimicrobium** | ***Actinomadura*** | *Agromyces rhizospherae* | *Anaerosinus glycerini* | *Avibacterium volantium* |
| *Acidimicrobium* | *Actinomadura alba* | **Akkermansia** | **Anaerovirgula** | **Azoarcus** |
| *ferrooxidans* | *Actinomadura atramentaria* | | *Anaerovirgula multivorans* | Azoarcus *indigens* |
| | *Actinomadura* | *Akkermansia muciniphila* | | |
| | *bangladeshensis* | | | Azoarcus *tolulyticus* |
| **Acidiphilium** | *Actinomadura catellatispora* | **Albidiferax** | **Ancalomicrobium** | Azoarcus *toluvorans* |
| *Acidiphilium acidophilum* | *Actinomadura chibensis* | *Albidiferax ferrireducens* | *Ancalomicrobium adetum* | |
| *Acidiphilium angustum* | *Actinomadura chokoriensis* | | | **Azohydromonas** |
| *Acidiphilium cryptum* | *Actinomadura citrea* | **Albidovulum** | **Ancylobacter** | *Azohydromonas australica* |
| *Acidiphilium multivorum* | *Actinomadura coerulea* | *Albidovulum inexpectatum* | *Ancylobacter aquaticus* | *Azohydromonas lata* |
| *Acidiphilium organovorum* | *Actinomadura echinospora* | **Alcaligenes** | **Aneurinibacillus** | |
| *Acidiphilium rubrum* | *Actinomadura fibrosa* | *Alcaligenes denitrificans* | *Aneurinibacillus aneurinilyticus* | **Azomonas** |
| | *Actinomadura formosensis* | *Alcaligenes faecalis* | | *Azomonas agilis* |
| **Acidisoma** | *Actinomadura hibisca* | | *Aneurinibacillus migulanus* | *Azomonas insignis* |
| *Acidisoma sibiricum* | *Actinomadura kijaniata* | **Alcanivorax** | *Aneurinibacillus thermoaerophilus* | *Azomonas macrocytogenes* |
| *Acidisoma tundrae* | *Actinomadura latina* | *Alcanivorax borkumensis* | | **Azorhizobium** |
| **Acidisphaera** | *Actinomadura livida* | *Alcanivorax jadensis* | | *Azorhizobium caulinodans* |
| *Acidisphaera rubrifaciens* | *Actinomadura luteofluorescens* | | **Angiococcus** | |
| | | **Algicola** | *Angiococcus disciformis* | **Azorhizophilus** |
| **Acidithiobacillus** | *Actinomadura macra* | *Algicola bacteriolytica* | | *Azorhizophilus paspali* |
| *Acidithiobacillus albertensis* | *Actinomadura madurae* | | **Angulomicrobium** | |
| *Acidithiobacillus caldus* | *Actinomadura oligospora* | **Alicyclobacillus** | *Angulomicrobium tetraedrale* | **Azospirillum** |
| *Acidithiobacillus ferrooxidans* | *Actinomadura pelletieri* | *Alicyclobacillusdisulfidooxidans* | **Anoxybacillus** | *Azospirillum brasilense* |
| *Acidithiobacillus thiooxidans* | *Actinomadura rubrobrunea* | *Alicyclobacillus* | *Anoxybacillus pushchinoensis* | *Azospirillum halopraeferens* |
| | *Actinomadura rugatobispora* | | | *Azospirillum irakense* |
| | *Actinomadura umbrina* | *sendaiensis* | | |
| **Acidobacterium** | *Actinomadura verrucosospora* | *Alicyclobacillus vulcanalis* | **Aquabacterium** | **Azotobacter** |
| *Acidobacterium capsulatum* | | | *Aquabacterium commune* | *Azotobacter beijerinckii* |
| | *Actinomadura vinacea* | **Alishewanella** | *Aquabacterium parvum* | *Azotobacter chroococcum* |
| | *Actinomadura viridilutea* | *Alishewanella fetalis* | | *Azotobacter nigricans* |
| | *Actinomadura viridis* | **Alkalibacillus** | | *Azotobacter salinestris* |
| | *Actinomadura yumaensis* | *Alkalibacillus haloalkaliphilus* | | *Azotobacter vinelandii* |
| | | | | |
| **Bacillus** | **Bacteroides** | **Bibersteinia** | **Borrelia** | **Brevinema** |
| [see below] | *Bacteroides caccae* | *Bibersteinia trehalosi* | *Borrelia afzelii* | *Brevinema andersonii* |
| | *Bacteroides coagulans* | | *Borrelia americana* | |
| | *Bacteroides eggerthii* | **Bifidobacterium** | *Borrelia burgdorferi* | **Brevundimonas** |
| **Bacteriovorax** | *Bacteroides fragilis* | *Bifidobacterium adolescentis* | *Borrelia carolinensis* | *Brevundimonas alba* |
| *Bacteriovorax stolpii* | *Bacteroides galacturonicus* | *Bifidobacterium angulatum* | *Borrelia coriaceae* | *Brevundimonas aurantiaca* |
| | *Bacteroides helcogenes* | *Bifidobacterium animalis* | *Borrelia garinii* | *Brevundimonas diminuta* |
| | *Bacteroides ovatus* | *Bifidobacterium asteroides* | *Borrelia japonica* | *Brevundimonas intermedia* |
| | *Bacteroides pectinophilus* | *Bifidobacterium bifidum* | | *Brevundimonas subvibrioides* |
| | *Bacteroides pyogenes* | *Bifidobacterium boum* | | *Brevundimonas vancanneytii* |
| | *Bacteroides salyersiae* | *Bifidobacterium breve* | | |
| *Bacteroides stercoris* | *Bifidobacterium catenulatum* | | | *Brevundimonas variabilis* |
| *Bacteroides suis* | *Bifidobacterium choerinum* | **Bosea** | | *Brevundimonas vesicularis* |
| *Bacteroides tectus* | *Bifidobacterium coryneforme* | *Bosea minatitlanensis* | | |
| *Bacteroides thetaiotaomicron* | *Bifidobacterium cuniculi* | *Bosea thiooxidans* | | **Brochothrix** |
| *Bacteroides uniformis* | *Bifidobacterium dentium* | | | *Brochothrix campestris* |
| *Bacteroides ureolyticus* | *Bifidobacterium gallicum* | **Brachybacterium** | | *Brochothrix thermosphacta* |
| *Bacteroides vulgatus* | *Bifidobacterium gallinarum* | *Brachybacterium alimentarium* | | |
| | *Bifidobacterium indicum* | | | **Brucella** |
| **Balnearium** | *Bifidobacterium longum* | *Brachybacterium faecium* | | *Brucella canis* |
| *Balnearium lithotrophicum* | *Bifidobacterium magnumBifidobacterium merycicum* | *Brachybacterium paraconglomeratum* | | *Brucella neotomae* |
| | | *Brachybacterium rhamnosum* | | **Bryobacter** |
| **Balneatrix** | | | | *Bryobacter aggregatus* |
| *Balneatrix alpica* | *Bifidobacterium minimum* | *Brachybacterium tyrofermentans* | | |
| **Balneola** | *Bifidobacterium pseudocatenulatum* | | | **Burkholderia** |
| *Balneola vulgaris* | | **Brachyspira** | | *Burkholderia ambifaria* |
| | *Bifidobacterium pseudolongum* | *Brachyspira alvinipulli* | | *Burkholderia andropogonis* |
| **Barnesiella** | | *Brachyspira hyodysenteriae* | | *Burkholderia anthina* |
| *Barnesiella viscericola* | *Bifidobacterium pullorum* | *Brachyspira innocens* | | *Burkholderia caledonica* |
| | *Bifidobacterium ruminantium* | *Brachyspira murdochii* | | *Burkholderia caryophylli* |
| **Bartonella** | *Bifidobacterium saeculare* | *Brachyspira pilosicoli* | | *Burkholderia cenocepacia* |
| *Bartonella alsatica* | *Bifidobacterium subtile* | | | *Burkholderia cepacia* |
| *Bartonella bacilliformis* | *Bifidobacterium thermophilum* | | *Burkholderia cocovenenans* | |
| *Bartonella clarridgeiae* | | | *Burkholderia dolosa* | |
| *Bartonella doshiae* | | **Bradyrhizobium** | *Burkholderia fungorum* | |
| *Bartonella elizabethae* | **Bilophila** | | *Burkholderia glathei* | |
| *Bartonella grahamii* | *Bilophila wadsworthia* | *Bradyrhizobium canariense elkanii* | *Burkholderia glumae* | |
| *Bartonella henselae* | **Biostraticola** | *Bradyrhizobium* | *Burkholderia graminis* | |
| *Bartonella rochalimae* | *Biostraticola tofi* | *Bradyrhizobium japonicum* | *Burkholderia kururiensis* | |
| *Bartonella vinsonii* | | *Bradyrhizobium liaoningense* | *Burkholderia multivorans* | |
| | **Bizionia** | **Brenneria** | *Burkholderia phenazinium* | |
| **Bavariicoccus** | *Bizionia argentinensis* | *Brenneria alni* | *Burkholderia plantarii* | |
| *Bavariicoccus seileri* | | *Brenneria nigrifluens* | *Burkholderia pyrrocinia* | |
| **Bdellovibrio** | **Blastobacter** | *Brenneria quercina* | *Burkholderia silvatlantica* | |
| | *Blastobacter capsulatus* | *Brenneria quercina* | *Burkholderia stabilis* | |
| *Bdellovibrio bacteriovorus* | *Blastobacter denitrificans* | *Brenneria salicis* | *Burkholderia thailandensis* | |
| *Bdellovibrio exovorus* | | | *Burkholderia tropica* | |
| **Beggiatoa** | **Blastococcus** | **Brevibacillus** | *Burkholderia unamae* | |
| *Beggiatoa alba* | *Blastococcus aggregatus* | *Brevibacillus agri* | *Burkholderia vietnamiensis* | |
| | *Blastococcus saxobsidens* | *Brevibacillus borstelensis* | | |
| **Beijerinckia** | | *Brevibacillus brevis* | **Buttiauxella** | |
| *Beijerinckia derxii* | **Blastochloris** | *Brevibacillus centrosporus* | *Buttiauxella agrestis* | |
| *Beijerinckia fluminensis* | *Blastochloris viridis* | *Brevibacillus choshinensis* | *Buttiauxella brennerae* | |
| *Beijerinckia indica* | | *Brevibacillus invocatus* | *Buttiauxella ferragutiae* | |
| *Beijerinckia mobilis* | **Blastomonas** | *Brevibacillus laterosporus* | *Buttiauxella gaviniae* | |
| | *Blastomonas natatoria* | *Brevibacillus parabrevis* | *Buttiauxella izardii* | |
| **Belliella** | | *Brevibacillus reuszeri* | *Buttiauxella noackiae* | |
| *Belliella baltica* | **Blastopirellula** | | *Buttiauxella warmboldiae* | |
| | *Blastopirellula marina* | **Brevibacterium** | | |
| **Bellilinea** | | *Brevibacterium abidum* | **Butyrivibrio** | |
| *Bellilinea caldifistulae* | **Blautia** | *Brevibacterium album* | *Butyrivibrio fibrisolvens* | |
| | *Blautia coccoides* | *Brevibacterium aurantiacum* | *Butyrivibrio hungatei* | |
| **Belnapia** | *Blautia hansenii* | *Brevibacterium celere* | *Butyrivibrio proteoclasticus* | |
| *Belnapia moabensis* | *Blautia producta* | *Brevibacterium epidermidis* | | |
| **Bergeriella** | *Blautia wexlerae* | *Brevibacterium frigoritolerans* | | |
| *Bergeriella denitrificans* | **Bogoriella** | | | |
| | *Bogoriella caseilytica* | *Brevibacterium halotolerans* | | |
| **Beutenbergia** | | *Brevibacterium iodinum* | | |
| *Beutenbergia cavernae* | **Bordetella** | *Brevibacterium linens* | | |
| | *Bordetella avium* | *Brevibacterium lyticum* | | |
| | *Bordetella bronchiseptica* | *Brevibacterium mcbrellneri* | | |
| | *Bordetella hinzii* | *Brevibacterium otitidis* | | |
| | *Bordetella holmesii* | *Brevibacterium oxydans* | | |
| | *Bordetella parapertussis* | | | |
| | | *Bordetella pertussis* | *Brevibacterium paucivorans* | |
| | | *Bordetella petrii* | *Brevibacterium stationis* | |
| | | *Bordetella trematum* | | |
| **Bacillus** | | | | |
| *B. acidiceler* | *B. aminovorans* | *B. glucanolyticus* | *B. taeanensis* | *B. lautus* |
| *B. acidicola* | *B. amylolyticus* | *B. gordonae* | *B. tequilensis* | *B. lehensis* |
| *B. acidiproducens* | *B. andreesenii* | *B. gottheilii* | *B. thermantarcticus* | *B. lentimorbus* |
| *B. acidocaldarius* | *B. aneurinilyticus* | *B. graminis* | *B. thermoaerophilus* | *B. lentus* |
| *B. acidoterrestris* | *B. anthracis* | *B. halmapalus* | *B. thermoamylovorans* | *B. licheniformis* |
| *B. aeolius* | *B. aquimaris* | *B. haloalkaliphilus* | *B. thermocatenulatus* | *B. ligniniphilus* |
| *B. aerius* | *B. arenosi* | *B. halochares* | *B. thermocloacae* | *B. litoralis* |
| *B. aerophilus* | *B. arseniciselenatis* | *B. halodenitrificans* | *B. thermocopriae* | *B. locisalis* |
| *B. agaradhaerens* | *B. arsenicus* | *B. halodurans* | *B. thermodenitrificans* | *B. luciferensis* |
| *B. agri* | *B. aurantiacus* | *B. halophilus* | *B. thermoglucosidasius* | *B. luteolus* |
| *B. aidingensis* | *B. arvi* | *B. halosaccharovorans* | *B. thermolactis* | *B. luteus* |
| *B. akibai* | *B. aryabhattai* | *B. hemicellulosilyticus* | *B. thermoleovorans* | *B. macauensis* |
| *B. alcalophilus* | *B. asahii* | *B. hemicentroti* | *B. thermophilus* | *B. macerans* |
| *B. algicola* | *B. atrophaeus* | *B. herbersteinensis* | *B. thermoruber* | *B. macquariensis* |
| *B. alginolyticus* | *B. axarquiensis* | *B. horikoshii* | *B. thermosphaericus* | *B. macyae* |
| *B. alkalidiazotrophicus* | *B. azotofixans* | *B. horneckiae* | *B. thiaminolyticus* | *B. malacitensis* |
| *B. alkalinitrilicus* | *B. azotoformans* | *B. horti* | *B. thioparans* | *B. mannanilyticus* |
| *B. alkalisediminis* | *B. badius* | *B. huizhouensis* | *B. thuringiensis* | *B. marisflavi* |
| *B. alkalitelluris* | *B. barbaricus* | *B. humi* | *B. tianshenii* | *B. marismortui* |
| *B. altitudinis* | *B. bataviensis* | *B. hwajinpoensis* | *B. trypoxylicola* | *B. marmarensis* |
| *B. alveayuensis* | *B. beijingensis* | *B. idriensis* | *B. tusciae* | *B. massiliensis* |
| *B. alvei* | *B. benzoevorans* | *B. indicus* | *B. validus* | *B. megaterium* |
| *B. amyloliquefaciens* | *B. beringensis* | *B. infantis* | *B. vallismortis* | *B. mesonae* |
| | *B. berkeleyi* | *B. infernus* | *B. vedderi* | *B. methanolicus* |
| • *B.* | *B. beveridgei* | *B. insolitus* | *B. velezensis* | *B. methylotrophicus* |
| *a.* subsp. *amyloliquefaciens* | *B. bogoriensis* | *B. invictae* | *B. vietnamensis* | *B. migulanus* |
| • *B. a.* subsp. *plantarum* | *B. boroniphilus* | *B. iranensis* | *B. vireti* | *B. mojavensis* |
| | *B. borstelensis* | *B. isabeliae* | *B. vulcani* | *B. mucilaginosus* |
| *B. dipsosauri* | *B. brevis Migula* | *B. isronensis* | *B. wakoensis* | *B. muralis* |
| | *B. butanolivorans* | *B. jeotgali* | *B. weihenstephanensis* | *B. murimartini* |
| *B. drentensis* | *B. canaveralius* | *B. kaustophilus* | *B. xiamenensis* | *B. mycoides* |
| *B. edaphicus* | | | | |
| *B. ehimensis* | *B. carboniphilus* | *B. kobensis* | *B. xiaoxiensis* | *B. naganoensis* |
| *B. eiseniae* | *B. cecembensis* | *B. kochii* | *B. zhanjiangensis* | *B. nanhaiensis* |
| *B. enclensis* | *B. cellulosilyticus* | *B. kokeshiiformis* | | *B. nanhaiisediminis* |
| *B. endophyticus* | *B. centrosporus* | *B. koreensis* | *B. peoriae* | *B. nealsonii* |
| *B. endoradicis* | *B. cereus* | *B. korlensis* | *B. persepolensis* | *B. neidei* |
| *B. farraginis* | *B. chagannorensis* | *B. kribbensis* | *B. persicus* | *B. neizhouensis* |
| *B. fastidiosus* | *B. chitinolyticus* | *B. krulwichiae* | *B. pervagus* | *B. niabensis* |
| *B. fengqiuensis* | *B. chondroitinus* | *B. laevolacticus* | *B. plakortidis* | *B. niacini* |
| *B. firmus* | *B. choshinensis* | *B. larvae* | *B. pocheonensis* | *B. novalis* |
| *B. flexus* | *B. chungangensis* | *B. laterosporus* | *B. polygoni* | *B. oceanisediminis* |
| *B. foraminis* | *B. cibi* | *B. salexigens* | *B. polymyxa* | *B. odysseyi* |
| *B. fordii* | *B. circulans* | *B. saliphilus* | *B. popilliae* | *B. okhensis* |
| *B. formosus* | *B. clarkii* | *B. schlegelii* | *B. pseudalcalophilus* | *B. okuhidensis* |
| *B. fortis* | *B. clausii* | *B. sediminis* | *B. pseudofirmus* | *B. oleronius* |
| *B. fumarioli* | *B. coagulans* | *B. selenatarsenatis* | *B. pseudomycoides* | *B. oryzaecorticis* |
| *B. funiculus* | *B. coahuilensis* | *B. selenitireducens* | *B. psychrodurans* | *B. oshimensis* |
| *B. fusiformis* | *B. cohnii* | *B. seohaeanensis* | *B. psychrophilus* | *B. pabuli* |
| *B. galactophilus* | *B. composti* | *B. shacheensis* | *B. psychrosaccharolyticus* | *B. pakistanensis* |
| *B. galactosidilyticus* | *B. curdlanolyticus* | *B. shackletonii* | *B. psychrotolerans* | *B. pallidus* |
| *B. galliciensis* | *B. cycloheptanicus* | *B. siamensis* | *B. pulvifaciens* | *B. pallidus* |
| *B. gelatini* | *B. cytotoxicus* | *B. silvestris* | *B. pumilus* | *B. panacisoli* |
| *B. gibsonii* | *B. daliensis* | *B. simplex* | *B. purgationiresistens* | *B. panaciterrae* |
| *B. ginsengi* | *B. decisifrandis* | *B. siralis* | *B. pycnus* | *B. pantothenticus* |
| *B. ginsengihumi* | *B. decolorationis* | *B. smithii* | *B. qingdaonensis* | *B. parabrevis* |
| *B. ginsengisoli* | *B. deserti* | *B. soli* | *B. qingshengii* | *B. paraflexus* |
| *B. globisporus (eg, B.* | | *B. solimangrovi* | *B. reuszeri* | *B. pasteurii* |
| g. subsp. *Globisporus;* or *B.* | | *B. solisalsi* | *B. rhizosphaerae* | *B. patagoniensis* |
| g. subsp. *Marinus)* | | *B. songklensis* | *B. rigui* | |
| | | *B. sonorensis* | *B. ruris* | |
| | | *B. sphaericus* | *B. safensis* | |
| | | *B. sporothermodurans* | *B. salarius* | |
| | | *B. stearothermophilus* | | |
| | | *B. stratosphericus* | | |
| | | *B. subterraneus* | | |
| | | *B. subtilis (eg, B. s.* subsp. *Inaquosorum;* or *B.* s. subsp. *Spizizeni;* or *B. s.* subsp. *Subtilis)* | | |
| | | | | |
| | | | | |
| | | | | |
| **Caenimonas** | **Campylobacter** | **Cardiobacterium** | **Catenuloplanes** | **Curtobacterium** |
| *Caenimonas koreensis* | *Campylobacter coli* | *Cardiobacterium hominis* | *Catenuloplanes atrovinosus* | *Curtobacterium albidum* |
| | *Campylobacter concisus* | | *Catenuloplanes castaneus* | |
| **Caldalkalibacillus** | *Campylobacter curvus* | **Carnimonas** | *Catenuloplanes crispus* | *Curtobacterium citreus* |
| *Caldalkalibacillus uzonensis* | *Campylobacter fetus* | *Carnimonas nigrificans* | *Catenuloplanes indicus* | |
| **Caldanaerobacter** | *Campylobacter gracilis* | ***Carnobacterium*** | *Catenuloplanes japonicus* | |
| *Caldanaerobacter subterraneus* | *Campylobacter helveticus* | | *Catenuloplanes nepalensis* | |
| | *Campylobacter hominis* | *Carnobacterium alterfunditum* | *Catenuloplanes niger* | |
| | *Campylobacter hyointestinalis* | | | |
| | *Campylobacter jejuni* | *Carnobacterium divergens* | | |
| **Caldanaerobius** | *Campylobacter lari* | *Carnobacterium funditum* | **Chryseobacterium** | |
| *Caldanaerobius fijiensis* | *Campylobacter mucosalis* | *Carnobacterium gallinarum* | *Chryseobacterium balustinum* | |
| *Caldanaerobius polysaccharolyticus* | *Campylobacter rectus* | *Carnobacterium maltaromaticum* | | |
| | *Campylobacter showae* | | | |
| *Caldanaerobius zeae* | | *Carnobacterium mobile* | **Citrobacter** | |
| | *Campylobacter sputorum* | | *C**.** amalonaticus* | |
| **Caldanaerovirga** | *Campylobacter upsaliensis* | *Carnobacterium viridans* | *C. braakii* | |
| *Caldanaerovirga acetigignens* | **Capnocytophaga** | **Caryophanon** | *C. diversus* | |
| **Caldicellulosiruptor** | *Capnocytophaga canimorsus* | *Caryophanon latum* | *C. farmeri* | |
| *Caldicellulosiruptor bescii* | *Capnocytophaga cynodegmi* | *Caryophanon tenue* | *C. freundii* | |
| *Caldicellulosiruptor kristjanssonii* | *Capnocytophaga gingivalis* | | *C. gillenii* | |
| *Caldicellulosiruptor owensensis* | *Capnocytophaga granulosa* | **Catellatospora** | *C. koseri* | |
| | *Capnocytophaga haemolytica* | *Catellatospora citrea* | *C. murliniae* | |
| | *Capnocytophaga ochracea* | *Catellatospora methionotrophica* | *C. pasteurii*^{[1]} | |
| | *Capnocytophaga sputigena* | | *C. rodentium* | |
| | | | *C. sedlakii* | |
| | | **Catenococcus** | *C. werkmanii* | |
| | | *Catenococcus thiocycli* | *C. youngae* | |
| | | | **Clostridium** (see below) | |
| | **Coccochloris** | | | |
| | *Coccochloris elabens* | | | |
| | **Corynebacterium** | | | |
| | *Corynebacterium flavescens* | | | |
| | *Corynebacterium variabile* | | | |
| **Clostridium** | | | | |
| *Clostridium absonum, Clostridium aceticum, Clostridium acetireducens, Clostridium acetobutylicum, Clostridium acidisoli, Clostridium aciditolerans, Clostridium acidurici, Clostridium aerotolerans, Clostridium aestuarii, Clostridium akagii, Clostridium aldenense, Clostridium aldrichii, Clostridium algidicarni, Clostridium algidixylanolyticum, Clostridium algifaecis, Clostridium algoriphilum, Clostridium alkalicellulosi, Clostridium aminophilum, Clostridium aminovalericum, Clostridium amygdalinum, Clostridium amylolyticum, Clostridium arbusti, Clostridium arcticum, Clostridium argentinense, Clostridium asparagiforme, Clostridium aurantibutyricum, Clostridium autoethanogenum, Clostridium baratii, Clostridium barkeri, Clostridium bartlettii, Clostridium beijerinckii, Clostridium bifermentans, Clostridium bolteae, Clostridium bornimense, Clostridium botulinum, Clostridium bowmanii, Clostridium bryantii, Clostridium butyricum, Clostridium cadaveris, Clostridium caenicola, Clostridium caminithermale, Clostridium carboxidivorans, Clostridium carnis, Clostridium cavendishii, Clostridium celatum, Clostridium celerecrescens, Clostridium cellobioparum, Clostridium cellulofermentans, Clostridium cellulolyticum, Clostridium cellulosi, Clostridium cellulovorans, Clostridium chartatabidum, Clostridium chauvoei, Clostridium chromiireducens, Clostridium citroniae, Clostridium clariflavum, Clostridium clostridioforme, Clostridium coccoides, Clostridium cochlearium, Clostridium colletant, Clostridium colicanis, Clostridium colinum, Clostridium collagenovorans, Clostridium cylindrosporum, Clostridium difficile, Clostridium diolis, Clostridium disporicum, Clostridium drakei, Clostridium durum, Clostridium estertheticum, Clostridium estertheticum estertheticum, Clostridium estertheticum laramiense, Clostridium fallax, Clostridium felsineum, Clostridium fervidum, Clostridium fimetarium, Clostridium formicaceticum, Clostridium frigidicarnis, Clostridium* | | | | |
| *frigoris, Clostridium ganghwense, Clostridium gasigenes, Clostridium ghonii, Clostridium glycolicum, Clostridium glycyrrhizinilyticum, Clostridium grantii Clostridium haemolyticum, Clostridium halophilum, Clostridium hastiforme, Clostridium hathewayi, Clostridium herbivorans, Clostridium hiranonis, Clostridium histolyticum, Clostridium homopropionicum, Clostridium huakuii, Clostridium hungatei, Clostridium hydrogeniformans, Clostridium hydroxybenzoicum, Clostridium hylemonae, Clostridium jejuense, Clostridium indolis, Clostridium innocuum, Clostridium intestinale, Clostridium irregulare, Clostridium isatidis, Clostridium josui, Clostridium kluyveri, Clostridium lactatifermentans, Clostridium lacusfryxellense, Clostridium laramiense, Clostridium lavalense, Clostridium lentocellum, Clostridium lentoputrescens, Clostridium leptum, Clostridium limosum, Clostridium litorale, Clostridium lituseburense, Clostridium ljungdahlii, Clostridium lortetii, Clostridium lundense, Clostridium magnum, Clostridium malenominatum, Clostridium mangenotii, Clostridium mayombei, Clostridium methoxybenzovorans, Clostridium methylpentosum, Clostridium neopropionicum, Clostridium nexile, Clostridium nitrophenolicum, Clostridium novyi, Clostridium oceanicum, Clostridium orbiscindens, Clostridium oroticum, Clostridium oxalicum, Clostridium papyrosolvens, Clostridium paradoxum, Clostridium paraperfringens (Alias: C. welchii), Clostridium paraputrificum, Clostridium pascui, Clostridium pasteurianum, Clostridium peptidivorans, Clostridium perenne, Clostridium perfringens, Clostridium pfennigii, Clostridium phytofermentans, Clostridium piliforme, Clostridium polysaccharolyticum, Clostridium populeti, Clostridium propionicum, Clostridium proteoclasticum, Clostridium proteolyticum, Clostridium psychrophilum, Clostridium puniceum, Clostridium purinilyticum, Clostridium putrefaciens, Clostridium putrificum, Clostridium quercicolum, Clostridium quinii, Clostridium ramosum, Clostridium rectum, Clostridium roseum, Clostridium saccharobutylicum, Clostridium saccharogumia, Clostridium saccharolyticum, Clostridium saccharoperbutylacetonicum, Clostridium sardiniense, Clostridium sartagoforme, Clostridium scatologenes, Clostridium schirmacherense, Clostridium scindens, Clostridium septicum, Clostridium sordellii, Clostridium sphenoides, Clostridium spiroforme, Clostridium sporogenes, Clostridium sporosphaeroides, Clostridium stercorarium, Clostridium stercorarium leptospartum, Clostridium stercorarium stercorarium, Clostridium stercorarium thermolacticum, Clostridium sticklandii, Clostridium straminisolvens, Clostridium subterminale, Clostridium sufflavum, Clostridium sulfidigenes, Clostridium symbiosum, Clostridium tagluense, Clostridium tepidiprofundi, Clostridium termitidis, Clostridium tertium, Clostridium tetani, Clostridium tetanomorphum, Clostridium thermaceticum, Clostridium thermautotrophicum, Clostridium thermoalcaliphilum, Clostridium thermobutyricum, Clostridium thermocellum, Clostridium thermocopriae, Clostridium thermohydrosulfuricum, Clostridium thermolacticum, Clostridium thermopalmarium, Clostridium* | | | | |
| *thermopapyrolyticum, Clostridium thermosaccharolyticum, Clostridium thermosuccinogenes, Clostridium thermosulfurigenes, Clostridium thiosulfatireducens, Clostridium tyrobutyricum, Clostridium uliginosum, Clostridium ultunense, Clostridium villosum, Clostridium vincentii, Clostridium viride, Clostridium xylanolyticum, Clostridium xylanovorans* | | | | |
| **Dactylosporangium** | **Deinococcus** | **Delftia** | **Echinicola** | |
| *Dactylosporangium aurantiacum* | *Deinococcus aerius* | *Delftia acidovorans* | *Echinicola pacifica* | |
| *Dactylosporangium fulvum* | *Deinococcus apachensis* | **Desulfovibrio** | *Echinicola vietnamensis* | |
| *Dactylosporangium matsuzakiense* | *Deinococcus aquaticus* | *Desulfovibrio desulfuricans* | | |
| *Dactylosporangium roseum* | *Deinococcus aquatilis* | **Diplococcus** | | |
| *Dactylosporangium thailandense* | *Deinococcus caeni* | *Diplococcus pneumoniae* | | |
| *Dactylosporangium vinaceum* | *Deinococcus radiodurans* | | | |
| | *Deinococcus radiophilus* | | | |
| **Enterobacter** | *Enterobacter kobei* | **Faecalibacterium** | **Flavobacterium** | |
| *E. aerogenes* | *E. ludwigii* | *Faecalibacterium prausnitzii* | *Flavobacterium antarcticum* | |
| *E. amnigenus* | *E. mori* | **Fangia** | *Flavobacterium aquatile* | |
| *E. agglomerans* | *E. nimipressuralis* | *Fangia hongkongensis* | *Flavobacterium aquidurense* | |
| *E. arachidis* | *E. oryzae* | **Fastidiosipila** | | |
| *E. asburiae* | *E. pulveris* | *Fastidiosipila sanguinis* | *Flavobacterium balustinum* | |
| *E. cancerogenous* | *E. pyrinus* | **Fusobacterium** | *Flavobacterium croceum* | |
| *E. cloacae* | *E. radicincitans* | *Fusobacterium nucleatum* | *Flavobacterium cucumis* | |
| *E. cowanii* | *E. taylorae* | | *Flavobacterium* | |
| *E. dissolvens* | *E. turicensis* | | *daejeonense* | |
| *E. gergoviae* | *E. sakazakii Enterobacter soli* | | *Flavobacterium defluvii* | |
| *E. helveticus* | **Enterococcus** | | *Flavobacterium degerlachei* | |
| *E. hormaechei* | *Enterococcus durans* | | *Flavobacterium denitrificans* | |
| *E. intermedius* | *Enterococcus faecalis* | | | |
| | *Enterococcus faecium* | | *Flavobacterium filum* | |
| | **Erwinia** | | *Flavobacterium flevense* | |
| | *Erwinia hapontici* | | *Flavobacterium frigidarium* | |
| | **Escherichia** | | *Flavobacterium mizutaii* | |
| | *Escherichia coli* | | *Flavobacterium okeanokoites* | |
| **Gaetbulibacter** | **Haemophilus** | **Ideonella** | **Janibacter** | |
| *Gaetbulibacter saemankumensis* | *Haemophilus aegyptius* | *Ideonella azotifigens* | *Janibacter anophelis* | |
| **Gallibacterium** | *Haemophilus aphrophilus* | **Idiomarina** | *Janibacter corallicola* | |
| *Gallibacterium anatis* | *Haemophilus felis* | *Idiomarina abyssalis* | *Janibacter limosus* | |
| **Gallicola** | *Haemophilus gallinarum* | *Idiomarina baltica* | *Janibacter melonis* | |
| *Gallicola barnesae* | *Haemophilus haemolyticus* | *Idiomarina fontislapidosi* | *Janibacter terrae* | |
| **Garciella** | *Haemophilus influenzae* | *Idiomarina loihiensis* | **Jannaschia** | |
| *Garciella nitratireducens* | *Haemophilus paracuniculus* | *Idiomarina ramblicola* | *Jannaschia cystaugens* | |
| **Geobacillus** | *Haemophilus parahaemolyticus* | *Idiomarina seosinensis* | *Jannaschia helgolandensis* | |
| *Geobacillus thermoglucosidasius* | *Haemophilus parainfluenzae* | *Idiomarina zobellii* | *Jannaschia pohangensis* | |
| *Geobacillus stearothermophilus* | *Haemophilus paraphrohaemolyticus* | **Ignatzschineria** | *Jannaschia rubra* | |
| **Geobacter** | | *Ignatzschineria larvae* | | |
| *Geobacter bemidjiensis* | *Haemophilus parasuis* | | **Janthinobacterium** | |
| *Geobacter bremensis* | *Haemophilus pittmaniae* | **Ignavigranum** | *Janthinobacterium agaricidamnosum* | |
| *Geobacter chapellei* | **Hafnia** | *Ignavigranum ruoffiae* | | |
| *Geobacter grbiciae* | *Hafnia alvei* | **Ilumatobacter** | *Janthinobacterium lividum* | |
| *Geobacter hydrogenophilus* | **Hahella** | *Ilumatobacter fluminis* | **Jejuia** | |
| *Geobacter lovleyi* | *Hahella ganghwensis* | **Ilyobacter** | *Jejuia pallidilutea* | |
| *Geobacter metallireducens* | **Halalkalibacillus** | *Ilyobacter delafieldii* | **Jeotgalibacillus** | |
| *Geobacter pelophilus* | *Halalkalibacillus halophilus* | *Ilyobacter insuetus* | *Jeotgalibacillus alimentarius* | |
| *Geobacter pickeringii* | **Helicobacter** | *Ilyobacter polytropus* | | |
| *Geobacter sulfurreducens* | *Helicobacter pylori* | *Ilyobacter tartaricus* | **Jeotgalicoccus** | |
| **Geodermatophilus** | | | *Jeotgalicoccus halotolerans* | |
| *Geodermatophilus obscurus* | | | | |
| **Gluconacetobacter** | | | | |
| *Gluconacetobacter xylinus* | | | | |
| **Gordonia** | | | | |
| *Gordonia rubripertincta* | | | | |
| **Kaistia** | **Labedella** | *Listeria ivanovii* | **Micrococcus** | **Nesterenkonia** |
| *Kaistia adipata* | *Labedella gwakjiensis* | *L. marthii* | *Micrococcus luteus* | *Nesterenkonia holobia* |
| *Kaistia soli* | **Labrenzia** | *L. monocytogenes* | *Micrococcus lylae* | **Nocardia** |
| **Kangiella** | *Labrenzia aggregata* | *L. newyorkensis* | **Moraxella** | *Nocardia argentinensis* |
| *Kangiella aquimarina* | *Labrenzia alba* | *L. riparia* | *Moraxella bovis* | *Nocardia corallina* |
| *Kangiella koreensis* | *Labrenzia alexandrii* | *L. rocourtiae* | *Moraxella nonliquefaciens* | *Nocardia otitidiscaviarum* |
| | *Labrenzia marina* | *L. seeligeri* | *Moraxella osloensis* | |
| **Kerstersia** | **Labrys** | *L. weihenstephanensis* | **Nakamurella** | |
| *Kerstersia gyiorum* | *Labrys methylaminiphilus* | *L. welshimeri* | *Nakamurella multipartita* | |
| **Kiloniella** | *Labrys miyagiensis* | **Listonella** | **Nannocystis** | |
| *Kiloniella laminariae* | *Labrys monachus* | *Listonella anguillarum* | *Nannocystis pusilla* | |
| **Klebsiella** | *Labrys okinawensis* | **Macrococcus** | **Natranaerobius** | |
| *K. granulomatis* | *Labrys portucalensis* | *Macrococcus bovicus* | *Natranaerobius thermophilus* | |
| *K oxytoca* | | **Marinobacter** | | |
| *K. pneumoniae* | **Lactobacillus** | *Marinobacter algicola* | *Natranaerobius trueperi* | |
| *K. terrigena* | **[see below]** | *Marinobacter bryozoorum* | **Naxibacter** | |
| *K. variicola* | **Laceyella** | *Marinobacter flavimaris* | *Naxibacter alkalitolerans* | |
| **Kluyvera** | *Laceyella putida* | **Meiothermus** | **Neisseria** | |
| *Kluyvera ascorbata* | **Lechevalieria** | *Meiothermus ruber* | *Neisseria cinerea* | |
| | *Lechevalieria aerocolonigenes* | | *Neisseria denitrificans* | |
| | **Legionella** | | *Neisseria gonorrhoeae* | |
| **Kocuria** | [see below] | **Methylophilus** | *Neisseria lactamica* | |
| *Kocuria roasea* | **Listeria** | *Methylophilus methylotrophus* | *Neisseria mucosa* | |
| *Kocuria varians* | *L. aquatica* | | *Neisseria sicca* | |
| **Kurthia** | *L. booriae* | **Microbacterium** | *Neisseria subflava* | |
| *Kurthia zopfii* | *L. cornellensis* | *Microbacterium ammoniaphilum* | **Neptunomonas** | |
| | *L. fleischmannii* | | *Neptunomonas japonica* | |
| | *L. floridensis* | *Microbacterium arborescens* | | |
| | *L. grandensis* | *Microbacterium liquefaciens* | | |
| | *L. grayi* | *Microbacterium oxydans* | | |
| | *L. innocua* | | | |
| ***Lactobacillus*** | | | | |
| *L. acetotolerans* | *L. catenaformis* | *L. mali* | *L. parakefiri* | *L.sakei* |
| *L. acidifarinae* | *L. ceti* | *L. manihotivorans* | *L. paralimentarius* | *L.salivarius* |
| *L. acidipiscis* | *L. coleohominis* | *L. mindensis* | *L. paraplantarum* | *L.sanfranciscensis* |
| *L. acidophilus* | *L. collinoides* | *L. mucosae* | *L. pentosus* | *L.satsumensis* |
| *Lactobacillus agilis* | *L. composti* | *L. murinus* | *L. perolens* | *L.secaliphilus* |
| *L. algidus* | *L. concavus* | *L. nagelii* | *L. plantarum* | *L.sharpeae* |
| *L. alimentarius* | *L. coryniformis* | *L. namurensis* | *L. pontis* | *L.siliginis* |
| *L. amylolyticus* | *L. crispatus* | *L. nantensis* | *L. protectus* | *L.spicheri* |
| *L. amylophilus* | *L. crustorum* | *L. oligofermentans* | *L. psittaci* | *L.suebicus* |
| *L. amylotrophicus* | *L. curvatus* | *L. oris* | *L. rennini* | *L. thailandensis* |
| *L. amylovorus* | *L. delbrueckii subsp. bulgaricus* | *L. panis* | *L. reuteri* | *L. ultunensis* |
| *L. animalis* | | *L. pantheris* | *L. rhamnosus* | *L. vaccinostercus* |
| *L. antri* | *L. delbrueckii subsp. delbrueckii* | *L. parabrevis* | *L. rimae* | *L. vaginalis* |
| *L. apodemi* | | *L. parabuchneri* | *L. rogosae* | *L. versmoldensis* |
| *L. aviarius* | *L. delbrueckii subsp. lactis* | *L. paracasei* | *L. rossiae* | *L. vini* |
| *L. bifermentans* | *L. dextrinicus* | *L. paracollinoides* | *L. ruminis* | *L. vitulinus* |
| *L. brevis* | *L. diolivorans* | *L. parafarraginis* | *L. saerimneri* | *L. zeae* |
| *L. buchneri* | *L. equi* | *L. homohiochii* | *L. jensenii* | *L. zymae* |
| *L. camelliae* | *L. equigenerosi* | *L. iners* | *L. johnsonii* | *L. gastricus* |
| *L. casei* | *L. farraginis* | *L. ingluviei* | *L. kalixensis* | *L. ghanensis* |
| *L. kitasatonis* | *L. farciminis* | *L. intestinalis* | *L. kefiranofaciens* | *L. graminis* |
| *L. kunkeei* | *L. fermentum* | *L. fuchuensis* | *L. kefiri* | *L. hammesii* |
| *L. leichmannii* | *L. fornicalis* | *L. gallinarum* | *L. kimchii* | *L. hamsteri* |
| *L. lindneri* | *L. fructivarans* | *L. gasseri* | *L. helveticus* | *L. harbinensis* |
| *L. malefermentans* | *L. frumenti* | | *L. hilgardii* | *L. hayakitensis* |
| **Legionella** | | | | |
| *Legionella adelaidensis* | *Legionella drancourtii* | *Candidatus* Legionella jeonii | *Legionella quinlivanii* | |
| *Legionella anisa* | *Legionella dresdenensis* | *Legionella jordanis* | *Legionella rowbothamii* | |
| *Legionella beliardensis* | *Legionella drozanskii* | *Legionella lansingensis* | *Legionella rubrilucens* | |
| *Legionella birminghamensis* | *Legionella dumoffii* | *Legionella londiniensis* | *Legionella sainthelensi* | |
| *Legionella bozemanae* | *Legionella erythra* | *Legionella longbeachae* | *Legionella santicrucis* | |
| *Legionella brunensis* | *Legionella fairfieldensis* | *Legionella lytica* | *Legionella shakespearei* | |
| *Legionella busanensis* | *Legionella fallonii* | *Legionella maceachernii* | *Legionella spiritensis* | |
| *Legionella cardiaca* | *Legionella feeleii* | *Legionella massiliensis* | *Legionella steelei* | |
| *Legionella cherrii* | *Legionella geestiana* | *Legionella micdadei* | *Legionella steigerwaltii* | |
| *Legionella cincinnatiensis* | *Legionella* genomospecies | *Legionella monrovica* | *Legionella taurinensis* | |
| *Legionella clemsonensis* | *Legionella gormanii* | *Legionella moravica* | *Legionella tucsonensis* | |
| *Legionella donaldsonii* | *Legionella gratiana* | *Legionella nagasakiensis* | *Legionella tunisiensis* | |
| | *Legionella gresilensis* | *Legionella nautarum* | *Legionella wadsworthii* | |
| | *Legionella hackeliae* | *Legionella norrlandica* | *Legionella waltersii* | |
| | *Legionella impletisoli* | *Legionella oakridgensis* | *Legionella worsleiensis* | |
| | *Legionella israelensis* | *Legionella parisiensis* | *Legionella yabuuchiae* | |
| | *Legionella jamestowniensis* | *Legionella pittsburghensis* | | |
| | | *Legionella pneumophila* | | |
| | | *Legionella quateirensis* | | |
| **Oceanibulbus** | **Paenibacillus** | **Prevotella** | **Quadrisphaera** | |
| *Oceanibulbus indolifex* | *Paenibacillus thiaminolyticus* | *Prevotella albensis* | *Quadrisphaera granulorum* | |
| | | *Prevotella amnii* | | |
| **Oceanicaulis** | **Pantoea** | *Prevotella bergensis* | **Quatrionicoccus** | |
| *Oceanicaulis alexandrii* | *Pantoea agglomerans* | *Prevotella bivia* | *Quatrionicoccus australiensis* | |
| **Oceanicola** | | *Prevotella brevis* | | |
| *Oceanicola batsensis* | **Paracoccus** | *Prevotella bryantii* | | |
| *Oceanicola granulosus* | *Paracoccus alcaliphilus* | *Prevotella buccae* | **Quinella** | |
| *Oceanicola nanhaiensis* | **Paucimonas** | *Prevotella buccalis* | *Quinella ovalis* | |
| **Oceanimonas** | *Paucimonas lemoignei* | *Prevotella copri* | | |
| *Oceanimonas baumannii* | **Pectobacterium** | *Prevotella dentalis* | **Ralstonia** | |
| **Oceaniserpentilla** | *Pectobacterium aroidearum* | *Prevotella denticola* | *Ralstonia eutropha* | |
| *Oceaniserpentilla haliotis* | *Pectobacterium atrosepticum* | *Prevotella disiens* | *Ralstonia insidiosa* | |
| **Oceanisphaera** | *Pectobacterium betavasculorum* | *Prevotella histicola* | *Ralstonia mannitolilytica* | |
| *Oceanisphaera donghaensis* | | *Prevotella intermedia* | *Ralstonia pickettii* | |
| *Oceanisphaera litoralis* | *Pectobacterium cacticida* | *Prevotella maculosa* | *Ralstonia pseudosolanacearum* | |
| **Oceanithermus** | *Pectobacterium carnegieana* | *Prevotella marshii* | | |
| *Oceanithermus desulfurans* | *Pectobacterium carotovorum* | *Prevotella melaninogenica* | *Ralstonia syzygii* | |
| *Oceanithermus profundus* | *Pectobacterium chrysanthemi* | *Prevotella micans* | *Ralstonia solanacearum* | |
| **Oceanobacillus** | *Pectobacterium cypripedii* | *Prevotella multiformis* | **Ramlibacter** | |
| *Oceanobacillus caeni* | *Pectobacterium rhapontici* | *Prevotella nigrescens* | *Ramlibacter henchirensis* | |
| **Oceanospirillum** | *Pectobacterium wasabiae* | *Prevotella oralis* | *Ramlibacter tataouinensis* | |
| *Oceanospirillum linum* | **Planococcus** | *Prevotella oris* | | |
| | *Planococcus citreus* | *Prevotella oulorum* | | |
| **Planomicrobium** | *Prevotella pallens* | **Raoultella** | | |
| *Planomicrobium okeanokoites* | *Prevotella salivae* | *Raoultella ornithinolytica* | | |
| **Plesiomonas** | *Prevotella stercorea* | *Raoultella planticola* | | |
| *Plesiomonas shigelloides* | *Prevotella tannerae* | *Raoultella terrigena* | | |
| **Proteus** | *Prevotella timonensis* | **Rathayibacter** | | |
| *Proteus vulgaris* | *Prevotella veroralis* | *Rathayibacter caricis* | | |
| | **Providencia** | *Rathayibacter festucae* | | |
| | *Providencia stuartii* | *Rathayibacter iranicus* | | |
| | **Pseudomonas** | *Rathayibacter rathayi* | | |
| | *Pseudomonas aeruginosa* | *Rathayibacter toxicus* | | |
| | *Pseudomonas alcaligenes* | *Rathayibacter tritici* | | |
| | *Pseudomonas anguillispetica* | **Rhodobacter** | | |
| | *Pseudomonas fluorescens* | *Rhodobacter sphaeroides* | | |
| | *Pseudoalteromonas* | **Ruegeria** | | |
| | *haloplanktis* | *Ruegeria gelatinovorans* | | |
| | *Pseudomonas mendocina* | | | |
| | *Pseudomonas pseudoalcaligenes* | | | |
| | *Pseudomonas putida* | | | |
| | *Pseudomonas tutzeri* | | | |
| | *Pseudomonas syringae* | | | |
| | | **Psychrobacter** | | |
| | | *Psychrobacter faecalis* | | |
| | | *Psychrobacter phenylpyruvicus* | | |
| | | | | |
| **Saccharococcus** | **Sagittula** | **Sanguibacter** | **Stenotrophomonas** | **Tatlockia** |
| *Saccharococcus thermophilus* | *Sagittula stellata* | *Sanguibacter keddieii* | *Stenotrophomonas maltophilia* | *Tatlockia maceachernii* |
| | | *Sanguibacter suarezii* | | *Tatlockia micdadei* |
| **Saccharomonospora** | **Salegentibacter** | | **Streptococcus** | **Tenacibaculum** |
| *Saccharomonospora azurea* | *Salegentibacter salegens* | **Saprospira** | | *Tenacibaculum amylolyticum* |
| *Saccharomonospora cyanea* | **Salimicrobium** | *Saprospira grandis* | [also see below] | |
| *Saccharomonospora viridis* | *Salimicrobium album* | **Sarcina** | | *Tenacibaculum discolor* |
| | | | **Streptomyces** | *Tenacibaculum gallaicum* |
| **Saccharophagus** | **Salinibacter** | *Sarcina maxima* | *Streptomyces achromogenes* | |
| *Saccharophagus degradans* | *Salinibacter ruber* | *Sarcina ventriculi* | | *Tenacibaculum lutimaris* |
| **Saccharopolyspora** | | **Sebaldella** | *Streptomyces cesalbus* | |
| *Saccharopolyspora erythraea* | **Salinicoccus** | *Sebaldella termitidis* | *Streptomyces cescaepitosus* | *Tenacibaculum mesophilum* |
| *Saccharopolyspora gregorii* | *Salinicoccus alkaliphilus* | | *Streptomyces cesdiastaticus* | |
| | *Salinicoccus hispanicus* | | *Streptomyces cesexfoliatus* | *Tenacibaculum skagerrakense* |
| *Saccharopolyspora hirsuta* | *Salinicoccus roseus* | | *Streptomyces fimbriatus* | |
| *Saccharopolyspora hordei* | | | *Streptomyces fradiae* | |
| *Saccharopolyspora rectivirgula* | | | *Streptomyces fulvissimus* | **Tepidanaerobacter** |
| *Saccharopolyspora spinosa* | **Salinispora** | **Serratia** | *Streptomyces griseoruber* | *Tepidanaerobacter syntrophicus* |
| *Saccharopolyspora taberi* | *Salinispora arenicola* | *Serratia fonticola* | *Streptomyces griseus* | |
| | *Salinispora tropica* | *Serratia marcescens* | *Streptomyces lavendulae* | **Tepidibacter** |
| **Saccharothrix** | | | *Streptomyces phaeochromogenes* | *Tepidibacter formicigenes* |
| *Saccharothrix australiensis* | ***Salinivibrio*** | **Sphaerotilus** | | |
| *Saccharothrix coeruleofusca* | *Salinivibrio costicola* | *Sphaerotilus natans* | *Streptomyces thermodiastaticus* | *Tepidibacter thalassicus* |
| *Saccharothrix espanaensis* | **Salmonella** | **Sphingobacterium** | | |
| *Saccharothrix longispora* | *Salmonella bongori* | *Sphingobacterium multivorum* | *Streptomyces tubercidicus* | **Thermus** |
| *Saccharothrix mutabilis* | *Salmonella enterica* | | | *Thermus aquaticus* |
| *Saccharothrix syringae* | *Salmonella subterranea* | **Staphylococcus** | | *Thermus filiformis* |
| *Saccharothrix tangerinus* | *Salmonella typhi* | [see below] | | *Thermus thermophilus* |
| *Saccharothrix texasensis* | | | | |
| **Staphylococcus** | | | | |
| *S. arlettae* | | *S. microti* | | |
| *S. agnetis* | *S. equorum* | *S. muscae* | *S. schleiferi* | |
| *S. aureus* | *S. felis* | *S. nepalensis* | *S. sciuri* | |
| *S. auricularis* | *S. fleurettii* | *S. pasteuri* | *S. simiae* | |
| *S. capitis* | *S. gallinarum* | *S. petrasii* | *S. simulans* | |
| *S. caprae* | *S. haemolyticus* | *S. pettenkoferi* | *S. stepanovicii* | |
| *S. carnosus* | *S. hominis* | *S. piscifermentans* | *S. succinus* | |
| *S. caseolyticus* | *S. hyicus* | *S. pseudintermedius* | *S. vitulinus* | |
| *S. chromogenes* | *S. intermedius* | *S. pseudolugdunensis* | *S. warneri* | |
| *S. cohnii* | *S. kloosii* | *S. pulvereri* | S. *xylosus* | |
| *S. condimenti* | *S. leei* | *S. rostri* | | |
| *S. delphini* | *S. lentus* | *S. saccharolyticus* | | |
| *S. devriesei* | *S. lugdunensis* | *S. saprophyticus* | | |
| *S. epidermidis* | *S. lutrae* | | | |
| | *S. lyticans* | | | |
| | *S. massiliensis* | | | |
| **Streptococcus** | | | | |
| *Streptococcus agalactiae* | *Streptococcus infantarius* | *Streptococcus orisratti* | *Streptococcus thermophilus* | |
| *Streptococcus anginosus* | *Streptococcus iniae* | *Streptococcus parasanguinis* | *Streptococcus sanguinis* | |
| *Streptococcus bovis* | *Streptococcus intermedius* | *Streptococcus peroris* | *Streptococcus sobrinus* | |
| *Streptococcus canis* | *Streptococcus lactarius* | *Streptococcus pneumoniae* | *Streptococcus suis* | |
| *Streptococcus constellatus* | *Streptococcus milleri* | *Streptococcus pseudopneumoniae* | *Streptococcus uberis* | |
| *Streptococcus downei* | *Streptococcus mitis* | | *Streptococcus vestibularis* | |
| *Streptococcus dysgalactiae* | *Streptococcus mutans* | *Streptococcus pyogenes* | *Streptococcus viridans* | |
| *Streptococcus equines* | *Streptococcus oralis* | *Streptococcus ratti* | *Streptococcus zooepidemicus* | |
| *Streptococcus faecalis* | *Streptococcus tigurinus* | *Streptococcus salivariu* | | |
| *Streptococcus ferus* | | | | |
| **Uliginosibacterium** | **Vagococcus** | **Vibrio** | **Virgibacillus** | **Xanthobacter** |
| | *Vagococcus carniphilus* | *Vibrio aerogenes* | *Virgibacillus halodenitrificans* | *Xanthobacter agilis* |
| *Uliginosibacterium gangwonense* | *Vagococcus elongatus* | *Vibrio aestuarianus* | | *Xanthobacter aminoxidans* |
| **Ulvibacter** | *Vagococcus fessus* | *Vibrio albensis* | *Virgibacillus pantothenticus* | |
| *Ulvibacter litoralis* | *Vagococcus fluvialis* | *Vibrio alginolyticus* | | *Xanthobacter autotrophicus* |
| **Umezawaea** | *Vagococcus lutrae* | *Vibrio campbellii* | | |
| *Umezawaea tangerina* | *Vagococcus salmoninarum* | *Vibrio cholerae* | **Weissella** | *Xanthobacter flavus* |
| **Undibacterium** | | *Vibrio cincinnatiensis* | *Weissella cibaria* | *Xanthobacter tagetidis* |
| *Undibacterium pigrum* | **Variovorax** | *Vibrio coralliilyticus* | *Weissella confusa* | *Xanthobacter viscosus* |
| **Ureaplasma** | *Variovorax boronicumulans* | *Vibrio cyclitrophicus* | *Weissella halotolerans* | **Xanthomonas** |
| *Ureaplasma urealyticum* | *Variovorax dokdonensis* | *Vibrio diazotrophicus* | *Weissella hellenica* | *Xanthomonas albilineans* |
| | *Variovorax paradoxus* | *Vibrio fluvialis* | *Weissella kandleri* | |
| **Ureibacillus** | *Variovorax soli* | *Vibrio furnissii* | *Weissella koreensis* | *Xanthomonas alfalfae* |
| *Ureibacillus composti* | | *Vibrio gazogenes* | *Weissella minor* | *Xanthomonas arboricola* |
| *Ureibacillus suwonensis* | **Veillonella** | *Vibrio halioticoli* | *Weissella paramesenteroides* | |
| *Ureibacillus terrenus* | *Veillonella atypica* | *Vibrio harveyi* | | *Xanthomonas axonopodis* |
| *Ureibacillus thermophilus* | *Veillonella caviae* | *Vibrio ichthyoenteri* | *Weissella soli* | |
| *Ureibacillus thermosphaericus* | *Veillonella criceti* | *Vibrio mediterranei* | *Weissella thailandensis* | *Xanthomonas campestris* |
| | *Veillonella dispar* | *Vibrio metschnikovii* | *Weissella viridescens* | |
| | *Veillonella montpellierensis* | | | |
| *Veillonella parvula* | *Vibrio mytili* | | *Xanthomonas citri* | |
| *Veillonella ratti* | *Vibrio natriegens* | **Williamsia** | *Xanthomonas codiaei* | |
| *Veillonella rodentium* | *Vibrio navarrensis* | *Williamsia marianensis* | *Xanthomonas cucurbitae* | |
| | *Vibrio nereis* | *Williamsia maris* | | |
| **Venenivibrio** | *Vibrio nigripulchritudo* | *Williamsia serinedens* | *Xanthomonas euvesicatoria* | |
| *Venenivibrio stagnispumantis* | *Vibrio ordalii* | | | |
| | *Vibrio orientalis* | **Winogradskyella** | *Xanthomonas fragariae* | |
| | *Vibrio parahaemolyticus* | *Winogradskyella thalassocola* | *Xanthomonas fuscans* | |
| **Verminephrobacter** | *Vibrio pectenicida* | | *Xanthomonas gardneri* | |
| *Verminephrobacter eiseniae* | *Vibrio penaeicida* | **Wolbachia** | *Xanthomonas hortorum* | |
| | *Vibrio proteolyticus* | *Wolbachia persica* | *Xanthomonas hyacinthi* | |
| | *Vibrio shilonii* | | *Xanthomonas perforans* | |
| | *Vibrio splendidus* | | *Xanthomonas phaseoli* | |
| **Verrucomicrobium** | *Vibrio tubiashii* | | *Xanthomonas pisi* | |
| *Verrucomicrobium spinosum* | *Vibrio vulnificus* | **Wolinella** | *Xanthomonas populi* | |
| | | *Wolinella succinogenes* | *Xanthomonas theicola* | |
| | | | *Xanthomonas translucens* | |
| | | **Zobellia** | | |
| | | *Zobellia galactanivorans* | *Xanthomonas vesicatoria* | |
| | | *Zobellia uliginosa* | | |
| | | | **Zoogloea** | **Xylella** |
| | | | *Zoogloea ramigera* | *Xylella fastidiosa* |
| | | | *Zoogloea resiniphila* | **Xylophilus** |
| | | | | *Xylophilus ampelinus* |
| **Xenophilus** | **Yangia** | *Yersinia mollaretii* | **Zooshikella** | **Zobellella** |
| *Xenophilus azovorans* | *Yangia pacifica* | *Yersinia philomiragia* | *Zooshikella ganghwensis* | *Zobellella denitrificans* |
| **Xenorhabdus** | | *Yersinia pestis* | | *Zobellella taiwanensis* |
| *Xenorhabdus beddingii* | **Yaniella** | *Yersinia pseudotuberculosis* | **Zunongwangia** | |
| *Xenorhabdus bovienii* | *Yaniella flava* | *Yersinia rohdei* | *Zunongwangia profunda* | |
| *Xenorhabdus cabanillasii* | *Yaniella halotolerans* | *Yersinia ruckeri* | | |
| *Xenorhabdus doucetiae* | **Yeosuana** | | **Zymobacter** | **Zeaxanthinibacter** |
| *Xenorhabdus griffiniae* | *Yeosuana aromativorans* | **Yokenella** | *Zymobacter palmae* | *Zeaxanthinibacter enoshimensis* |
| *Xenorhabdus hominickii* | | *Yokenella regensburgei* | **Zymomonas** | |
| *Xenorhabdus koppenhoeferi* | **Yersinia** | **Yonghaparkia** | *Zymomonas mobilis* | **Zhihengliuella** |
| *Xenorhabdus nematophila* | *Yersinia aldovae* | *Yonghaparkia alkaliphila* | | *Zhihengliuella halotolerans* |
| *Xenorhabdus poinarii* | *Yersinia bercovieri* | | **Zymophilus** | |
| **Xylanibacter** | *Yersinia enterocolitica* | **Zavarzinia** | *Zymophilus paucivorans* | **Xylanibacterium** |
| *Xylanibacter oryzae* | *Yersinia entomophaga* | *Zavarzinia compransoris* | *Zymophilus raffinosivorans* | *Xylanibacterium ulmi* |
| | *Yersinia frederiksenii* | | | |
| *Yersinia intermedia* | | | | |
| *Yersinia kristensenii* | | | | |

**Table 2: SaPIs**

| **Element** | **Staphylococcal genome** | **Baba*** | **Lindsay and Holden** 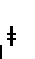 | **Size (kb)** | **Inducing phages** |
|---|---|---|---|---|---|
| SaPI4 | *S. aureus* str. MRSA252 | NA | SaPI4 | 15.1 | Endogenous prophage |
| SaPI1028 | *S. aureus* str. NY940 | NA | NA | 15.6 | Endogenous prophage |
| SaPIbov 1 | *S. aureus* str. RF122 | vSa2 | NA | 15.8 | φ11 and 80α |
| SaPIbov2 | *S. aureus* str. V329 | NA | NA | 27 | Endogenous prophage |
| SaPIm4 | *S. aureus* str. mu50 | vSa3 type I | NA | 14.4 | Endogenous prophage |
| SaPImw2 | *S. aureus* str. mw2 | vSa3 type II | SaPI3 | 14.4 | Not known |
| SePI1 | *S. aureus* str. FR1909 | NA | NA | 9.9 | Not known |
| ShPI2 | *S. haemolyticus* | vSh2 | NA | 16.6 | 80α and φ13 |
| SaPI1 | *S. aureus* str. RN4282 | vSa1 | NA | 15.2 | Not known |
| SaPI3 | *S. aureus* str. COL | vSa1 | SaPI1 | 15.6 | Not known |
| SaPI5 | *S. aureus* str. USA300 | NA | NA | 14.0 | 80α |
| SaPIn1 and SaPIm 1 | *S. aureus* str. n315 and *S. aureus* str. mu50. respectively | vSa4 type I | SaP12 | 15 | 80 and 80α |
| SaPI2 | *S. aureus* str. RN3984 | NA | NA | 14.7 | Not known |
| SaRlfusB | *S. aureus* European fusidic acid-resistant impetigo clone CS6 | NA | NA | 20.7 | Endogenous prophage |
| SaPI122 | *S. aureus* str. RF122 | NA | NA | 17.9 | Not known |
| SaPI6Δ | *S. aureus* strains 8325. COL, USA300, MSSA476. Newman and mw2 | vSa4 type Il | NA | 3.14 | Not known |
| SsPl15305 | *S. saprophyticus str.* 15305 | vSs15305 | NA | 16.7 | Endogenous prophage |

| | | | | | |
|---|---|---|---|---|---|
| [N/A, not applicable; *Nomenclature proposed by Baba *et al;* 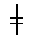 Nomenclature propsed by Lindsey & Holden *et al]* | | | | | |

**Table 3: Titers of SA100 packaged CGVs after production**

| | **SA100** | **Native phage** |
|---|---|---|
| **strain** | TFU/mL | PFU/mL |
| **EMG2** | 1.4E+10 | n.d |
| **C-1a** | 2.2E+10 | <20 |
| **C-1792** | 5.2E+09 | <20 |

**Table 4: Groups (Example 5)**

| **Group** | **Preparation (per 500 *µ*l)** |
|---|---|
| vehicle | 139 ul 9% Sod.carb + 361 *µ*l H₂O |
| SA100 | 139 ul 9% Sod.carb + 330 *µ*l p114 + 31 *µ*l H₂O |
| SA100 + induction of CRISPR system | 170 *µ*l Inducers+Sod.carb + 330 *µ*l p114 + 31 *µ*l H₂O |

**Table 5: Clinical Scores**

| | |
|---|---|
| Score 0 | healthy |
| Score 1 | Minor clinical signs of infection (slower movements, light piloerection in the skin) |
| Score 2 | Moderate signs of infection (slower movements, slightly pinched eyes, lack of curiosity or changed activity) |
| Score 3 | Severe signs of infection (reduced movements, pinched eyes, changed body posture) |
| Score 4 | Severe signs of infection (stiff movements, forced ventilation, pinched eyes) Sacrificed. |
| Score 5 | The mouse does not move, is cold, lying on the side. Sacrificed |
| Score 6 | The mouse is dead |

**Table 6: Sequences**

| **SEQ ID NO:** | **DESCRIPTION** | **SEQUENCE** |
|---|---|---|
| 1 | *terS* nucleotide sequence | AATTGGCAGTAAAGTGGCAGTTTTTGATACCTAAAATGAGATATTATGATAGTGTAGGATAT TGACTATCTTACTGCGTTTCCCTTATCGCAATTAGGAATAAAGGATCTATGTGGGTTGGCTG ATTATAGCCAATCCTTTTTTAATTTTAAAAGCGTATAGCGCGAGAGTTGGTGGTAAATGAA ATGAACGAAAAACAAAAGAGATTCGCAGATGAATATATAATGAATGGATGTAATGGTAAAAA AGCAGCAATTTCAGCAGGTTATAGTAAGAAAACAGCAGAGTCTTTAGCAAGTCGATTGTTAA GAAATGTTAATGTTTCGGAATATATTAAAGAACGATTAGAACAGATACAAGAAGAGCGTTTA ATGAGCATTACAGAAGCTTTAGCGTTATCTGCTTCTATTGCTAGAGGAGAACCTCAAGAGGC TTACAGTAAGAAATATGACCATTTAAACGATGAAGTGGAAAAAGAGGTTACTTACACAATCA CACCAACTTTTGAAGAGCGTCAGAGATCTATTGACCACATACTAAAAGTTCATGGTGCGTAT ATCGACAAAAAAGAAATTACTCAGAAGAATATTGAGATTAATATTGGTGAGTACGATGACGA AAGTTAAATTAAACTTTAACAAACCATCTAATGTTTTCAACAG |
| 2 | Packaging signal sequence | GCATGCGTTTTCCTGCCTCATTTCTGCAAACCGCGCCATTCCCGGCGCGGTCTGAGCGTGTCAGTGCAACTGCATTAAAACCG CCCCGCAAAGCGGGCGGGCGAGGCGGGGAAAGCACCGCGCAAACCCAGAAGTTAGTTAATTATTTGTGTAGTCAAAGTGC CTTGACTACATACCTCGTTAATACATTGGAGCATAATGAAGAAAATCTATGGCCTATGGTCCAAAACTGTCTTTTTGATGGCAC TATCCTGAAAAATATGCAAAAAATAGATTGATGTAAGGTGGTTCTTGTCAGTGTCGCAAGATCCTTAAGAATTC |
| 3 | Cos packaging signal sequence | GCATGCGTTTTCCTGCCTCATTTCTGCAAACCGCGCCATTCCCGGCGCGGTCTGAGCGTGTCAGTGCAACTGCATTAAAACCG CCCCGCAAAGCGGGCGGGCGAGGCGGGGAAAGCACCGCGCGCAAACCGACAAGTTAGTTAATTATTTGTGTAGTCAAAGTGC CTTCAGTACATACCTCGTTAATACATTGGAGCATAATGAAGAAAATCTATGGCCTATGGTC |
| 4 | P2/P4 packaging signal sequence | TGCATTAAAACCGCCCCGCAAAGCGGGCGGGCGAGGCGGGGAAAGCACCGCGCGC |
| 5 | *sid* | ATGTCTGACCACACTATCCCTGAATATCTGCAACCCGCACTGGCACAACTGGAAAAGGCCAGAGCCGCCCATCTTGAGAACGC CCGCCTGATGGATGAGACCGTCACGGCCATTGAACGGGCAGAGCAGGAAAAAAATGCGCTGGCGCAGGCCGACGGAAACGA CGCTGACGACTGGCGCACGGCCTTTCGTGCAGCCGGTGGTGTCCTGAGCGACGAGCTGAAACAGCGCCACATTGAGCGCGTG GCACGCCGGGAGCTGGTACAGGAATATGACAATCTGGCCGTGGTGCTGAATTTCGAACGTGAACGCCTGAAAGGGGCGTGTG ACAGCACGGCCACCGCCTACCGGAAGGCACATCATCACCTTCTGAGTCTGTATGCAGAGCATGAGCTGGAACACGCCCTGAAT GAAACCTGTGAGGCGCTTGTCCGGGCAATGCATCTGAGCATTCTGGTACAGGAAAATCCGCTCGCCAACACCACCGGCCATCA GGGCTACGTCGCACCGGAAAAGGCTGTCATGCAGCAGGTGAAATCATCGCTGGAACAGAAAATTAAACAGATGCAAATCAGC CTCACCGGCGAGCCGGTTCTCCGGCTGACCGGACTGTCAGCGGCAACACTCCCGCACATGGATTATGAGGTGGCAGGCACACC GGCACAGCGCAAGGTGTGGCAGGACAAAATAGACCAGCAGGGAGCAGAGCTTAAGGCCAGAGGGCTGCTGTCATGA |
| 6 | *psu* | ATGGAAAGCACAGCCTTACAGCAGGCCTTTGACACCTGTCAGAATAACAAAGCAGCATGGCTGCAACGCAAAAATGAGCTGG CAGCGGCCGAACAGGAATATCTGCGGCTTCTGTCAGGAGAAGGCAGAAACGTCAGTCGCCTGGACGAATTACGCAATATTATC GAAGTCAGAAAATGGCAGGTGAATCAGGCCGCCGGTCGTTATATTCGTTCGCATGAAGCCGTTCAGCACATCAGCATCCGCGA CCGGCTGAATGATTTTATGCAGCAGCACGGCACAGCACTGGCGGCCGCACTGGCACCGGAGCTGATGGGCTACAGTGAGCTG ACGGCCATTGCCCGAAACTGTGCCATACAGCGTGCCACAGATGCCCTGCGTGAAGCCCTTCTGTCCTGGCTTGCGAAGGGTGA AAAAATTAATTATTCCGCACAGGATAGCGACATTTTAACGACCATCGGATTCAGGCCTGACGTGGCTTCGGTGGATGACAGCC GTGAAAAATTCACCCCTGCGCAGAACATGATTTTTCGCGTAAAAGTGCGCAACTGGCATCACGTCAGTCAGTGTAA |
| 7 | *Delta* | ATGATTTACTGTCCGTCGTGTGGACATGTTGCTCACACCCGTCGCGCACATTTCATGGACGATGGCACCAAGATAATGATTGCA CAGTGCCGGAATATTTATTGCTCTGCGACATTTGAAGCGAGTGAAAGCTTTTTCTCTGACAGTAAAGATTCAGGAATGGAATAC ATTTCAGGCAAACAGAGATACCGCGATTCACTGACGTCAGCCTCCTGCGGTATGAAACGCCCGAAAAGAATGCTTGTTACCGG |
| | | ATATTGTTGTCGGAGATGTAAAGGCCTTGCACTGTCAAGAACATCGCGGCGTCTGTCTCAGGAAGTCACCGAGCGTTTTTATGT GTGCACGGATCCGGGCTGTGGTCTGGTGTTTAAAACGCTTCAGACCATCAACCGCTTCATTGTCCGCCCGGTCACGCCGGACG AACTGGCAGAACGCCTGCATGAAAAACAGGAACTGCCGCCAGTACGGTTAAAAACACAATCATATTCGCTGCGTCTGGAATGA |
| 8 | *Q* through *S* of P2 | TCAGTCGTTGTCAGTGTCCAGTGAGTAGTTTTAAAGCGGATGACCTCCTGACCGAGCCAGCCGTTTATCTCGCGGATCCTGTC CTGTAACGGGATAAGCTCATTGCGGACAAAGACCTTTGCCACTTTCTCAATATCACCCAGCGACCCGACGTTCTCCGGCTTGCC ACCCATCAACTGAAAGGGGATGCGGTGCGCGTCCAGCAGGTCAGCGGCGCTGGCTTTTTTGATATTAAAAAATCGTCCTTCG TCGCCACTTCACTGAGGGGGATAATTTTAATGCCGTCGGCTTTCCCCTGTGGGGCATAGAGAAACAGGTTTTTAAAGTTGTTGC GGCCTTTCGACTTGACCATGTTTTCGCGAAGCATTTCGATATCGTTGCGATCCTGCACGGCATCGGTGACATACATGATGTATCC GGCATGTGCGCCATTTTCGTAATACTTGCGGCGGAACAACGTGGCCGACTCATTCAGCCAGGCAGAGTTAAGGGCGCTGAGAT ATTCCGGCAGGCCGTACAGCTCCTGATTAATATCCGGCTCCAGCAGGTGAAACACGGAGCCGGGCGCGAAGGCTGTCGGCTC GTTGAAGGACGGCACCCACCAGTAAACATCCTCTTCCACGCCACGGCGGGTATATTTGCCGGTGAGGTTTCCAGTCTGATGAC CTTACCGGTGGTGCTGTAACGCTTTTCCAGAAACGCATTACCGAACACCAGAAAATCCAGCACAAAGCGGCTGAAATCCTGCT GGGAAAGCCATGGATGCGGGATAAATGTCGAGGCCAGAATATTGCGTTTGACGTAAATCGGCGAGCTGTGATGCACGGCAGC CCGCAGGCTTTTGCCAGACCGGTAAAGCTGACCGGTGGCTCATACCATCTGCCGTTACTGATGCACTCGACGTAATCCAGAAT GTCACGGCGGTCGAGTACCGGCACCGGCTCACCAAAGGTGAATGCCTCCATTTCGGGCCGCTGGCGGTCATTGTTTTGCCG CAGGTTGCGGTGTTTCCCTTTTTCTTGCTCATCAGTAAAACTCCAGAATGGTGGATGTCAGCGGGGTGCTGATACCGGCGGT GAGTGGCTCATTTAACAGGGCGTGCATGGTCGCCCAGGCGAGGTCGGCGTGGCTGGCTTCCTCGCTGCGGCTGGCCTCATAG GTGGCGCTGCGTCCGCTGCTGGTCATGGTCTTGCGGATAGCCATAAACGAGCTGGTGATGTCGGTGGCGCTGACGTCGTATTC CAGACAGCCACGGCGGATAACGTCTTTTGCCTTGAGCACCATTGCGGTTTTCATTTCCGGCGTGTAGCGGATATCACGCGCGGC GGGATAGAACGAGCGCACGAGCTGGAACACGCCGACACCGAGGCCGGTGGCATCAATACCGATGTATTCGACGTTGTATTTT TCGGTGAGTTTGCGGATGGATTCCGCCTGGGTGGCAAAGTCCATGCCTTTCCACTGGTGACGCTCAAGTATTCTGAATTTGCCA CCGGCCACCACCGGCGGTGCCAGTACCACGCATCCGGCGCTGTCGCCACGGTGTGACGGGTCGTAACCAATCCATACCGGGC GGGAGCCGAACGGATTGGCGGCAAACGGCGCATAGTCTTCCCATTCTTCCAGCGTGTCGACCATGCAGCGTTGCAGCTCCTCG AACGGGAACACCGATGCCTTGTCGTCAACAAATTCACACATGAACAGGTTTTTAAAATCGTCGGCGCTGTTTTCGCGTTTGAGC TGCTCAATGTCGAACAGCGTGCAGCCGCCTTTCAGGGCGTCCTCAATGGTGACAATCTGTCGCCACTGGCCGTCCGCACAGAG AAGCCCACCGGCAAGGGCGTTATGACTGACGTCGATTTCCACGCGTTCGGCGGCTGGCGCGTCCCCGGTTAAACAGTTCAC |
| | | CCGACCAGAACGGGTAGGCGTCGTGCCAGCGTGGACGGGGTGGAGAAATAGGTCGAGCGCAGGTGACTCTGTGAGGCCA TACCTGATGCCACCTTACGCAGTACCTGAAAATTCGGGATCCAGAAAATCTCATCGACGTACAGGTCGCCGTTATGACTCTGCG CGGTGTTGGAGTTGGTGCCGAGAAAAATCAGTTTTGCGCCGTTATTGCCCAGGACAATCGGGTCACCGGTCAGGTCAACGTCA ACCAGACGGGCAAAGGCGATGATGTATTCGCGGAACACATACGCCTGTGTTTACTGGCCGACAGAAAAATCTGGTTATGACC GGTTTTCAGGGCACGCAGCAGCGCCTCGCGGAAAAATAAAACGTCGCGCCAATCTGGCGGGATTTCAGGATATCGCGGATG CGGTGCTCAAGCCCGGCACGATACCAGTGCAGCTGATAGTCGAAAGACTGCTCAAAGAAAATCTGCTCCAGCTTTTCGATGGC CTCGTCACTGAAAAAATTCTTTTCGGTTTGCGCCGTCCGCCTTTGTTACGGTTAGCGACGTTCGGATTAAGGTCTGCCTCGTTG CCGGTCTGGCTGTAGCGGTTGACCCGTGCCAGTCGTTCAATCTGGCGTCCCAGCAGGTCAATTTCCTTGAAGTCACCGCCGGTT TTCTGTGGTTTGATGATGAGCTGGGTCAGCCGCGCTTCCAGACTCATTTCGACACGGCTGATGGGGGCAACGCTGTCCCAGCC GTCGCGCTGTTTCCAGCTCTGCACTGTCGGGCGTTTCATCTGCAACATGGCGGCAATCTGCGGCACGGAAAACCCCTGCCAGTA CAGCAGCGCCGCCTGACGACGCGGGTCGTGTAAAAGAGTGGTGTCTGTGGTGATGGTCATGAATACCTCGCCGTGATGAATA CACGGCAAGGCTACTGAGTCGCGCCCCGCGATTCGCTAAGGTGCTGTTGTGTCAGTGATAAGCCATCCGGGACTGATGGCGG AGGATGCGCATCGTCGGGAAACTGATGCCGACATGTGACTCCTCTAATCACTATTCAGGACTCCTGACAATGGCAAAAAAAGT CTCAAAATTCTTTCGTATCGGCGTTGAGGGTGACACCTGTGACGGGCGTGTCATCAGTGCGCAGGATATTCAGGAAATGGCCG AAACCTTTGACCCGCGTGTCTATGGTTGCCGCATTAACCTGGAACATCTGCGCGGCATCCTGCCTGACGGTATTTTTAAGCGTT ATGGCGATGTGGCCGAACTGAAGGCCGAAAAGATTGACGATGATTCGGCGCTGAAAGGCAAATGGGCGCTGTTTGCGAAAAT CACCCCGACCGATGACCTTATCGCGATGAACAAGGCCGCGCAGAAGGTCTACACCTCAATGGAAATTCAGCCGAACTTTGCCA ACACCGGCAAATGTTATCTGGTGGGTCTGGCCGTCACCGATGACCCGGCAAGCCTCGGCACGGAATACCTGGAATTCTGCCGC ACGGCAAAACACAACCCCCTGAACCGCTTCAAATTAAGCCCTGAAAACCTGATTTCAGTGGCAACGCCTGTTGAGCTGGAATTT GAAGACCTGCCTGAAACCGTGTTCACCGCCCTGACCGAAAAGGTGAAGTCCATTTTTGGCCGCAAACAGGCCAGCGATGATGC CCGTCTGAATGACGTGCATGAAGCGGTGACCGCTGTTGCTGAACATGTGCAGGAAAAACTGAGCGCCACTGAGCAGCGCCTC GCTGAGATGGAAACCGCCTTTTCTGCACTTAAGCAGGAGGTGACTGACAGGGCGGATGAAACCAGCCAGGCATTCACCCGCC TGAAAAACAGTCTCGACCACACCGAAAGTCTGACCCAGCAGCGCCGCAGCAAAGCCACCGGCGGTGGCGGTGACGCCCTGAT GACGAACTGCTGACCGGCGTCAGTCAGTCCGGGAAAACCTTCACGATTAACCCTTAATTTCAGGAAAAACTATGCGCCAGGAA ACCCGCTTTAAATTTAATGCCTACCTGTCCCGTGTTGCCGAACTGAACGGCATCGACGCCGGTGATGTGTCGAAAAAATTCACC GTTGAACCGTCGGTCACCCAGACCCTGATGAACACCATGCAGGAGTCCTCTGACTTTCTGACCCGCATCAATATTGTGCCGGTC AGCGAAATGAAAGGGGAAAAAATTGGTATCGGTGTCACCGGCTCCATCGCCAGCACTACCGACACTGCCGGTGGTACCGAGC |
| | | GTCAGCCGAAGGACTTCTCGAAGCTGGCGTCAAACAAGTACGAATGCGACCAGATTAACTTCGATTTTATATCCGCTACAAAA CGCTGGACCTGTGGGCGCGTTATCAGGATTTCCAGCTCCGTATCCGTAACGCCATTATCAAACGCCAGTCCCTTGATTTCATCAT GGCCGGTTTTAACGGCGTGAAGCGTGCCGAAACCTCTGACCGCAGCAGCAATCCGATGTTGCAGGATGTGGCGGTCGGCTGG CTGCAGAAATACCGCAATGAAGCACCGGCGCGTGATGAGCAAGGTCACTGACGAGGAAGGCCGCACCACCTCTGAGGTTA TCCGCGTGGGTAAGGGCGGTGATTATGCCAGCCTTGATGCACTGGTGATGGATGCGACCAACAACCTGATTGAACCGTGGTAT CAGGAAGACCCTGACCTTGTGGTGATTGTGGGGCGTCAGCTACTGGCGGACAAGTATTTCCCCATCGTCAACAAGGAGCAGG ACAACAGCGAAATGCTGGCCGCTGACGTCATCATCAGCCAGAAACGCATCGGTAACCTACCAGCGGTACGCGTCCCGTACTTC CCGGCGGATGCGATGCTCATCACGAAGCTGGAAAACCTGTCCATCTACTACATGGATGACAGCCATCGCCGCGTGATTGAGGA AAACCCGAAACTCGACCGCGTGGAGAACTACGAGTCAATGAACATTGATTACGTGGTGGAAGACTACGCCGCCGGTTGTCTG GTGGAAAAAATCAAGGTCGGTGACTTCTCCACACCGGCTAAGGCGACCGCAGAGCCGGGAGCGTAACCGATGACGAGTCCCG CACAGCGCCACATGATGCGGGTCTCGGCAGCGATGACCGCGCAGCGGGAAGCCGCCCCGCTGCGACATGCAACTGTCTATGA GCAGATGCTGGTTAAGCTCGCCGCAGACCAGCGCACACTGAAAGCGATTTACTCAAAAGAGCTGAAGGCCGCAAAAAAACGC GAACTGCTGCCGTTCTGGTTGCCGTGGGTGAACGGCGTGCTGGAGCTGGGCAAAGGTGCACAGGATGACATTCTGATGACGG TCATGCTGTGGCGTCTGGATACCGGCGATATTGCCGGTGCGCTGGAGATTGCCCGTTATGCCCTGAAGTACGGTCTGACCATG CCGGGTAAACACCGCCGTACCCCGCCGTACATGTTCACCGAGGAGGTAGCGCTTGCGGCCATGCGCGCTCACGCTGCCGGTGA GTCTGTGGATACCCGCCTGCTGACGGAGACCCTTGAACTGACCGCCACGGCTGACATGCCTGATGAAGTGCGCGCAAAGCTGC ACAAAATCACCGGTCTGTTTCTGCGTGACGGTGGTGATGCCGCCGGTGCGCTGGCGCACCTGCAACGTGCGACACAGCTCGAC TGTCAGGCAGGCGTCAAAAAAGAGATTGAACGACTGGAGCGGGAGCTGAAACCGAAGCCGGAGCCGCAGCCCAAAGCGGCC ACCCGCGCCCCGCGTAAGACCCGGAGCGTGACACCGGCAAAACGTGGACGCCCGAAAAAGAAAGCCAGTTAACAACCGAATG CGCCCCGCGCCAGGGCGGCACGCCGGTCAGTGACGGTGAATCACCTGACACTGCACCGGCGTCCACCGCCCGACTTTTCAGAG GTAGTCATGATGACGCTGATTATTCCGCGAAAGGAGGCTCCCGTGTCCGGTGAGGGTACGGTGGTCATCCCGCAACCGGCAG GCGACGAGCCGGTGATTAAAAACACGTTCTTTTTTCCCGATATCGACCCGAAGCGCGTCCGGGAACGTATGCGCCTTGAGCAG ACCGTCGCCCCCGCCCGTCTGCGTGAGGCCATCAAGTCAGGCATGGCTGAAACGAATGCGGAGCTGTACGAGTACCGCGAAC AGAAAATTGCCGCCGGTTTTACGCGTCTGGCTGACGTCCCGGCGGACGATATCGACGGTGAAAGCATCAAGGTTTTTTACTAC GAGCGCGCCGTGTGTGCGATGGCGACCGCGTCGCTTTATGAGCGTTATCGCGGTGTGGATGCCAGTGCGAAAGGCGACAAGA AGGCTGACAGCATTGACAGCACCATTGATGAGCTGTGGCGGGATATGCGCTGGGCGGTGGCGCGCATCCAGGGCAAGCCGC GCTGCATCGTGAGTCAAATCTGATGAAGACCTTTGCGCTACAGGGCGACACGCTCGACGCCATTTGTGTCCGCTATTACGGGC |
| | | GCACTGAGGGCGTGGTTGAGACCGTGCTCGCCGCAAATCCGGGACTGGCTGAACTGGGGGCGGTGCTGCCACACGGCACCG CCGTCGAACTGCCCGACGTTCAGACCGCGCCCGTGGCTGAAACTGTCAATCTGTGGGAGTAACGCATGACAGCAGAAGAAAA AAGCGTCCTGTCGCTTTTCATGATTGGGGTGCTGATTGTTGTCGGCAAGGTGCTTGCCGGTGGTGAACCTATCACCCCGCGTCT GTTTATCGGGCGCATGTTGCTCGGTGGTTTTGTCTCGATGGTTGCCGGTGTTGTTCTGGTGCAGTTTCCTGACCTGTCACTGCCA GCGGTGTGCGGCATCGGCTCCATGCTGGGTATCGCCGGTTATCAGGTGATTGAGATTGCCATTCAGCGCCGCTTTAAGGGCAG GGGGAAACAGTAATGCCGGTAATTAACACGCATCAGAATATCGCCGCCTTTCTCGACATGCTGGCCGTGTCCGAAGGGACGGC GAATCATCCACTGACGAAAAACCGGGGCTATGACGTGATAGTCACCGGACTGGACGGAAGCCGGAAATTTTCACCGACTAC AGTGACCACCCGTTCGCACATGGCCGACCGGCGAAGGTGTTTAACCGTCGCGGTGAAAAATCCACGGCCTCCGGTCGCTATCA GCAGCTTTACCTGTTCTGGCCGCATTACCGCAAACAGCTTGCCCTGCCGGATTTCAGTCCGTTGTCACAGGACAGACTCGCCAT TCAGTTGATCCGCGAACGCGGAGCACTGGATGACATCCGGGCGGACGCATTGAGCGCGCCATTTCACGCTGTCGCAATATCT GGGCGTCCCTGCCGGGTGCCGGTTACGGTCAGCGTGAGCATTCACTGGAAAAACTGGTCACCGTCTGGCGTACCGCTGGCGG CGTACCGGCTTAAACGGAGTAAATACCATGAAGAAATTATCCCTTTCACTGATGCTGAACGTGTCGCTGGCGCTGATGCTGGC ACTGTCCCTGATTTACCCGCAGAGCGTGGCCGTCAATTTTGTCGCTGCCTGGGCGATTCTGGCGACGGTTATCTGTGTGGTTGC CGGTGGTGTGGGCGTGTATGCCACTGAGTATGTGCTGGAACGCTACGGGCGGGAGCTGCCGCCGGAATCGCTGGCCGTGAA GATTGTCACGTCGCTGTTTTTGCAGCCGGTGCCGTGGCGCAGACGGGCGGCGGCTCTGGTAGTGGTGGTGGCGACGTTTATCT CGCTGGTCGCTGCCGGGTGGATTTTTACCGCGCTGATTTATCTTGTGGTGTCGCTGTTTTTCCGGCTGATACGTAAAGCCTGTC GTCAGCGTCTTGAGGGGCGGGAACCATGTCAAGGCTGATGATTGTGCTGGTCGTGTTGTTATCGCTGGCGGTGGCCGGTCTGT TTCTGGTGAAACACAAAAATGCCAGCCTGCGCGCCTCGCTGGACAGGGCGAACAACGTCGCCAGCGGTCAGCAGACGACCAT CACCATGCTGAAAAATCAGCTTCATGTTGCGCTCACCAGGGCAGATAAAAACGAGCTGGCGCAGGTGGCACTGCGTCAGGAA CTGGAGAACGCCGCGAAACGTGAAGCACAGCGCGAGAAAACCATCACGAGGTTACTTAATGAGAACGAAGATTTTCGCCGCT GGTACGGTGCTGACCTGCCTGATGCTGTGCGCCGGTTGCACCAGCGCCCCGCCTGCACCGACGCCAGTGATTGTCCCCAACGC ATGCCCGAAAGTGAGCCTTTGCCCGATGCCGGGCAGTGACCCGCAGACGAACGGCGATTTAAGTGCCGATATCCGGCAGCTT GAGAACGCGCTGGCACGCTGTGCCAGCCAGGTAAAAATGATTAAACACTGTCAGGACGAAAACGATGCTCAAACCCGACAGC CTGCGCAGGGCGCTGACTGATGCCGTCACGGTGCTGAAAACTAACCCCGATATGCTGCGGATATTCGTGGATAACGGGAGTAT TGCCTCCACACTGGCGGCGTCGCTGTCATTCGAAAAGCGTTACACGCTCAATGTGATTGTGACCGACTTTACCGGTGATTTTGA CCTGCTCATTGTGCCGGTGCTGGCGTGGCTGCGGGAAAATCAGCCCGACATCATGACCACCGACGAAGGCCAGAAAAAGGGC TTCACGTTTTATGCAGACATCAACAATGACAGCAGCTTTGATATCAGTATCAGCCTGATGCTGACCGAGCGCACGCTGGTCAGT |
| | | GAGGTGGACGGCGCACTGCATGTGAAGAATATCTCGGAACCCCCGCCGCCGGAGCCGGTCACCCGCCCGATGGAGCTGTATA TCAATGGCGAACTGGTGAGTAAGTGGGATGAATGAGTTTAAGCGTTTTGAAGACCGGCTGACCGGACTGATTGAATCGCTGTC ACCGTCAGGGCGTCGGCGACTGAGTGCCGAACTGGCGAAACGTCTGCGGCAGAGTCAGCAGCGTCGGGTGATGGCACAGAA AGCCCCGGACGGCACACCCTACGCCACGCCAGCAGCAGAGCGTCAGAAAAAGACCGGTCGCGTTAAGCGAAAAATGTTT GCGAAACTTATTACCAGTCGTTTTTGCATATCCGTGCCAGCCCGGAGCAGGCATCAATGGAATTTTACGGCGGGAAGTCGCC GAAAATCGCCAGTGTGCATCAGTTTGGTCTGTCGGAAGAAAACCGGAAAGACGGTAAGAAAATTGATTATCCGGCGCGTCCCC TGCTCGGCTTTACCGGTGAGGATGTGCAGATGATTGAAGAGATTATCCTGGCTCACCTTGAGCGTTAG |
| 9 | *V* through *G* of P2 | ATGAACACTCTCGCAAATATTCAGGAACTCGCGCGCACTGCGCAACATGATTCGCACTGGCATTATCGTCGAAACCGACCTT AACGCCGGTCGCTGCCGCGTGCAGACCGGCGGCATGTGCACCGACTGGCTTCAGTGGCTGACCCATCGCGCAGGACGTTCGC GCACATGGTGGGCACCTTCCGTGGGGAACAGGTGCTGATTCTGGCCGTGGGTGGTGAACTCGACACGGCGTTCGTTCTGCC GGGGATTTATTCCGGCGATAACCCCTCGCCGTCTGTGTCGGCGGATGCCCTGCATATCCGTTTCCCTGACGGGGCGGTGATTG AATATGAACCCGAAACCAGTGCACTCACGGTAAGCGGAATTAAAACGGCCAGCGTGACGGCTTCCGGTTCTGTTACTGCCACG GTGCCGGTGGTCATGGTGAAAGCATCAACCCGCGTCACCCTGGACACCCCGGAGGTGGTCTGCACCAACAGGCTGATTACCG GCACGCTGGAAGTGCAGAAAGGCGGACGATGCGCGGCAACATTGAACACACCGGCGGTGAACTCTCATCAAACGGTAAGG TACTGCATACCCATAAACACCCCGGCGACAGCGGCGGCACAACCGGGAGTCCTTTATGACAGCGCGTTATCTCGGAATGAATC GCAGTGATGGCCTGACTGTCACTGACCTTGAGCATATCAGCCAGAGTATCGGCGATATCCTGCGCACACCGGTCGGCTCACGG GTGATGCGTCGTGATTACGGCTCGTTGCTGGCGTCAATGATTGACCAGCCGCAGACCCCGGCGCTTGAGTTGCAGATTAAAGT CGCCTGTTACATGGCAGTGCTGAAATGGGAACCCCGCGTCACCCTGTCATCCGTCACCACGGCGCGCAGTTTTGACGGGCGAA TGACGGTCACGTTAACCGGCCAGCACAACGACACCGGCCAGCCACTTTCATTAACCATCCCTGTGAGTTGAAACCATGCCGATT ATCGACCTGAACCAGCTACCCGCACCGGATGTGGTCGAGGAGCTGGACTTTGAAAGCATTCTCGCTGAACGCAAGGCGACACT GATTTCCCTTTACCCGGAAGATCAGCAGGAGGCGGTCGCCCGTACCCTGACACTGGAATCTGAGCCTCTCGTCAAACTGCTGG AAGAAAATGCTTATCGTGAGCTTATCTGGCGTCAGCGTGTGAATGAGGCCGCACGGGCGGTGATGCTGGCCTGTGCCGCCGG TAATGACCTTGATGTGATTGGTGCCAATTACAACACCACGCGCCTGACTATCACCCCGGCAGATGATTCGACCATCCCGCCGAC ACCGGCAGTGATGGAATCTGACACCGATTATCGTCTGCGTATTCAGCAGGCTTTTGAGGGCTTAAGCGTCGCCGGGTCAGTGG GAGCCTATCAGTATCATGGTCGCAGTGCTGACGGCGTGTCGCGGATATTTCTGTCACCAGTCCGTCTCCGGCCTGTGTCACCA TCTCTGTGCTGTCACGTGAAAATAACGGCGTCGCATCCGAAGACCTGCTGGCTGTGGTGCGTAACGCCCTTAATGGCGAGGAC |
| | | GTCAGGCCGGTGGCCGACCGCGTGACCGTGCAGTCTGCCGCCATCGTTGAATACCAGATAAACGCCACGCTTTACCTTTACCCT GGTCCCGAAAGCGAACCCATCCGCGCTGCCGCTGTGAAAAAGCTGGAAGCGTATATCACGGCACAGCACCGGCTGGGGCGCG ACATCCGTCTGTCTGCCATTTATGCCGCTTTGCATGTGGAAGGTGTGCAGCGTGTCGAACTGGCTGCACCACTGGCCGACATCG TGCTCAACAGTACGCAGGCGTCTTTCTGTACCGAATACCGCGTCGTGACCGGAGGCTCGGATGAGTGATTCGCGACTGCTGCC GACCGGCTCATCACCGCTTGAGGTCGCCGCCGCAAAAGCCTGTGCGGAAATTGAAAAACGCCGGTCAGTATTCGTGAACTGT GGAACCCGGACACCTGTCCGGCAAATCTGCTGCCGTGGCTGGCGTGGGCGTTTTCGGTCGACAGGTGGGATGAAAAGTGGCC GGAAGCGACAAAACGCGCCGTTATCCGCGATGCCTATTTCATCCACTGTCATAAGGGCACGATAGGTGCAATCCGGCGTGTGG TGGAGCCGCTCGGCTATCTCATCAACGTGACGGAGTGGTGGGAAAACAGTGACCCGCCCGGCACCTTCCGGCTTGATATTGGT GTACTGGAAAGCGGTATCACAGAGGCAATGTATCAGGAAATGGAACGGCTGATTGCTGATGCCAAACCTGCAAGCCGTCACC TTATTGGCCTGAACATTACCCGGGACATTCCCGGCTATCTGTTCGCCGGTGGTGTGGCTTACGACGGCGATGTAATTACGGTTT ACCCCGGATAAGTGAGGAATAATGAGCATAAAATTCAGAACCGTTATCACCACTGCCGGTGCAGCAAAGCTGGCAGCGGCAA CCGCGCCGGGAAGGCGGAAGGTCGGCATTACCACGATGGCCGTCGGGGATGGCGGTGGTAAATTGCCTGTCCCGGATGCCG GACAGACCGGGCTTATCCATGAAGTCTGGCGACATGCGCTGAACAAAATCAGCCAGGACAAACGAAACAGTAATTATATTATC GCCGAGCTGGTTATTCCGCCGGAGGTGGGCGGTTTCTGGATGCGTGAGCTTGGCCTGTACGATGATGCGGGAACGTTAATTG CCGTGGCGAACATGGCCGAAAGCTATAAGCCAGCCCTTGCCGAAGGCTCAGGACGTTGGCAGACCTGTCGCATGGTCATCATC GTCAGCAGTGTGGCCTCAGTGGAGCTGACCATTGACACCACAACGGTGATGGCGACGCAGGATTACGTTGATGACAAAATTG CAGAGCACGAACAGTCACGACGTCACCCGGACGCCTCGCTGACAGCAAAAGGTTTTACTCAGTTAAGCAGTGCGACCAACAGC ACGTCTGAAACACTGGCCGCAACGCCGAAAGCGGTAAAGGCCGCGTATGACCTGGCTAACGGGAAATATACCGCACAGGACG CCACCACAGCGCGAAAAGGCCTTGTCCAGCTTAGTAGCGCCACCAACAGCACGTCTGAAACGCTCGCCGCAACACCAAAAGCC GTTAAGACGGTAATGGATGAAACGAACAAAAAAGCGCCATTAAACAGCCCTGCACTGACCGGAACGCCAACGACGCCAACTG CGCGACAGGGAACGAATAATACTCAGATCGCAAACACGGCTTTCGTTATGGCCGCGATTGCCGCCCTTGTAGACTCGTCGCCT GACGCACTGAATACGCTGAACGAGCTGGCGGCGGCGCTGGGCAATGACCCGAATTTTGCTACCACCATGACTAATGCGCTTGC GGGTAAGCAACCGAAAGATGCTACCCTGACGGCGCTGGCGGGGCTTGCTACTGCGGCAGACAGGTTTCCGTATTTTACGGGG AATGATGTTGCCAGCCTGGCGACCCTGACAAAAGTCGGGCGGGATATTCTGGCTAAATCGACCGTTGCCGCCGTTATCGAATA TCTCGGTTTACAGGAAACGGTAAACCGAGCCGGGAACGCCGTGCAAAAAAATGGCGATACCTTGTCCGGTGGACTTACTTTTG AAAACGACTCAATCCTTGCCTGGATTCGAAATACTGACTGGGCGAAGATTGGATTTAAAAATGATGCCGATGGTGACACTGAT TCATACATGTGGTTTGAAACGGGGGATAACGGCAATGAATATTTCAAATGGAGAAGCCGCCAGAGTACCACAACAAAAGACC |
| | | TGATGACGTTGAAATGGGATGCACTAAATATTCTTGTTAATGCCGTCATTAATGGCTGTTTTGGAGTTGGTACGACGAATGCAC TAGGTGGTAGCTCTATTGTTCTTGGTGATAATGATACCGGATTTAAACAGAATGGAGACGGTATTCTTGATGTTTATGCTAACA GTCAGCGTGTATTCCGTTTTCAGAATGGAGTGGCTATTGCTTTTAAAAATATTCAGGCAGGTGATAGTAAAAAGTTCTCGCTAT CCAGCTCTAATACATCCACGAAGAATATTACCTTTAATTTATGGGGTGCTTCCACCCGTCCAGTGGTTGCAGAGTTAGGCGATG AGGCCGGATGGCATTTCTATAGCCAGCGAAATACAGATAACTCGGTAATATTTGCTGTTAACGGTCAGATGCAACCCAGCAAC TGGGGAAATTTTGATTCCCGCTATGTGAAAGATGTTCGCCTGGGTACGCGAGTTGTTCAATTGATGGCGCGAGGTGGTCGTTA TGAAAAAGCCGGACACACGATTACCGGATTAAGAATCATTGGTGAAGTAGATGGCGATGATGAAGCCATCTTCAGGCCGATA CAAAAATACATCAATGGCACATGGTATAACGTTGCGCAGGTGTAAGTTATGCAGCATTTAAAGAACATTAAGTCAGGTAATCC AAAAACAAAAGAGCAATATCAGCTAACAAAGAATTTTGATGTTATCTGGTTATGGTCCGAAGACGGAAAAAACTGGTATGAGG AAGTGAAGAACTTTCAGCCAGACACAATAAAGATTGTTTACGATGAAAATAATATTATTGTCGCTATCACCAGAGATGCTTCAA CGCTTAATCCTGAAGGTTTTAGCGTTGTTGAGGTTCCTGATATTACCTCCAACCGACGTGCTGACGACTCAGGTAAATGGATGT TTAAGGATGGTGCTGTGGTTAAACGGATTTATACGGCAGATGAACAGCAACAACAGGCAGAATCACAAAAGGCCGCGTTACTT TCCGAAGCGGAAAACGTTATTCAGCCACTGGAACGCGCTGTCAGGCTGAATATGGCGACGGATGAGGAACGTGCACGACTGG AGTCATGGGAACGTTACAGCGTTCTGGTCAGCCGTGTGGATCCTGCAAATCCTGAATGGCCGGAAATGCCGCAATAA |
| 10 | *FI* through *ogr* of P2 | ATGAGTGACTATCATCACGGCGTGCAGGTGCTGGAGATTAACGAGGGCACCCGCGTCATTTCCACCGTATCCACGGCCATTGT CGGCATGGTCTGCACGGCCAGCGATGCAGATGCGGAAACCTTCCCCCTCAATAAACCTGTGCTGATTACCAATGTGCAGAGCG CAATTTCAAAGGCCGGTAAAAAGGCACGCTGGCGGCATCGTTGCAGGCCATCGCTGACCAGTCAAAACCGGTCACCGTTGTC ATGCGCGTGGAAGACGGCACCGGTGATGACGAGGAAACGAAACTCGCGCAGACCGTTTCCAATATCATCGGCACCACCGATG AAAACGGTCAGTACACCGGACTAAAAGCCATGCTGGCGGCGGAGTCGGTAACCGGTGTTAAACCGCGTATTCTCGGCGTGCC GGGACTGGATACCAAAGAGGTGGCTGTTGCACTGGCATCAGTCTGTCAGAAGCTGCGTGCTTTCGGGTATATCAGCGCATGG GGCTGTAAAACCATTTCCGAGGTGAAAGCCTATCGTCAGAATTTCAGCCAGCGTGAGCTGATGGTCATCTGGCCGGATTTCCTC GCATGGGATACGGTCACCAGTACCACCGCCACCGCGTATGCCACCGCCCGTGCGCTGGGGCTGCGCGCTAAAATCGACCAGG AGCAGGGCTGGCATAAAACGCTGTCCAATGTCGGGGTGAACGGTGTTACCGGCATCAGCGCATCTGTATTCTGGGATTTGCAG GAGTCCGGCACCGATGCTGACCTGCTTAACGAGTCAGGCGTCACTACGCTGATTCGCCGCGACGGTTTCCGCTTCTGGGGTAA CCGTACCTGCTCTGATGACCCGCTGTTCCTCTTTGAAAACTACACCCGCACCGCGCAGGTCGTGGCCGACACGATGGCTGAGGC GCACATGTGGGCGGTGGACAAGCCCATCACTGCAACGCTGATTCGCGACATCGTTGACGGCATCAATGCCAAATTCCGTGAGC |
| | | TGAAAACAAACGGCTATATCGTGGATGCGACCTGCTGGTTCAGCGAAGAATCCAACGATGCGGAAACCCTCAAGGCCGGAAA ACTGTATATCGACTACGACTATACACCGGTGCCTCCTCTCGAAAACCTGACCCTGCGCCAGCGTATTACCGATAAATACCTGGC AAATCTGGTCACCTCGGTTAACAGCAATTAAGGAGCCTGACCGATGGCAATGCCGCGCAAACTCAAGTTAATGAACGTCTTTCT GAACGGCTACAGCTATCAGGGCGTTGCAAAGTCCGTCACGCTGCCAAAACTGACCCGTAAGCTCGAAAACTATCGCGGTGCGG GGATGAACGGCAGCGCACCGGTAGACCTCGGCCTTGATGACGATGCGCTGTCAATGGAGTGGTCGCTCGGTGGCTTCCCGGA TTCGGTTATCTGGGAGCTTTACGCCGCAACCGGTGTGGATGCCGTGCCGATTCGTTTTGCAGGCTCTTACCAGCGCGACGATAC CGGCGAAACGGTGGCCGTCGAAGTGGTCATGCGTGGACGTCAGAAAGAAATCGACACCGGCGAGGGTAAACAGGGAGAAG ACACTGAGTCGAAAATCTCCGTGGTCTGCACCTATTTCCGGCTGACGATGGACGGTAAGGAGCTGGTCGAAATTGACACCATC AACATGATTGAGAAGGTGAACGGCGTCGATCGGCTGGAGCAACACCGCCGCAATATCGGCCTGTGATTTTCATCCGGTCAGCC TGGCTGGCCGGTTAACCCTGATTCAGAAGTGAGAAAACCATGAACAAAGAAAATGTCATTACCCTGGACAATCCGGTCAAACG TGGTGAGCAGGTTATCGAACAGGTCACGCTGATGAAACCCAGTGCCGGGACGCTACGCGGTGTCAGTCTGGCTGCGGTTGCA AACTCCGAAGTCGATGCACTGATTAAGGTGCTGCCGCGCATGACGGCACCGATGCTGACCGAGCAGGAAGTCGCCGCGCTGG AACTGCCTGACCTTGTGGCGCTGGCCGGTAAGGTGGTCGGTTTTTTGTCGCCGAACTCGGTGCAGTGACGTTTCCGAAAAATC TCTCGGTCGATGACCTGATGGCGGATGTGGCAGTGATATTTCACTGGCCGCCATCAGAACTGTATCCCATGAGCCTGACCGAA CTCATCACATGGCGCGAAAAGGCGCTCCGGCGAAGCGGAAACACGAATGAGTAACAATGTAAAATTACAGGTATTGCTCAGG GCTGTTGACCAGGCATCCCGCCCGTTTAAATCCATCCGCACAGCGAGCAAGTCGCTGTCGGGGGATATCCGGGAAACACAAAA ATCACTGCGCGAGCTGAACGGTCACGCATCCCGTATTGAGGGATTCCGCAAGACCAGTGCACAGCTCGCCGTGACTGGTCATG CACTTGAAAAGGCACGGCAGGAGGCCGAAGCCCTTGCCACACAGTTTAAAAACACCGAACGTCCGACCCGTGCTCAGGCGAA AGTCCTGGAATCCGCAAAGCGTGCGGCGGAGGACTTACAGGCGAAATATAACCGCCTGACAGATTCCGTTAAACGCCAGCAG CGGGAACTGGCCGCTGTGGGAATTAATACCCGCAATCTTGCACATGATGAGCAGGGACTGAAAAACCGTATCAGTGAAACCA CCGCACAGCTTAACCGTCAGCGTGATGCGCTGGTGCGTGTCAGTGCGCAACAGGCAAAACTTAACGCAGTAAAACAGCGTTAT CAGGCCGGAAAGGAACTGGCCGGAAATATGGCCTCAGTGGGCGCTGCCGGTGTGGGGATTGCGGCGGCGGGAACGATGGC CGGTGTTAAGCTACTGATGCCCGGTTATGAGTTTGCGCAGAAAAACTCAGAATTACAGGCTGTGATCGGAGTGGCAAAAGACT CCGCCGAAATGGCCGCACTCCGCAAGCAGGCGCGCCAGCTCGGCGACAATACCGCCGCCTCGGCAGATGATGCAGCCGGTGC GCAGATTATTATTGCGAAAGCCGGTGGGGATGTTGATGCCATTCAGGCGGCAACGCCGGTCACGCTGAACATGGCGCTGGCG AACCGTCGCACAATGGAAGAAAACGCCGCCCTGCTGATGGGGATGAAATCCGCCTTTCAGCTTTCAAACGATAAGGTCGCTCA TATCGGGGATGTTCTCTCCATGACGATGAACAAAACCGCCGCCGATTTTGACGGCATGAGCGATGCGCTGACCTATGCCGCAC |
| | | CTGTGGCAAAAAATGCCGGTGTCAGCATTGAAGAAACCGCCGCAATGGTCGGGGCGCTGCATGATGCAAAAATCACAGGCTC AATGGCGGGGACGGAAGCCGTGCCGTGTTAAGCCGCCTGCAGGCACCGACGGAAAAGCATGGGATGCACTCAAAGAGCT TGGAGTGAAAACCTCAGACAGCAAAGGAAACACCCGGCCAATATTTACCATTCTGAAAGAAATGCAGGCCAGTTTTGAGAAAA ACCGGCTCGGTACTGCCCAGCAGGCTGAATACATGAAAACTATTTTCGGGGAGGAGGCCAGCTCAGCCGCTGCCGTGCTGAT GACTGCCGCCTCAACCGGAAAGCTGGACAAACTGACCGCTGCGTTTAAAGCCTCAGACGGAAGACCGCCGAGCTGGTAAAT ATCATGCAGGACAACCTAGGCGGTGACTTTAAAGCGTTTCAGTCCGCTTATGAGGCGGTGGGGACTGACCTGTTTGACCAGCA GGAAGGCGCGCTGCGTAAGCTCACGCAGACGGCCACAAAGTATGTGTTAAAACTCGACGGCTGGATACAGAAAAACAAATCA CTGGCGTCAACCATCGGCATCATTGCCGGCGGTGCACTGGCGCTTACTGGCATCATCGGTGCCATTGGCCTCGTAGCCTGGCC GGTTATCACCGGCATCAATGCCATCATCGCGGCAGCAGGCGCAATGGGGGCAGTCTTCACGACGGTTGGCAGTGCTGTTATGA CCGCCATCGGGGCTATTAGCTGGCCGGTTGTGGCCGTGGTGGCTGCCATTGTCGCCGGTGCGTTGCTTATCCGTAAATACTGG GAGCCTGTCAGCGCATTCTTTGGTGGTGTGGTTGAAGGGCTGAAAGCGGCATTTGCGCCGGTGGGGGAACTGTTCACGCCAC TTAAACCGGTTTTTGACTGGCTGGGCGAAAAGTTACAGGCCGCGTGGCAGTGGTTTAAAAACCTGATTGCCCCGGTCAAAGCC ACCCAGGACACCCTGAACCGTTGCCGTGACACGGGCGTCATGTTCGGGCAGGCACTGGCTGACGCGTTGATGCTGCCGCTTAA TGCGTTCAACAAACTGCGCAGTGGTATTGACTGGGTACTGGAAAAACTCGGTGTTATCAACAAAGAGTCAGACACACTTGACC AGACCGCCGCCAGAACTCATACCGCCACGTATGGTACCGGTGACTATATTCCGGCGACCAGCTCTTATGCAGGCTATCAGGCTT ATCAGCCGGTCACGGCACCGGCTGGCCGCTCTTATGTAGACCAGAGTAAAAACGAATATCACATCAGCCTGACGGGGGGGAC TGCGCCGGGGACACAGCTTGACCGCCAGTTACAGGATGCGCTCGAAAAATACGAGCGGGATAAACGTGCGCGCGCCCGTGCC AGCATGATGCATGACGGTTAAGGAGGTGACGAAAAATGATGCTCGCGTTAGGTATGTTTGTTTTTATGCGCCAGACGCTGCCA CACCAGACCATGCAGCGTGAATCAGATTATCGCTGGCCGTCAAATTCCCGTATCGGTAAACGGGATGCCTTTCAGTTTCTCGGT GTGGGTGAGGAAAACATCACGCTGGCCGGTGTGCTTTATCCCGAACTGACCGGCGGCAAGCTGACGATGACCACGCTCAGGC TGATGGCAGAGGAGGGGCGGGCGTGGCCGTTGCTGGATGGCACCGGCATGATTTACGGCATGTATGTCATCAGCAGGGTGA GTGAAACAGGGAGTATTTTCTTTGCAGACGGCACACCCCGGAAAATTGATTTTACGCTGTCACTCACCCGCGTTGATGAATCAC TGGCCGCGCTTTATGGCGATATCGGTAAACAGGCGGAATCGCTCATCGGTAAGGCCGGCAGTATGGCGACCAGATTCACAGG TATGACGGGGGCGGGATAATGCTGGATGCGCTGACATTTGATGCAGGCAGTACGCTGACGCCGGATTACATGCTGATGCTCG ACAGCAGGGATATTACCGGCAATATCAGCGACCGTCTGATGAGCATGACCCTGACGGATAACCGGGGCTTTGAGGCTGACCA GCTTGATATTGAACTGAACGATGCCGACGGGCAGGTCGGGCTGCCGGTTCGTGGCGCTGTCCTGACGGTGTATATCGGCTGG AAAGGTTTTGCCCTGGTATGCAAAGGGAAATTTACCGTTGATGAGGTTGAACACCGGGGCGCACCGGATGTAGTCACCATCCG |
| | | CGCCCGGAGTGCAGATTTCGCGGACGCTCAATTCCCGCCGGGAAGGCTCCTGGCATGACACCACGCTCGGTGCGATTGTTA AGGCGATAGCCACCCGTAACAGGCTGGAAGCCAGTGTCGCTCCGTCACTGGCCGGAATAAAAATTCCACACATCGACCAGTCG CAGGAGTCTGATGCGAAATTCCTGACCCGTCTTGCAGAACGCAACGGCGGTGAGGTGTCGGTAAAAATGGGAAAACTGTTGT TTCTCAAAGCGGGGCAGGGAGTGACGGCCAGCGGTAAAAAAATCCCGCAGGTCACCATAACCCGCAGCGACGGCGACCGCCA TCATTTTGCGATTGCTGACCGTGGAGCCTACACCGGTGTAACGGCAAAATGGCTACACACTAAAGACCCGAAGCCGCAAAAGC AGAAGGTAAAACTGAAACGCAAAAAGAAAGAGAAACACCTGCGCGCACTGGAGCACCCGAAAGCGAAACCGGTCAGGCAGA AGAAAGCGCCTAAAGTACCGGAAGCGCGTGAAGGTGAATACATGGCCGGTGAGGCTGACAACGTTTTTGCCCTGACCACGGT ATATGCCACGAAAGCGCAGGCCATGCGCCGCTCAGGCGAAGTGGGATAAACTGCAACGGGGCGTTGCGGAGTTCTCTATC AGCCTGGCTACCGGTCGGGCAGATATTTACACGGAAACACCGGTCAAAGTGTCTGGCTTTAAGCGCGTCATAGACGAGCAGG ACTGGACAATCACTAAGGTGACACATTTTCTGAATAATAGCGGCTTCACGACGTCCTTAGAGCTTGAGGTCAGGCTTTCTGATG TGGAGTACGAAACAGAAGATGATGAGTGATGTTTTTGTTTTATCTGTTTGTTTTGTAAGGATAAATTAACTAAAATGGCACCAT CAACAAAACCGGAAGAGGTGCTCGCGATGTTTCATTGTCCTTTATGCCAGCATGCCGCACATGCGCGTACAAGTCGCTATATCA CTGACACGACAAAAGAGCGTTATCATCAGTGCCAGAACGTGAATTGCAGCGCCACGTTCATCACTTATGAGTCGGTACAGCGA TACATCGTGAAGCCGGGAGAAGTCCACGCCGTAAGGCCGCACCCGTTGCCATCAGGGCAGCAAATTATGTGGATGTAA |
| 11 | S. aureus phi11 packaging signal | ANGATTTANTCC |
| 12 | *cpmA* | MKTESYFKEYNQFVLDQHKAIQELEQERNALESKIKLDKSTYKQLIMDGQDDKADNLYQATDADEKKLKALNKRLETKKSVSKEVKY QKTIELLKHQSELSSLYESEKQSAIEKLKKAVDAYNEIIDEIEDINDRYEDEHQQYASVYSQEQLYDDKEARKALNGHFKENIFTSFINGN DLPYEHNNKLFLKC |
| 13 | *cpmB* | MKTKYELNNTKKVANAFCLNEEDTNLLINAVDLDIKNNMQEISSELQQAEQSKQKQYGTTLQNLAKQNRIIK |
| 14 | *ptiA* | MDKQQIKDFVCDYHERTRSDVLIDDDINTDEFFSIADENSNEWMADDNIDDHIVKNHLEMIVDRVANDKEFYIFDSLIQGRSYQDIS GVLDCSEQSVRFWYETLLDKIVEVIE |
| 15 | *ptiB* | MESIAEKETYHLPTEHLQVFNVIKNTSNKYITKTILNQLGYEYNSSNERWLRRVINSLVYDGYPIGCSYKPSERGYYIITTEQEKQQA MRSIKKLADGSMKRYEALKRIEV |
| 16 | *ptiM* | MIAYPIRVGSVYRGEQMKLLKTKNCLYYRNGDNKLSEYQLLTQFNPTFINKKIRMCEFQIESMYHMSASTTTCDEMMGVVSVSYPIE KLVIKIIETKARLQNYKNRSISNMVLLKTVLNHYTEKEQKKVVKYMRSNGRYKPYNVIERLQVDLYQASIKQRSERQKQRNIAIENSKIA RVNAYHQSSYVKVV |
| 17 | gene #29 (*terS*) through gene #53 (*lysin*) of phi11 | atgaacgaaaaacaaaagagattcgcagatgaatatataatgaatggatgtaatggtaaaaaagcagcaattacagcaggttatagtaagaaaacagc agagtctttagcaagtcgattgttaagaaatgttaatgtttcggaatatattaaagaacgattagaacagatacaagaagagcgtttaatgagtattacagaagcttta gcgttatctgcttctattgctagaggagaacctcaagaggcttacagtaagaaatatgaccatttaaacgatgaagtggaaaaagaggttacttacacaatcacacca acttttgaagagcgtcagagatctattgaccacatactaaaagtacatggtgcgtatatcgataaaaaagaaattactcagaagaatattgagattaatattggtgagt acgatgacgaaagttaaattaaactttaacaaaccgtctaatgttttcaatagaaacatattcgaaatactaaccaattacgataacttcactgaagtacattacggtg gaggttcgagcggtaagtctcacggcgttatacaaaaagttgtactcaaagcattgcaagattggaaatatcctaggcgtatactgtggcttagaaaagtacaatcaa caattaaagatagtttgttcgaagatgttaaagattgtttgataaactttggtatttgggacatgtgcctttggaataagactgataacaaagttgaattgccaaacggc gcagtttttttgtttaaaggattagataacccagagaaaataaagtcgataaaaggcatatcagacatagtcatggaagaagcgtctgaattcacactaaatgattac acgcaattaacgttgcgtttgagggagcgtaaacacgtgaataagcaaatatttttgatgtttaacccagtatctaaactgaattgggtttataagtatttctttgaacat ggtgaaccaatggaaaatgtcatgattagacaatctagttatcgagataataagtttcttgatgaaatgacacgacaaaacttagagttgttagcaaatcgtaatcca gcatattacaaaatttatgcgttaggtgaatttgctacactagacaaattggttttccctaagtatgaaaaacgtttaataaataaagatgagttaagacatttaccttct tattttggattggactttggctacgttaatgatcctagtgcttttatacattctaaaatagatgtaaagaaaaagaagttatacatcattgaagagtatgttaaacaaggt atgctgaatgatgaaatagctaatgtcataaagcaacttggttatgctaaagaagaaattacagcagatagtgcagaacaaaaaagtatagctgaattaaggaatct agggcttaaaaggatttaccaaccaaaaaagggaagggctcggttgtacaagggttacaattcttaatgcaatttgaaatcattgttgatgaacgttgtttcaagact attgaagagtttgacaactacacatggcaaaaggacaaagatacaggtgaatataccaatgaaccagtagatacatacaatcattgtatcgattcgttgcgttattca gtggaacgattctacagaccggttagaaaacgcacaaatctcagttcgaaagttgacacaataaaatctctaggattataggagggaacaaatgttaaaagtaaacg aatttgaaacagatacagatctacggggaaacataaattacttatttaatgatgaagccaatgttgtttacacatatgacgggacggaatccgatttattacaaaacgt taatgaagtaagtaaatacattgaacatcacatggattaccaacgacctagattgaaagtgttaagtgattattacgaaggtaaaactaagaacttagttgagttaac acgacgcaaagaagagtacatggcagataaccgtgtagcgcatgattacgcatcttatattagcgattttatcaacggctatttcttgggtaatccaattcaatatcaa [00119] |
| | | gatgatgacaaagatgtattagaagttattgaggcgttcaatgatttaaatgatgttgagtcacacaatagatctttaggattagatttgtcaatttatggcaaagcttat gagttaatgattagaaaccaagatgatgaaacgcgtttatacaagagtgatgcaatgagtacttttgtcatatacgacaatacaattgaacgtaatagtatcgcaggc gttagatatttaagaactaaaccaatagacaagactgacgaagatgaagtgtttacagttgatttattcacttcacacggtgtttatagatatcttaccagtagaacaa atggattgaagctcacaccacgtgaaaacggttttgaatcacactctttcgaacgtatgcctattacagaatttagcaacaacgaaagaagaaaaggggattatgag aaagtaatcactttaattgatttgtatgataatgctgaatcagatactgctaactatatgagtgatttaaatgacgctatgttacttattaaaggtaatttaaatttagatc ctgtagaagttagaaaacaaaaggaagctaacgtgttgtttttagaaccgactgtttatgctgatagcgaaggtagagaaacagaaggctctgttgatggtggttatat ttataagcaatacgatgtacaaggtaccgaagcttataaagaccgtttaaacagtgatatacacatgtttaccaacacgcctaacatgaaagatgataactttagcgg cactcaatcgggcgaggcaatgaaatacaaattatttggattggaacaacgtactaaaactaaagaaggattgtttactaaagggttaagacgtcgtgctaagttgtt agagacaatacttaaaaatacatggtcgattgacgctaacaaagatttcaatactgttagatacgtatacaacagaaacttacctaaatcattgattgaagaattaaa agcttatattgattctggtgggaagattagccaaacaactttaatgtctctattctcgttcttccaagaccctgaattagaagttaagaaaatcgaagaagatgagaaa gaatctattaaaaaagctcaaaaaggtatttataaagaccctagagacatcaatgatgacgaacaagatgatgatacaaaagatactgttgataaaaaggaatgatt gtaattgcctaacaaaaacactcaagaatattgggaagaacgcggacgcaaagcaatcgagaatgagttgaagcgtgataaaactaaagctgaagaaatagaacg tatattgaatatgatgattaagcgcattgaaaaagagatcaatgcgtttattgtcaagtacggagattttgcaggcgttacattacaagaagcacaaaagattattgat gagttcgatgtaaaagcgtttcaagaagaagcaaaaagattggtcgaaaacaaggagtttagcgatagagcaaatgaagaattaaagaagtataacacgaaaatg tatgtatctagagaacagatgttaaagattcaaatagaattcttaattgcttatgcaacagctcaaacagaattatcgatgagggaatatttcgaatcaacagcttatc gtgtgttcagtgatcaagcgggtatttaggtgaaggtgtacaagtagctaaagaagttatagatacaatcgttgatacacaatttcatggtgtcgtttggtcagagcga ttatggactaataccgaagcaatgaaacaagaagtagaagaaataattgctaatgtagttattagaggtcgacatcctaatgaatatgttaaagatatgcgcaagca cttaaataaattcgaaggcacagcacgacaaaagaccgcagcaattaaatcattgctttatacggaatcggcacgtgttcacgcacaatcaagcattgacagcatga aagaaatttcaccggaaggatattatatgtatattgcaaaaatcgataatagaacaactaaagtatgcaaaggcttaatggagaaatattcaaagttaaagacgct aaaattggtgttaatttctatcctatgcatatcaattgtcgttcagattgcgctttactacctaaatctatgtggccgaaaaaaccaagcaagaaacgaaaaacaaaat acttcggagggaaagtgaaaagcggtgattgatttaaaagtgaagtttttaaaggcaagttagttttgtatgacagtaaattaaatgtttggaggatactaatatgag taatactgacaaataccttagagacatagcaagagaattaaaaggtatacgtaaagagttacaaaagcgaaacgaaacagttattattgatgcaaacttagacagtt taaggtcggcagtattagccgataaagaaaaatcgaaatataatgaacctctcttttaatagctagcacttaattgtgttggctatttttatgtccaaaacgtgctgatg acataaaaagcacgcatggaaaaacagtcgacagactataaatggaggtatatctcatggaagaaaataaacttaagtttaatttgcaatttttgcagaccaatcag atgatccggacgaaccaggcggagatggtaaaaaggaaatcctgataagaaagaaaatgacgaaggtactgaaataactttcacgccagagcaacaaaagaaa gttgatgaaatacttgaacgtcgtgtagcccacgaaaagaaaaaagctgatgagtatgcaaaagaaaaagcagcagaagctgctaaagaagctgctaaattagcg aaaatgaacaaggatcaaaaagatgaatatgaacgcgaacaaatggaaaaagaactggaacaattacgttcagaaaaacaattaaacgaaatgcgttcagaagc |
| | | acgaaaaatgttgagtgaagcggaagttgattcatcagatgaggttgtcaatttagttgtaacagatactgctgaacaaactaaattgaatgttgaagctttttctaatg cagtaaaaaaagcggttaatgaagcggttaaggttaacgctagacaatcgccattgactggtggagattcatttaatcactcgactaaaaataaaccgcaaaactta gctgaaatagctagacaaaaaagaattattaaaaattaacggaggcatttaaatggaacaaacacaaaaattaaaattaaatttgcaacattttgcaagtaacaatg ttaaaccacaagtatttaaccctgacaatgtaatgatgcatgaaaagaaagatggcacgttgttaaacgactttacaacacctatcttacaagaggttatggaaaact ctaaaatcatgcaattaggtaagtacgaaccaatggaaggtactgagaagaagtttactttttgggctgataaaccaggtgcttactgggtaggtgaaggtcaaaaaa tcgaaacgtctaaggctacttgggttaatgctacaatgagagcgtttaaattaggggttatcttaccagtaacaaaagaattcttgaattacacttattcacaattctttg aagaaatgaaacctatgattgctgaagctttctataaaaagtttgacgaggcaggtattttgaatcaaggtaacaatccgttcggtaaatcaattgcacaatcaattga aaaaactaataaggttattaaaggtgacttcacacaagataacattattgatttagaggcattgcttgaagatgacgaattagaagcaaatgcatttatctcaaaaac acaaaacagaagcttgttacgtaaaattgtagatcctgaaacgaaagaacgtatttatgaccgtaacagtgattcgttagacggtctacctgtggttaaccttaaatca agcaacttaaaacgtggtgaattaatcactggtgacttcgacaaattgatttatggtatccctcaattaatcgaatacaaaatcgatgaaactgcacaattatctacagt taaaaacgaagatggcacacctgtaaacttgtttgaacaagacatggtggcattacgtgcaactatgcatgtagcattgcatattgctgatgataaagcgtttgctaag ttagttcctgctgacaaaagaacagattcagttccaggagaagtttaataaataattaggagtggtaacatgcccgaaatcattggaattgttaaagtagattttacag atttagaagataacagacatgtctatatgaaagggcatgtctaccctcgtaaaggttataatcctacagatgaacgtatcaaagctttagctagtgttgaaaataaacg caacaaacaaatgatttacattgtaaatgacaaattaaccaaaaaagaacttgtcgaaatagcaagtgttgctggcttacaagttgatgaaaaacaaacaaaagctg aaattatcaatgcttttgagtcactagagtaggtggttatatgactacgctagctgatgtaaaaaaacgtattggtcttaaagatgaaaagcaagatgaacaattaga agaaatcataaaaagttgtgaaagccagttgttatcaatgttacctattgaagttgaacaaataccggaaaggtttagttacatgattaaagaagttgcagttaaacgc tacaacaggattggtgctgaaggtatgacatcagaagcggttgacggacgtagcaatgcgtatgaattgaacgatttcaaggagtatgaagctattattgataattac tttaatgctagaacgagaactaaaaaggaagggctgtgttcttttgagatatgaagatagagttatttttcaattagaacaagtagcaacttacaatcctaaaactag caaaaaagaaaacacactaatcacttatgatgcgataccatgcaatattaaccccatttctagagcaagaaagcaacttgaatttggtgatgtaaaaaacgatgtaa gtgttctgaggataaaagaatcaatatcttaccctgttagccacgtgttggttaatggcattcgctacaagatagttgatacaaggatatacagacacgaaacgtcata ttatatcgaagaggtcaattgatgaatatagatggattagacgcactgttaaaccaatttcacgatatgaaaaccaacattgatgatgatgtagatgatattttacagg aaaacgccaaagaatatgtagtacgagctaaattgaaagctagagaagtaatgaataagggttattggactggtaatttatcacgcaatatcagatataaaaaaact ggcgatttgcaatacactatcacatcgcacgcagcttatagtggtttcttagaatttggtactcgatacatggaggctgaaccttttatgtggccggtatacgaagtgatt aggaaatcaactgtagaagaattgaaagcgttgtttgaataggagataaaagcatgacaccgaacttacaactttataataaagcgtatgaaacgctacaaggatat ggattccctgttatttctcgtaaagagatgcaacaagagattccgtatcctttttttgtaataaaaatgccggagtcaaatagaagtaagtacacgtttgatagttattct ggcgatacgaatttagttattgatatttggagtgtaagcgatgatttaggacatcatgacggacttgttaaaagatgtattgatgatttaacacctagcgttaaaacaaa cgattatgactttgaagaagaagatactaacatcacacagttagttgatgatactaccaatcaagaattgctacacacatcagtaacgatatcttacaaaacatttaa |
| | | aaaacggaggaatattgaatggcaaatatgaaaaatagtaatgatcgtattattttatttagaaaagctggcgaaaaagtagatgctactaaaatgctttttttaactg aatacggcttatcacatgaagctgatacagatacagaggatacaatggacggttcttataacactggtggttctgttgagtcaacaatgtctggtactgctaaaatgttt tatggtgacgattttgcagatgaaattgaagatgcagttgtagatcgcgtattgtatgaagcttgggaagttgaaagtagaataccaggcaaaaatggggattccgct aaatttaaagcgaaatatttccaaggtttccacaataaatttgaattaaaagcagaagctaacggtattgatgaatatgaatatgaatatggagtgaatggtcgtttcc aacgtggatttgcaacactacctgaggctgtaacaaagaaacttaaggcgactggatacagattccacgacactacaaaagcagatgcattaactggcgaagattta acagcaattccacaacctaaagtagattcaccaccggttgcaccaagagaggtataaaaatagggcgttaagcccttttttatttgtttaaattaattatgaatggagat tttaagttatgaatgtagaaattaacggaaagtcattagaattaagttttggttttaaatttttaagagaaatcgataaccgattaggtttaaaagttgaacaagcttcta tcggtcaaggtgtatcaatgttgcctgtaggtttagaaagtggaaatccggttgtgattggcgaagttttaatcgcagctacatctcacttaaaaaaacaagcaattact attaataacattgatgaagcattagatgaaatcgcagaaaatatcggactagaagaattcggttcggatattttaacggagttgggaaagcgacctatgacccgaaa cctagtcgaagtagtggaaacggaagaaaaaccagcggaagcctaataacttacgacagaatcgttataacttgtatgtcaacacttggtattacagatttgaacgtt attgagcaaatgacattaacagaatataactatcgaatgtatgcgaaagagtatgaaatgctaacccaagaattcgaacgttacaaacttgcgtttgctattcgtgatg ctgcagctactaaaaatgttgggacagaaaataaacctaaagaggaatatgtttttaacaatgcaaacgacgtattgccttatgaagaaaatatccaacggcttaacg aaggtaaagatataagatttagtagcgaacgtgatgaatacgaaccacaaaataatgaattctttaaagttatagcagaatttaataagcaatagaaagagaggtgt taatgtgacggaatataaaattaaagcgactattgaagctagtgtagccaaattcaaaaggcaaattgatagtgcggttaagtctgtgcaaagatttaaacgagtagc agatcaaactaaagatgttgaattaaacgctaacgataaaaatttacaaaaaactatcaaagttgctaaaaagtctttagatgcctttagtaacaaaaatgtaaaagc taaattagatgctagtatacaagatttacaacaaaaggtactagaatcgaattttgaactagacaaactaaactctaaagaagttacaccagaagttaagttgcaaa aacaaaagttgattaaagatatcgctgaaacagaagctaaattatcagaattagaaaagaaacgtgtcaatattgacgtcaatgcagataacagtaaattcaatcga gtgttaaaagtatctaaagctagtctcgaagcattaaataggtctaaagccaaagctattatagacgtggacaatggtgttgctaactctaaaatcaaacgtactaaa gaagaacttaaaagtattccgaacaaaactagatctcgacttgatgtagatacagggctttctataccaactatttatgcgtttaaaaaatcattagacgcattgccga acaaaaaaacaacaaaggtagatgtcgatactaatggtttaaagaaagcttatgcctacataataaaagcaaatgacaattttcaaagacagatggggaatttagct aatatgttccgtgtgttcggtactgtaggttctaatatggttggtggattacttacatcatcttttagtatcttaatacctgtaatagcgagcgtagtacctgtagtatttgc gctattaaacgctatcaaagtgttaactggtggtgtacttgctttaggtggtgccgtagcaatagcgggagcaggatttgtagcgtttggcgcaatggctatcagcgcta taaagatgcttaatgatggcactttacaagctagctcagcaacaaacgaatacaaaaaagcgttagatggcgtaaagtcagcatggactgatattataaagcaaaat caatccgctatcttcacaactcttgcaaatggtttaaatactgttaaaactgcaatgcagagcttacaaccgtttttagtggtatttcaagaggaatggaagaagcgtc tcaaagcgtgcttaaatgggctgaaaatagcagtgtagcttcaagattctttaatatgatgaatacaacgggtgtttcggtatttaacaagctattaagtgctgcaggtg gttttggtgacggattagtcaatgtattcacgcaattagcaccactgtttcaatggtcggctgattggttggatagattaggtcaatctttctctaactgggctaatagtgc agctggagaaaattcgataactcgttttattgaatacacaaaaacaaacttacctatcattggtaatatttcaaaaatgttttcgttggaattaacaatttgatgaatgc |
| | | attcagcggatcatcaactggcatattccaatctcttgaacaaatgacagctaagtttagggaatggtctgaacaagtaggacaatctcaagggtttaaagactttgtc agttatatacaaacaaatggaccactaataatgcaattgattggaaacatcgcaagaggattagttgcattcgcaacagcaatggctcctatagctagtgcagtatta cgcgttgcagttgcaataactggttggatagctaacttgtttgaggcgcatccagctacagcacaattagttggtgtcattataactttagttggtgcatttagatttttaa taccgattattcttgctgtatctaactttatgggtggcggattaataggtagaatcattgcattagtaagtaagttcggtttattaagagcgggattaacaattttaaaag gtgcgttcatgttattaaaaggaccattaaaaattatatcagttatattccaattgttattcggtaagattggattaattagaaatgctatcacaggactagtaactgtgtt tggtattttaggcggtccaataacaatagtaattggtgtaattgctgcattaatagctatattcgttttattgtggaataaaaatgaaggattcagaaactttattataaa tgcttggaatgcgataaaaacgtttatggttaatgtttggaatgtattaaaagctgtagcttcggttgtatggaatgctattttaacagctatcactacagcagtatcgaa tgtttacaattttataatgattgtttggaatcaaatagtcgcttatttacaagggctatggaatggaattatcgctattgcaacaacagtatggaacctttagttacaatc attacaactgttttcacgacgataatgacaatagttatgacgatatggacagctatttggacgttcttaagtacaatctggaatacgataattacaatcgctactacgat ttggaatttgttagtcactgtaataactacagtgtttaccacaattatgactatcgcaataacaatttggaacgctatttggacgttcttacaaacgttgtggaacactat agttactgtggcaactaaggtttggaacgctatcactacagctatatctactgcgttacaagcggcatggagttttatttctaatatatggaatacgatttggagtttctta tctggtatattaacgacaatttggaataaagttgtaagcatattcacacaagttgtttcaactatatcagacaaaatgtctcaagcttggaacttcattgtcactaaaggt atgcaatgggtatctactataacaagtacgctaattaactttgttaatagagttgttcaaggattcgttaatgttgtaaacaaagttagtcaaggtatgacaaatgcagt aaataaagttaaaagctttgtggatgactttgtatcagcaggtgctgatatgatccgtggtttgatgagaggtattggtaatatggctagagacttagctgaaaaagca gctagtgtagcaaaaggtgctttaaatgcagccaaaagagcgctaggtattcactcaccttcacgtgaattcatggatgttggtatgtattcaatgttaggtttcgttaa aggtatagataatcattcaagtaaagttatccgtaatgtttctaatgttgcagataaagtagttgatgcatttcaacctacattaaacgcacctgacatttctagtattac aggaaacttaagtaatttaggtggaaatataaatgcgcaagtacaacacacacattctattgaaacatcaccgaacatgaaaactgttaaagttgaattcgatgtcaa taacgatgcgcttactagtattgttaacggcagaaatgctaaacgcaattctgagtattacttataaaggaggttacaaatggacatagaattaacaaaaaaagatgg tactgtaatcaaattaagtgaatacgggtttatcgttaacgatatagtaattgatagcatgcaaatcaacacaaagtatcaagacaaagaaaatatgaacggtcgtat attaatggggagcaattatatcagtagagatatagttgttccttgtttttgtaaagttaaaaatcgttcagacattgcttatatgcgagatatgttgtattcgttaacgaca gacatagaacctatgtatttgcgagaaatcagaagaaaagaagagttgaattacaggtttactcaaccaacttctgatgattacgtgaaattagataaaaacaacttc ccggattacgaatattcaagacacgatcaacaaatttatgtaaatggtaaacagtataaagttatttttaacggagttataaaccctaaacaaaaaggtaataaagttt cttttgaactaaaattcgaaactacagaattaccatacggtgaaagtattggaacaagcctagagttagaagaaacaaaaaggttggattgtggtcgtttgattttaa tattgattggcatgcaggcggagacaaaagaaagtatacatttgaaaatttgagcaaaggtacagtttactatcatggtagtgctcctaacgaccaattcaacatgtat aaaaagataacaattattttaggcgaagatacagaatcgtttgtatggaatttaacgcatgctgaaataatgaaaatcgaaggatcaaactaaaagctggagacag aattgtttatgatagcttccgagtttataaaaacggtgttgaaataagtaccgaaacgaatatagcccaaccaaaatttaaatacggagctaataaatttgagtttaat caaacggtacaaaaagttcagtttgatttgaaattttattataagtaggtgtcagaatgacaataactattaaaccacctaaaggtaatggcgcacctgtaccagtaga |
| | | aacaactttagtaaaaaaagttaatgctgacggtgtattaacttttgatattctagaaaataaatatacttatgaagttattaacgctatagggaaaagatggattgtta gtcatgtcgaaggtgaaaacgacaagaaagaatatgtaataactgtcattgataggaaatcagaaggcgacagacaactggttgaatgtactgctagagagattccc atagacaagttaatgattgatagaatttatgttaatgtaacaggatcttttacagtagaaagatattttaacattgtgtttcaaggtactggaatgctttttgaagtcgag ggcaaagttaaatcttcaaagtttgaaaatggtggtgaaggcgatacaaggttagaaatgtttaaaaagggattagaacatttcggtttagaatataaaataacgtat gacaaaaagaaagacagatataagtttgtattgacgccttttgcaaatcaaaaagcgtcttatttatttctgacgaagtcaacgccaacgctataaaactcgaggaa gatgcaagtgatttcgccaccttcattagaggatatggtaattattcaggagaagaaacattcgaacacgctgggctcgtaatggaagctagaagtgcattagctgaa atatacggcgacatccacgcagaaccatttaaagatggtaaagtgactgaccaagaaactatggataaagaattacaatcgagattgaaaaagtcgttaaaacaat ctttgtctttggactttttggtgttaagagaatcatatccagaagcagacccacaacccggagacatagtacaaataaaatctaccaaactaggtttgaatgatttagtc cgtatagtacaagttaaaacgattaggggtataaacaatgtaattgttaagcaagatgtaacgcttggtgagtttaatcgagaacaacgatatatgaaaaaagttaat actgcagctaactatgtttctggattaaatgatgttaacctttctaatcctagtaaagcggcagaaaacttgaagtctaaagtagcgtcaatagctaaatcaacactcg atttgatgagtagaactgatttgattgaagataaacaacagaaggtaagctctaaaactgtgactacatctgacggcactatcgttcatgattttatagataaatcaaa cattaaagatgtaaaaacgattggaacgattggcgattctgtagctagaggatcacatgcgaaaactaatttcacagaaatgttaggcaagaagttaaaagctaaaa cgaccaaccttgcaagaggtggcgcaacaatggcaacagttccaataggtaaagaagcggtagaaaacagcatttatagacaagcagagcaaataagaggagac ctaatcatattacaaggtacagatgatgactggttacatggttattggcaggcgtaccgataggcactgataaaaccgacactaaaacgttttacggcgccttttgttc tgcaattgaagttatcaggaaaaataatccagcttcaaaaatacttgtaatgacagctactaggcaatgccctatgagtggtacaacgatacgccgtaaagatacgga caaaaacaaactagggttaactttagaggattatgtcaatgctcagatattggcttgtagtgaattggatgtaccagtatatgatgcttatcacacagattatttcaaac catataatccagcatttagaaaatctagcatgcctgatggattacatcctaatgaaagaggtcatgaagttattatgtatgagcttattaaaaattattatcagttttatg gatagtaaaggaggaaaacatgagtaataaactaattacagatttaagtagagtctttgactacagatatgtagatgaaaatgagtataactttaaacttatttcaga catgctgacggattttaatttctctcttgaataccacagaaataaagaggtattcgcacatgatggagaacaataaagtatgaacatttaaatgttacaagtaacgtc tctgactttttaacatatttaaacggtcgatttagcaacatggtactaggtcataacggcgacggtatcaacgaagtaaaagacgcgcgcgttgataatacaggttatg gtcataagacattgcaagatcgtttgtatcatgattattcaacactagatgttttcactaaaaaggttgagaaagctgtagatgaacactataaagaatatcgagcgac agaataccgattcgaaccaaaagagcaagaaccggaatttatcactgatttatcgccatatacaaatgcagtaatgcaatcattttgggtagaccctagaacgaaaat tatttatatgacgcaagctcgtccaggtaatcattacatgttatctagattgaagcccaacggacaatttattgatagattgcttgttaaaaacggcggtcacggtacac acaatgcgtatagatacattgatggagaattatggatttattcagctgtattggacagtaacaaaaacaacaagtttgtacgtttccaatatagaactggagaaataac ttatggtaatgaaatgcaagatgtcatgccgaatatatttaacgacagatatacgtcagcgatttataatccggtagaaaatttaatgatttttagacgtgaatataaac ccactgaaagacaacttaagaattcgttgaactttgttgaggttagaagtgctgacgatattgataaaggtatagacaaagtattgtatcaaatggatatacctatgga atacacttcagatacacaacctatgcaaggtatcacttatgatgcaggtatcttatattggtatacaggtgattcgaatacagccaaccctaactacttacaaggcttcg |
| | | atatcaaaacgaaagaattgttatttaaacgtcgcatcgatataggcggtgaataacaactttaaaggagatttccaagaggctgagggtctagatatgtattacg atctagaaacaggacgtaaagcacttctaatcggggtaactattggacctggtaacaacagacatcattcaatttattctatcggtcaaagaggtgtaaaccaattctt gaaaaacatcgcacctcaagtatcaatgactgattcaggcggacgtgttaaaccgttaccaatacagaacccagcatatctaagtgatattacggaagttggtcatta ctatatctatacgcaagacacacaaaatgcattagatttcccgttaccgaaagcgtttagagatgcagggtggttcttggatgtactgcctggacactataatggtgctc taagacaagtacttaccagaaacagcacaggtagaaatatgcttaaattcgaacgtgtcattgacattttcaataagaaaaacaacggagcatggaatttctgcccgc aaaacgccggttattgggaacatatccctaagagtattacaaaattatcagatttaaaaatcgttggtttagatttctatatcactactgaagaatcaaaccgatttact gattttcctaaagactttaaaggtattgcaggttggatattagaagtaaaatcgaatacaccaggtaatacaacacaagtattaagacgtaataacttcccgtctgcac atcaattttagttagaaactttggtactggtggcgttggtaaatggagtttattcgaaggaaaggtggttgaataatggtagtagataatttttcgaaagatgataactt aatcgagttacaaacaacatcacaatataatccggttattgacacaaacatcagtttctatgaatcagatagaggaactggtgttttaaattttgcagtaactaagaat aacagacccttatctataagttctgaacatgttaaaacatctatcgtgttaaaaaccgatgattataacgtagatagaggcgcttatatttcagacgaattaacgatagt agacgcaattaatgggcgtttgcagtatgtgataccgaatgaatttttaaaacattcaggcaaggtgcatgctcaggcattctttacacaaaacgggagtaataatgtt gttgttgaacgtcaatttagcttcaatattgaaaatgatttagttagtgggtttgatggtataacaaagcttgtttatatcaaatctattcaagatactatcgaagctgtcg gtaaagattttaaccaattaaagcaaaatatggctgatacacaaacgttaatagcaaaagtgaatgatagtgcgacaaaaggcattcaacaaatcgaaatcaagca aaacgaagctatacaagctattactgcgacgcaaactagtgcaacacaagctgttacagctgaattcgataaaatagttgaaaaagagcaagcgattttgaacgtg ttaacgaagttgaacaacaaatcaatggcgctgaccttgttaaaggtaattcaacaacgaattggcaaaagtctaaacttacagatgattacggtaaagcaattgaat cgtatgagcagtccatagatagcgttttaagcgcagttaacacatctaggattattcatattactaatgcaacagatgcgccagaaaagacggatataggcacgttag agaagcctggacaagatggtgttgatgacggttcttcgttcgatgaatcaacttatacatcaagcaaatctggtgtgttagttgtttatgttgttgataataatactgctc gtgcaacatggtacccagacgattcaaacgatgagtacacaaaatacaaaatctacggcacatggtacccgttttataaaaagaatgatggaaacttaactaagcaa tttgttgaagaaacgtctaacaacgctttaaatcaagctaagcagtatgtagatgataaattcggaacaacgagctggcaacaacataagatgacagaggcgaatgg tcaatcaattcaagttaacttaaataatgcgcaaggcgatttgggatatttaactgctggtaattactatgcaacaagagtgccggatttaccaggtagcgttgaaagtt atgagggttatttatcggtattcgttaaagatgatacaaacaagctatttaacttcacaccttataactctaaaaagatttacacacgatcaatcacaaacggcagactt gagcaacagtggacagttcctaatgaacataaatcaacggtattgttcgacggtggcgcaaatggtgtaggtacaacaatcaatctaactgaaccgtacacaaactat tctattttgttggtaagtggaacttatccaggtggcgttattgagggattcggactaaccgcattacctaacgcgattcaattgagtaaagcgaatgtagttgactcaga cggcaacggtggcggtatttatgagtgcttactatccaaaacaagtagcactactttaagaatagataacgatgtgtactttgatttaggtaaaacatcaggttctgga gcgaatgccaacaaagttactataactaaaattatggggtggaaataatgaaaatcacagtaaacgataaaaacgaagttatcggattcgttaatactggcggtttac gcaatagtttagatgtagatgataacaatgtgcctattaaatttaaagaagagttcgaacctagaaagtttgttttcactaacggcgaaattaaatacaatagcaatttc gaaaaagaagacgtaccgaatgcatcaaaccaacaaagtgcgtcagatttaagtgatgaggaacttcgcggaatggttgcgagtatgcaaatgcaggtggcacaag |
| | | taaacgtattaacaatggaattagctcaacaaaacgctatgttaacacaacagttgactgaactgaaaactaacaaaacaagtactgagggggacgtttaaataatg aagatgatttatccaacttttaaagacattaaaactttttatgtttggggttactataaaaacgagcaaattaagtggtacgtagacaagggtttaatcgataaagaag aatacgctttaatcactggagaaaaatatccagaaacaaaagatgaaaagtcacaggtgtaatgcttgtggctttttaatttgaataaagtgggtggcataatgtttgg atttaccaaacgacatgaacaagattggcgtttaacgcgattagaagaaaatgataagactatgtttgaaaaattcgacagaatagaagatagtcttagagcgcaag aaaagatttatgacaaattagatagaaatttgaagaattaaagcgcgacaaggtagaagatgaaaagaataaagaaaagaatgccaagaatattagagacataa aaatgtggattctaggtttgatagggactatcttcagtacgattgtcatagctttactaagaactgtttttggtatttaaaggaggtgattaccatgcttaaaggattttta ggatatagcttctgggcgtgcttctggtttggtaaatgtaaataacagttaagagtcagtgcttcggcactggctttttattttgattgaaatgaggtgcatacatgggatt acctaatccgaaaaatagaaagcccacagctagtgaagtggttgaatgggcgttatatatcgctaaaaacaaaatagctattgatgtacctggttctggaatgggagc acaatgctgggatttacctaattatttactcgataaatattgggggtttagaacatggggaaatgctgatgctatggctcaaaaatccaattatagaggtagagatttca agataattagaaatacaaaagattttgtaccacaaccaggcgactggggtgtttggactggtggttgggcaggacatgtaaacattgtagtgggaccatgcacaaaa gactattggtatggcgtagatcaaaactggtatacaaataacgcaacaggaagtccaccttataaaattaaacactcttatcatgatggaccaggtggagggttaaa tattttgttagaccaccatatcatccagacaaaactacaccggcacctaaaccagaagatgatagtgatgataacgaaaaaaataataaaaaagttccaatttggaa agatgtaacaactataaagtacactatttctagccaagaggttaattatccagaatatatttatcactttatagtagaaggtaatcgacgactcgaaaaacctaaagga ataatgattagaaacgcacaaacgatgagctcggtagaaagtttatataacagtaggaagaaatacaaacaggatgtagaatatccccacttttatgttgatagacat aatatttgggcacctagaagagctgtatttgaagttcctaatgaacctgattatatagttatagacgtatgtgaagattatagtgcgagtaaaaatgaatttatttttaat gagattcacgcaatggttgtagctgtagatatgatggccaaatatgagatacctctaagtattgaaaatttaaaagtagacgacagcatttggcgttcaatgttggaac atgttaattggaatatgattgacaacggtgttcctcctaaagataaatacgaagcattagaaaaggcattacttaatatatttaaaaacagagaaaaattattaaattc tataactaagccaacagtaacaaaatctagaataaaagttatggtagataataaaaacgctgatatagctaatgtaagagactcgtcaccaacagccaacaatggtt cggcatctaaacaaccgcagatcataacagaaacgagtccttatacattcaaacaagcactggataaacaaatggcaagaggtaacccgaaaaaatctaatgcttg gggttggctaacgctacacgagcacaaacgagttcagcaatgaatgtaaagcgtatatgggaaagtaacacacaatgctaccaaatgcttaatttaggcaagtatc aaggtgtttcagttagcgcacttaataagatacttaaaggtaagggaacattgaataatcaaggtaaagcgttcgcagaagcttgtaaaaagcacaacattaatgaa atttatttaatcgcgcatgctttcttagaaagtggatatggaacaagtaacttcgctaacggaaaagatggagtatacaactacttcggcattggcgcttacgacaaca atcctaactacgcaatgacgtttgcaaggaataaaggttggacatctccagcaaaagcaatcatgggcggtgctagcttcgtaagaaaggattacatcaataaaggt caaaacacattgtaccgaattagatggaatcctaagaatccagctacccaccaatacgctactgctatagagtggtgccaacatcaagcaagtacaatcgctaagtta tataaacaaatcggcttaaaaggtatctacttcacaagggataaatataaataaagaggtgtgtaaatgtacaaaataaaagatgttgaaacgagaataaaaaatg atggtgttgacttaggtgacattggctgtcgattttacactgaagatgaaaatacagcatctataagaataggtatcaatgacaaacaaggtcgtatcgatctaaaagc acatggcttaacacctagattacatttgtttatggaagatggctctatattcaaaaatgagccccttattatcgacgatgttgtaaaaggtttccttacctacaagatacc |
| | | taaaaaggttatcaaacacgctggttatgttcgctgtaagctgtttttagagaaagaagaagaaaaaatacatgtcgcaaacttttctttcaatatcgttgatagtggta ttgaatctgctgtagcaaaagaaatcgatgttaaattggtagatgatgctattacgagaattttaaaagataacgcgacagatttattgagcaaagactttaaagaga aaatagataaagatgtcatttcttacatcgaaaagaatgaaagtagatttaaaggtgcgaaaggtgataaaggtgaaccgggacaacctggagcaaaaggtgaagc aggtaaaaaaggagaacaaggcgcacccggtaaaaacggtactgtagtatcaatcaatcctgacactaaaatgtggcaaattgatggtaaagatacagatatcaaa gcagaacctgagttattggacaaaatcaatatcgcaaatgttgaagggttagaaaataaattgcaagaagttgaaaaaatcaaagatacaactctcaacgactctaa aacgtatacggatacaaaaattgctgaactagttgatagcgcgcctgaatctatgaacacattaagagaattagcagaagcaatacaaaacaactctatttcagaaa gtgtattgcaacagattggctcaaaagttaatacagaagattttgaggaattcaaacaaacactaaatgatttatatgctccaaaaaatcataatcatgacgagcggt atgttttgtcatctcaagcttttactaaacaacaagcggataatttatatcaactaaaaagcgcatctcaaccgacggttaaaatttggacaggaacagaaaatgaata taactatatatatcaaaaagacccgaatacgttatatttaattaaagggtgatttttatggaaggtaattttaaaaatgtaaagaagtttatttacgaaggtgaagaata tacaaaagtatatgctggaaatatccaagtatggaaaaagccttcatcttttgtaataaaacccttacctaaaaataaatatccggatagcatagaagaatcaacagc aaaatggacaataaatggagttgaacccaataaaagttatcaggtgacaatagaaaatgtacgtagcggtataatgaggatttcgcaaactaatttagggtcaagtg atttaggaatatcaggagtcaatagcggagttgcaagtaaaaatatcaactttagtaatccttcagggatgttgtacgtcactataagtgatgtttattcaggatctccg acattgaccattgaataattttaaacgactaattttagtcgtttttttattttggataaaggagcaaacaaatggatattaactggaaattgagattcaaaaacaaag cagtactaactggtttagttggagcattgttgctatttatcaagcaagtcacggatttattcggattagatttatctactcaattaaatcaagctagcgcaattataggcg ctatcctcacgttacttacaggtattggcgttattactgacccaacgtcaaaaggcgtctcagattcatctatagcacagacatatcaagcgcctagagatagcaaaaa agaagaacaacaagttacgtggaaatcatcacaagacagtagtttaacgccggaattaagcgcgaaagcaccaaaagaatatgatacatcacaacctttcacagac gcctctaacgatgttggctttgatgtgaatgagtatcatcatggaggtggcgacaatgcaagcaaaattaactaaaaatgagtttatagagtggttgaaaacttctgag ggaaaacaattcaatgtggacttatggtatggatttcaatgctttgattatgccaatgctggttggaaagttttgtttggattacttctaaaaggtttaggtgcaaaagat attccgttcgctaacaacttcgacggattagctactgtataccaaaatacaccggacttcttagcacaacctggcgacatggtggtattcggtagcaactacggtgctg gatatggtcacgttgcatgggtaattgaagcaactttagattacatcattgtatatgagcagaattggctaggcggtggctggactgacggaatcgaacaacccggctg gggttgggaaaaagttacaagacgacaacatgcttatgatttccctatgtggtttatccgtccgaattttaaaagtgagacagcgccacgatcagttcaatctcctacac aagcacctaaaaaagaaacagctaagccacaacctaaagcagtagaacttaaaatcatcaaagatgtggttaaaggttatgacctacctaagcgtggtagtaaccct aaaggtatagttatacacaacgacgcagggagcaaaggggcgactgctgaagcatatcgtaacggattagtaaatgcacctttatcaagattagaagcgggcattgc gcatagttacgtatcaggcaacacagtttggcaagccttagatgaatcacaagtaggttggcataccgctaatcaaataggtaataaatattattacggtattgaagta tgtcaatcaatgggcgcagataacgcgacattcttaaaaaatgaacaggcaactttccaagaatgcgctagattgttgaaaaaatggggattaccagcaaacagaaa tacaatcagattgcacaatgaatttacttcaacatcatgccctcatagaagttcggttttacacactggttttgacccagtaactcgcggtctattgccagaagacaagc ggttgcaacttaaagactactttatcaagcagattagggcgtacatggatggtaaaataccggttgccactgtctctaatgagtcaagcgcttcaagtaatacagttaa |
| | | accagttgcaagtgcatggaaacgtaataaatatggtacttactacatggaagaaagtgctagattcacaaacggcaatcaaccaatcacagtaagaaaagtgggg ccattcttatcttgtccagtgggttatcagttccaacctggtgggtattgtgattatacagaagtgatgttacaagatggtcatgtttgggtaggatatacatgggagggg caacgttattacttgcctattagaacatggaatggttctgccccacctaatcagatattaggtgacttatggggagaaatcagttagaatgacatagtcatgtctattta agcaggtgcgttacatacctgctttctatttacatttaaagataaaatgtgctattattttactagaactttttaacatttctctcaagatttaaatgtagataacaggcag gtactacggtacttgcctatttttttatgcaaattttaaaaaacactttactaataaacatttgtttagtataattatatttgtaggttagttgatgacttacaaattatgtgt aaggaggtgaaaagcctcatgctagacataataaaaacacttctagaacatcaagtattggcagtactgataattccagaagtgttaaaacaacttagagaatggca tctcggctacctagaccgaaagccaaacaacaaagattaacattatgcttggagcctgatggctcctccttacacttatataatataatattatttggaggttttcaatta tgacagaacaaatgtatttaatattgtttttattaagcctaccattgttattatttatcgggagaaagacacatttttattgtttagataaaaagaatggacgtagataat atgagtgattataaattaaaaataattgaattgatcaaaagtgatataacaggttaccaaattcacaaacaaactggcgtagcgcaatatgtaatttcacaattaagg caaggaaagcgcgaagtagataacttaactttaaatacaactgaaaaactatacagttacgcacgacaagtgttataatataaatgtgaaatggtcattcttgaaatg actcggtcgctactggcacagaccgtttaaagtgtcaccacaacatgaactgagaattcatatgacgttgctgacgagcgacaaagctctgtgttcctgaatgggagta ggtttgtgtggtggtataatttagtaacagcatagactgtctatagcaaagttgccgaagagattctaaacgtatttataaatacgtggcccttgctagataaccgcatc ttaactgatgcggttatttttatccccacacaaccaacaaaaccacaccacctattaatttaggagtgtggttgttttaatatgtgaagctaaaataactacaaatgata ccatttttgataccatttgttgtaaaacagaaaaaataaggaaaataaaaaaggcaaaaaaacgcattaaatcaacgtttattgtctcatgaaatttaaatgtatata aatttca |
| 18 | Cloned P4 region able to activate P2 phage (P4 sequence added to CGV) | AATTTTCTTAACCTGAAGTGACGAGGAGCCGGAAAATGTCTGACCACACTATCCCTGAATATCTGCAACCCGCACTGGCACAAC TGGAAAAGGCCAGAGCCGCCCATCTTGAGAACGCCCGCCTGATGGATGAaACCGTCACGGCCATTGAACGGGCAGAGCAGGA AAAAAATGCGCTGGCGCAGGCCGACGGAAACGACGCTGACGACTGGCGCACGGCCTTTCGTGCAGCCGGTGGTGTCCTGAGC GACGAGCTGAAACAGCGCCACATTGAGCGCGTGGCACGCCGGGAGCTGGTACAGGAATATGACAATCTGGCCGTGGTGCTG AATTTCGAACGTGAACGCCTGAAAGGGGCGTGTGACAGCACGGCCACCGCCTACCGGAAGGCACATCATCACCTTCTGAGTCT GTATGCAGAGCATGAGCTGGAACACGCCCTGAATGAAACCTGTGAGGCGCTTGTCCGGGCAATGCATCTGAGCATTCTGGTAC AGGAAAATCCGCTCGCCAACACCACCGGCCATCAGGGCTACGTCGCACCGGAAAAGGCTGTCATGCAGCAGGTGAAATCATC GCTGGAACAGAAAATTAAACAGATGCAAATCAGCCTCACCGGCGAGCCGGTTCTCCGGCTGACCGGACTGTCAGCGGCAACA CTCCCGCACATGGATTATGAGGTGGCAGGCACACCGGCACAGCGCAAGGTGTGGCAGGACAAAATAGACCAGCAGGGAGCA GAGCTTAAGGCCAGAGGGCTGCTGTCATGATTTACTGTCCGTCGTGTGGACATGTTGCTCACACCCGTCGCGCACATTTCATGG ACGATGGCACCAAGATAATGATTGCACAGTGCCGGAATATTTATTGCTCTGCGACATTTGAAGCGAGTGAAAGCTTTTTCTCTG |
| | | ACAGTAAAGATTCAGGAATGGAATACATTTCAGGCAAACAGAGATACCGCGATTCACTGACGTCAGCCTCCTGCGGTATGAAA CGCCCGAAAAGAATGCTTGTTACCGGATATTGTTGTCGGAGATGTAAAGGCCTTGCACTGTCAAGAACATCGCGGCGTCTGTC TCAGGAAGTCACCGAGCGTTTTTATGTGTGCACGGATCCGGGCTGTGGTCTGGTGTTTAAAACGCTTCAGACCATCAACCGCTT CATTGTCCGCCCGGTCACGCCGGACGAACTGGCAGAACGCCTGCATGAAAAACAGGAACTGCCGCCAGTACGGTTAAAAACA CAATCATATTCGCTGCGTCTGGAATGAGGGCTGCCGGTTAACACCGGCCGTCGCCGCACACCGTATTTTTATTCTTCAGCATGA TGAGAAAGAGATAACGATGGAAAGCACAGCCTTACAGCAGGCCTTTGACACCTGTCAGAATAACAAAGCAGCATGGCTGCAA CGCAAAAATGAGCTGGCAGCGGCCGAACAGGAATATCTGCGGCTTCTGTCAGGAGAAGGCAGAAACGTCAGTCGCCTGGACG AATTACGCAATATTATCGAAGTCAGAAAATGGCAGGTGAATCAGGCCGCCGGTCGTTATATTCGTTCGCATGAAGCCGTTCAG CACATCAGCATCCGCGACCGGCTGAATGATTTATGCAGCAGCACGGCACAGCACTGGCGGCCGCACTGGCACCGGAGCTGA TGGGCTACAGTGAGCTGACGGCCATTGCCCGAAACTGTGCCATACAGCGTGCCACAGATGCCCTGCGTGAAGCCCTTCTGTCC TGGCTTGCGAAGGGTGAAAAAATTAATTATTCCGCACAGGATAGCGACATTTTAACGACCATCGGATTCAGGCCTGACGTGGC TTCGGTGGATGACAGCCGTGAAAAATTCACCCCTGCGCAGAACATGATTTTTCGCGTAAAAGTGCGCAACTGGCATCACGTC AGTCAGTGTAAAATTCCCCGAAAATCCGCCCGTTTTTACTGAAAAAAGCCATGCATCGATAAGGTGCATGGCTTTGCATGCGTT TTCCTGCCTCATTTTCTGCAAACCGCGCCATTCCCGGCGCGGTCTGAGCGTGTCAGTGCAACTGCATTAAAACCGCCCCGCAAA GCGGGCGGGCGAGGCGGGGAAAGCACCGCGCAAACCGACAAGTTAGTTAATTATTTGTGTAGTCAAAGTGCCTTCAGTAC ATACCTCGTTAATACATTGGAGCATAATGAAGAAAATCTATGGCCTATGGTCCAAAACTGTCTTTTTTGATGGCACTATCCTGAA AAATATGCAAAAAATAGATTGATGTAAGGTGGTTCTTGTCAGTGTCGCAAGATC |
| 19 | Native P2 (acc nr: NC_001895) | GGCGAGGCGGGGAAAGCACTGCGCGCTGACGGTGGTGCTGATTGTATTTTTCAGCGTCTCAGCGCGTCGTGACGGCACTTA GTCTGCCCGTTGAGGCGTTGTGTGTCTGCGGGGTGTTTGTGCGGTGGTGAGCGTGTGAGGGGGGATGACGGGGTGTAAAA AAGCCGCCCGCAGGCGGCGATGTTCAGTCGTTGTCAGTGTCCAGTGAGTAGTTTTTAAAGCGGATGACCTCCTGACCGAGCCA GCCGTTTATCTCGCGGATCCTGTCCTGTAACGGGATAAGCTCATTGCGGACAAAGACCTTTGCCACTTTCTCAATATCACCCAGC GACCCGACGTTCTCCGGCTTGCCACCCATCAACTGAAAGGGGATGCGGTGCGCGTCCAGCAGGTCAGCGGCGCTGGCTTTTTT GATATTAAAAAAATCGTCCTTCGTCGCCACTTCACTGAGGGGGATAATTTTAATGCCGTCGGCTTTCCCCTGTGGGGCATAGAG AAACAGGTTTTTAAAGTTGTTGCGGCCTTTCGACTTGACCATGTTTTCGCGAAGCATTTCGATATCGTTGCGATCCTGCACGGCA TCGGTGACATACATGATGTATCCGGCATGTGCGCCATTTTCGTAATACTTGCGGCGGAACAACGTGGCCGACTCATTCAGCCAG GCAGAGTTAAGGGCGCTGAGATATTCCGGCAGGCCGTACAGCTCCTGATTAATATCCGGCTCCAGCAGGTGAAACACGGAGC |
| | | CGGGCGCGAAGGCTGTCGGCTCGTTGAAGGACGGCACCCACCAGTAAACATCCTCTTCCACGCCACGGCGGGTATATTTTGCC GGTGAGGTTTCCAGTCTGATGACCTTACCGGTGGTGCTGTAACGCTTTTCCAGAAACGCATTACCGAACACCAGAAAATCCAGC ACAAAGCGGCTGAAATCCTGCTGGGAAAGCCATGGATGCGGGATAAATGTCGAGGCCAGAATATTGCGTTTGACGTAAATCG GCGAGCTGTGATGCACGGCAGCCCGCAGGCTTTTTGCCAGACCGGTAAAGCTGACCGGTGGCTCATACCATCTGCCGTTACTG ATGCACTCGACGTAATCCAGAATGTCACGGCGGTCGAGTACCGGCACCGGCTCACCAAAGGTGAATGCCTCCATTTTCGGGCC GCTGGCGGTCATTGTTTTTGCCGCAGGTTGCGGTGTTTTCCCTTTTTTCTTGCTCATCAGTAAAACTCCAGAATGGTGGATGTCA GCGGGGTGCTGATACCGGCGGTGAGTGGCTCATTTAACAGGGCGTGCATGGTCGCCCAGGCGAGGTCGGCGTGGCTGGCTTC CTCGCTGCGGCTGGCCTCATAGGTGGCGCTGCGTCCGCTGCTGGTCATGGTCTTGCGGATAGCCATAAACGAGCTGGTGATGT CGGTGGCGCTGACGTCGTATTCCAGACAGCCACGGCGGATAACGTCTTTTGCCTTGAGCACCATTGCGGTTTTCATTTCCGGCG TGTAGCGGATATCACGCGGCGGGATAGAACGAGCGCACGAGCTGGAACACGCCGACACCGAGGCCGGTGGCATCAATAC CGATGTATTCGACGTTGTATTTTTCGGTGAGTTTGCGGATGGATTCCGCCTGGGTGGCAAAGTCCATGCCTTTCCACTGGTGAC GCTCAAGTATTCTGAATTTGCCACCGGCCACCACCGGCGGTGCCAGTACCACGCATCCGGCGCTGTCGCCACGGTGTGACGGG TCGTAACCAATCCATACCGGGCGGGAGCCGAACGGATTGGCGGCAAACGGCGCATAGTCTTCCCATTCTTCCAGCGTGTCGAC CATGCAGCGTTGCAGCTCCTCGAACGGGAACACCGATGCCTTGTCGTCAACAAATTCACACATGAACAGGTTTTTAAAATCGTC GGCGCTGTTTTCGCGTTTGAGCTGCTCAATGTCGAACAGCGTGCAGCCGCCTTTCAGGGCGTCCTCAATGGTGACAATCTGTCG CCACTGGCCGTCCGCACAGAGAAGCCCACCGGCAAGGGCGTTATGACTGACGTCGATTTCCACGCGTTCGGCGGCGCTGGCG CGTCCCCGGTTAAACAGTTCACCCGACCAGAACGGGTAGGCGTCGTGCGCCAGCGTGGACGGGGTGGAGAAATAGGTCGAG CGCAGGTGACTCTGTGAGGCCATACCTGATGCCACCTTACGCAGTACCTGAAAATTCGGGATCCAGAAAATCTCATCGACGTA CAGGTCGCCGTTATGACTCTGCGCGGTGTTGGAGTTGGTGCCGAGAAAAATCAGTTTTGCGCCGTTATTGCCCAGGACAATCG GGTCACCGGTCAGGTCAACGTCAACCAGACGGGCAAAGGCGATGATGTATTCGCGGAACACATACGCCTGTGTTTTACTGGCC GACAGAAAAATCTGGTTATGACCGGTTTTCAGGGCACGCAGCAGCGCCTCGCGGGAAAAATAAAACGTCGCGCCAATCTGGC GGGATTTCAGGATATCGCGGATGCGGTGCTCAAGCCCGGCACGATACCAGTGCAGCTGATAGTCGAAAGACTGCTCAAAGAA AATCTGCTCCAGCTTTTCGATGGCCTCGTCACTGAAAAAATTCTTTTTCGGTTTGCGCCGTCCGCCTTTGTTACGGTTAGCGACG TTCGGATTAAGGTCTGCCTCGTTGCCGGTCTGGCTGTAGCGGTTGACCCGTGCCAGTCGTTCAATCTGGCGTCCCAGCAGGTCA ATTTCCTTGAAGTCACCGCCGGTTTTCTGTGGTTTGATGATGAGCTGGGTCAGCCGCGCTTCCAGACTCATTTCGACACGGCTG ATGGGGGCAACGCTGTCCCAGCCGTCGCGCTGTTTCCAGCTCTGCACTGTCGGGCGTTTCATCTGCAACATGGCGGCAATCTGC GGCACGGAAAACCCCTGCCAGTACAGCAGCGCCGCCTGACGACGCGGGTCGTGTAAAAGAGTGGTGTCTGTGGTGATGGTCA |
| | | TGAATACCTCGCCGTGATGAATACACGGCAAGGCTACTGAGTCGCGCCCCGCGATTCGCTAAGGTGCTGTTGTGTCAGTGATA AGCCATCCGGGACTGATGGCGGAGGATGCGCATCGTCGGGAAACTGATGCCGACATGTGACTCCTCTAATCACTATTCAGGAC TCCTGACAATGGCAAAAAAAGTCTCAAAATTCTTTCGTATCGGCGTTGAGGGTGACACCTGTGACGGGCGTGTCATCAGTGCG CAGGATATTCAGGAAATGGCCGAAACCTTTGACCCGCGTGTCTATGGTTGCCGCATTAACCTGGAACATCTGCGCGGCATCCT GCCTGACGGTATTTTTAAGCGTTATGGCGATGTGGCCGAACTGAAGGCCGAAAAGATTGACGATGATTCGGCGCTGAAAGGC AAATGGGCGCTGTTTGCGAAAATCACCCCGACCGATGACCTTATCGCGATGAACAAGGCCGCGCAGAAGGTCTACACCTCAAT GGAAATTCAGCCGAACTTTGCCAACACCGGCAAATGTTATCTGGTGGGTCTGGCCGTCACCGATGACCCGGCAAGCCTCGGCA CGGAATACCTGGAATTCTGCCGCACGGCAAAACACAACCCCCTGAACCGCTTCAAATTAAGCCCTGAAAACCTGATTTCAGTGG CAACGCCTGTTGAGCTGGAATTTGAAGACCTGCCTGAAACCGTGTTCACCGCCCTGACCGAAAAGGTGAAGTCCATTTTTGGCC GCAAACAGGCCAGCGATGATGCCCGTCTGAATGACGTGCATGAAGCGGTGACCGCTGTTGCTGAACATGTGCAGGAAAAACT GAGCGCCACTGAGCAGCGCCTCGCTGAGATGGAAACCGCCTTTTCTGCACTTAAGCAGGAGGTGACTGACAGGGCGGATGAA ACCAGCCAGGCATTCACCCGCCTGAAAAACAGTCTCGACCACACCGAAAGTCTGACCCAGCAGCGCCGCAGCAAAGCCACCGG CGGTGGCGGTGACGCCCTGATGACGAACTGCTGACCGGCGTCAGTCAGTCCGGGAAAACCTTCACGATTAACCCTTAATTTCA GGAAAAACTATGCGCCAGGAAACCCGCTTTAAATTTAATGCCTACCTGTCCCGTGTTGCCGAACTGAACGGCATCGACGCCGG TGATGTGTCGAAAAAATTCACCGTTGAACCGTCGGTCACCCAGACCCTGATGAACACCATGCAGGAGTCCTCTGACTTTCTGAC CCGCATCAATATTGTGCCGGTCAGCGAAATGAAAGGGGAAAAAATTGGTATCGGTGTCACCGGCTCCATCGCCAGCACTACCG ACACTGCCGGTGGTACCGAGCGTCAGCCGAAGGACTTCTCGAAGCTGGCGTCAAACAAGTACGAATGCGACCAGATTAACTTC GATTTTTATATCCGCTACAAAACGCTGGACCTGTGGGCGCGTTATCAGGATTTCCAGCTCCGTATCCGTAACGCCATTATCAAAC GCCAGTCCCTTGATTTCATCATGGCCGGTTTTAACGGCGTGAAGCGTGCCGAAACCTCTGACCGCAGCAGCAATCCGATGTTGC AGGATGTGGCGGTCGGCTGGCTGCAGAAATACCGCAATGAAGCACCGGCGCGCGTGATGAGCAAGGTCACTGACGAGGAAG GCCGCACCACCTCTGAGGTTATCCGCGTGGGTAAGGGCGGTGATTATGCCAGCCTTGATGCACTGGTGATGGATGCGACCAAC AACCTGATTGAACCGTGGTATCAGGAAGACCCTGACCTTGTGGTGATTGTGGGGCGTCAGCTACTGGCGGACAAGTATTTCCC CATCGTCAACAAGGAGCAGGACAACAGCGAAATGCTGGCCGCTGACGTCATCATCAGCCAGAAACGCATCGGTAACCTACCA GCGGTACGCGTCCCGTACTTCCCGGCGGATGCGATGCTCATCACGAAGCTGGAAAACCTGTCCATCTACTACATGGATGACAG CCATCGCCGCGTGATTGAGGAAAACCCGAAACTCGACCGCGTGGAGAACTACGAGTCAATGAACATTGATTACGTGGTGGAA GACTACGCCGCCGGTTGTCTGGTGGAAAAAATCAAGGTCGGTGACTTCTCCACACCGGCTAAGGCGACCGCAGAGCCGGGAG CGTAACCGATGACGAGTCCCGCACAGCGCCACATGATGCGGGTCTCGGCAGCGATGACCGCGCAGCGGGAAGCCGCCCCGCT |
| | | GCGACATGCAACTGTCTATGAGCAGATGCTGGTTAAGCTCGCCGCAGACCAGCGCACACTGAAAGCGATTTACTCAAAAGAGC TGAAGGCCGCAAAAAAACGCGAACTGCTGCCGTTCTGGTTGCCGTGGGTGAACGGCGTGCTGGAGCTGGGCAAAGGTGCAC AGGATGACATTCTGATGACGGTCATGCTGTGGCGTCTGGATACCGGCGATATTGCCGGTGCGCTGGAGATTGCCCGTTATGCC CTGAAGTACGGTCTGACCATGCCGGGTAAACACCGCCGTACCCCGCCGTACATGTTCACCGAGGAGGTAGCGCTTGCGGCCAT GCGCGCTCACGCTGCCGGTGAGTCTGTGGATACCCGCCTGCTGACGGAGACCCTTGAACTGACCGCCACGGCTGACATGCCTG ATGAAGTGCGCGCAAAGCTGCACAAAATCACCGGTCTGTTTCTGCGTGACGGTGGTGATGCCGCCGGTGCGCTGGCGCACCT GCAACGTGCGACACAGCTCGACTGTCAGGCAGGCGTCAAAAAAGAGATTGAACGACTGGAGCGGGAGCTGAAACCGAAGCC GGAGCCGCAGCCCAAAGCGGCCACCCGCGCCCCGCGTAAGACCCGGAGCGTGACACCGGCAAAACGTGGACGCCCGAAAAA GAAAGCCAGTTAACAACCGAATGCGCCCCGCGCCAGGGCGGCACGCCGGTCAGTGACGGTGAATCACCTGACACTGCACCGG CGTCCACCGCCCGACTTTTCAGAGGTAGTCATGATGACGCTGATTATTCCGCGAAAGGAGGCTCCCGTGTCCGGTGAGGGTAC GGTGGTCATCCCGCAACCGGCAGGCGACGAGCCGGTGATTAAAAACACGTTCTTTTTCCCGATATCGACCCGAAGCGCGTCC GGGAACGTATGCGCCTTGAGCAGACCGTCGCCCCGCCCGTCTGCGTGAGGCCATCAAGTCAGGCATGGCTGAAACGAATGC GGAGCTGTACGAGTACCGCGAACAGAAAATTGCCGCCGGTTTTACGCGTCTGGCTGACGTCCCGGCGGACGATATCGACGGT GAAAGCATCAAGGTTTTTTACTACGAGCGCGCCGTGTGTGCGATGGCGACCGCGTCGCTTTATGAGCGTTATCGCGGTGTGGA TGCCAGTGCGAAAGGCGACAAGAAGGCTGACAGCATTGACAGCACCATTGATGAGCTGTGGCGGGATATGCGCTGGGCGGT GGCGCGCATCCAGGGCAAGCCGCGCTGCATCGTGAGTCAAATCTGATGAAGACCTTTGCGCTACAGGGCGACACGCTCGACG CCATTTGTGTCCGCTATTACGGGCGCACTGAGGGCGTGGTTGAGACCGTGCTCGCCGCAAATCCGGGACTGGCTGAACTGGG GGCGGTGCTGCCACACGGCACCGCCGTCGAACTGCCCGACGTTCAGACCGCGCCCGTGGCTGAAACTGTCAATCTGTGGGAG TAACGCATGACAGCAGAAGAAAAAAGCGTCCTGTCGCTTTTCATGATTGGGGTGCTGATTGTTGTCGGCAAGGTGCTTGCCGG TGGTGAACCTATCACCCCGCGTCTGTTTATCGGGCGCATGTTGCTCGGTGGTTTTGTCTCGATGGTTGCCGGTGTTGTTCTGGT GCAGTTTCCTGACCTGTCACTGCCAGCGGTGTGCGGCATCGGCTCCATGCTGGGTATCGCCGGTTATCAGGTGATTGAGATTG CCATTCAGCGCCGCTTTAAGGGCAGGGGGAAACAGTAATGCCGGTAATTAACACGCATCAGAATATCGCCGCCTTTCTCGACA TGCTGGCCGTGTCCGAAGGGACGGCGAATCATCCACTGACGAAAAACCGGGGCTATGACGTGATAGTCACCGGACTGGACGG GAAGCCGGAAATTTTCACCGACTACAGTGACCACCCGTTCGCACATGGCCGACCGGCGAAGGTGTTTAACCGTCGCGGTGAAA AATCCACGGCCTCCGGTCGCTATCAGCAGCTTTACCTGTTCTGGCCGCATTACCGCAAACAGCTTGCCCTGCCGGATTTCAGTCC GTTGTCACAGGACAGACTCGCCATTCAGTTGATCCGCGAACGCGGAGCACTGGATGACATCCGGGCGGACGCATTGAGCGC GCCATTTCACGCTGTCGCAATATCTGGGCGTCCCTGCCGGGTGCCGGTTACGGTCAGCGTGAGCATTCACTGGAAAAACTGGT |
| | | CACCGTCTGGCGTACCGCTGGCGGCGTACCGGCTTAAACGGAGTAAATACCATGAAGAAATTATCCCTTTCACTGATGCTGAA CGTGTCGCTGGCGCTGATGCTGGCACTGTCCCTGATTTACCCGCAGAGCGTGGCCGTCAATTTTGTCGCTGCCTGGGCGATTCT GGCGACGGTTATCTGTGTGGTTGCCGGTGGTGTGGGCGTGTATGCCACTGAGTATGTGCTGGAACGCTACGGGCGGGAGCTG CCGCCGGAATCGCTGGCCGTGAAGATTGTCACGTCGCTGTTTTTGCAGCCGGTGCCGTGGCGCAGACGGGCGGCGGCTCTGG TAGTGGTGGTGGCGACGTTTATCTCGCTGGTCGCTGCCGGGTGGATTTTTACCGCGCTGATTTATCTTGTGGTGTCGCTGTTTTT CCGGCTGATACGTAAAGCCTGTCGTCAGCGTCTTGAGGGGCGGGAACCATGTCAAGGCTGATGATTGTGCTGGTCGTGTTGTT ATCGCTGGCGGTGGCCGGTCTGTTTCTGGTGAAACACAAAAATGCCAGCCTGCGCGCCTCGCTGGACAGGGCGAACAACGTC GCCAGCGGTCAGCAGACGACCATCACCATGCTGAAAAATCAGCTTCATGTTGCGCTCACCAGGGCAGATAAAAACGAGCTGGC GCAGGTGGCACTGCGTCAGGAACTGGAGAACGCCGCGAAACGTGAAGCACAGCGCGAGAAAACCATCACGAGGTTACTTAAT GAGAACGAAGATTTTCGCCGCTGGTACGGTGCTGACCTGCCTGATGCTGTGCGCCGGTTGCACCAGCGCCCCGCCTGCACCGA CGCCAGTGATTGTCCCCAACGCATGCCCGAAAGTGAGCCTTTGCCCGATGCCGGGCAGTGACCCGCAGACGAACGGCGATTTA AGTGCCGATATCCGGCAGCTTGAGAACGCGCTGGCACGCTGTGCCAGCCAGGTAAAAATGATTAAACACTGTCAGGACGAAA ACGATGCTCAAACCCGACAGCCTGCGCAGGGCGCTGACTGATGCCGTCACGGTGCTGAAAACTAACCCCGATATGCTGCGGAT ATTCGTGGATAACGGGAGTATTGCCTCCACACTGGCGGCGTCGCTGTCATTCGAAAAGCGTTACACGCTCAATGTGATTGTGA CCGACTTTACCGGTGATTTTGACCTGCTCATTGTGCCGGTGCTGGCGTGGCTGCGGGAAAATCAGCCCGACATCATGACCACC GACGAAGGCCAGAAAAAGGGCTTCACGTTTTATGCAGACATCAACAATGACAGCAGCTTTGATATCAGTATCAGCCTGATGCT GACCGAGCGCACGCTGGTCAGTGAGGTGGACGGCGCACTGCATGTGAAGAATATCTCGGAACCCCCGCCGCCGGAGCCGGTC ACCCGCCCGATGGAGCTGTATATCAATGGCGAACTGGTGAGTAAGTGGGATGAATGAGTTTAAGCGTTTTGAAGACCGGCTG ACCGGACTGATTGAATCGCTGTCACCGTCAGGGCGTCGGCGACTGAGTGCCGAACTGGCGAAACGTCTGCGGCAGAGTCAGC AGCGTCGGGTGATGGCACAGAAAGCCCCGGACGGCACACCCTACGCGCCACGCCAGCAGCAGAGCGTCAGAAAAAAGACCG GTCGCGTTAAGCGAAAAATGTTTGCGAAACTTATTACCAGTCGTTTTTTGCATATCCGTGCCAGCCCGGAGCAGGCATCAATGG AATTTTACGGCGGGAAGTCGCCGAAAATCGCCAGTGTGCATCAGTTTGGTCTGTCGGAAGAAAACCGGAAAGACGGTAAGAA AATTGATTATCCGGCGCGTCCCCTGCTCGGCTTTACCGGTGAGGATGTGCAGATGATTGAAGAGATTATCCTGGCTCACCTTGA GCGTTAGTTTATCCAGGCAGAGGCTGATGCGCAATTAAACATTGAGCGGCCATGCTGGTCGCTCAATGTTTAGAGGTTTATG AGTGATTTTTATTTGATGCTTTGTATTCTAAAACCTTCTTATTGGCGTAAAAGAATTTTGTATATGACAGGAATATAACCAGACC TGAAGTGAAATAGACGAGGGATAGTATTAATAATGCTTTTTTGTGACTGTTATTATCTTTAATCTCCTGGCTTAACCATTCGGAG TCCTCCTCGTTTAGCTGTAAGAGCTTATTGCAGGCGATCTCAGGAAGTGTGTCTTTTATAAATACGTTTTGCAGTCTCTTGCAAT |
| | | CGGCAAGGCTATAAGTTTATTAAATTCAACTGCTTTATTTTGAAGGATAAAAGAACTTTGTCACTATAAACATAGTACATCAT GTTTTTATATGGTATGCCTGTGGCATCCCTTACTATAACGGATTGTTCGTTGTGTATGTAACATGCGAGGAGAATGTAAAAAT ACTGGCCAGAATTACAATTATTGTTTTAATTATGTGTGGTTTTGTTATGTCACCCCAGATACGAGTAAGGAAAAAATACGA TGTTTTTAGTTTTCCATCAATCAGTCCCTGCTGTATCATTCTCACATTTTCAATGCCTGATACATTGATTCCGTTAATTATTTTAAA TAGTTGAATGTCGCGCCACTCGCGGTCAAGTCTTTTTAATTTTTGTCTGAATATCCAAAATTGAAATAATGTGCAATAAGCCTC ATAAGGTTACTTTTACCAAAGCTAAAAATGCTAATACTGCAAAGCTACAAAGGAAAAAACGATTAGCCCCACACATTAGTC ACATTATAGCTGACCATTACGCTCTCCTTGAATGTTGTCTGGTAGTTCTACAAATGAATCCAGATAGCATAACTTTTATATATTGT GCAATCTCACATGCATGAACACTCTCGCAAATATTCAGGAACTCGCGCGCACTGCGCAACATGATTCGCACTGGCATTATCG TCGAAACCGACCTTAACGCCGGTCGCTGCCGCGTGCAGACCGGCGGCATGTGCACCGACTGGCTTCAGTGGCTGACCCATCGC GCAGGACGTTCGCGCACATGGTGGGCACCTTCCGTGGGGGAACAGGTGCTGATTCTGGCCGTGGGTGGTGAACTCGACACGG CGTTCGTTCTGCCGGGGATTTATTCCGGCGATAACCCCTCGCCGTCTGTGTCGGCGGATGCCCTGCATATCCGTTTCCCTGACG GGGCGGTGATTGAATATGAACCCGAAACCAGTGCACTCACGGTAAGCGGAATTAAAACGGCCAGCGTGACGGCTTCCGGTTC TGTTACTGCCACGGTGCCGGTGGTCATGGTGAAAGCATCAACCCGCGTCACCCTGGACACCCCGGAGGTGGTCTGCACCAACA GGCTGATTACCGGCACGCTGGAAGTGCAGAAAGGCGGGACGATGCGCGGCAACATTGAACACACCGGCGGTGAACTCTCATC AAACGGTAAGGTACTGCATACCCATAAACACCCCGGCGACAGCGGCGGCACAACCGGGAGTCCTTTATGACAGCGCGTTATCT CGGAATGAATCGCAGTGATGGCCTGACTGTCACTGACCTTGAGCATATCAGCCAGAGTATCGGCGATATCCTGCGCACACCGG TCGGCTCACGGGTGATGCGTCGTGATTACGGCTCGTTGCTGGCGTCAATGATTGACCAGCCGCAGACCCCGGCGCTTGAGTTG CAGATTAAAGTCGCCTGTTACATGGCAGTGCTGAAATGGGAACCCCGCGTCACCCTGTCATCCGTCACCACGGCGCGCAGTTTT GACGGGCGAATGACGGTCACGTTAACCGGCCAGCACAACGACACCGGCCAGCCACTTTCATTAACCATCCCTGTGAGTTGAAA CCATGCCGATTATCGACCTGAACCAGCTACCCGCACCGGATGTGGTCGAGGAGCTGGACTTTGAAAGCATTCTCGCTGAACGC AAGGCGACACTGATTTCCCTTTACCCGGAAGATCAGCAGGAGGCGGTCGCCCGTACCCTGACACTGGAATCTGAGCCTCTCGT CAAACTGCTGGAAGAAAATGCTTATCGTGAGCTTATCTGGCGTCAGCGTGTGAATGAGGCCGCACGGGCGGTGATGCTGGCC TGTGCCGCCGGTAATGACCTTGATGTGATTGGTGCCAATTACAACACCACGCGCCTGACTATCACCCCGGCAGATGATTCGACC ATCCCGCCGACACCGGCAGTGATGGAATCTGACACCGATTATCGTCTGCGTATTCAGCAGGCTTTTGAGGGCTTAAGCGTCGC CGGGTCAGTGGGAGCCTATCAGTATCATGGTCGCAGTGCTGACGGGCGTGTCGCGGATATTTCTGTCACCAGTCCGTCTCCGG CCTGTGTCACCATCTCTGTGCTGTCACGTGAAAATAACGGCGTCGCATCCGAAGACCTGCTGGCTGTGGTGCGTAACGCCCTTA ATGGCGAGGACGTCAGGCCGGTGGCCGACCGCGTGACCGTGCAGTCTGCCGCCATCGTTGAATACCAGATAAACGCCACGCT |
| | | TTACCTTTACCCTGGTCCCGAAAGCGAACCCATCCGCGCTGCCGCTGTGAAAAAGCTGGAAGCGTATATCACGGCACAGCACC GGCTGGGGCGCGACATCCGTCTGTCTGCCATTTATGCCGCTTTGCATGTGGAAGGTGTGCAGCGTGTCGAACTGGCTGCACCA CTGGCCGACATCGTGCTCAACAGTACGCAGGCGTCTTTCTGTACCGAATACCGCGTCGTGACCGGAGGCTCGGATGAGTGATT CGCGACTGCTGCCGACCGGCTCATCACCGCTTGAGGTCGCCGCCGCAAAAGCCTGTGCGGAAATTGAAAAACGCCGGTCAG TATTCGTGAACTGTGGAACCCGGACACCTGTCCGGCAAATCTGCTGCCGTGGCTGGCGTGGGCGTTTTCGGTCGACAGGTGGG ATGAAAAGTGGCCGGAAGCGACAAAACGCGCCGTTATCCGCGATGCCTATTTCATCCACTGTCATAAGGGCACGATAGGTGCA ATCCGGCGTGTGGTGGAGCCGCTCGGCTATCTCATCAACGTGACGGAGTGGTGGGAAAACAGTGACCCGCCCGGCACCTTCC GGCTTGATATTGGTGTACTGGAAAGCGGTATCACAGAGGCAATGTATCAGGAAATGGAACGGCTGATTGCTGATGCCAAACC TGCAAGCCGTCACCTTATTGGCCTGAACATTACCCGGGACATTCCCGGCTATCTGTTCGCCGGTGGTGTGGCTTACGACGGCGA TGTAATTACGGTTTACCCCGGATAAGTGAGGAATAATGAGCATAAAATTCAGAACCGTTATCACCACTGCCGGTGCAGCAAAG CTGGCAGCGGCAACCGCGCCGGGAAGGCGGAAGGTCGGCATTACCACGATGGCCGTCGGGGATGGCGGTGGTAAATTGCCT GTCCCGGATGCCGGACAGACCGGGCTTATCCATGAAGTCTGGCGACATGCGCTGAACAAAATCAGCCAGGACAAACGAAACA GTAATTATATTATCGCCGAGCTGGTTATTCCGCCGGAGGTGGGCGGTTTCTGGATGCGTGAGCTTGGCCTGTACGATGATGCG GGAACGTTAATTGCCGTGGCGAACATGGCCGAAAGCTATAAGCCAGCCCTTGCCGAAGGCTCAGGACGTTGGCAGACCTGTC GCATGGTCATCATCGTCAGCAGTGTGGCCTCAGTGGAGCTGACCATTGACACCACAACGGTGATGGCGACGCAGGATTACGTT GATGACAAAATTGCAGAGCACGAACAGTCACGACGTCACCCGGACGCCTCGCTGACAGCAAAAGGTTTTACTCAGTTAAGCAG TGCGACCAACAGCACGTCTGAAACACTGGCCGCAACGCCGAAAGCGGTAAAGGCCGCGTATGACCTGGCTAACGGGAAATAT ACCGCACAGGACGCCACCACAGCGCGAAAAGGCCTTGTCCAGCTTAGTAGCGCCACCAACAGCACGTCTGAAACGCTCGCCGC AACACCAAAAGCCGTTAAGACGGTAATGGATGAAACGAACAAAAAAGCGCCATTAAACAGCCCTGCACTGACCGGAACGCCA ACGACGCCAACTGCGCGACAGGGAACGAATAATACTCAGATCGCAAACACGGCTTTCGTTATGGCCGCGATTGCCGCCCTTGT AGACTCGTCGCCTGACGCACTGAATACGCTGAACGAGCTGGCGGCGGCGCTGGGCAATGACCCGAATTTTGCTACCACCATGA CTAATGCGCTTGCGGGTAAGCAACCGAAAGATGCTACCCTGACGGCGCTGGCGGGGCTTGCTACTGCGGCAGACAGGTTTCC GTATTTTACGGGGAATGATGTTGCCAGCCTGGCGACCCTGACAAAAGTCGGGCGGGATATTCTGGCTAAATCGACCGTTGCCG CCGTTATCGAATATCTCGGTTTACAGGAAACGGTAAACCGAGCCGGGAACGCCGTGCAAAAAAATGGCGATACCTTGTCCGGT GGACTTACTTTTGAAAACGACTCAATCCTTGCCTGGATTCGAAATACTGACTGGGCGAAGATTGGATTTAAAAATGATGCCGAT GGTGACACTGATTCATACATGTGGTTTGAAACGGGGGATAACGGCAATGAATATTTCAAATGGAGAAGCCGCCAGAGTACCA CAACAAAAGACCTGATGACGTTGAAATGGGATGCACTAAATATTCTTGTTAATGCCGTCATTAATGGCTGTTTTGGAGTTGGTA |
| | | CGACGAATGCACTAGGTGGTAGCTCTATTGTTCTTGGTGATAATGATACCGGATTTAAACAGAATGGAGACGGTATTCTTGAT GTTTATGCTAACAGTCAGCGTGTATTCCGTTTTCAGAATGGAGTGGCTATTGCTTTTAAAAATATTCAGGCAGGTGATAGTAAA AAGTTCTCGCTATCCAGCTCTAATACATCCACGAAGAATATTACCTTTAATTTATGGGGTGCTTCCACCCGTCCAGTGGTTGCAG AGTTAGGCGATGAGGCCGGATGGCATTTCTATAGCCAGCGAAATACAGATAACTCGGTAATATTTGCTGTTAACGGTCAGATG CAACCCAGCAACTGGGGAAATTTTGATTCCCGCTATGTGAAAGATGTTCGCCTGGGTACGCGAGTTGTTCAATTGATGGCGCG AGGTGGTCGTTATGAAAAAGCCGGACACACGATTACCGGATTAAGAATCATTGGTGAAGTAGATGGCGATGATGAAGCCATC TTCAGGCCGATACAAAAATACATCAATGGCACATGGTATAACGTTGCGCAGGTGTAAGTTATGCAGCATTTAAAGAACATTAA GTCAGGTAATCCAAAAACAAAAGAGCAATATCAGCTAACAAAGAATTTTGATGTTATCTGGTTATGGTCCGAAGACGGAAAAA ACTGGTATGAGGAAGTGAAGAACTTTCAGCCAGACACAATAAAGATTGTTTACGATGAAAATAATATTATTGTCGCTATCACCA GAGATGCTTCAACGCTTAATCCTGAAGGTTTTAGCGTTGTTGAGGTTCCTGATATTACCTCCAACCGACGTGCTGACGACTCAG GTAAATGGATGTTTAAGGATGGTGCTGTGGTTAAACGGATTTATACGGCAGATGAACAGCAACAACAGGCAGAATCACAAAA GGCCGCGTTACTTTCCGAAGCGGAAAACGTTATTCAGCCACTGGAACGCGCTGTCAGGCTGAATATGGCGACGGATGAGGAA CGTGCACGACTGGAGTCATGGGAACGTTACAGCGTTCTGGTCAGCCGTGTGGATCCTGCAAATCCTGAATGGCCGGAAATGCC GCAATAAGTTGTATGACCTCTGTTGTGAACTTACATATCTATGGCACAGAGTAAAGCCTAATCTGAAGTCCACTCTGTGCCAAA AGCGGACCTTATAAACAAGAACAATGGTCATATCAGTGTGTTTTGATTGCATAGCTAACGTGCGTCTTCCTGTACAGAATCATA AGATGATAGGGCATAGGAGATGATTATTATCGCGTGTTTTAAAAATAGCTTTCTGCATCAAGTGTCACTTGCGAAGAGGTATTG GCGCTATGATTGGCATCACTCAGTTCAGATAATATTTTAAATATTTGGTAAATGTTCAGATTACACAATTCGGTCTGCGTCCCGT TAAAATTATCTACTATTAGTTATTACTATGAGGTGAATGGCAAGTGTTTTCCACTCGCCATTTTTGTTTTTTATTTTTTTAAAGCCT TTCCGACGTTTAAGGATTTTGCCAAGCCATAAAATATTTGATATTTCATTCCTGTTTTTATTTTTGGTGAGATATTTGCGTAACTT CTTTCTCTAAAACACCAACGTATAAACCTATTATTATCTTCGGTTGTTTCTATATAGCATATTACATTGAGACCAAAAAGAAACTG AAGGAAGTCATTTGCAGTTGTCATGAATTTTGGTTTTGTTGTGGAAATGGAACTAATATGAGTTTCAAGTTGTTTATATGCTAA GAGGTATTCAGCGTAATCAAAAGTATCTTTTCCAGAGAGGAATTCAAAGAACTTTAAAAAAATCTCATAATGTTCACTTCCATA ATAAAATGATAAATGATCTTTTATTTCTCCAAGTAAATAGTTAGAGTAATCTCTTTGCATGCTAGGACTTTCAAAGTCATCTAGA CTAAATTGATTTTTCCCATCATCACCGCTGGCGTGTGATTTGATAATTTCGAGCATCCGTAATATATCTCTTGGGCGATAATATG ACCAGCGAAGGAAATTAATAAATGATGTGGGGTGGTTATAATTATCATGGACGTTGGGTGAATCCCAAGGGAAATAATGGTC CCATGCCTGTCCCTTTTTAAGGGCCATGTCTTGCTGAGTCCTAAGCAGATGATCTAATACCTCAAATAATTGAGAGCTACGGTG GTTAACATATTCTGTCCGCCAGTCCAAGAATACTGAATTATCTCTGATTTTTGCGTTTTGATTTTGTAATCCTATGGATTCAAATA |
| | | TATCTGGCCTGATCAAGAGAACGGCCTTCATTCTCCCTTTTCCTCCCTTTATACTTGGGAAGAAATCGTTGTTAATCTCCCAGACT GCGTGAGCTAATCCTTTTATACATTCTAGATAATCATCGTATGGAATTGAGGAAGGGCGGATATCAATACCATCGATAAATAAA ATATGATTTTGAGATAAACGTATTTGGCTAAAGGCCTCTTCAAATTTTTTTGTATATAAAATAAATTTATCTGAAATCTGCTCTC AGAAAAAGATAAAGACTCTCTTTCTTCACCTTTTAAGGTTGCATGCTTATGAAGTAGTTCGGCTGCAACTTTTGACTCTTGAACA AAACTGAGGGCTTGAATTATTTCTGGTGAAAATGCCTTAAGATAATACTCATCTATTGCATCATTAAGTGATGAGAATTTTGAAT AATTTAGTAACTTTGAAATTTTCCCTTCTTTATCTAATACTTGTTTTGATATTAATAAATATAGAATAACTTTCCATATGCCAGAGA AGTCAGACAAATTTAAATGCTTTTCAGATTTAAGAGTGATAAATTTTTGATATTCTGTCTCTCTTATATATTTAGTTGTAGCTAGC GTATTGTTAATATTGTTATTTGAGAGATAGATAGAGTATGCGGTTTTACCAGTACCTTTCTCTCCTACAAGAAACGATATATTTG GTTCACATAATCTAGATAGGTGATTATCACGTATGAACACTTTGTTAAGTAATTCTTTATTCTCCCTTCTCTTATAATTTTCAGCAT CAGCATAGCCAAGAGTTAGATCTTTGATTGGTATCATTATTACCTCAACTAAAGCAATTGAAAAACATATGGTTATAAAAACAT AGCGCTGCTCTGTCGCATATTGTGGATATCAACATATGAAATGTAAAGGAACTATTTGTGGATGAATGAACCGAATCGCTGTA GCTTCGGTAGTCATCTTTCAGACTTGTATAAATGAACAACTTCCGCTTCTCGCTCAAAGGAAACTGTCAGATTTGATAGCTTTTG GGCTATGTAAACTGTCAGTCGGAAAATGAGTGAGTACAAATCAGGGCAGGTGAGCGAATTGCCCGCCTTTTCTTTACCGGTGG TTGTGCTGTCGATTAGCCAACCGGGACAAATAGCCTGACATCTCCGGCGCAACTGAAAATACCACTCACCCATTAACCACGGAG TTAAACGGATGAGTGACTATCATCACGGCGTGCAGGTGCTGGAGATTAACGAGGGCACCCGCGTCATTTCCACCGTATCCACG GCCATTGTCGGCATGGTCTGCACGGCCAGCGATGCAGATGCGGAAACCTTCCCCCTCAATAAACCTGTGCTGATTACCAATGTG CAGAGCGCAATTTCAAAGGCCGGTAAAAAGGCACGCTGGCGGCATCGTTGCAGGCCATCGCTGACCAGTCAAAACCGGTCA CCGTTGTCATGCGCGTGGAAGACGGCACCGGTGATGACGAGGAAACGAAACTCGCGCAGACCGTTTCCAATATCATCGGCAC CACCGATGAAAACGGTCAGTACACCGGACTAAAAGCCATGCTGGCGGCGGAGTCGGTAACCGGTGTTAAACCGCGTATTCTC GGCGTGCCGGGACTGGATACCAAAGAGGTGGCTGTTGCACTGGCATCAGTCTGTCAGAAGCTGCGTGCTTTCGGGTATATCA GCGCATGGGGCTGTAAAACCATTTCCGAGGTGAAAGCCTATCGTCAGAATTTCAGCCAGCGTGAGCTGATGGTCATCTGGCCG GATTTCCTCGCATGGGATACGGTCACCAGTACCACCGCCACCGCGTATGCCACCGCCCGTGCGCTGGGGCTGCGCGCTAAAAT CGACCAGGAGCAGGGCTGGCATAAAACGCTGTCCAATGTCGGGGTGAACGGTGTTACCGGCATCAGCGCATCTGTATTCTGG GATTTGCAGGAGTCCGGCACCGATGCTGACCTGCTTAACGAGTCAGGCGTCACTACGCTGATTCGCCGCGACGGTTTCCGCTTC TGGGGTAACCGTACCTGCTCTGATGACCCGCTGTTCCTCTTTGAAAACTACACCCGCACCGCGCAGGTCGTGGCCGACACGATG GCTGAGGCGCACATGTGGGCGGTGGACAAGCCCATCACTGCAACGCTGATTCGCGACATCGTTGACGGCATCAATGCCAAATT CCGTGAGCTGAAAACAAACGGCTATATCGTGGATGCGACCTGCTGGTTCAGCGAAGAATCCAACGATGCGGAAACCCTCAAG |
| | | GCCGGAAAACTGTATATCGACTACGACTATACACCGGTGCCTCCTCGAAAACCTGACCCTGCGCCAGCGTATTACCGATAAA TACCTGGCAAATCTGGTCACCTCGGTTAACAGCAATTAAGGAGCCTGACCGATGGCAATGCCGCGCAAACTCAAGTTAATGAA CGTCTTTCTGAACGGCTACAGCTATCAGGGCGTTGCAAAGTCCGTCACGCTGCCAAAACTGACCCGTAAGCTCGAAAACTATCG CGGTGCGGGGATGAACGGCAGCGCACCGGTAGACCTCGGCCTTGATGACGATGCGCTGTCAATGGAGTGGTCGCTCGGTGGC TTCCCGGATTCGGTTATCTGGGAGCTTTACGCCGCAACCGGTGTGGATGCCGTGCCGATTCGTTTTGCAGGCTCTTACCAGCGC GACGATACCGGCGAAACGGTGGCCGTCGAAGTGGTCATGCGTGGACGTCAGAAAGAAATCGACACCGGCGAGGGTAAACAG GGAGAAGACACTGAGTCGAAAATCTCCGTGGTCTGCACCTATTTCCGGCTGACGATGGACGGTAAGGAGCTGGTCGAAATTG ACACCATCAACATGATTGAGAAGGTGAACGGCGTCGATCGGCTGGAGCAACACCGCCGCAATATCGGCCTGTGATTTTCATCC GGTCAGCCTGGCTGGCCGGTTAACCCTGATTCAGAAGTGAGAAAACCATGAACAAAGAAAATGTCATTACCCTGGACAATCCG GTCAAACGTGGTGAGCAGGTTATCGAACAGGTCACGCTGATGAAACCCAGTGCCGGGACGCTACGCGGTGTCAGTCTGGCTG CGGTTGCAAACTCCGAAGTCGATGCACTGATTAAGGTGCTGCCGCGCATGACGGCACCGATGCTGACCGAGCAGGAAGTCGC CGCGCTGGAACTGCCTGACCTTGTGGCGCTGGCCGGTAAGGTGGTCGGTTTTTTGTCGCCGAACTCGGTGCAGTGACGTTTCC GAAAAATCTCTCGGTCGATGACCTGATGGCGGATGTGGCAGTGATATTTCACTGGCCGCCATCAGAACTGTATCCCATGAGCC TGACCGAACTCATCACATGGCGCGAAAAGGCGCTCCGGCGAAGCGGAAACACGAATGAGTAACAATGTAAAATTACAGGTAT TGCTCAGGGCTGTTGACCAGGCATCCCGCCCGTTTAAATCCATCCGCACAGCGAGCAAGTCGCTGTCGGGGGATATCCGGAA ACACAAAAATCACTGCGCGAGCTGAACGGTCACGCATCCCGTATTGAGGGATTCCGCAAGACCAGTGCACAGCTCGCCGTGAC TGGTCATGCACTTGAAAAGGCACGGCAGGAGGCCGAAGCCCTTGCCACACAGTTTAAAAACACCGAACGTCCGACCCGTGCTC AGGCGAAAGTCCTGGAATCCGCAAAGCGTGCGGCGGAGGACTTACAGGCGAAATATAACCGCCTGACAGATTCCGTTAAACG CCAGCAGCGGGAACTGGCCGCTGTGGGAATTAATACCCGCAATCTTGCACATGATGAGCAGGGACTGAAAAACCGTATCAGT GAAACCACCGCACAGCTTAACCGTCAGCGTGATGCGCTGGTGCGTGTCAGTGCGCAACAGGCAAAACTTAACGCAGTAAAAC AGCGTTATCAGGCCGGAAAGGAACTGGCCGGAAATATGGCCTCAGTGGGCGCTGCCGGTGTGGGGATTGCGGCGGCGGGAA CGATGGCCGGTGTTAAGCTACTGATGCCCGGTTATGAGTTTGCGCAGAAAAACTCAGAATTACAGGCTGTGATCGGAGTGGCA AAAGACTCCGCCGAAATGGCCGCACTCCGCAAGCAGGCGCGCCAGCTCGGCGACAATACCGCCGCCTCGGCAGATGATGCAG CCGGTGCGCAGATTATTATTGCGAAAGCCGGTGGGGATGTTGATGCCATTCAGGCGGCAACGCCGGTCACGCTGAACATGGC GCTGGCGAACCGTCGCACAATGGAAGAAAACGCCGCCCTGCTGATGGGGATGAAATCCGCCTTTCAGCTTTCAAACGATAAGG TCGCTCATATCGGGGATGTTCTCTCCATGACGATGAACAAAACCGCCGCCGATTTTGACGGCATGAGCGATGCGCTGACCTAT GCCGCACCTGTGGCAAAAAATGCCGGTGTCAGCATTGAAGAAACCGCCGCAATGGTCGGGGCGCTGCATGATGCAAAAATCA |
| | | CAGGCTCAATGGCGGGACGGAAGCCGTGCCGTGTTAAGCCGCCTGCAGGCACCGACGGAAAAGCATGGATGCACTCA AAGAGCTTGGAGTGAAAACCTCAGACAGCAAAGGAAACACCCGGCCAATATTTACCATTCTGAAAGAAATGCAGGCCAGTTTT GAGAAAAACCGGCTCGGTACTGCCCAGCAGGCTGAATACATGAAAACTATTTTCGGGGAGGAGGCCAGCTCAGCCGCTGCCG TGCTGATGACTGCCGCCTCAACCGGAAAGCTGGACAAACTGACCGCTGCGTTTAAAGCCTCAGACGGGAAGACCGCCGAGCT GGTAAATATCATGCAGGACAACCTAGGCGGTGACTTTAAAGCGTTTCAGTCCGCTTATGAGGCGGTGGGGACTGACCTGTTTG ACCAGCAGGAAGGCGCGCTGCGTAAGCTCACGCAGACGGCCACAAAGTATGTGTTAAAACTCGACGGCTGGATACAGAAAAA CAAATCACTGGCGTCAACCATCGGCATCATTGCCGGCGGTGCACTGGCGCTTACTGGCATCATCGGTGCCATTGGCCTCGTAGC CTGGCCGGTTATCACCGGCATCAATGCCATCATCGCGGCAGCAGGCGCAATGGGGGCAGTCTTCACGACGGTTGGCAGTGCT GTTATGACCGCCATCGGGGCTATTAGCTGGCCGGTTGTGGCCGTGGTGGCTGCCATTGTCGCCGGTGCGTTGCTTATCCGTAA ATACTGGGAGCCTGTCAGCGCATTCTTTGGTGGTGTGGTTGAAGGGCTGAAAGCGGCATTTGCGCCGGTGGGGGAACTGTTC ACGCCACTTAAACCGGTTTTTGACTGGCTGGGCGAAAAGTTACAGGCCGCGTGGCAGTGGTTTAAAAACCTGATTGCCCCGGT CAAAGCCACCCAGGACACCCTGAACCGTTGCCGTGACACGGGCGTCATGTTCGGGCAGGCACTGGCTGACGCGTTGATGCTG CCGCTTAATGCGTTCAACAAACTGCGCAGTGGTATTGACTGGGTACTGGAAAAACTCGGTGTTATCAACAAAGAGTCAGACAC ACTTGACCAGACCGCCGCCAGAACTCATACCGCCACGTATGGTACCGGTGACTATATTCCGGCGACCAGCTCTTATGCAGGCTA TCAGGCTTATCAGCCGGTCACGGCACCGGCTGGCCGCTCTTATGTAGACCAGAGTAAAAACGAATATCACATCAGCCTGACGG GGGGGACTGCGCCGGGGACACAGCTTGACCGCCAGTTACAGGATGCGCTCGAAAAATACGAGCGGGATAAACGTGCGCGCG CCCGTGCCAGCATGATGCATGACGGTTAAGGAGGTGACGAAAAATGATGCTCGCGTTAGGTATGTTTGTTTTTATGCGCCAGA CGCTGCCACACCAGACCATGCAGCGTGAATCAGATTATCGCTGGCCGTCAAATTCCCGTATCGGTAAACGGGATGCCTTTCAGT TTCTCGGTGTGGGTGAGGAAAACATCACGCTGGCCGGTGTGCTTTATCCCGAACTGACCGGCGGCAAGCTGACGATGACCAC GCTCAGGCTGATGGCAGAGGAGGGCGGGCGTGGCCGTTGCTGGATGGCACCGGCATGATTTACGGCATGTATGTCATCAGC AGGGTGAGTGAAACAGGGAGTATTTTCTTTGCAGACGGCACACCCCGGAAAATTGATTTTACGCTGTCACTCACCCGCGTTGA TGAATCACTGGCCGCGCTTTATGGCGATATCGGTAAACAGGCGGAATCGCTCATCGGTAAGGCCGGCAGTATGGCGACCAGA TTCACAGGTATGACGGGGGCGGGATAATGCTGGATGCGCTGACATTTGATGCAGGCAGTACGCTGACGCCGGATTACATGCT GATGCTCGACAGCAGGGATATTACCGGCAATATCAGCGACCGTCTGATGAGCATGACCCTGACGGATAACCGGGGCTTTGAG GCTGACCAGCTTGATATTGAACTGAACGATGCCGACGGGCAGGTCGGGCTGCCGGTTCGTGGCGCTGTCCTGACGGTGTATAT CGGCTGGAAAGGTTTTGCCCTGGTATGCAAAGGGAAATTTACCGTTGATGAGGTTGAACACCGGGGCGCACCGGATGTAGTC ACCATCCGCGCCCGGAGTGCAGATTTTCGCGGACGCTCAATTCCCGCCGGGAAGGCTCCTGGCATGACACCACGCTCGGTGC |
| | | GATTGTTAAGGCGATAGCCACCCGTAACAGGCTGGAAGCCAGTGTCGCTCCGTCACTGGCCGGAATAAAAATTCCACACATCG ACCAGTCGCAGGAGTCTGATGCGAAATTCCTGACCCGTCTTGCAGAACGCAACGGCGGTGAGGTGTCGGTAAAAATGGGAAA ACTGTTGTTTCTCAAAGCGGGGCAGGGAGTGACGGCCAGCGGTAAAAAAATCCCGCAGGTCACCATAACCCGCAGCGACGGC GACCGCCATCATTTTGCGATTGCTGACCGTGGAGCCTACACCGGTGTAACGGCAAAATGGCTACACACTAAAGACCCGAAGCC GCAAAAGCAGAAGGTAAAACTGAAACGCAAAAAGAAAGAGAAACACCTGCGCGCACTGGAGCACCCGAAAGCGAAACCGGT CAGGCAGAAGAAAGCGCCTAAAGTACCGGAAGCGCGTGAAGGTGAATACATGGCCGGTGAGGCTGACAACGTTTTTGCCCTG ACCACGGTATATGCCACGAAAGCGCAGGCCATGCGCGCCGCTCAGGCGAAGTGGGATAAACTGCAACGGGGCGTTGCGGAG TTCTCTATCAGCCTGGCTACCGGTCGGGCAGATATTTACACGGAAACACCGGTCAAAGTGTCTGGCTTTAAGCGCGTCATAGAC GAGCAGGACTGGACAATCACTAAGGTGACACATTTCTGAATAATAGCGGCTTCACGACGTCCTTAGAGCTTGAGGTCAGGCT TTCTGATGTGGAGTACGAAACAGAAGATGATGAGTGATGTTTTTGTTTTATCTGTTTGTTTTGTAAGGATAAATTAACTAAAAT GGCACCATCAACAAAACCGGAAGAGGTGCTCGCGATGTTTCATTGTCCTTTATGCCAGCATGCCGCACATGCGCGTACAAGTC GCTATATCACTGACACGACAAAAGAGCGTTATCATCAGTGCCAGAACGTGAATTGCAGCGCCACGTTCATCACTTATGAGTCG GTACAGCGATACATCGTGAAGCCGGGAGAAGTCCACGCCGTAAGGCCGCACCCGTTGCCATCAGGGCAGCAAATTATGTGGA TGTAATTACAAACAGAAAGCCCCTCAGTCGAGGGGCTTTTTTGTCGATGTGGTCAATGTGTGGACGTGACCAGAAATAAATCC TTTTATTTCAATTTATTGTACGTAAAAAATAAGCCCGTGTAAGGGAGATTTAGGGTGTCACCAGTAGGGGCTTTCAACGGTACA ATGCGGGTTTGAGCGGCATAAATTACCACTGAAAGCCCTTAAACGTTACTCTACTGTGGACACTGTGTGGACACTCTCGGCCTC AGTACCACCTCTTAGCGGATTAAGAGAAATGGCGTCCTGAAGGTACTCTGGCGCAAAATGAGCGTAAACCATAGTTTGCTCAA TCCGCGTGTGACCTAGTATCCGTTGTAGCGTGATAATACTTCCTCCATTAATCATGAAATGAGTGGCAAAGCTGTGCCTTAGTG CATGTGTGGCTTGCCCCATTGGCAAATCCGGTTTTATTGCTTTCATTGTTCGTCTGAAGCGAGGGTAATCAGCATCAGGGAATA AAAAACCTCGTTTGTTATCCGCGATCATTTTGGCAACAGCCTCTGAGATCGGGACGGTGCGTGGTTTGTTTGTTTTCGTTTTAAC AAACGTGACGCGGTTATGGATGATATTTTCTGCTTTCAAACGAGCTGCTTCTCCCCAACGTGCTCCTGTACTCAGGCAAAGAAT CGCAATCTTTTTATTGTCGCCGTCAAGTGCTGCAAGCAGTAAGGCAATTTCTTCCTGTGTTAGATAGCCTGTTTCTGGTTTTTCCT CCTTAAGCCTTTTTGTCCCTCTGATAGGGTGCTCACCAAAGAATAACTCCGCTTCAATCAGGGCTGTAAACATGCCGCTAATACA TGTTAAATCACGATTGATACTCGAAGGTTTAATACCCTGACTTCTTCGGGTGGCGCAGTACTGGCTGATAAGCGATTTCGTAAT TTGAAATGCGCATGGGTCATTCGTTATTTTTGTGAAGATTTCAATTTTTCCAAGATTAGATTTCCCATGCTCTTCGTGTTTACCCT TTAAATCCCACCAGATCTGTGTCAGCTCCGACAGACGTCGCTTGTCTGTTGGTTTTGATAGCCATTCTTTATTGTGGTGGTTGTA CAACGTGTATTTCTCGAAAGCGACAGCTTCGCTTTTCTTATCAAACTTCCTACGGATGCGTTTTCCATTACGTCCAGTAGGGCGG |
| | | ATGTCCACTTCATATCGACCATCATCGAGTTTTTGATTGCCATCAGAAAACCCTCCGAGTGGTGTTTTTTTGCGACTACTAAT CGCTTTTTCGTGGTGGCTGAAATTAGCCACCAATAGTAGGCACTTGTGATGAATATATTCACGATGAATTGTTAACCAGTCTT TTGACCGGAGTGGGGCGACGTTGTTTCGTTTGCCCAAAGTGTGCGAGAGCGGGCGCAATTTGCCCGGACTCAGGAGCGATC TGATTGGTCATGAACCATAAAGTGTATTTGGTGAATTGTGGGGTCTGCAGGATGTTCATCATGACATCTGTTGGAGGTGTTGA ACGACCACTTTCATAGTAACTCAGCGTGCCATACGGAACCCCTGTTAAATCAGCAAGTTGTTGTCTGCTCAAATACTCTGATTTT CGCATTAAGACTATCTTCTCGCTTATCGTGTTTGACATGGTGTTTAGATCTCAATAGTATTTAGTTTAGATGTAGATTGTTTAGTG CTTGGATGTGGGCACTAAAAGGCATTATAAGACATTAAACGCAATTCATGAGGGCTAGAGGACGACATGAGCAAGCAAGTAA CACTCATGACTGATGCGATTCCTTATCAGGAGTTCGCAAAACTAATAGGAAAATCGACAGGAGCGGTTCGTCGGATGATCGAT AAAGGAAAGCTGCCTGTAATTGATATGACCGATCCACAATCAGCTTCAGGTCGTGCAGGTGAATATTGGGTATACCTTCCGGC ATGGAATAACGGACTAAAACTGGCTTATGAAAGCCGCCCTAAAGAGATTCGTGACGGCTGGTTGATGTGGTTAGGTCTCGGTG AACCACGTTAAGGAGAACCGTATGAATGAGCCTCGTTGTATTGCTCAGTTACTGCGTAACGAAAGCCCCAGGGCGATTGACTT CACCATCACCCACGGTAAGGGGCGTAAGGAATCATTATCCGCACCAAAAAACAGAGTCCGTTAAAAAGGCTCTGACCTTTC TGAAAAGCCGGAGGGTATGGAAATGACAGTGATGACGCTCAATCTCGTTGAAAAACAGCCAGCAGCTATGCGCCGGATAATT GGTAAGCATCTTGCCGTTCCTCGCTGGCAGGATACATGTGATTATTATAATCAGATGATGGAGCGCGAACGGTTAACGGTTTG CTTCCATGCTCAGTTAAAACAGCGTCACGCAACGATGTGTTTTGAAGAAATGAACGACGTCGAACGTGAACGACTGGTATGTG CAATTGATGAATTGCGTGGTGCATTCTCAAAACGCCGTCAGGTTGGCGCAAGTGAGTATGCATATATTAGTTTTTTAACAGTCA GTCAGCGTCGTACTTTATTTATGCATGCCGGATTGACTGAAAAAGAATTCAACCAGCCATACTGGCGAATTAATGAAGAGTCAT GTTACTGGCGTGATGCTTTATTCCGTGCATTACGTGAATTATTCAGTCTGTTTGAGTATGCACCGACAATTCTGACGTCGGTAAA ACCGGAGCAATATCTGCATTAAGTAATTAACCAGAGTTTTTAACGCACTTAATTGTGCGGGGCTTCTTTTTGCCTGGAGAAAGT TATGCATACAGTTTCTGAAAATCAGTGCGGTATATACGCATTACTGCTGCAACAGGCCAGAACCGAAGCACAGGCCGACGCTG CGACGCGCTTTTCTTCTCATCTTGATGCCATGATTCGCCACATCACAAAGGCGGAGTTATCCCGCGTGGAGATAGTCGAGCTGC TCAGTCAGGAGTCGGAAAAATTTCACAATATCGGATTGTCTCGCGGGGAGGTGCTTTGATGTTCTGTTCTCGTGCAGTTGTATT ACTGAATAACGCCTTAAAAATCGCCGTTATGAAAAATGGCGATTTGTCTCTTATTCAACTTGGTCTTGATAAAGAAAAACGCGA AATAACTGAATCTGTTATCGCGATTTATCAGAGTGAATTAAACCTCCTGTCTGATGTGGTCAATTTACTTGTTAAACGCGCTGTA TTTCACAAGCAAATTTCCTCCGTGGATGAACTGACGAAATTAACGACAGAAATCGCCAGCTATTGCGCTGATGAATTTAAAAAA CTGAACGACAAAAGGAACTGGTAATGCCGGACAACGTAGATTTTATTCAGGAACAACAGGCTGAATTACTGGAGCGCCAGATT AACGCGGCAAGGGTAAAACATTGCGGTGCTTCTGCGCTGGTTTGCGAAGAGTGTGACGCGCCAATACCTGCTGCCCGTCGTGC |
| | | GGCTTACCCGTCAGCCACGCGTTGTGTTTCCTGTCAGTCAGTCTTTGAAGCAAAAACAAACATTACCGGAGAACGGCATGAG TATTCGTATTGAAATTGGCGAACGTTATGTCGTTACCAGTGACAGCTTTCAGTTTATTCTCCACGAGAAAAGAGAGCGGAAAG CGGTAAAAACGCCGGTCAGGAATGGCTGGCGGTGGTTGGTTATTACCCGAAATTAAGCCAGCTCGTTTCAGGCCTGATGCATC ACGATATTCTGACCGGAAGCGCAAAGTCTTTTGCTGATTTAAACGTGCAGGTTGAGCAACTCAGCAAGCGTTGTTCAGAGGCT TTTGGCTCATATGGCCGTTAAAGCCTCCGGGCGTTTTGTCCCTCCGTCAGCATTTGCCGCAGGCACCGGTAAGATGTTTACCGG TGCTTATGCATGGAACGCGCCACGCGAGGCCGTCGGGCGAAAGACCCCTTACACGTGACGAGATGCGTCAGATGCAAGGT GTTTTATCCACGATTAACCGCCTGCCTTACTTTTGCGCTCGCTGTTTACTTCACGCTATGACTACATCCGGCGCAATAAAAGCCC GGTGCACGGGTTTATTTCCTCACATCCACTTTTCAGCGTCGTTTATGGCCGCGCATTGAGCGTGTGAATCAGCGCCATGAAAT GAACACCGACGCGTCGTTGCTGTTTCTGGCAGAGCGTGACCACTATGCGCGCCTGCCGGGAATGAATGACAAGGAGCTGAAA AAGTTTGCCGCCCGTATCTCATCGCAGCTTTTCATGATGTATGAGGAACTCAGCGATGCCTGGGTGGATGCACATGGCGAAAA AGAATCGCTGTTTACGGATGAGGCGCAGGCTCACCTCTATGGTCATGTTGCTGGCGCTGCACGTGCTTTCAATATTTCCCCGCT TTACTGGAAAAAATACCGTAAAGGACAGATGACCACGAGGCAGGCATATTCTGCCATTGCCCGTCTGTTTAACGATGAGTGGT GGACTCATCAGCTCAAAGGCCAGCGTATGCGCTGGCATGAGACGTTACTGATTGCTGTCGGGGAGGTGAATAAAGACCGTTCT CCTTATGCCAGTAAACATGCCATTCGTGATGTGCGTGCACGCCGCCAAGCAAATCTGGAATTTCTTAAATCGTGTGACCTTGAA AACAGGGAAACCGGCGAGCGCATCGACCTTATCAGTAAGGTGATGGGCAGTATTTCTAATCCTGAAATTCGCCGGATGGAGCT GATGAACACCATTGCCGGTATTGAGCGTTACGCCGCCGCAGAGGGTGATGTGGGGATGTTTATCACGCTTACCGCGCCTTCAA AGTATCACCCGACACGTCAGGTCGGAAAAGGCGAAAGTAAAACCGTCCAGCTAAATCACGGCTGGAACGATGAGGCATTTAA TCCAAAGGATGCGCAGCGTTATCTCTGCCATATCTGGAGCCTGATGCGCACGGCATTCAAAGATAATGATTTACAGGTCTACG GTTTGCGTGTCGTCGAGCCACACCACGACGGAACGCCGCACTGGCATATGATGCTTTTTTGTAATCCACGCCAGCGTAACCAGA TTATCGAAATCATGCGTCGCTATGCGCTCAAAGAGGATGGCGACGAAAGAGGAGCCGCGCGAAACCGTTTTCAGGCAAAACA CCTTAACCAGGGCGGTGCTGCGGGGTATATCGCGAAATACATCTCAAAAAACATCGATGGCTATGCACTGGATGGTCAGCTCG ATAACGATACCGGCAGACCGCTGAAAGACACTGCTGCGGCTGTTACCGCATGGGCGTCAACGTGGCGCATCCCACAATTTAAA ACGGTTGGTCTGCCGACAATGGGGGCTTACCGTGAACTACGCAAATTGCCTCGCGGCGTCAGCATTGCTGATGAGTTTGACGA GCGCGTCGAGGCTGCACGCGCCGCCGCAGACAGTGGTGATTTTGCGTTGTATATCAGCGCGCAGGGTGGGGCAAATGTCCCG CGCGATTGTCAGACTGTCAGGGTCGCCCGTAGTCCGTCGGATGAGGTTAACGAGTACGAGGAAGAAGTCGAGAGAGTGGTC GGCATTTACGCGCCGCATCTCGGCGCGCGTCATATTCATATCACCAGAACGACGGACTGGCGCATTGTGCCGAAAGTTCCGGT CGTTGAGCCTCTGACTTTAAAAAGCGGCATCGCCGCGCCTCGGAGTCCTGTCAATAACTGTGGAAAGCTCACCGGTGGTGATA |
| | | CTTCGTTACCGGCTCCCACACCTTCTGAGCACGCCGCAGCAGTGCTTAATCTGGTTGATGACGGTGTTATTGAATGGAATGAAC CGGAGGTCGTGAGGGCGCTCAGGGGCGCATTAAAATACGACATGAGAACGCCAAACCGTCAGCAAAGAAACGGAAGCCCGT TAAAACCGCATGAAATTGCACCATCTGCCAGACTGACCAGGTCTGAACGATTGCAGATCACCCGTATCCGCGTTGACCTTGCTC AGAACGGTATCAGGCCTCAGCGATGGGAACTTGAGGCGCTGGCGCGTGGAGCAACCGTAAATTATGACGGGAAAAAATTCAC GTATCCGGTCGCTGATGAGTGGCCGGGATTCTCAACAGTAATGGAGTGGACATGATGGCAAAAATTCACGAGGTAAAGCTGC ACGCAAAATATTTCGACCTTGTACTGGAAGGAAAGAAACGCGCAGAGTTTCGGAAAAAAGATCGTAATTATGAGCGCGGGGA CACGTTGATTTTGCATGAATGGGTGCAGGGTGTGTTTACGGGGCGAAAGGTTGAAGCCCGGATAACAGATGTTACTGACCTGT CAGACTGGCTGGAAGATTATGTCTTGCTGCACAGAATACTAGGTGAGATACTGCCGCTGACTGTCAAGCTGGCAGTATCCTCC ACATTTTTATAAATCTAAGGCGAAGAACGGTGGCAATATATTTGCTTTAAATTGTAAATAAGCAAATTATTCTAAGCCACAAAT GATATCAATTGAGTATCGATTTTCGTTCGAATATAACCATAACGCCAAGCGTCAAATGTAAGTCATCTATATGTGATTCGTATTC ATATTGGTCAGCATCAGGAGATAGTAAATGCTCTGCTGCTGTAAAAACATCAATTGTAACAGATATATCGTCTGGAATTTCTTC ATTTAATTCGGATATGTATTTTTATATTTGTTTTTAATTTCCTTATCATGTCTTCCTTATGGTGGATTGTAGTGCAGTAATTTTT TTAGTTGAATCATTGTATGAATGGAATGAACAGTTTAATGCTAGGCCAAGACTTGAATTTCCTGTAGGGCCTGTGTGTTTAAAG TATTCAAGATTTCTTCTTATGTCAGAAAATAAAAGACTTTACAGGGGGCATTTCCTTTAACTTCTATTGCACATATTGGAATGTC GAAAAATGGTCTGCTGTCCAATATGGCAATATCAATTCTGCCTGATCTTGTTGTGTTTGTGTTTTTTCTGACAGTTTTTTTGAGA ATATTTTTTGGGGGTCAGACCGTTTCGATAAAGGAACTGTTGCATTAATGAATTTATTTGTATGGTACTCAAATATAACTTTGTA ATCACCATGGCGAAAAGATTTTATTTCTAACAACGATAGACCTATAGATACGGTAGCTACATATTCAGTGTTTATTTTTGCACCA GACCTGAAATCCATTAAATAAGTTGCGTCGGCTGACTTTTTCATTCCATCTTTTACAGCTTTCACAATATCCATGTTATTCATATC AACACCTTTTATATTCCAGAATTATAAATCTTAATGATTGATTTGTTTGTGTGTTTAGGTTACTAGATTGACGTACTTATAGCACA TTCTGATTTTGAAATCACCATCCTTGGGTAAAGCACAGCCTATCACTATTGGTTTAGTACTGTGCTGTAATAGTATCAGATTAAG TTAAATCCATTTTATGAAATCTTCCATTTCATCATATTTTTTTATTACTGCTCTAACATCTTTGCTTTCATCACATAAACAATCTAAA AGACTATCCCATTCAGTTTGTTTTTTACCAAATCCATAAACAGCCTCTATATCACCGGATTTCCATATATAGATTCCGTTATCTTTC AATTTTTGATGTATTTCATGTATATGCTTTTGTGTTTTTTCATGGTTACATATTTTGATGAAGTCTTTGCTTGAAATGCTTTTGAAT GACTCAAAAGTAGTAACTTTTGTATTTAAACTAAGCTCTCCTGAGTTGATTGATTCAATCAAAGCAGTCAATAGATTATCACATT CAGTACTAAGTAAGTCTTTATGCTCTGTTAATAGAATATTTGATAAGAAATCACAATCAGCTAGAATCCTTGTTTTTATGCCGAT GGCATTGATGATTTGTGACATCTTAAATAAACTACCCTTACCGTCAACGGCAACAATGCAGATTTTACTCGGGTTGAGTTCATGT CCGTTAATTTTTTTATAAAGTGCATATAGAACGTTTGTCTCTGTTTTCCCTTCAACAAGCAAAACTTCTTCAGAAAATAAAAGGTA |
| | | TGATGAATTAGAAAGCGTGAATGCTGAGTGCAATTGCGGTGATGAAGATTTATATAATTCTTCGATTTTTCAGATATAGTCTTC CTTGCTATGGTTCCATTAGAATCCTTACAAACCTGAATCGCATTTGCTGCATGCTTTGCAGAAAGCATACTGGCTGAGTGAGTT GATATTATAACCTGATACCCTGATTCACTTAATGTGACAAGTGATTCTCTGACAGAATTAATGGCTGAAGGGTGTAAATATAAC TCAGGTTCATCAATGAAAATCAAAGTGTTTGATTTTTTTGATTCGCTGTTTTCTTTTTTTATTTCGGCCAGGTATTGAATTAATGC CATTTGAATGGAACGTTGTGTTCCGTGACCAAATCGGCTGATATCTCTCATTACCGGTTCATCTTCCCGAGACTCAAAAACTTTC AGAGTGCCGGATTTAAATATCTCATCTAATGTCGGTGTGGGAAAGTGTAACTTTACACTTACGTCAGGAAAAAATTGGTTTACT TTTTTATTTACACCTGAGTCTATTTTATTAAGGCTTTCTAATCTGTTCTCACCGTTGTGAGAAAGATATTTACCTATTTCTGATATG TTTTTTGAAAATTTTTCTTCGTGTTCTTGTTTTATTTCAGAAACAATTGCGGAAAGTATCTTTCCTATTGTGGTCGTGTTTTTGCAT TTTGTTGAGTCTTCGACAGCGTCAGACATTGCAGGGATATGAATTGGTTCCGGAAATATATTGGAGATTGCACCATCTATGCCG CCAGGGTTTTTCTTCCACGTGGTACCGTCATATACATCCAGACTTTTTTGGCTTTTCCTGTTTCCTTATTAAATTCCTGTCTTCTT GCAAAGGTAAGAGTCCCGTCAATTATAAACGGAGCTATTTTTTGTTGATTCTCTTCTGTTAACAAAGACAGAGTATCATCTGTTA TACCTTGAATAACGCCTTCAACGGATACGGGGTGTGTAGGATCGTACACATCTGATTCTGAAATCAAAGAGCCATCTAACAGCC ACTTAATTGCTAAGATAATATTTGATTTTCCTGCGTTATTATAACCAACTAAAGCAGTGAAGGGGCGCAAAATAGCCGATGTTG ACTTACAAGAACGAAAGTTGCTAATTGAAACTGAAGCAAGACGTACAGTCATTATTTTTCCTTAAATGTGCTATTTGTATGCAAT GAGTTCATACGAAACGCTTTTTTACATTTTATAGTCGTTGCATTCAAGGGTGCATGAGATTGCATTAAGGGAAACTGTGATATG GCTTGGCTTTTGACTGGAAATACTGATGGCTCATTAGTTTTATTAAGGTGCATTAAAACCGCCCCGTGAAGCGGGCGGGCGAG GCGGGGAAAGCAC |
| 20 | Native P4 (acc nr: X51522) | TTTGCCGCTGCCGCCGGGACCGGTCACCTCCAGAAAGAGCTGCCAGTCGTAGCGGTTTGCCAGCACCATAAACAGTGCAGCCA GAATCACGTCGCGTTTTTCCGCACGGCCACCGGCGGCACGGTCAAGCCAGCGCCAGAAGGCGGGGGCGTGGGTTTCCAGCGT TTCACCGTCCACCGGCGGGGTGAAATCCACATCGCACAGGGTGCGCATCCAGTGTGACGGACTGTGCGGGTGGAACGTGCCG TTCTGCGTGTCGAGCACGCCGTTACGAAAGCCAATCAGGCGGCGGGAGGGGGCTTCCTGCTGCGGAATAATCAGCTTCAGGG TATCCACCACGGAGGCCACCTTCCCGGAGGAGAACGGCGCACGCAGACGCTGAAACAGCCCGGCCACATCCCGGGCAAAGTC CTGTGGCGGCAGCACCTTCCAGACACCATTTTCATAGCGGGACAGAAGCTGGCCGTTGGCATCGACCGCGAGCGCCTCGCCGT AATGCTCATAGATACGCATGGCCTTTTCGCTGGTACTCATGGCGGAAAACTCCGCTTCGCTCATGGTGTCGAACGGGCTTTCAG CCGGTGGCCGGATGGCATCGTAAATGGCCTTACGGGTGGCCTCCCCGCCGTACTGCGTGAAGGCATCATTCCAGTCACCGAAG ACCGGCGGCAGGGCAACAACACCTTCACACGCATCTGCGGCTGCGGCGGCTTTTTCTGGCCGTCACCACTGAGGTCACGGTC |
| | | AGCGGCAAGGACAATCTGACAGGCGGGGTGCTTCTGCCGGGCAAGGCTGGCCAGAGAAAGGAGGTTCACGGAAGAAAGCG CCACCATCACCGTTTCACCGGTCAGGTGATGTACGGTAAGTGCGGTCGCGTATCCCTCCGCTATCCACAGACGTTTCCGGCCT GATTCTGTCCTTCAAGGGTGTGACAGGTGCCCCTGACCTGTCCGCCTTTCAGGGTGCGCTTACGGCCGTCAGCACTGATTAACT GAAGGTTAACCAGTTCGCCGCTGTCGTCATACAGTGGCACCACAAGGTCACCGGCGCGCCAGCTCACGCCACCGGCTCTGTGT GTGCCGGTCAGCATCCGGCATTCCCGGCCGGGAAAGCCCTTGCGGGTCAGGTAGGCGTTACCGGTTCCGGTACGGGTTTTCGC CATCAGGGTTTGTGCCAGTGCGGCGGCGTTCTTCCGGGCAGCGTCTGTTTCATCAACGGCGGCGGTCGTCACTGCCGGGTCAG CCGGTGGCAGGCTGCCGGTCACGGCAGCCACCTTTGCGGCCGCGTCGGACGGGGAAACACCAAAAACCTTTTCAACCAGTTTC AGGCCGTCACCGGCACCACACTGATTGCAGTACCAGGTGCCGCGCCCCTCCCTGTCATCAAAACGGAAGCGGTCACTCCCGCC ACAGACCGGACAGGGCTGATGACGGTTCTTCAGCACCTGAATCCCCAGCGCCGGGAGAATACGCGGCCAGTGGCCGAGCGCA TGGCTGACGGTGGCGGTTACGTTCATTTTCATGGTGTTGTTCTCCTTCAGTGCAGTACCGGCGCTTTTATGTGACGGGCACAGA GTTCATCCATCACAACCAGCCCGAGAAAGGACAGCGACGGCGCGGCCTTCAGGGGGCCGGATTCCATTAAATCTTCCAGCAGG GCACAGGCTATCTGACGCCCTTTTTCCTCACCGTGCTGGCGCAGATAAAAGCCTTCCAGCTCAGCGGCGATGGCCGCCTCCAGT GACTCAAGGGTGAGATGCGGGTAGCGGTGCTGACGTTCGCACACGGTCAGCCAGGCACAGGCGACAGCGCGACGGTAAAGG GCAGCGCGTAAGACGGGCGGTAAGGGTGTTTTCATTTGCTTTTCTCCCTGTGACAGATGACTGCATTCCGTGCCGGTTGCATTA ACTGATAAGGCATATCTGCGTCTCCTGAAGACGTGCGTATCCCTGCGCGAATACGCACATTTAATTTTTCGGGGGTCGTTTTTA ATTACAGATAATTGCGGTAACTGTTATCCGGGGTGGTTTCCGGGTCAGGCTCCGTGCGGGGAATTTCCCGCCATTCCCGCGCCA CCGGTGCTGCCCGGCTGACCGGAACAGTGTCCTGCGGGTAAATATCCAGATATTTTTCCCGCCATTTCTGTAATTCCGGGTCTC CGGCCATTTCTTTCAGTACCGCATGCCGGTTTACGGGGCTGCGTTTAAACAGGTCAGGACGGTCACAGGTAAATTCCCGCAGA AAACGCCCCAGCGGGATGTCTGTGGTGCGTCCGTCAGCGAGGATACGCACAAGGATACTGAATTTACGGCGGTACGGGTTCC AGACAATGTCCGGGCAGCGGTACGGCATTTCCCACGGAATACCGTCTTCCAGAATGCCGACCACGGCCACATCGGGAAAACCG GCAGAACGGTAAATCTCACCGGGCTGGGGAAAATCAAACATGCGTCCTGTCTCCCCGGTCTTTCTGCTGGGCGAGAAAATCGC GGCACAGGCCTTTGGCTTTCAGCTCATTCAGCACAAAATCAATATCTTCATTCAGGTAGCTGAAAATATGTGGAATGTAGAGCT GATGCAGGCCGGAGAGTTCACGGTGAATCAAATCACCCCCAACAAACCGGGATACGGCGCTGGCGCGGTTGAGCTTATGGTA AGCCTCAATGCTGAGGTGTTCACGGGCGTCATGACGCGCTGAGACGGTCTGAGGGGCTTTTTATTACGCACGGGACACCTCC ACCACCGGCAGACGGGCAGCAAGGAGAGCACATAGTCACGGACAAGGAACGGCGGGCACTGCGTTCATCACCGGCGACG GTGCGAAGCATACAGATACGGGGATGACGGTCTGCGCGACGGACAGCCGCAAACACAAAGACAAATTCAGGGTGTGAGGGG GTAAGGGTTGTAGCCATGATGGCAGCCTCCTGTGAATAGCAAATAACGCTATCGCCGGAGTTCTCACGCTCGATGGCGATAGC |
| | | CCAGACGGGGGTGAGAATACCGGCTTCACAGGATACCGGCCAGCCCGGAGGCTGCCCCGCCTGAGCTACCATTGACTCTGCG GCATAATGAGCGGACGCGGGCAGGATGCACGGAATGCCATCTGCACGACTGACCACACCACACCATAATCTGGCGCTCTGT GGCATTGATTGCGACACAAAAAAAGACGCGTGGCGTCATATGTCGCCTGTGAATTGCTCGGGTTCTCACGCCCGGCTGCCG ATTTTGCGGCAGGCGAAAAACTATATCCGCAAATGCCGGAAAAAGGCAAGCCAGAAAAAGGGAGTTTTTGCAGAGCGGCAT CATCATGCGTCGTACCCCCGTTTGCGTCCGGCAATGCGTCCGGCCATCCATGCGGTGACTTCAGAGTGCAGCCAGGCCACATTT TTACCGCCAAGACTCACCTGCGGCGGAAATTCCCCCTTACGGATGAGTTCGTAGATGGTCGAGCGTGACAGGCCGCACAGGTG CATCACTTCCGGCACACGTAAAAAACGCTCCTGCGTGATGTCCGGCAGCGGCATCAGTGGCGTCACTGGGGCGGAGACGGG GAAGAAAAAACAGCTTGCATCGGGCTACCTCGTTAATGTCCATACAGCACCGGATAAGTCCGTCCGGCTTCGGGTAGCGCTTT ATTTTGTGAATATTTTCAGCAGACGCAACAGGGGGGATTTGTTCAGGCTGTCTTACAATGGCTGTGTGTTTTTTGTTCATCTCCA CTTAAAGTCATTTAAAGCCACTTAAAGCAATTTGTAATTTTTATAGTGAAATACAAATCGTTTCTTCTTATTCATTCCCGGCGAAT TAATAAAAACAAACAGTAGTAAACAGCACAAAAAGCCCATCAACGGGTGAACAGTGGTGAACAGACGGTGAACAGTCATTAC TGCGATTGTTCACCCTTTAACTTACTGTATTACTTATCTTTTTATTAAGGTGAACAGAGGTGAACAGTAAAATATAAAAAAACA AACAGTAAGCCGGTTTTTCCTGCGACCTTTTCCTGGCTTGCCGGTCTGAGGATGAGTCTCCTGTGTCAGGGCTGGCACATCTGC AATGCGTCGTGTTGTTGTCCGGTGTACGTCACAATTTTCTTAACCTGAAGTGACGAGGAGCCGGAAAATGTCTGACCACACTAT CCCTGAATATCTGCAACCCGCACTGGCACAACTGGAAAAGGCCAGAGCCGCCCATCTTGAGAACGCCCGCCTGATGGATGAGA CCGTCACGGCCATTGAACGGGCAGAGCAGGAAAAAAATGCGCTGGCGCAGGCCGACGGAAACGACGCTGACGACTGGCGCA CGGCCTTTCGTGCAGCCGGTGGTGTCCTGAGCGACGAGCTGAAACAGCGCCACATTGAGCGCGTGGCACGCCGGGAGCTGGT ACAGGAATATGACAATCTGGCCGTGGTGCTGAATTTCGAACGTGAACGCCTGAAAGGGGCGTGTGACAGCACGGCCACCGCC TACCGGAAGGCACATCATCACCTTCTGAGTCTGTATGCAGAGCATGAGCTGGAACACGCCCTGAATGAAACCTGTGAGGCGCT TGTCCGGGCAATGCATCTGAGCATTCTGGTACAGGAAAATCCGCTCGCCAACACCACCGGCCATCAGGGCTACGTCGCACCGG AAAAGGCTGTCATGCAGCAGGTGAAATCATCGCTGGAACAGAAAATTAAACAGATGCAAATCAGCCTCACCGGCGAGCCGGT TCTCCGGCTGACCGGACTGTCAGCGGCAACACTCCCGCACATGGATTATGAGGTGGCAGGCACACCGGCACAGCGCAAGGTG TGGCAGGACAAAATAGACCAGCAGGGAGCAGAGCTTAAGGCCAGAGGGCTGCTGTCATGATTTACTGTCCGTCGTGTGGACA TGTTGCTCACACCCGTCGCGCACATTTCATGGACGATGGCACCAAGATAATGATTGCACAGTGCCGGAATATTTATTGCTCTGC GACATTTGAAGCGAGTGAAAGCTTTTTCTCTGACAGTAAAGATTCAGGAATGGAATACATTTCAGGCAAACAGAGATACCGCG ATTCACTGACGTCAGCCTCCTGCGGTATGAAACGCCCGAAAAGAATGCTTGTTACCGGATATTGTTGTCGGAGATGTAAAGGC CTTGCACTGTCAAGAACATCGCGGCGTCTGTCTCAGGAAGTCACCGAGCGTTTTTATGTGTGCACGGATCCGGGCTGTGGTCT |
| | | GGTGTTTAAAACGCTTCAGACCATCAACCGCTTCATTGTCCGCCCGGTCACGCCGGACGAACTGGCAGAACGCCTGCATGAAA AACAGGAACTGCCGCCAGTACGGTTAAAAACACAATCATATTCGCTGCGTCTGGAATGAGGGCTGCCGGTTAACACCGGCCGT CGCCGCACACCGTATTTTTATTCTTCAGCATGATGAGAAAGATAACGATGGAAAGCACAGCCTTACAGCAGGCCTTTGACAC CTGTCAGAATAACAAAGCAGCATGGCTGCAACGCAAAAATGAGCTGGCAGCGGCCGAACAGGAATATCTGCGGCTTCTGTCA GGAGAAGGCAGAAACGTCAGTCGCCTGGACGAATTACGCAATATTATCGAAGTCAGAAAATGGCAGGTGAATCAGGCCGCCG GTCGTTATATTCGTTCGCATGAAGCCGTTCAGCACATCAGCATCCGCGACCGGCTGAATGATTTTATGCAGCAGCACGGCACAG CACTGGCGGCCGCACTGGCACCGGAGCTGATGGGCTACAGTGAGCTGACGGCCATTGCCCGAAACTGTGCCATACAGCGTGC CACAGATGCCCTGCGTGAAGCCCTTCTGTCCTGGCTTGCGAAGGGTGAAAAAATTAATTATTCCGCACAGGATAGCGACATTTT AACGACCATCGGATTCAGGCCTGACGTGGCTTCGGTGGATGACAGCCGTGAAAAATTCACCCCTGCGCAGAACATGATTTTTT CGCGTAAAAGTGCGCAACTGGCATCACGTCAGTCAGTGTAAAATTCCCCGAAAATCCGCCCGTTTTTACTGAAAAAGCCATGC ATCGATAAGGTGCATGGCTTTGCATGCGTTTTCCTGCCTCATTTTCTGCAAACCGCGCCATTCCCGGCGCGGTCTGAGCGTGTC AGTGCAACTGCATTAAAACCGCCCCGCAAAGCGGGCGGGCGAGGCGGGGAAAGCACCGCGCGCAAACCGACAAGTTAGTTA ATTATTTGTGTAGTCAAAGTGCCTTCAGTACATACCTCGTTAATACATTGGAGCATAATGAAGAAAATCTATGGCCTATGGTCC AAAACTGTCTTTTTTGATGGCACTATCCTGAAAAATATGCAAAAAATAGATTGATGTAAGGTGGTTCTTGTCAGTGTCGCAAGA TCCTTAAGAATTCGTGGCATGAGAGAGTTAAAGGATGCTGAATCATGTATATGGATTAATTGGCGTTGTTGGGACCATAATCA CTATTGCTACTAGCTTCATCCGCAACCAAGATATTTCACAATGGTTGCTCGTTTGTTCTGGATGGTTAGCTGCTTTACTTATTGGA TGGTTTACACACAGAACGATTAAAGCAATTAGCAACAACCACACGAATGTTATAAAAAGTAATATGGAGGTAATCAACAGTCA TAATGAATCCAATCAAAACTTGATGGCTGAAAACAAAGAATTGATTAAAGAACTTGCAGAACTTCAGAACAGAAAGAGAAGA TGGAAAGTATAGCTGCTTATCTTGCTACTCAGAACCCCCAAATTAACGCTATGCCAAGAACAGCAAGCCGACCAGAAAATATCG ATTCGGAGGCTAATTAAATATGAAAGTATATTTTGAAAATTACTCATACTATCCAGCCCTAAGAACACGATCAGCAGAAATGAC CGGACTGAATAATTTATCATATGAGAATAAGAAAAAAATATTACCTTTAATTTCTTTGGGGAAATGGCCTCGCTCGGAAGAAAT ACAGGTATCGTTAGATAAAAGTTTAGAAGTGATGAGTAATCTCCCATTTATTTTGGATGTCACAAAGGATAATTCTCATCATTGC GCGTCAAGCTTCGAACTACTCTCTCCTGAGAATGGTTTTAAAAACTGGATAGAGTTTTGTTCCAGAAATGATAATATTATTCCAG TAGTCCAGATGCCGGATTCAGCAAAACTTAGAGATATATCTATCCAAGCCAGGGTTTTAGAGGAACTTAAAGGTTCAATTGCAT TTAGAATTAGAAATCTAAATACAGACATTAATAAAACCCTGACGTCACTAGTATCGATGAACTCTCCAGAAAATGCCATAGTTTT CATAGATTTGGGCTACATTAGAGGGAATGTATCTGCTATAACAGCTGCAGCAATAAATTCGATTAATCAGATTAGAACTGAGAT TCCTGAAGCCATAATTAGTGTGCTAGCAACAAGCTTCCCTAGTTCAGTTACAAATTTTTGCCGTGAAAATGGACAGTCCGGATA |
| | | TATTGATGTTATTGAGAGAGAATTGCATCAAAATATTGGTGGAAGTGATGTCGCTATTTATGGCGATCATGGTTCAATTCATTC AGTAGTGTATGACAACATAATTGGGCGATATGTTCCTAGAATTGATATAGCGTTAAATGATTCATGGTATTTCGAGCGCCGCCC TGGGATGAATAAAGAAGGGTTCATTGAAGCTGCGAAATCAATTTTGGCAGAGTATCCACATTATCAAAGGGAGGACTCTTGG GGAGCTGCAATGATAAGAAATGCTGCTATAGGGGATATAGCAGGTATGGAAGTCCAGCTAAATGGATTGCTGTTCGAGTGA ATCTACACTTAAATAAGCAAATTGAGCTATCTGAAGCTCTACAGTATGGCTTTGATCATGATGAGGAAGATCTTATCTAATAAA AAACTAGGGAAGGTTAGCCTTCCCTAGCCCACAACCCTTTTAAGTACTTTTTCCGGAATATAGGAAACATCATCGCCATATTTCC TAACTATAGAGTCAACATCAACCTTATGGTATTCATTTAAAGATTTCTCCGAACAAGCCTTAAACTCAAACTTAACCAAAGAATT ATTCCTTCTTTTAGCTTTGCTCAATAAGGCAAGATCGTTTATAGATATTTCTTGATTACCATGGAGTCTAGATAAAAAATCATTAG ATGTCGCATGATACCTATTTTTAATAGATGTAATGACAAATTCTCTAATCTTGTTTAAAGATAACTTAGATGCCAAAACCTCAAG ACAATCCCTATGTAGTTCATTTGAAAATGTAATTCCATACTGATTAAGCAGATCTTTAAGCTCAGACTTAAGCAAAAAACCCAAA TAAACAAATTTATTTAAGCGAGAGCAAACCCTTCCTTTTTGAATTATTTTAAAGCGTACGCTACTATTATTAGATTGAAATTCTAC AACGCCAACATCTGATGAAACCCGTTTAGATACTTCACTGGTAAATTTTGGCGCGCACACAATTGTAACTTTTTCGAAGCTAGAT TTAAAAATTTTTAACTGACCATCCAAGCGCTTAAGAGAGTCAAAATCGCTTTTTATTTCAAAAGCTTGTAGGCTTCCATTGGCAA TAGCCAAATCAGCTCTTCTGCTCCAGTTAGCAACAACCATTTCATTAATGATCGTTGCATCACTAAGCATGCCTTTTTTATATAAC CATTCAATTAGGGCTATTTTAACTTCCTGCTCTCGCAATAACTCAGCCATGTTAATCACACTCACTTCACGTTGATTTTTATTATA GCAACTAACAATTAAAAACTCCAGATTATGTTACTTAACACTCATTTTAAAAATCAACCAATAACTATTTTTATTTAAGTATTTCTT TGTATTTTGAATAATCATAAGGCTAATATATTTATTACGGTTCATATCCAAATAATCAGCCCACCACTGCATCATTAAACGCCGC TCATCCAAATGCTCGGAGGTATGGATATATGCCGCACGTACATTATTACGCTCTGAGTGGCTCAGTTGCCTCTCTATCGCGTCAT CACTCCATAACCCCGACTCCCCCAACGCACCACGCGCCATAGTCCTAAACCCATGCCCACACACCTCGGTTTTAGTATCATAGCC CATCGCACGCAATGCGCTGTTTACCGTGTTTTCACTCATAACCTTAGTTGCGTCATGATCCCCCGGAAAAAGCAGCTCTTTATCA CCACTAATCTGCTTTAACTGGTTTAATAAAATCATCGCCTGTCGACTAAGCGGAACGATATGTTCCTCTTTCATCTTCATGCCTCG GTACGAATAACGCACACCTTTAATTTCTTCTCGCTTTGCAGGTATACGCCAAAGAGATTTATCGAAGTCGAATTCATCCCAACGT GCGAAACGTAATTCACTGGAACGCACAAAAGTTAGCAAGGAAAGCTTGACCGCAATCCGTGTCATTACACGGCCACGATATGC AGCAAGACGTGCAAGAAACTCAGGGAATCGGCTAGAAGGTAAAGCAGGGTAATGTCGCGCTTTGGTTGTCGATAGCGCACCA GCCATATCGCTGGCTGGATTTGAGTCGATGTAATCGTTCTGTACGGCATAACGCATAATGGCTGTGACGCGCTGTTGCAGGCG CTGAGCGACATCGTGTTTGCCACTGGCATCAACTTTTTTAATCGGGGCTAACAGGTGGCTGGTTTTAAGCTGCCGAATGTCGGA CGAACCGATATGAGGGAATATATAAAGCTCAAGATAGCGAAGAACGCGCGATCGATGGTCTTCACTCCAGCGCTTGTTACTGG |
| | | CATGCCATTCTCGGGCGATAGCTTCAAAAGTATATGCCCCCGAATTCTCGGCCCGAGCTTCTTTTGTACGACTTTTGGGTCTAT GCCCTGTACTAAAAGCTTTTTAGCTTCATCGCGTTTTGCTCTTGCCTGAGCAAGCGTCACAGTAGGCCAAACACCAAACGCAAG ACGATCCTCTTTTTTGTCAGAGGGACGTCTGTATTTCATGCGCCAGTATTTGGAACCTTTGGCCGAAACCTCAAGATACAAACCA CCACCATCGGCCATTTTGTAGGTTTTGTCTTTTGGCTTTGCGGTCTCGACCTGTCTGGCGTTGAGCTTCATTTGGGGGCACATTT CTAATCGAAGTTAAGATGCCCCCAATTATGCCCCCAATGATATCCGGATTTCAACGGACAACCTCGGAAGACGCAGGACGAAA AAATCGCTGCAAGCATTGATTTTAAAGGGATATTTGGACTTTCTCGGATGGTCTTGGAAGTAAGAATGGTGCCGAAGGCCGGA CTCAAACATCAAAATAAGTTAATGATAAAAAACAAATAATAAAACACAACAATGAAATATGCCCCCTTTTGTGCCCCCACTGTTT TTCTGACCAATCTATTTTCAGCCCATCAATAAATCGGAAAGTTAAATCATTTTTAATCAGTAAGTTTGGATCCGTAGCTCGGATC CAAACCAGTGCATCTTTTATCCACATAAAAAATTTTTTTCGAAAGAACTGTTCACACTGTTCACCTTTCTGTTTTCTCCTTTTATT TCAGAGTGATAGGTGGTGAATAATGGGTGAAGGGTGAACATTCGATTCTTCACCTCCGGCATTCTGCCGATGTGACTCATACC GGTGATTAATCCTCCGCACTGAAATCACTCAGGAAGAAAAAGTTTTTTTGATTTGATTGTTCACACTGTTCACCTTTCGTTTTT CTCTTTTAATTTCAGTGTGATAACGGGTGAATATACGGTGAAGGGTGAACAGTGGATTGTTCACCTTCGGGGGATATCGGAT AAAAAAAGACCGGCAGATGCCGGTCAGGTGGGTCAGGCTGTTGTAGGGTCGTCACATTTTGGCAGCCAGTCGCCGTAGCTTTC CTCTTTCAGCGTCAGGTTGGTCTGTATCCCCTGTTTGGTATGGCGTTTCTCGTAATTCAGTCCGTATTCCTTCAGCATCACCGGCA GCCCCAGCCCGAACATTTTCAGACTGAGTACATTCCGGTAGCCGTTTGCCTCCATGTAGGCCAGATAGGCGTGATAGAGGTATT TACGGTAATTGCGCGGGATGATACTGGCGTTCCCCATATACATGCCGCTGGTCTGCGGCAGGGTTTCCAGATAGCCGATAAAA TCAAACGTCGGGTCGGCATCCCGTTTGATGTTCAGTGCCTCGTCTGAGTTCTGCTGGGACTGAAGCAGTGACCGGGCGAGCAT CGGGTCGCTGAACTTCTGCATCAGGTGACGCACGATGACCGCCAGCTCGCGGGTGATTTTGTCCTTAAGCTGCGGGTCGCGCT CCTGCGGGGCTATCTGTTCCGGGAAGTGAATAATCACCCGTCGGCGTGACACGCCGCCGCTGCGGTCGGTGAAGCGCATCGG GTTATTGTTCACGGCCAGAATCACCGCCGGGATGTGCGTGGAGTACGCATCCCGGTATTTCGGGTCAACGGACACCGCATCGC CGCCGGTGATGGCCTTGAGTCCGGCACCGTCGCCGCTCCATTTTCCTGGTCCGGCAGGCGTATCAGTGAGAAGCCAGTTAAC GCGGCACGTTCACGCGGGGATTCCAGCGTCTCGATGGTGGCCGACGTGGCGTTATCCTCCCCGGCCAGCAGGGTGGCTATTTC GGCCATGATACT |
| 21 | Sequence removed from P2 (replaced | TTTGAGCGGCATAAATTACCACTGAAAGCCCTTAAACGTTACTCTACTGTGGACACTGTGTGGACACTCTCGGCCTCAGTACCA CCTCTTAGCGGATTAAGAGAAATGGCGTCCTGAAGGTACTCTGGCGCAAAATGAGCGTAAACCATAGTTTGCTCAATCCGCGT GTGACCTAGTATCCGTTGTAGCGTGATAATACTTCCTCCATTAATCATGAAATGAGTGGCAAAGCTGTGCCTTAGTGCATGTGT |
| | with kanamycin marker; Example 3) | GGCTTGCCCCATTGGCAAATCCGGTTTTATTGCTTTCATTGTTCGTCTGAAGCGAGGGTAATCAGCATCAGGGAATAAAAAACC TCGTTTGTTATCCGCGATCATTTGGCAACAGCCTCTGAGATCGGGACGGTGCGTGGTTTGTTTGTTTTCGTTTTAACAAACGTG ACGCGGTTATGGATGATATTTCTGCTTTCAAACGAGCTGCTTCTCCCAACGTGCTCCTGTACTCAGGCAAAGAATCGCAATCT TTTTATTGTCGCCGTCAAGTGCTGCAAGCAGTAAGGCAATTTCTTCCTGTGTTAGATAGCCTGTTTCTGGTTTTCCTCCTTAAGC CTTTTTGTCCCTCTGATAGGGTGCTCACCAAAGAATAACTCCGCTTCAATCAGGGCTGTAAACATGCCGCTAATACATGTTAAAT CACGATTGATACTCGAAGGTTTAATACCCTGACTTCTTCGGGTGGCGCAGTACTGGCTGATAAGCGATTTCGTAATTTGAAATG CGCATGGGTCATTCGTTATTTTTGTGAAGATTTCAATTTTTCCAAGATTAGATTTCCCATGCTCTTCGTGTTTACCCTTTAAATCCC ACCAGATCTGTGTCAGCTCCGACAGACGTCGCTTGTCTGTTGGTTTTGATAGCCATTCTTTATTGTGGTGGTTGTACAACGTGTA TTTCTCGAAAGCGACAGCTTCGCTTTTCTTATCAAACTTCCTACGGATGCGTTTTCCATTACGTCCAGTAGGGCGGATGTCCACT TCATATCGACCATCATCGAGTTTTTTGATTGCCATCAGAAAACCCTCCGAGTGGTGTGTTTTTTTGCGACTACTAATCGCTTTTTT CGTGGTGGCTGAAATTTAGCCACCAATAGTAGGCACTTGTGATGAATATATTCACGATGAATTGTTAACCAGTCTTTTGACCGG AGTGGGGCGACGTTGTTTCGTTTTGCCCAAAGTGTGCGAGAGCGGGCGCAATTTGCCCGGACTCAGGAGCGATCTGATTGGTC ATGAACCATAAAGTGTATTTGGTGAATTGTGGGGTCTGCAGGATGTTCATCATGACATCTGTTGGAGGTGTTGAACGACCACTT TCATAGTAACTCAGCGTGCCATACGGAACCCCTGTTAAATCAGCAAGTTGTTGTCTGCTCAAATACTCTGATTTTCGCATTAAGA CTATCTTCTCGCTTATCGTGTTTGACATGGTGTTTAGATCTCAATAGTATTTAGTTTAGATGTAGATTGTTTAGTGCTTGGATGTG GGCACTAAAAGGCATTATAAGACATTAAACGCAATTCATGAGGGCTAGAGGACGACATGAGCAAGCAAGTAACACTCATGAC TGATGCGATTCCTTATCAGGAGTTCGCAAAACTAATAGGAAAATCGACAGGAGCGGTTCGTCGGATGATCGATAAAGGAAAG CTGCCTGTAATTGATATGACCGATCCACAATCAGCTTCAGGTCGTGCAGGTGAATATTGGGTATACCTTCCGGCATGGAATAAC GGACTAAAACTGGCTTATGAAAGCCGCCCTAAAGAGATTCGTGACGGCTGGTTGATGTGGTTAGGTCTCGGTGAACCACGTTA AGGAGAACCGTATGAATGAGCCTCGTTGTATTGCTCAGTTACTGCGTAACGAAAGCCCCAGGGCGATTGACTTCACCATCACC CACGGTAAGGGGCGTAAGGGAATCATTATCCGCACCAAAAAACAGAGTCCGTTAAAAAAGGCTCTGACCTTTCTGAAAAGCCG GAGGGTATGGAAATGACAGTGATGACGCTCAATCTCGTTGAAAAACAGCCAGCAGCTATGCGCCGGATAATTGGTAAGCATCT TGCCGTTCCTCGCTGGCAGGATACATGTGATTATTATAATCAGATGATGGAGCGCGAACGGTTAACGGTTTGCTTCCATGCTCA GTTAAAACAGCGTCACGCAACGATGTGTTTTGAAGAAATGAACGACGTCGAACGTGAACGACTGGTATGTGCAATTGATGAAT TGCGTGGTGCATTCTCAAAACGCCGTCAGGTTGGCGCAAGTGAGTATGCATATATTAGTTTTTAACAGTCAGTCAGCGTCGTA CTTTATTTATGCATGCCGGATTGACTGAAAAAGAATTCAACCAGCCATACTGGCGAATTAATGAAGAGTCATGTTACTGGCGTG ATGCTTTATTCCGTGCATTACGTGAATTATTCAGTCTGTTTGAGTATGCACCGACAATTCTGACGTCGGTAAAACCGGAGCAAT |
| | | ATCTGCATTAAGTAATTAACCAGAGTTTTTAACGCACTTAATTGTGCGGGGCTTCTTTTTGCCTGGAGAAAGTTATGCATACAGT TTCTGAAAATCAGTGCGGTATATACGCATTACTGCTGCAACAGGCCAGAACCGAAGCACAGGCCGACGCTGCGACGCTTTT CTTCTCATCTTGATGCCATGATTCGCCACATCACAAAGGCGGAGTTATCCCGCGTGGAGATAGTCGAGCTGCTCAGTCAGGAGT CGGAAAAATTTCACAATATCGGATTGTCTCGCGGGGAGGTGCTTTGATGTTCTGTTCTCGTGCAGTTGTATTACTGAATAACGC CTTAAAAATCGCCGTTATGAAAAATGGCGATTTGTCTCTTATTCAACTTGGTCTTGATAAAGAAAAACGCGAAATAACTGAATC TGTTATCGCGATTTATCAGAGTGAATTAAACCTCCTGTCTGATGTGGTCAATTTACTTGTTAAACGCGCTGTATTTCACAAGCAA ATTTCCTCCGTGGATGAACTGACGAAATTAACGACAGAAATCGCCAGCTATTGCGCTGATGAATTTAAAAAACTGAACGACAA AAGGAACTGGTAATGCCGGACAACGTAGATTTATTCAGGAACAACAGGCTGAATTACTGGAGCGCCAGATTAACGCGGCAA GGGTAAAACATTGCGGTGCTTCTGCGCTGGTTTGCGAAGAGTGTGACGCGCCAATACCTGCTGCCCGTCGTGCGGCTTACCCG TCAGCCACGCGTTGTGTTTCCTGTCAGTCAGTCTTTGAAGCAAAAAACAAACATTACCGGAGAACGGCATGAGTATTCGTATTG AAATTGGCGAACGTTATGTCGTTACCAGTGACAGCTTTCAGTTTATTCTCCACGAGAAAAAGAGAGCGGAAAGCGGTAAAAAC GCCGGTCAGGAATGGCTGGCGGTGGTTGGTTATTACCCGAAATTAAGCCAGCTCGTTTCAGGCCTGATGCATCACGATATTCT GACCGGAAGCGCAAAGTCTTTTGCTGATTTAAACGTGCAGGTTGAGCAACTCAGCAAGCGTTGTTCAGAGGCTTTTGGCTCAT ATGGCCGTTAAAGCCTCCGGGCGTTTTGTCCCTCCGTCAGCATTTGCCGCAGGCACCGGTAAGATGTTTACCGGTGCTTATGCA TGGAACGCGCCACGCGAGGCCGTCGGGCGCGAAAGACCCCTTACACGTGACGAGATGCGTCAGATGCAAGGTGTTTTATCCA CGATTAACCGCCTGCCTTACTTTTTGCGCTCGCTGTTTACTTCACGCTATGACTACATCCGGCGCAATAAAAGCCCGGTGCACGG GTTTTATTTCCTCACATCCACTTTTCAGCGTCGTTTATGGCCGCGCATTGAGCGTGTGAATCAGCGCCATGAAATGAACACCGAC GCGTCGTTGCTGTTTCTGGCAGAGCGTGACCACTATGCGCGCCTGCCGGGAATGAATGACAAGGAGCTGAAAAAGTTTGCCG CCCGTATCTCATCGCAGCTTTTCATGATGTATGAGGAACTCAGCGATGCCTGGGTGGATGCACATGGCGAAAAAGAATCGCTG TTTACGGATGAGGCGCAGGCTCACCTCTATGGTCATGTTGCTGGCGCTGCACGTGCTTTCAATATTTCCCCGCTTTACTGGAAA AAATACCGTAAAGGACAGATGACCACGAGGCAGGCATATTCTGCCATTGCCCGTCTGTTTAACGATGAGTGGTGGACTCATCA GCTCAAAGGCCAGCGTATGCGCTGGCATGAGACGTTACTGATTGCTGTCGGGGAGGTGAATAAAGACCGTTCTCCTTATGCCA GTAAACATGCCATTCGTGATGTGCGTGCACGCCGCCAAGCAAATCTGGAATTTCTTAAATCGTGTGACCTTGAAAACAGGGAA ACCGGCGAGCGCATCGACCTTATCAGTAAGGTGATGGGCAGTATTTCTAATCCTGAAATTCGCCGGATGGAGCTGATGAACAC CATTGCCGGTATTGAGCGTTACGCCGCCGCAGAGGGTGATGTGGGGATGTTTATCACGCTTACCGCGCCTTCAAAGTATCACC CGACACGTCAGGTCGGAAAAGGCGAAAGTAAAACCGTCCAGCTAAATCACGGCTGGAACGATGAGGCATTTAATCCAAAGGA TGCGCAGCGTTATCTCTGCCATATCTGGAGCCTGATGCGCACGGCATTCAAAGATAATGATTTACAGGTCTACGGTTTGCGTGT |
| | | CGTCGAGCCACACCACGACGGAACGCCGCACTGGCATATGATGCTTTTTGTAATCCACGCCAGCGTAACCAGATTATCGAAAT CATGCGTCGCTATGCGCTCAAAGAGGATGGCGACGAAAGAGGAGCCGCGAAACCGTTTTCAGGCAAAACACCTTAACCAG GGCGGTGCTGCGGGGTATATCGCGAAATACATCTCAAAAAACATCGATGGCTATGCACTGGATGGTCAGCTCGATAACGATAC CGGCAGACCGCTGAAAGACACTGCTGCGGCTGTTACCGCATGGGCGTCAACGTGGCGCATCCCACAATTTAAAACGGTTGGTC TGCCGACAATGGGGGCTTACCGTGAACTACGCAAATTGCCTCGCGGCGTCAGCATTGCTGATGAGTTTGACGAGCGCGTCGAG GCTGCACGCGCCGCCGCAGACAGTGGTGATTTTGCGTTGTATATCAGCGCGCAGGGTGGGGCAAATGTCCCGCGCGATTGTC AGACTGTCAGGGTCGCCCGTAGTCCGTCGGATGAGGTTAACGAGTACGAGGAAGAAGTCGAGAGAGTGGTCGGCATTTACGC GCCGCATCTCGGCGCGCGTCATATTCATATCACCAGAACGACGGACTGGCGCATTGTGCCGAAAGTTCCGGTCGTTGAGCCTC TGACTTTAAAAAGCGGCATCGCCGCGCCTCGGAGTCCTGTCAATAACTGTGGAAAGCTCACCGGTGGTGATACTTCGTTACCG GCTCCCACACCTTCTGAGCACGCCGCAGCAGTGCTTAATCTGGTTGATGACGGTGTTATTGAATGGAATGAACCGGAGGTCGT GAGGGCGCTCAGGGGCGCATTAAAATACGACATGAGAACGCCAAACCGTCAGCAAAGAAACGGAAGCCCGTTAAAACCGCAT GAAATTGCACCATCTGCCAGACTGACCAGGTCTGAACGATTGCAGATCACCCGTATCCGCGTTGACCTTGCTCAGAACGGTATC AGGCCTCAGCGATGGGAACTTGAGGCGCTGGCGCGTGGAGCAACCGTAAATTATGACGGAAAAAATTCACGTATCCGGTCG CTGATGAGTGGCCGGGATTCTCAACAGTAATGGAGTGGACATGATGGCAAAAATTCACGAGGTAAAGCTGCACGCAAAATAT TTCGACCTTGTACTGGAAGGAAAGAAACGCGCAGAGTTTCGGAAAAAAGATCGTAATTATGAGCGCGGGGACACGTTGATTT TGCATGAATGGGTGCAGGGTGTGTTTACGGGGCGAAAGGTTGAAGCCCGGATAACAGATGTTACTGACCTGTCAGACTGGCT GGAAGATTATGTCTTGCTGCACAGAATACTAGGTGAGATACTGCCGCTGACTGTCAAGCTGGCAGTATCCTCCACATTTTTAT AAATCTAAGGCGAAGAACGGTGGCAATATATTTGCTTTAAATTGTAAATAAGCAAATTATTCTAAGCCACAAATGATATCAATT GAGTATCGATTTTCGTTCGAATATAACCATAACGCCAAGCGTCAAATGTAAGTCATCTATATGTGATTCGTATTCATATTGGTCA GCATCAGGAGATAGTAAATGCTCTGCTGCTGTAAAAACATCAATTGTAACAGATATATCGTCTGGAATTTCTTCATTTAATTCGG ATATGTATTTTTTATATTTGTTTTTAATTTCCTTATCATGTCTTCCTTATGGTGGATTGTAGTGCAGTAATTTTTTTTAGTTGAATC ATTGTATGAATGGAATGAACAGTTTAATGCTAGGCCAAGACTTGAATTTCCTGTAGGGCCTGTGTGTTTAAAGTATTCAAGATT TCTTCTTATGTCAGAAAATAAAAGACTTTTACAGGGGGCATTTCCTTTAACTTCTATTGCACATATTGGAATGTCGAAAAATGGT CTGCTGTCCAATATGGCAATATCAATTCTGCCTGATCTTGTTGTGTTTGTGTTTTTTCTGACAGTTTTTTTGAGAATATTTTTTGG GGGTCAGACCGTTTCGATAAAGGAACTGTTGCATTAATGAATTTATTTGTATGGTACTCAAATATAACTTTGTAATCACCATGGC GAAAAGATTTTATTTCTAACAACGATAGACCTATAGATACGGTAGCTACATATTCAGTGTTTATTTTTGCACCAGACCTGAAATC CATTAAATAAGTTGCGTCGGCTGACTTTTTCATTCCATCTTTTACAGCTTTCACAATATCCATGTTATTCATATCAACACCTTTTAT |
| | | ATTCCAGAATTATAAATCTTAATGATTGATTTGTTTGTGTGTTTAGGTTACTAGATTGACGTACTTATAGCACATTCTGATTTTGA AATCACCATCCTTGGGTAAAGCACAGCCTATCACTATTGGTTTAGTACTGTGCTGTAATAGTATCAGATTAAGTTAAATCCATTT TATGAAATCTTCCATTTCATCATATTTTTTATTACTGCTCTAACATCTTTGCTTTCATCACATAAACAATCTAAAAGACTATCCCA TTCAGTTTGTTTTTACCAAATCCATAAACAGCCTCTATATCACCGGATTTCCATATATAGATTCCGTTATCTTTCAATTTTTGATG TATTTCATGTATATGCTTTTGTGTTTTTCATGGTTACATATTTTGATGAAGTCTTTGCTTGAAATGCTTTTGAATGACTCAAAAG TAGTAACTTTTGTATTTAAACTAAGCTCTCCTGAGTTGATTGATTCAATCAAAGCAGTCAATAGATTATCACATTCAGTACTAAG TAAGTCTTTATGCTCTGTTAATAGAATATTTGATAAGAAATCACAATCAGCTAGAATCCTTGTTTTATGCCGATGGCATTGATG ATTTGTGACATCTTAAATAAACTACCCTTACCGTCAACGGCAACAATGCAGATTTTACTCGGGTTGAGTTCATGTCCGTTAATTT TTTTATAAAGTGCATATAGAACGTTTGTCTCTGTTTTCCCTTCAACAAGCAAAACTTCTTCAGAAAATAAAAGGTATGATGAATT AGAAAGCGTGAATGCTGAGTGCAATTGCGGTGATGAAGATTTATATAATTCTTCGATTTTTTCAGATATAGTCTTCCTTGCTATG GTTCCATTAGAATCCTTACAAACCTGAATCGCATTTGCTGCATGCTTTGCAGAAAGCATACTGGCTGAGTGAGTTGATATTATA ACCTGATACCCTGATTCACTTAATGTGACAAGTGATTCTCTGACAGAATTAATGGCTGAAGGGTGTAAATATAACTCAGGTTCA TCAATGAAAATCAAAGTGTTTGATTTTTTTGATTCGCTGTTTTCTTTTTTTATTTCGGCCAGGTATTGAATTAATGCCATTTGAAT GGAACGTTGTGTTCCGTGACCAAATCGGCTGATATCTCTCATTACCGGTTCATCTTCCCGAGACTCAAAAACTTTCAGAGTGCC GGATTTAAATATCTCATCTAATGTCGGTGTGGGAAAGTGTAACTTTACACTTACGTCAGGAAAAAATTGGTTTACTTTTTTATTT ACACCTGAGTCTATTTTATTAAGGCTTTCTAATCTGTTCTCACCGTTGTGAGAAAGATATTTACCTATTTCTGATATGTTTTTTGA AAATTTTTCTTCGTGTTCTTGTTTTATTTCAGAAACAATTGCGGAAAGTATCTTTCCTATTGTGGTCGTGTTTTTGCATTTTGTTGA GTCTTCGACAGCGTCAGACATTGCAGGGATATGAATTGGTTCCGGAAATATATTGGAGATTGCACCATCTATGCCGCCAGGGT TTTTCTTCCACGTGGTACCGTCATATACATCCAGACTTTTTTTGGCTTTTCCTGTTTCCTTATTAAATTCCTGTCTTCTTGCAAAGG TAAGAGTCCCGTCAATTATAAACGGAGCTATTTTTTGTTGATTCTCTTCTGTTAACAAAGACAGAGTATCATCTGTTATACCTTG AATAACGCCTTCAACGGATACGGGGTGTGTAGGATCGTACACATCTGATTCTGAAATCAAAGAGCCATCTAACAGCCACTTAAT TGCTAAGATAATATTTGATTTTCCTGCGTTATTATAACCAACTAAAGCAGTGAAGGGGCGCAAAATAGCCGATGTTGACTTACA AGAACGAAAGTTGCTAATTGAAACTGAAGCAAGACGTACAGTCATTATTTTTCCTTAAATGTGCTATTTGTATGCAATGAGTTC ATACGAAACGCTTTTTTACATTTATAGTCGTTGCATTCAAGGGTGCATGAGATTGCATTAAGGGAAACTGTGATATGGCTTGG CTTTTGACTGGAAATACTGATGGCTCATTAGTTTATTAAGGTGCATTAAAACCGCCCCGTGAAGCGGGCGGGCGAGGCGGGG |
| | | AAAGCACGGCGAGGCGGGGAAAGCACTGCGCGCTGACGGTGGTGCTGATTGTATTTTTCAGCGTCTCAGCGCGTCGTGACG GCACTTAGTCTGCCCGTTGAGGCGTTGTGTGTCTGCGGGGTGTTTTGTGCGGTGGT |
| 22 | Wild-type cloDF13 ori | GCGCTGCGGACACATACAAAGTTACCCACAGATTCCGTGGATAAGCAGGGGACTAACATGTGAGGCAAAACAGCAGGGCCGC GCCGGTGGCGTTTTTCCATAGGCTCCGCCCTCCTGCCAGAGTTCACATAAACAGACGCTTTTCCGGTGCATCTGTGGGAGCCGT GAGGCTCAACCATGAATCTGACAGTACGGGCGAAACCCGACAGGACTTAAAGATCCCCACCGTTTCCGGCGGGTCGCTCCCTC TTGCGCTCTCCTGTTCCGACCCTGCCGTTTACCGGATACCTGTTCCGCCTTTCTCCCTTACGGGAAGTGTGGCGCTTTCTCATAGC TCACACACTGGTATCTCGGCTCGGTGTAGGTCGTTCGCTCCAAGCTGGGCTGTAAGCAAGAACTCCCCGTTCAGCCCGACTGCT GCGCCTTATCCGGTAACTGTTCACTTGAGTCCAACCCGGAAAAGCACGGTAAAACGCCACTGGCAGCAGCCATTGGTAACTGG GAGTTCGCAGAGGATTTGTTTAGCTAAACACGCGGTTGCTCTTGAAGTGTGCGCCAAAGTCCGGCTACACTGGAAGGACAGAT TTGGTTGCTGTGCTCTGCGAAAGCCAGTTACCACGGTTAAGCAGTTCCCCAACTGACTTAACCTTCGATCAAACCACCTCCCCAG GTGGTTTTTTCGTTTACAGGGCAAAAGATTACGCGCAGAAAAAAAGGATCTCAAGAAGATCCTTTGATC |
| 23 | Mutant cloDF13 ori (higher copy number ori), cloD13_cop3; single mutation versus wild-type is shown underlined and in bold | GCGCTGCGGACACATACAAAGTTACCCACAGATTCCGTGGATAAGCAGGGGACTAACATGTGAGGCAAAACAGCAGGGCCGC GCCGGTGGCGTTTTTCCATAGGCTCCGCCCTCCTGCCAGAGTTCACATAAACAGACGCTTTTCCGGTGCATCTGTGGGAGCCGT GAGGCTCAACCATGAATCTGACAGTACGGGCGAAACCCGACAGGACTTAAAGATCCCCACCGTTTCCGGCGGGTCGCTCCCTC TTGCGCTCTCCTGTTCCGACCCTGCCGTTTACCGGATACCTGTTCCGCCTTTCTCCCTTACGGGAAGTGTGGCGCTTTCTCATAGC TCACACACTGGTATCTCGGCTCGGTGTAGGTCGTTCGCTCCAAGCTGGGCTGTAAGCAAGAACTCCCCGTTCAGCCCGACTGCT GCGCCTTATCCGGTAACTGTTCACTTGAGTCCAACCCGGAAAAGCACGGTAAAACGCCACTGGCAGCAGCCATTGGTAACTGG GAGTTCGCAGAGGATTTGTTTAGCTAAACACGCGGTTGCTCTTGAAGTGTGCGCCAAAGTCCGGCTACACTGGAAGGACAGAT TTGGTTGCTGTGCTCTGCGAAATCCAGTTACCACGGTTAAGCAGTTCCCCAACTGACTTAACCTTCGATCAAACCACCTCCCCAG GTGGTTTTTTCGTTTACAGGGCAAAAGATTACGCGCAGAAAAAAAGGATCTCAAGAAGATCCTTTGATC |

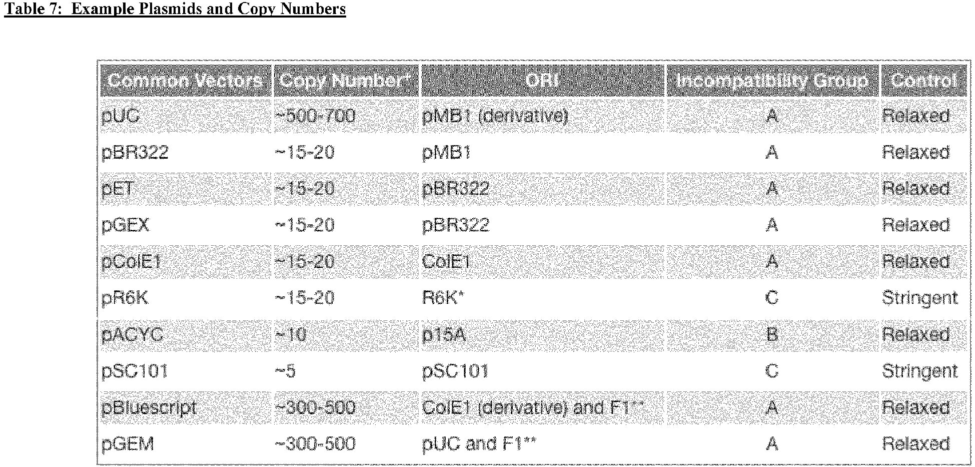

## Claims

1. An antibacterial composition comprising a population of first phages, wherein the first phages comprise a first DNA and require helper phages for replication of first phage particles, wherein the first DNA comprises a phage packaging signal for producing the first phage particles, wherein the first phages are capable of infecting target bacteria, and each first phage comprises antibacterial means for killing target bacteria,
wherein each first phage is a non self-replicative transduction particle,
wherein the phage DNA is comprised by a medium or high copy number plasmid,
and wherein
(a) the antibacterial means encodes a Cas nuclease, or
(b) wherein each first phage particle comprises a nucleotide sequence of interest (NSI) that is capable of expressing a protein or RNA in the target bacteria, wherein the presence in the target bacteria of the NSI-encoded protein or RNA mediates target cell killing, or downregulation of target cell growth or propagation,
wherein the NSI encodes a component of a CRISPR/Cas system that is toxic to the target bacteria, and wherein the component comprises
(i) a DNA sequence encoding a guide RNA (eg, a single guide RNA) or comprising a CRISPR array for producing guide RNA, wherein the guide RNA is capable of targeting the genome of the target bacteria; and/or
(ii) a DNA sequence encoding one or more components of Cascade.

2. A composition according to claim 1, wherein at least 95% of phage particles comprised by the composition are first phage particles.

3. A composition according to claim 1 or 2, wherein each first phage genome is devoid of genes encoding phage proteins.

4. A composition according to any preceding claim, wherein the Cas is Cpf1.

5. A composition according to any preceding claim, wherein the Cas is Cas 3.

6. A composition according to any preceding claim, wherein the Cas is Cas 9, optionally Cas9 protein from Streptococcus pyogenes (SpCas).

7. A composition according to any preceding claim, wherein the phage DNA is comprised by a medium copy number plasmid.

8. A composition according to any preceding claim, wherein the phage DNA is comprised by a high copy number plasmid.

9. A composition of any preceeding claim, wherein the first phage particles comprise no phage structural protein genes.

10. A composition according to any preceeding claim, wherein the first phages are capable of infecting *E. coli* target bacteria, and each first phage comprises antibacterial means for killing *E. coli* target bacteria.

11. The composition of any preceding claim for administration to a human or animal subject for medical use.

12. The composition for use according to claim 11 for administration to a human or animal subject for treating an infection of target bacterial cells, wherein the first phages are capable of infecting and killing the target cells, optionally wherein the infection is a gut microbiome infection.

13. The composition of any one of claims 1 to 10 for use in a contained method of treating a disease or condition of a human or animal subject, wherein the disease or condition is mediated by target bacteria and the target bacteria are comprised by the subject (optionally comprised by a gut microbiome), the method comprising administering the composition to the subject, whereby the target bacteria are exposed to the antibacterial means and killed and propagation of the first phage is contained.

14. A method of producing a phage composition, the method comprising expressing the phage proteins in a host bacterial cell comprising first and second DNAs, wherein
(i) the DNAs together comprise all phage structural protein genes required to produce a packaged phage particle comprising a copy of the first DNA;
(ii) the first DNA comprises none or at least one, but not all, of the genes; and wherein the one or more second DNAs comprise the remainder of the genes;
(iii) the first DNA comprises a phage packaging signal for producing the packaged phage particle; and
(iv) the second DNA is devoid of a nucleotide sequence required for packaging the second DNA into phage particles;
wherein packaged first phage particles are produced that comprise the first DNA, wherein the first phage require the second DNA for replication thereof to produce further first phage; and optionally separating an amount of first phage from cellular material wherein an amount of purified phage is obtained;
wherein each first phage comprises antibacterial means for killing target bacteria,
wherein the phage DNA is comprised by a medium or high copy number plasmid,
and wherein
(a) the antibacterial means encodes a Cas nuclease, or
(b) wherein each first phage particle comprises a nucleotide sequence of interest (NSI) that is capable of expressing a protein or RNA in the target bacteria, wherein the presence in the target bacteria of the NSI-encoded protein or RNA mediates target cell killing, or downregulation of target cell growth or propagation,
wherein the NSI encodes a component of a CRISPR/Cas system that is toxic to the target bacteria, and wherein the component comprises
(i) a DNA sequence encoding a guide RNA (eg, a single guide RNA) or comprising a CRISPR array for producing guide RNA, wherein the guide RNA is capable of targeting the genome of the target bacteria; and/or
(ii) a DNA sequence encoding one or more components of Cascade.

15. The method according to claim 14, wherein the phage composition is according to any one of claims 1 to 10.

## Patentansprüche

1. Antibakterielle Zusammensetzung, die eine Population erster Phagen umfasst, wobei die ersten Phagen eine erste DNS umfassen und für eine Replikation erster Phagenpartikel Helferphagen benötigen,
wobei die erste DNS ein Phagenverpackungssignal zum Herstellen der ersten Phagenpartikel umfasst,
wobei die ersten Phagen dazu in der Lage sind, Zielbakterien zu infizieren und jeder erste Phage ein antibakterielles Mittel zum Abtöten der Zielbakterien umfasst,
wobei jeder erste Phage ein nicht selbstreplizierender Transduktionspartikel ist,
wobei die Phagen-DNS von einem Plasmid mit mittlerer oder hoher Kopienzahl umfasst ist,
und wobei
(a) das antibakterielle Mittel eine Cas-Nuklease codiert oder
(b) wobei jeder erste Phagenpartikel eine Nukleotidsequenz von Interesse (NSI) umfasst, die in der Lage ist, ein Protein oder eine RNS in den Zielbakterien zu exprimieren, wobei die Anwesenheit in den Zielbakterien des NSI-codierten Proteins oder der NSI-codierten RNS Abtöten von Zielzellen oder Herunterregulieren des Wachstums oder der Vermehrung von Zielzellen vermittelt,
wobei die NSI eine Komponente eines CRISPR-/Cas-Systems codiert, die für die Zielbakterien toxisch ist, und wobei die Komponente Folgendes umfasst
(i) eine DNS-Sequenz, die eine Leit-RNS (z. B. eine einzelne Leit-RNS) codiert oder ein CRISPR-Array zum Herstellen von Leit-RNS umfasst, wobei die Leit-RNS in der Lage ist, auf das Genom der Zielbakterien abzuzielen; und/oder
(ii) eine DNS-Sequenz, die eine oder mehrere Komponenten einer Kaskade codiert.

2. Zusammensetzung nach Anspruch 1, wobei mindestens 95 % der Phagenpartikel, die von der Zusammensetzung umfasst sind, erste Phagenpartikel sind.

3. Zusammensetzung nach Anspruch 1 oder 2, wobei jedes erste Phagen-Genom frei von Genen ist, die Phagenproteine codieren.

4. Zusammensetzung nach einem vorhergehenden Anspruch, wobei das Cas Cpf1 ist.

5. Zusammensetzung nach einem vorhergehenden Anspruch, wobei das Cas Cas 3 ist.

6. Zusammensetzung nach einem vorhergehenden Anspruch, wobei das Cas Cas 9 ist, optional ein Cas9-Protein aus Streptococcus pyogenes (SpCas).

7. Zusammensetzung nach einem vorhergehenden Anspruch, wobei die Phagen-DNS von einem Plasmid mit mittlerer Kopienzahl umfasst ist.

8. Zusammensetzung nach einem vorhergehenden Anspruch, wobei die Phagen-DNS von einem Plasmid mit hoher Kopienzahl umfasst ist.

9. Zusammensetzung nach einem vorhergehenden Anspruch, wobei die ersten Phagenpartikel keine Phagenstrukturproteingene umfassen.

10. Zusammensetzung nach einem vorhergehenden Anspruch, wobei die ersten Phagen dazu in der Lage sind, *E*. *coli*-Zielbakterien zu infizieren und jeder erste Phage ein antibakterielles Mittel zum Abtöten der *E*. *coli*-Zielbakterien umfasst.

11. Zusammensetzung nach einem vorhergehenden Anspruch zur Verabreichung an ein menschliches oder tierisches Subjekt zur medizinischen Verwendung.

12. Zusammensetzung zur Verwendung nach Anspruch 11 zur Verabreichung an ein menschliches oder tierisches Subjekt zum Behandeln einer Infektion mit Zielbakterienzellen, wobei die ersten Phagen dazu in der Lage sind, die Zielzellen zu infizieren und abzutöten, optional wobei die Infektion eine Infektion des Darmmikrobioms ist.

13. Zusammensetzung nach einem der Ansprüche 1 bis 10 zur Verwendung in einem enthaltenen Verfahren zum Behandeln einer Krankheit oder eines Zustands eines menschlichen oder tierischen Subjekts, wobei die Krankheit oder der Zustand vermittelt wird durch Zielbakterien und die Zielbakterien von dem Subjekt umfasst sind (optional von einem Darmmikrobiom umfasst sind), wobei das Verfahren Verabreichen der Zusammensetzung an das Subjekt umfasst, wobei die Zielbakterien dem antibakteriellen Mittel ausgesetzt und abgetötet werden und eine Ausbreitung des ersten Phagen enthalten ist.

14. Verfahren zum Herstellen einer Phagenzusammensetzung, wobei das Verfahren Folgendes umfasst: Exprimieren der Phagenproteine in einer bakteriellen Wirtszelle, die erste und zweite DNS umfasst, wobei
(i) die DNS zusammen alle Phagenstrukturproteingene umfassen, die zum Herstellen eines verpackten Phagenpartikels erforderlich sind, der eine Kopie der ersten DNS umfasst.
(ii) die erste DNS keines oder mindestens eines, jedoch nicht alle Gene umfasst; und wobei die eine oder die mehreren zweiten DNS die übrigen Gene umfassen;
(iii)die erste DNS ein Phagenverpackungssignal zum Herstellen des verpackten Phagenpartikels umfasst; und
(iv) die zweite DNS frei von einer Nukleotidsequenz ist, die für die Verpackung der zweiten DNS in Phagenpartikel erforderlich ist.
wobei verpackte erste Phagenpartikel hergestellt werden, die die erste DNS umfassen, wobei der erste Phage die zweite DNS für seine Replikation benötigen, um weitere erste Phage herzustellen; und optional Abtrennen einer Menge des ersten Phagen aus dem Zellmaterial, wobei eine Menge gereinigter Phagen erhalten wird;
wobei jeder erste Phage ein antibakterielles Mittel zum Abtöten der Zielbakterien umfasst,
wobei die Phagen-DNS von einem Plasmid mit mittlerer oder hoher Kopienzahl umfasst ist,
und wobei
(a) das antibakterielle Mittel eine Cas-Nuklease codiert oder
(b) wobei jeder erste Phagenpartikel eine Nukleotidsequenz von Interesse (NSI) umfasst, die in der Lage ist, ein Protein oder eine RNS in den Zielbakterien zu exprimieren, wobei die Anwesenheit in den Zielbakterien des NSI-codierten Proteins oder der NSI-codierten RNS Abtöten von Zielzellen oder Herunterregulieren des Wachstums oder der Vermehrung von Zielzellen vermittelt,
wobei die NSI eine Komponente eines CRISPR-/Cas-Systems codiert, die für die Zielbakterien toxisch ist, und wobei die Komponente Folgendes umfasst
(i) eine DNS-Sequenz, die eine Leit-RNS (z. B. eine einzelne Leit-RNS) codiert oder ein CRISPR-Array zum Herstellen von Leit-RNS umfasst, wobei die Leit-RNS in der Lage ist, auf das Genom der Zielbakterien abzuzielen; und/oder
(ii) eine DNS-Sequenz, die eine oder mehrere Komponenten einer Kaskade codiert.

15. Verfahren nach Anspruch 14, wobei die Phagenzusammensetzung einem der Ansprüche 1 bis 10 entspricht.

## Revendications

1. Composition antibactérienne comprenant une population de premiers phages, dans laquelle les premiers phages comprennent un premier ADN et nécessitent des phages auxiliaires pour la réplication de premières particules de phage,
dans laquelle le premier ADN comprend un signal d'encapsidage de phage pour produire les premières particules de phage,
dans laquelle les premiers phages sont capables d'infecter des bactéries cibles, et chaque premier phage comprend des moyens antibactériens pour tuer des bactéries cibles,
dans laquelle chaque premier phage est une particule de transduction non auto-réplicative,
dans laquelle l'ADN de phage est compris par un plasmide à nombre de copies moyen ou élevé,
et dans laquelle
(a) les moyens antibactériens codent pour une nucléase Cas, ou
(b) dans laquelle chaque première particule de phage comprend une séquence nucléotidique d'intérêt (SNI) qui est capable d'exprimer une protéine ou un ARN dans les bactéries cibles, dans laquelle la présence dans les bactéries cibles de la protéine ou de l'ARN codé par la SNI médie la mort des cellules cibles, ou la régulation à la baisse de la croissance ou de la propagation des cellules cibles,
dans laquelle la SNI code pour un composant d'un système CRISPR/Cas qui est toxique pour les bactéries cibles, et dans laquelle le composant comprend
(i) une séquence d'ADN codant pour un ARN guide (par exemple, un ARN guide unique) ou comprenant un réseau CRISPR pour produire un ARN guide, dans laquelle l'ARN guide est capable de cibler le génome des bactéries cibles ; et/ou
(ii) une séquence d'ADN codant pour un ou plusieurs composants de Cascade.

2. Composition selon la revendication 1, dans laquelle au moins 95 % des particules de phage comprises par la composition sont des premières particules de phage.

3. Composition selon la revendication 1 ou 2, dans laquelle chaque premier génome de phage est dépourvu de gènes codant pour des protéines de phage.

4. Composition selon l'une quelconque des revendications précédentes, dans laquelle la Cas est Cpf1.

5. Composition selon l'une quelconque des revendications précédentes, dans laquelle la Cas est Cas 3.

6. Composition selon l'une quelconque des revendications précédentes, dans laquelle la Cas est Cas 9, éventuellement la protéine Cas9 provenant de Streptococcus pyogenes (SpCas).

7. Composition selon l'une quelconque des revendications précédentes, dans laquelle l'ADN de phage est compris par un plasmide à nombre de copies moyen.

8. Composition selon l'une quelconque des revendications précédentes, dans laquelle l'ADN de phage est compris par un plasmide à nombre de copies élevé.

9. Composition selon l'une quelconque des revendications précédentes, dans laquelle les premières particules de phage ne comprennent aucun gène de protéine structurale de phage.

10. Composition selon l'une quelconque des revendications précédentes, dans laquelle les premiers phages sont capables d'infecter des bactéries cibles *E. coli,* et chaque premier phage comprend des moyens antibactériens pour tuer des bactéries cibles *E. coli.*

11. Composition selon l'une quelconque des revendications précédentes pour administration à un sujet humain ou animal pour une utilisation médicale.

12. Composition pour une utilisation selon la revendication 11 pour administration à un sujet humain ou animal pour le traitement d'une infection de cellules bactériennes cibles, dans laquelle les premiers phages sont capables d'infecter et de tuer les cellules cibles, éventuellement dans laquelle l'infection est une infection du microbiome intestinal.

13. Composition selon l'une quelconque des revendications 1 à 10 pour une utilisation dans un procédé contenu de traitement d'une maladie ou d'une affection d'un sujet humain ou animal, dans laquelle la maladie ou l'affection est médiée par des bactéries cibles et les bactéries cibles sont comprises par le sujet (éventuellement comprises par un microbiome intestinal), le procédé comprenant l'administration de la composition au sujet, grâce à quoi les bactéries cibles sont exposées aux moyens antibactériens et tuées et la propagation du premier phage est contenue.

14. Procédé de production d'une composition de phage, le procédé comprenant les étapes consistant à exprimer les protéines de phage dans une cellule bactérienne hôte comprenant des premier et second ADN, dans lequel
(i) les ADN comprennent ensemble tous les gènes de protéines structurales de phage requis pour produire une particule de phage encapsidée comprenant une copie du premier ADN ;
(ii) le premier ADN ne comprend aucun ou au moins un, mais pas tous, des gènes ; et dans lequel les un ou plusieurs seconds ADN comprennent le reste des gènes ;
(iii)le premier ADN comprend un signal d'encapsidage de phage pour produire la particule de phage encapsidée ; et
(iv) le second ADN est dépourvu d'une séquence nucléotidique requise pour l'encapsidage du second ADN dans des particules de phage ;
dans lequel des premières particules de phage encapsidées sont produites qui comprennent le premier ADN, dans lequel le premier phage nécessite le second ADN pour sa réplication afin de produire d'autres premiers phages ; et éventuellement la séparation d'une quantité de premier phage à partir d'un matériau cellulaire dans lequel une quantité de phage purifié est obtenue ;
dans lequel chaque premier phage comprend des moyens antibactériens pour tuer des bactéries cibles,
dans lequel l'ADN de phage est compris par un plasmide à nombre de copies moyen ou élevé,
et dans laquelle
(a) les moyens antibactériens codent pour une nucléase Cas, ou
(b) dans laquelle chaque première particule de phage comprend une séquence nucléotidique d'intérêt (SNI) qui est capable d'exprimer une protéine ou un ARN dans les bactéries cibles, dans laquelle la présence dans les bactéries cibles de la protéine ou de l'ARN codé par la SNI médie la mort des cellules cibles, ou la régulation à la baisse de la croissance ou de la propagation des cellules cibles,
dans laquelle la SNI code pour un composant d'un système CRISPR/Cas qui est toxique pour les bactéries cibles, et dans laquelle le composant comprend
(i) une séquence d'ADN codant pour un ARN guide (par exemple, un ARN guide unique) ou comprenant un réseau CRISPR pour produire un ARN guide, dans laquelle l'ARN guide est capable de cibler le génome des bactéries cibles ; et/ou
(ii) une séquence d'ADN codant pour un ou plusieurs composants de Cascade.

15. Procédé selon la revendication 14, dans lequel la composition de phage est selon l'une quelconque des revendications 1 à 10.
